# EUROPEAN PATENT SPECIFICATION

(11) **EP 1 689 751 B1**
(45) Date of publication and mention of the grant of the patent: **20.10.2010**
(21) Application number: 04798876.1
(22) Date of filing: 12.11.2004
(51) Int. Cl.: C07D 487/04, A61K 31/505, A61P 9/08

(54) **5,7-DIAMINOPYRAZOLO[4,3-D]PYRIMIDINES WITH PDE-5 INHIBITING ACTIVITY**
5,7-DIAMINOPYRAZOLO[4,3-D]PYRIMIDINE MIT PDE-5-HEMMENDER WIRKUNG
5,7-DIAMINOPYRAZOLO[4,3-D]PYRIMIDINES A EFFET INHIBITEUR PAR RAPPORT A LA PDE-5

(30) Priority: 24.11.2003 GB 0327319
(43) Date of publication of application: 16.08.2006
(73) Proprietor: Pfizer Limited, Sandwich, Kent CT13 9NJ (GB); Pfizer, Inc., New York, NY 10017 (US)
(72) Inventor: BELL, Andrew Simon, Pfizer Global R&D, Sandwich, Kent CT13 9NJ (GB); BROWN, David Graham, Pfizer Global R&D, Sandwich, Kent CT13 9NJ (GB); DACK, Kevin Neil, Pfizer Global R&D, Sandwich, Kent CT13 9NJ (GB); FOX, David Nathan A., Pfizer Global R&D, Sandwich, Kent CT13 9NJ (GB); MARSH, Ian Roger, Pfizer Global R&D, Sandwich, Kent CT13 9NJ (GB); MORRELL, Andrew Ian, Pfizer Global R&D, Sandwich, Kent CT13 9NJ (GB); PALMER, Michael John, Pfizer Global R&D, Sandwich, Kent CT13 9NJ (GB); WINSLOW, Carol Ann, Pfizer Global R&D, Sandwich, Kent CT13 9NJ (GB)
(74) Representative: Ruddock, Keith Stephen
(86) International application number: PCT/IB2004/003747
(87) International publication number: WO 2005/049616

(56) References cited:
- WO-A-00/24745
- WO-A-02/00660
- WO-A-2004/096810

## Description

The present invention relates to a series of novel 5,7-diaminopyrazolo[4,3-*d*] pyrimidines, which are cyclic guanylate monophosphate (cGMP)-specific phosphodiesterase type 5 inhibitors (hereinafter referred to as PDE-5 inhibitors) that are useful in the treatment of hypertension and other disorders, to processes for their preparation, intermediates used in their preparation, to compositions containing them and the uses of said compounds and compositions.

### i) Hypertension

Blood pressure (BP) is defined by a number of haemodynamic parameters taken either in isolation or in combination. Systolic blood pressure (SBP) is the peak arterial pressure attained as the heart contracts. Diastolic blood pressure is the minimum arterial pressure attained as the heart relaxes. The difference between the SBP and the DBP is defined as the pulse pressure (PP).

Hypertension, or elevated BP, has been defined as a SBP of at least 140mmHg and/or a DBP of at least 90mmHg. By this definition, the prevalence of hypertension in developed countries is about 20% of the adult population, rising to about 60-70% of those aged 60 or more, although a significant fraction of these hypertensive subjects have normal BP when this is measured in a non-clinical setting. Some 60% of this older hypertensive population have isolated systolic hypertension (ISH), i.e. they have an elevated SBP and a normal DBP. Hypertension is associated with an increased risk of stroke, myocardial infarction, atrial fibrillation, heart failure, peripheral vascular disease and renal impairment (Fagard, RH; Am. J. Geriatric Cardiology 11 (1), 23-28, 2002; Brown, MJ and Haycock, S; Drugs 59(Suppl2), 1-12, 2000).

The pathophysiology of hypertension is the subject of continuing debate. While it is generally agreed that hypertension is the result of an imbalance between cardiac output and peripheral vascular resistance, and that most hypertensive subjects have abnormal cardiac output and increased peripheral resistance there is uncertainty which parameter changes first (Beevers, G et al.; BMJ 322, 912-916, 2001).

Despite the large number of drugs available in various pharmacological categories, including diuretics, alpha-adrenergic antagonists, beta-adrenergic antagonists, calcium channel blockers, angiotensin converting enzyme (ACE) inhibitors and angiotensin receptor antagonists, the need for an effective treatment of hypertension is still not satisfied.

### ii) PDE5 inhibitors

Vascular endothelial cells secrete nitric oxide (NO). This acts on vascular smooth muscle cells and leads to the activation of guanylate cyclase and the accumulation of cyclic guanosine monophosphate (cGMP). The accumulation of cGMP causes the muscles to relax and the blood vessels to dilate. This dilation reduces vascular resistance and so leads to a reduction in blood pressure.

The cGMP is inactivated by hydrolysis to guanosine 5'-monophosphate (GMP) by a cGMP-specific phosphodiesterase. One important phosphodiesterase has been identified as Phosphodiesterase type 5 (PDE5). Inhibitors of PDE5 decrease the rate of hydrolysis of cGMP and so potentiate the actions of nitric oxide.

Inhibitors of PDE5 have been reported in several chemical classes, including: pyrazolo[4,3-d]pyrimidin-7-ones (e.g. published international patent applications WO 93/06104, WO 98/49166, WO 99/54333, WO 00/24745, WO 01/27112 and WO 01/27113); pyrazolo[3,4-d]pyrimidin-4-ones (e.g. published international patent application WO 93/07149); pyrazolo[4,3-d]pyrimidines (e.g. published international patent application WO 01/18004); quinazolin-4-ones (e.g. published international patent application WO 93/12095); pyrido[3,2-d]pyrimidin-4-ones (e.g. published international patent application WO 94/05661); purin-6-ones (e.g. published international patent application WO 94/00453); hexahydro-pyrazino[2',1':6,1]pyrido[3,4-b]indole-1,4-diones (e.g. published international application WO 95/19978) and imidazo[5,1-f][1,2,4]triazin-ones (e.g. published international application WO 99/24433).

Although they have been suggested as agents for the treatment of related conditions such as angina, PDE5 inhibitors have not yet been adopted as agents for the treatment of hypertension. PDE5 inhibitors are known for the treatment of male erectile dysfunction, e.g. sildenafil, tadalafil and vardenafil. There remains a demand for new PDE5 inhibitors, particularly with improved pharmacokinetic and pharmacodynamic properties. The compounds provided herein are potent inhibitors of PDE5 that have improved selectivity *in vitro* and/or an extended half-life *in vivo*.

WO 02/00660 and WO 01/18004 disclose pyrazolo[4,3-d]pyrimidines with a PDE-5 inhibiting effect, which can be used for treating disorders of the cardiovascular system.

According to a first aspect, the present invention provides compounds of formula (I) 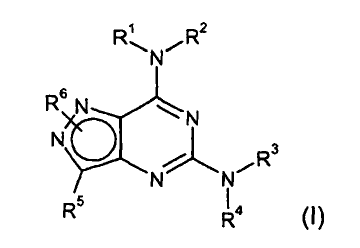 wherein
R¹ is a cyclic group selected from R^{A}, R^{B}, R^{C} and R^{D}, each of which is optionally substituted with one or more R⁷ groups;
R² is hydrogen or C₁-C₂ alkyl;
R³ and R⁴ are each independently C₁-C₈ alkyl, C₂-C₈ alkenyl, C₂-C₈ alkynyl or C₃-C₁₀ cycloalkyl, each of which is optionally substituted with one or more R⁸ groups, or R^{E}, which is optionally substituted with one or more R⁹ groups, or hydrogen;
or -NR³R⁴ forms R^{F}, which is optionally substituted with one or more R¹⁰ groups;
R⁵ is selected from -Y-CO₂R¹⁵ and -Y-R¹⁶;
R⁶, which may be attached at N¹ or N², is C₁-C₆ alkyl, C₁-C₆ haloalkyl, C₂-C₆ alkenyl or C₂-C₆ alkynyl, each of which is optionally substituted by C₁-C₆ alkoxy, C₁-C₆ haloalkoxy or a cyclic group selected from R^{J}, R^{K}, R^{L} and R^{M}, or R⁶ is R^{N}, C₃-C₇ cycloalkyl or C₃-C₇ halocycloalkyl, each of which is optionally substituted by C₁-C₆ alkoxy or C₁-C₆ haloalkoxy, or R⁶ is hydrogen;
R⁷ is halo, C₁-C₆ alkyl, C₁-C₆ haloalkyl, C₂-C₆ alkenyl, C₂-C₆ alkynyl, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹², CONR¹²R¹³ or CN;
R⁸ is halo, phenyl, C₁-C₆ alkoxyphenyl, OR¹², OC(O)R¹², NO₂, NR¹²R¹³, NR¹²C(O)R¹³, NR¹²CO₂R¹⁴, C(O)R¹², CO₂R¹², CONR¹²R¹³, CN, C₃-C₆ cycloalkyl, R^{G} or R^{H}, the last two of which are optionally substituted with one or more R⁹ groups;
R⁹ is C₁-C₆ alkyl, C₁-C₆ haloalkyl or CO₂R¹²,
R¹⁰ is halo, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, C₁-C₆ alkyl or C₁-C₆ haloalkyl, the last two of which are optionally substituted by R¹¹;
R¹¹ is phenyl, NR¹²R¹³ or NR¹²CO₂R¹⁴;
R¹² and R¹³ are each independently hydrogen, C₁₋C₆ alkyl or C₁-C₆ haloalkyl;
R¹⁴ is C₁₋C₆ alkyl or C₁-C₆ haloalkyl;
R¹⁵ is hydrogen or C₁-C₆ alkyl optionally substituted with one or more groups selected from phenyl, halo, OH, C₁-C₆ alkyloxy, NH₂, NH(C₁-C₆alkyl) and N(C₁-C₆ alkyl)₂;
R¹⁶ is a carboxylic acid isostere selected from tetrazol-5-yl, 5-trifluoromethyl-1,2,4-triazol-3-yl, 5-(methylsulfonyl)-1,2,4-triazol-3-yl, 2,5-dihydro-5-oxo-1,2,4-oxadiazol-3-yl, -SO₂NHR¹⁷ and -CONHR¹⁸;
R¹⁷ is selected from C₁-C₆ alkyl, phenyl, -CO-(C₁-C₆ alkyl) and -CO-phenyl;
R¹⁸ is selected from -SO₂-(C₁-C₆ alkyl) and -SO₂-phenyl;
R^{A} and R^{J} are each independently a C₃-C₁₀ cycloalkyl or C₃-C₁₀ cycloalkenyl group, each of which may be either monocyclic or, when there are an appropriate number of ring atoms, polycyclic and which may be fused to either
   (a) a monocyclic aromatic ring selected from a benzene ring and a 5- or 6-membered heteroaromatic ring containing up to three heteroatoms selected from nitrogen, oxygen and sulphur, or
   (b) a 5-, 6- or 7-membered heteroalicyclic ring containing up to three heteroatoms selected from nitrogen, oxygen and sulphur;
R^{B} and R^{K} are each independently a phenyl or naphthyl group, each of which may be fused to
   (a) a C₅-C₇ cycloalkyl or C₅-C₇ cycloalkenyl ring,
   (b) a 5-, 6- or 7-membered heteroalicyclic ring containing up to three heteroatoms selected from nitrogen, oxygen and sulphur, or
   (c) a 5- or 6-membered heteroaromatic ring containing up to three heteroatoms selected from nitrogen, oxygen and sulphur;
R^{C}, R^{L} and R^{N} are each independently a monocyclic or, when there are an appropriate number of ring atoms, polycyclic saturated or partly unsaturated ring system containing between 3 and 10 ring atoms, of which at least one is a heteroatom selected from nitrogen, oxygen and sulphur, which ring may be fused to a C₅-C₇ cycloalkyl or C₅-C₇ cycloalkenyl group or a monocyclic aromatic ring selected from a benzene ring and a 5- or 6-membered heteroaromatic ring containing up to three heteroatoms selected from nitrogen, oxygen and sulphur;
R^{D} and R^{M} are each independently a 5- or 6-membered heteroaromatic ring containing up to three heteroatoms independently selected from nitrogen, oxygen and sulphur, which ring may further be fused to
   (a) a second 5- or 6-membered heteroaromatic ring containing up to three heteroatoms selected from nitrogen, oxygen and sulphur;
   (b) C₅-C₇ cycloalkyl or C₅-C₇ cycloalkenyl ring;
   (c) a 5-, 6- or 7-membered heteroalicyclic ring containing up to three heteroatoms selected from nitrogen, oxygen and sulphur; or
   (d) a benzene ring;
R^{E}, R^{F} and R^{G} are each independently a monocyclic or, when there are an appropriate number of ring atoms, polycyclic saturated ring system containing between 3 and 10 ring atoms, of which at least one is a heteroatom selected from nitrogen, oxygen and sulphur;
R^{H} is a 5- or 6-membered heteroaromatic ring containing up to three heteroatoms independently selected from nitrogen, oxygen and sulphur; and
Y is a covalent bond, -CH₂-O-CH₂-, C₁-C₆ alkylenyl or C₃-C₇ cycloalkylenyl;
a tautomer thereof or a pharmaceutically acceptable salt or solvate of said compound or tautomer.

As used herein, alkylenyl indicates an alkyl-*m*,*n*-diyl unit where *m* and *n* are the same or different, such as methylene (-CH₂-), ethylene (-CH,CH₂-) and propane-1,2-diyl (-CH(CH₃)CH₂-).

As used herein, cycloalkylenyl indicates a cycloalkyl-*m*,*n*-diyl unit where *m* and *n* are the same or different, such as cydopropane-1,1-diyl and cyclohexane-1,4-diyl.

Unless otherwise indicated, an alkyl or alkoxy group may be straight or branched and contain 1 to 8 carbon atoms, preferably 1 to 6 and particularly 1 to 4 carbon atoms. Examples of alkyl include methyl, ethyl, n-propyl, isopropyl, n-butyl, isobutyl, sec-butyl, pentyl and hexyl. Examples of alkoxy include methoxy, ethoxy, isopropoxy and n-butoxy.

Unless otherwise indicated, an alkenyl or alkynyl group may be straight or branched and contain 2 to 8 carbon atoms, preferably 2 to 6 and particularly 2 to 4 carbon atoms and may contain up to 3 double or triple bonds which may be conjugated. Examples of alkenyl and alkynyl include vinyl, allyl, butadienyl and propargyl.

Unless otherwise indicated, a cycloalkyl or cycloalkoxy group may contain 3 to 10 ring-atoms, may be either monocyclic or, when there are an appropriate number of ring atoms, polycyclic. Examples of cycloalkyl groups are cyclopropyl, cyclopentyl, cyclohexyl and adamantyl.

Unless otherwise indicated, a cycloalkenyl group may contain 3 to 10 ring-atoms, may be either monocyclic or, when there are an appropriate number of ring atoms, polycyclic and may contain up to 3 double bonds. Examples of cycloalkenyl groups are cyclopentenyl and cyclohexenyl.

Aryl includes phenyl, naphthyl, anthracenyl and phenanthrenyl.

Unless otherwise indicated, a heteroalicyclyl group contains 3 to 10 ring-atoms up to 4 of which may be hetero-atoms such as nitrogen, oxygen and sulfur, and may be saturated or partially unsaturated. Examples of heteroalicyclyl groups are oxiranyl, azetidinyl, tetrahydrofuranyl, thiolanyl, pyrrolidinyl, pyrrolinyl, imidazolidinyl, imidazolinyl, sulfolanyl, dioxolanyl, dihydropyranyl, tetrahydropyranyl, piperidinyl, pyrazolinyl, pyrazolidinyl, dioxanyl, morpholinyl, dithianyl, thiomorpholinyl, piperazinyl, azepinyl, oxazepinyl, thiazepinyl, thiazolinyl and diazapanyl.

Unless otherwise indicated, a heteroaryl group contains 3 to 10 ring-atoms up to 4 of which may be hetero-atoms such as nitrogen, oxygen and sulfur. Examples of heteroaryl groups are furyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyrimidinyl, pyrazinyl, pyridazinyl, tetrazolyl, triazinyl. In addition, the term heteroaryl includes fused heteroaryl groups, for example benzimidazolyl, benzoxazolyl, imidazopyridinyl, benzoxazinyl, benzothiazinyl, oxazolopyridinyl, benzofuranyl, quinolinyl, quinazolinyl, quinoxalinyl, benzothiazolyl, phthalimido, benzofuranyl, benzodiazepinyl, indolyl and isoindolyl.

Halo means fluoro, chloro, bromo or iodo.

Haloalkyl includes monohaloalkyl, polyhaloalkyl and perhaloalkyl, such as 2-bromoethyl, 2,2,2-trifluoroethyl, chlorodifluoromethyl and trichloromethyl. Haloalkoxy includes monohaloalkoxy, polyhaloalkoxy and perhaloalkoxy, such as 2-bromoethoxy, 2,2,2-trifluoroethoxy, chlorodifluoromethoxy and trichloromethoxy. Halocycloalkyl includes monohalocycloalkyl, polyhalocycloalkyl and perhalocycloalkyl.

Unless otherwise indicated, the term substituted means substituted by one or more defined groups. In the case where groups may be selected from a number of alternative groups, the selected groups may be the same or different.

In one preferred embodiment, R¹ is R^{A}, which is optionally substituted with one or more R⁷ groups; and
R^{A} is a C₃-C₁₀ cycloalkyl group, which may be either monocyclic or, when there are an appropriate number of ring atoms, polycyclic, which may be fused to either
(a) a monocyclic aromatic ring selected from a benzene ring and a 5- or 6-membered heteroaromatic ring containing up to three heteroatoms selected from nitrogen, oxygen and sulphur, or
(b) a 5-, 6- or 7-membered heteroalicyclic ring containing up to three heteroatoms selected from nitrogen, oxygen and sulphur.

Preferably, R^{A} is a monocyclic C₃-C₈ cycloalkyl group.

More preferably, R^{A} is a monocyclic C₅-C₇ cycloalkyl group.

Most preferably, R^{A} is cyclopentyl or cyclohexyl.

In another preferred embodiment, R¹ is R^{B}, which is optionally substituted with one or more R⁷ groups.

Preferably, R^{B} is phenyl.

In another preferred embodiment, R¹ is R^{C}, which is optionally substituted with one or more R⁷ groups.

Preferably, R^{c} is a monocyclic saturated or partly unsaturated ring system containing between 3 and 8 ring atoms, of which at least one is a heteroatom selected from nitrogen, oxygen and sulphur.

More preferably, R^{C} is a monocyclic saturated or partly unsaturated ring system containing between 5 and 7 ring atoms, of which at least one is a heteroatom selected from nitrogen, oxygen and sulphur.

Most preferably, R^{C} is a monocyclic saturated ring system containing between 5 and 7 ring atoms, of which at least one is a heteroatom selected from nitrogen, oxygen and sulphur.

In another preferred embodiment, R¹ is R^{D}, which is optionally substituted with one or more R⁷ groups.

Preferably, R^{D} is a 5- or 6-membered heteroaromatic ring containing up to three heteroatoms independently selected from nitrogen, oxygen and sulphur.

More preferably, R^{D} is a 5-membered heteroaromatic ring containing a heteroatom selected from nitrogen, oxygen and sulphur and optionally up to two further nitrogen atoms in the ring, or a 6-membered heteroaromatic ring including 1, 2 or 3 nitrogen atoms.

More preferably R^{D} is furanyl, thienyl, pyrrolyl, pyrazolyl, imidazolyl, isoxazolyl, oxazolyl, isothiazolyl, thiazolyl, oxadiazolyl, pyridyl, pyridazinyl, pyrimidyl or pyrazinyl.

Most preferably, R^{D} is pyrazolyl, imidazolyl, isoxazolyl, oxazolyl, oxadiazolyl, pyridyl, pyridazinyl, pyrimidyl or pyrazinyl.

Preferably, R⁷ is halo, C₁-C₆ alkyl, C₁-C₆ haloalkyl, OR¹² or CONR¹²R¹³.

More preferably, R⁷ is halo, C₁-C₃ alkyl, C₁-C₃ alkoxy, hydroxy or CONH(C₁-C₃ alkyl).

Most preferably, R⁷ is fluoro, methyl, ethyl, hydroxy, methoxy, propoxy or CONHMe.

Preferably, R² is hydrogen or methyl.

More preferably, R² is hydrogen.

Preferably, R³ is hydrogen, C₁-C₆ alkyl, which is optionally substituted with one or more R⁸ groups, or R^{E}, which is optionally substituted with one or more R⁹ groups; and wherein R^{E} is a monocyclic or, when there are an appropriate number of ring atoms, polycyclic saturated ring system containing between 3 and 7 ring atoms, of which at least one is a heteroatom selected from nitrogen, oxygen and sulphur.

More preferably, R³ is hydrogen, C₁-C₄ alkyl, which is optionally substituted with one or more R⁸ groups, or R^{E}, which is optionally substituted with one or more R⁹ groups; and wherein R^{E} is a monocyclic saturated ring system containing between 3 and 7 ring atoms, of which at least one is a heteroatom selected from nitrogen, oxygen and sulphur.

In one preferred embodiment, R³ is R^{E} which is optionally substituted with one or more R⁹ groups and wherein R^{E} is a monocyclic saturated ring system containing between 3 and 7 ring atoms containing one nitrogen atom.

More preferably, R^{E} is azetidinyl, pyrrolidinyl or piperidinyl.

In another preferred embodiment, R³ is C₁-C₄ alkyl, which is optionally substituted with one or more R⁸ groups and wherein R⁸ is halo, phenyl, C₁-C₆ alkoxyphenyl, OR¹², NR¹²R¹³, NR¹²CO₂R¹⁴, CO₂R¹², CONR¹²R¹³, R^{G} or R^{H}, the last two of which are optionally substituted with one or more R⁹ groups.

More preferably, R⁸ is hydroxy, methoxy, methoxyphenyl, NH₂, NHMe, NMe₂, NHCO₂^{t}Bu, NMeCO₂^{t}Bu, CO₂H, CONHMe, R^{G} or R^{H}, the last two of which are optionally substituted with one or more R⁹ groups.

In one preferred embodiment, R⁸ is R^{G}. which is optionally substituted with one or more R⁹ groups and wherein R^{G} is a monocyclic saturated ring system containing between 3 and 7 ring atoms, of which at least one is a heteroatom selected from nitrogen, oxygen and sulphur.

More preferably, R^{G} is a monocyclic saturated ring system containing between 3 and 7 ring atoms containing one nitrogen atom and optionally one oxygen atom.

Most preferably, R^{G} is pyrrolidinyl, piperidinyl or morpholinyl.

In another preferred embodiment, R⁸ is R^{H}, which is optionally substituted with one or more R⁹ groups and wherein R^{H} is a 5- or 6-membered heteroaromatic ring containing up to two nitrogen atoms.

More preferably, R^{H} is pyrazolyl.

Preferably, R⁹ is methyl or CO₂^{t}Bu.

In another preferred embodiment, R³ is hydrogen or C₁-C₄ alkyl, which is optionally substituted with one or more R⁸ groups, or R³ is azetidinyl, pyrrolidinyl or piperidinyl, each of which is optionally substituted with one or more R⁹ groups, wherein R⁸ is hydroxy, methoxy, methoxyphenyl, NH₂, NHMe, NMe₂, NHCO₂^{t}Bu, NMeCO₂^{t}Bu, CO₂H, CONHMe, pyrrolidinyl, piperidinyl, morpholinyl or pyrazolyl, the last four of which are optionally substituted with one or more R⁹ groups and wherein R⁹ is methyl or CO₂^{t}Bu.

In one preferred embodiment, R⁴ is hydrogen, C₁-C₆ alkyl, C₁-C₆ haloalkyl, C₂-C₆ alkenyl or C₂-C₆ alkynyl.

More preferably, R⁴ is hydrogen, C₁-C₆ alkyl or C₁-C₆ haloalkyl.

Most preferably, R⁴ is hydrogen, methyl or ethyl.

In another preferred embodiment, -NR³R⁴ forms R^{F}, which is optionally substituted with one or more R¹⁰ groups and wherein R^{F} is a monocyclic or, when there are an appropriate number of ring atoms, polycyclic saturated ring system containing between 3 and 10 ring atoms containing at least one nitrogen atom and optionally one other atom selected from oxygen and sulphur.

More preferably, R^{F} is a monocyclic or, when there are an appropriate number of ring atoms, polycyclic saturated ring system containing between 3 and 10 ring atoms containing one or two nitrogen atoms and optionally one other atom selected from oxygen and sulphur.

Most preferably, R^{F} is selected from azetidinyl, pyrrolidinyl, piperidinyl, piperazinyl, morpholinyl, 3-azabicyclo[3.1.0]hex-3-yl, homopiperazinyl, 2,5-diazabicyclo[4.3.0]non-2-yl, 3,8-diazabicyclo[3.2.1]oct-3-yl, 3,8-diazabicyclo[3.2.1]oct-8-yl, 2,5-diazabicyclo[2.2.1]hept-2-yl, 1,4-diazabicyclo[4.3.0]non-4-yl and 1,4-diazabicyclo[3.2.2]non-4-yl.

Preferably R¹⁰ is halo, OR¹², NR¹²R¹³, NR¹²CO₂R¹⁴, CO₂R¹³, oxo, C₁-C₆ alkyl or C₁-C₆ haloalkyl, the last two of which are optionally substituted by R¹¹.

More preferably, R¹⁰ is halo, methyl, ethyl, isopropyl, hydroxy, methoxy, NH₂, NHMe, NMe₂, NHCO₂^{t}Bu, CO₂H, CO₂^{t}Bu, oxo, benzyl, -CH₂NH₂, -CH₂NHMe, CH₂NMe₂ or -CH₂NMeCO₂^{t}Bu.

In one preferred embodiment, R⁵ is -Y-CO₂R¹⁵. Preferably R¹⁵ is hydrogen or C₁-C₃ alkyl. More preferably R¹⁵ is hydrogen. Preferably Y is a covalent bond or C₁-C₆ alkylenyl. More preferably, Y is a covalent bond or methylene. Most preferably Y is a covalent bond

In another preferred embodiment, R⁵ is -Y-R¹⁶. Preferably R¹⁶ is a carboxylic acid isostere selected from -CONHR⁸, tetrazol-5-yl and 2,5-dihydro-5-oxo-1,2,4-oxadiazol-3-yl. Preferably Y is a covalent bond or C₁-C₆ alkylenyl. More preferably, Y is a covalent bond or methylene

Preferably, R⁶ is positioned on N¹ to give the compound of formula (I^{A}):

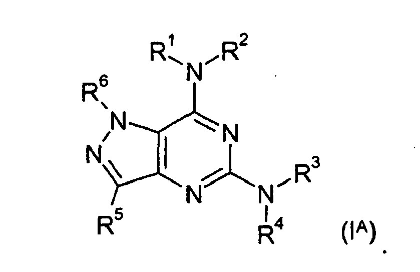

In an alternative embodiment of the present invention, R⁶ may be positioned on N² to give the compound of formula (I^{B}):

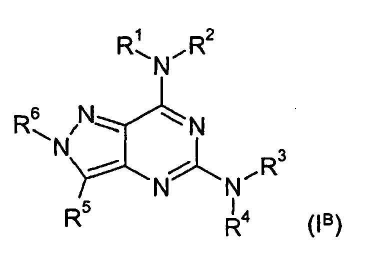

Preferably, R⁶ is C₁-C₆ alkyl or C₁-C₆ haloalkyl, each of which is optionally substituted by C₁-C₆ alkoxy, C₁-C₆ haloalkoxy or a cyclic group selected from R^{J}, R^{L} and R^{M}, or R⁶ is R^{N} or hydrogen;
R^{J} is a C₃-C₇ monocyclic cycloalkyl group;
R^{L} and R^{N} are each independently a monocyclic, saturated or partly unsaturated ring system containing between 4 and 7 ring atoms, of which at least one is a heteroatom selected from nitrogen, oxygen and sulphur; and
R^{M} is a 5- or 6-membered heteroaromatic ring containing up to three heteroatoms independently selected from nitrogen, oxygen and sulphur.

More preferably, R⁶ is C₁-C₄ alkyl or C₁-C₄ haloalkyl, each of which is optionally substituted by C₁-C₄ alkoxy, C₁-C₄ haloalkoxy or a cyclic group selected from R^{J}, R^{L} and R^{M}, or R⁶ is R^{N} or hydrogen;
R^{J} is cyclopropyl or cyclobutyl;
R^{L} and R^{N} are each independently a monocyclic saturated ring system containing either 5 or 6 ring atoms, of which at least one is a heteroatom selected from nitrogen, oxygen and sulphur; and
R^{M} is a 5- or 6-membered heteroaromatic ring containing a heteroatom selected from nitrogen, oxygen and sulphur.

More preferably, R⁶ is C₁-C₄ alkyl or C₁-C₄ haloalkyl, each of which is optionally substituted by C₁-C₄ alkoxy or a cyclic group selected from R^{J}, R^{L} and R^{M}, or R⁶ is R^{N} or hydrogen;
R^{J} is cyclopropyl or cyclobutyl;
R^{L} and R^{N} are each independently a monocyclic saturated ring system containing either 5 or 6 ring atoms containing one heteroatom selected from nitrogen, oxygen and sulphur; and
R^{M} is a 5- or 6-membered heteroaromatic ring containing one nitrogen atom.

More preferably, R⁶ is C₁-C₄ alkyl or C₁-C₄ haloalkyl, each of which is optionally substituted by C₁-C₄ alkoxy, cyclopropyl, cyclobutyl, tetrahydrofuranyl, tetrahydropyranyl or pyridinyl, or R⁶ is hydrogen or tetrahydropyranyl.

Most preferably, R⁶ is hydrogen, methyl, ethyl, isopropyl, isobutyl, methoxyethyl, methoxypropyl, ethoxyethyl, ethoxypropyl, propoxyethyl, 2,2,2-trifluoroethyl, tetrahydrofuranylmethyl, tetrahydropyranylmethyl, tetrahydropyranyl or pyridinylmethyl.

Preferred embodiments of compounds of formula (I) are those that incorporate two or more of the foregoing preferences.

Preferably R¹ is a cyclic group selected from R^{A}, R^{B}, R^{C} and R^{D}, each of which is optionally substituted with one or more R⁷ groups;
R² is hydrogen or C₁-C₂ alkyl;
R³ is hydrogen, C₁-C₄ alkyl, which is optionally substituted with one or more R⁸ groups, or R^{E}, which is optionally substituted with one or more R⁹ groups;
R⁴ is hydrogen, C₁-C₆ alkyl or C₁-C₆ haloalkyl;
or -NR³R⁴ forms R^{F}, which is optionally substituted with one or more R¹⁰ groups;
R⁵ is -Y-CO₂R¹⁵ or -Y-R¹⁶;
R⁶ is C₁-C₄ alkyl or C₁-C₄ haloalkyl, each of which is optionally substituted by C₁-C₄ alkoxy, C₁-C₄ haloalkoxy or a cyclic group selected from R^{J}, R^{L} and R^{M}, or R⁶ is R^{N} or hydrogen;
R⁷ is halo, C₁-C₆ alkyl, C₁-C₆ haloalkyl, C₂-C₆ alkenyl, C₂-C₆ alkynyl, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴ C(O)R¹², CO₂R¹², CONR¹²R¹³ or CN;
R⁸ is halo, phenyl, C₁-C₆ alkoxyphenyl, OR¹², OC(O)R¹², NO₂, NR¹²R¹³, NR¹²C(O)R¹³, NR¹²CO₂R¹⁴, C(O)R¹², CO₂R¹², CONR¹²R¹³, CN, R^{G} or R^{H}, the last two of which are optionally substituted with one or more R⁹ groups;
R⁹ is C₁-C₆ alkyl, C₁-C₆ haloalkyl or CO₂R¹²;
R¹⁰ is halo, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, C₁-C₆ alkyl or C₁-C₆ haloalkyl, the last two of which are optionally substituted by R¹¹;
R¹¹ is phenyl, NR¹²R¹³ or NR¹²CO₂R¹⁴;
R¹² and R¹³ are each independently hydrogen, C₁₋C₆ alkyl or C₁-C₆ haloalkyl;
R¹⁴ is C₁₋C₆ alkyl or C₁-C₆ haloalkyl;
R¹⁵ is hydrogen or C₁-C₃ alkyl:
R¹⁶ is tetrazol-5-yl, 5-trifluoromethyl-1,2,4-triazol-3-yl or 2,5-dihydro-5-oxo-1,2,4-oxadiazol-3-yl;
R^{A} is a monocyclic C₃-C₈ cycloalkyl group;
R^{B} is phenyl;
R^{C} is a monocyclic saturated or partly unsaturated ring system containing between 3 and 8 ring atoms, of which at least one is a heteroatom selected from nitrogen, oxygen and sulphur;
R^{D} is a 5- or 6-membered heteroaromatic ring containing up to three heteroatoms independently selected from nitrogen, oxygen and sulphur;
R^{E} is a monocyclic saturated ring system containing between 3 and 7 ring atoms, of which at least one is a heteroatom selected from nitrogen, oxygen and sulphur;
R^{F} and R^{G} are each independently a monocyclic or, when there are an appropriate number of ring atoms, polycyclic saturated ring system containing between 3 and 10 ring atoms, of which at least one is a heteroatom selected from nitrogen, oxygen and sulphur;
R^{H} is a 5- or 6-membered heteroaromatic ring containing up to three heteroatoms independently selected from nitrogen, oxygen and sulphur;
R^{J} is cyclopropyl or cyclobutyl;
R^{L} and R^{N} are each independently a monocyclic saturated ring system containing either 5 or 6 ring atoms, of which at least one is a heteroatom selected from nitrogen, oxygen and sulphur;
R^{M} is a 5- or 6-membered heteroaromatic ring containing a heteroatom selected from nitrogen, oxygen and sulphur; and
Y is a covalent bond or C₁-C₆ alkylenyl.

More preferably, R¹ is a cyclic group selected from R^{A}, R^{B}, R^{C} and R^{D}, each of which is optionally substituted with one or more R⁷ groups;
R² is hydrogen or C₁-C₂ alkyl;
R³ is hydrogen, C₁-C₄ alkyl, which is optionally substituted with one or more R⁸ groups, or R^{E}, which is optionally substituted with one or more R⁹ groups;
R⁴ is hydrogen, C₁-C₆ alkyl or C₁-C₆ haloalkyl;
or -NR³R⁴ forms R^{F}, which is optionally substituted with one or more R¹⁰ groups;
R⁵ is -Y-CO₂R¹⁵;
R⁶ is C₁-C₄ alkyl or C₁-C₄ haloalkyl, each of which is optionally substituted by C₁-C₄ alkoxy, C₁-C₄ haloalkoxy or a cyclic group selected from R^{J}, R^{L} and R^{M}, or R⁶ is R^{N} or hydrogen;
R⁷ is halo, C₁-C₆ alkyl, C₁-C₆ haloalkyl, OR¹² or CONR¹²R¹³;
R⁸ is halo, phenyl, C₁-C₆ alkoxyphenyl, OR¹², NR¹²R¹³, NR¹²CO₂R¹⁴, CO₂R¹², CONR¹²R¹³, R^{G} or R^{H}, the last two of which are optionally substituted with one or more R⁹ groups;
R⁹ is C₁-C₆ alkyl, C₁-C₆ haloalkyl or CO₂R¹²;
R¹⁰ is halo, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, C₁-C₆ alkyl or C₁-C₆ haloalkyl, the last two of which are optionally substituted by R¹¹;
R¹¹ is phenyl, NR¹²R¹³ or NR¹²CO₂R¹⁴;
R¹² and R¹³ are each independently hydrogen, C₁₋C₆ alkyl or C₁-C₆ haloalkyl;
R¹⁴ is C₁₋C₆ alkyl or C₁-C₆ haloalkyl;
R¹⁵ is hydrogen;
R^{A} is a monocyclic C₅-C₇ cycloalkyl group;
R^{B} is phenyl;
R^{c} is a monocyclic saturated ring system containing between 5 and 7 ring atoms, of which at least one is a heteroatom selected from nitrogen, oxygen and sulphur;
R^{D} is a 5-membered heteroaromatic ring containing a heteroatom selected from nitrogen, oxygen and sulphur and optionally up to two further nitrogen atoms in the ring, or a 6-membered heteroaromatic ring including 1, 2 or 3 nitrogen atoms;
R^{E} is a monocyclic saturated ring system containing between 3 and 7 ring atoms containing one nitrogen atom;
R^{F} is a monocyclic or, when there are an appropriate number of ring atoms, polycyclic saturated ring system containing between 3 and 10 ring atoms containing at least one nitrogen atom and optionally one other atom selected from oxygen and sulphur;
R^{G} is a monocyclic saturated ring system containing between 3 and 7 ring atoms, of which at least one is a heteroatom selected from nitrogen, oxygen and sulphur;
R^{H} is a 5- or 6-membered heteroaromatic ring containing up to two nitrogen atoms;
R^{L} and R^{N} are each independently a monocyclic saturated ring system containing either 5 or 6 ring atoms, of which at least one is a heteroatom selected from nitrogen, oxygen and sulphur;
R^{M} is a 5- or 6-membered heteroaromatic ring containing a heteroatom selected from nitrogen, oxygen and sulphur; and
Y is a covalent bond or methylene.

In an alternative embodiment of the present invention, the present invention provides compounds of formula (I-AA) 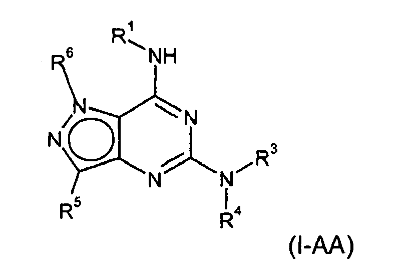 wherein
R¹ is a pyridyl optionally substituted with one or more C₁-C₆ alkyl groups;
R³ and R⁴ are each independently hydrogen or C₁-C₆ alkyl;
R⁵ is -CONHR¹⁸;
R⁶ is C₁-C₆ alkyl, optionally substituted by a substituent selected from -OH, C₃-C₆ cycloalkyloxy, C₁-C₆ alkoxy and C₁-C₆ haloalkoxy;
R¹⁸ is selected from the group consisting of -SO₂-(C₁-C₆ alkyl) and -SO₂-phenyl; and tautomers thereof or a pharmaceutically acceptable salts, or solvates of said compounds or tautomers.

In another embodiment of the compounds of formula I-AA, R¹ is 2-pyridinyl substituted with one or more methyl. In another embodiment of the compounds of formula I-AA, R³ and R⁴ are independently selected from the group consisting of methyl, ethyl, propyl, and isopropyl. In another embodiment of the compounds of formula I-AA, R¹⁸ is selected from the group consisting of -SO₂CH₃, and - SO₂CH₂CH₃. In another embodiment of the compounds of formula I-AA, R⁶ is ethyl, optionally substituted by a subsituent selected from the group consisting of hydroxyl, methoxy, ethoxy, propoxy, fluoromethoxy, fluoroethoxy, fluoropropoxy, difluoromethoxy, difluoroethoxy, difluoropropoxy, trifluoromethoxy, trifluoroethoxy, trifluoropropoxy, and cyclobutyloxy.

In another embodiment of the compounds of formula I-AA, R¹ pyridinyl is substituted with one or more methyl;
R³ and R⁴ are independently selected from the group consisting of hydrogen, methyl, ethyl, propyl and isopropyl;
R⁶ is ethyl, optionally substituted by a subsituent selected from the group consisting of -OH, C₃-C₆ cycloalkyloxy, C₁-C₆ alkoxy and C₁-C₆ haloalkoxy; and
R¹⁸ is selected from the group consisting of -SO₂CH₃, and -SO₂CH₂CH₃.

In another alternative embodiment of the present invention, the present invention provides compounds of formula (I-BB) 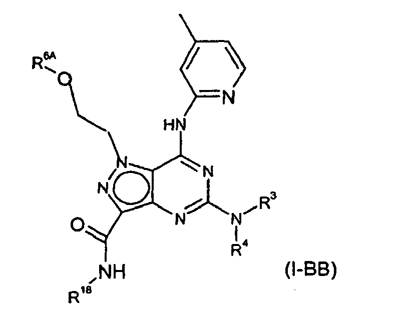 wherein
R³ and R⁴ are each independently selected from the group consisting of methyl, ethyl, and isopropyl;
R^{6A} is selected from the group consisting of methyl, ethyl, propyl, fluoromethyl, fluoroethyl, fluoropropyl, difluoroethyl, difluoropropyl, trifluoroethyl, and trifluoropropyl; and
R¹⁸ is selected from the group consisting of -SO₂CH₃, and -SO₂CH₂CH₃.

Most preferred compounds are:
methyl 5-((1*S*,4*S*)-2,5-diazabicyclo[2.2.1]hept-2-yl)-1-(2-ethoxyethyl)-7-(4-methyl-pyridin-2-ylamino)-1*H*-pyrazolo[4,3-*d*]pyrimidine-3-carboxylate,
methyl 1-(2-ethoxyethyl)-5-(*N*-isopropyl-*N*-methylamino)-7-(6-methylpyridin-2-ylamino)-1*H*-pyrazolo[4,3-*d*]pyrimidine-3-carboxylate,
ethyl 1-(2-ethoxyethyl)-5-(*N*-ethyl-*N*-methylamino)-7-(4-methylpyridin-2-ylamino)-1*H-*pyrazolo[4,3-*d*]pyrimidine-3-carboxylate,
2-(dimethylamino)ethyl 5-dimethylamino-1-(2-ethoxyethyl)-7-(4-methylpyridin-2-ylamino)-1*H*-pyrazolo[4,3-*d*]pyrimidine-3-carboxylate,
1-(2-ethoxyethyl)-5-(*N*-methyl-*N*-propylamino)-7-(4-methylpyridin-2-ylamino)-1*H-*pyrazolo[4,3-*d*]pyrimidine-3-carboxylic acid,
5-(*N*-isopropyl-*N*-methylamino)-7-(4-methylpyridin-2-ylamino)-1-(2-propoxy-ethyl)-1*H*-pyrazolo[4,3-*d*]pyrimidine-3-carboxylic acid,
7-(4,6-dimethylpyridin-2-ylamino)-1-(2-ethoxyethyl)-5-(*N*-isopropyl-*N*-methyl-amino)-1*H*-pyrazolo[4,3-*d*]pyrimidine-3-carboxylic acid,
5-(*N*-cyclobutyl-*N*-methylamino)-1-(2-ethoxyethyl)-7-(4-methylpyridin-2-ylamino)-1*H-*pyrazolo[4,3-*d*]pyrimidine-3-carboxylic acid,
1-(2-ethoxyethyl)-5-isopropylamino-7-(4-methylpyridin-2-ylamino)-1*H*-pyrazolo[4,3-*d*]pyrimidine-3-carboxylic acid,
1-(2-ethoxyethyl)-5-(*N*-ethyl-*N*-methylamino)-7-(2-methoxypyrimidin-4-ylamino)-1*H* pyrazolo[4,3-*d*]pyrimidine-3-carboxylic acid,
3-[1-(2-ethoxyethyl)-5-(*N*-isopropyl-*N*-methylamino)-7-(4-methylpyridin-2-ylamino)-1*H*-pyrazolo[4,3-*d*]pyrimidin-3-yl]-2*H*-1,2,4-oxadiazol-5-one,
3-[1-(2-ethoxyethyl)-5-(*N*-ethyl*-N*-methylamino)-7-(4-methylpyridin-2-ylamino)-1*H-*pyrazolo[4,3-*d*]pyrimidin-3-yi]-2*H*-1,2,4-oxadiazol-5-one,
1-(2-ethoxyethyl)-7-(4-fluoro-3-methylphnylamino)-5-(*N*-isopropyl-*N*-methylamino)-1*H*-pyrazolo[4,3-*d*]pyrimidine-3-carboxylic acid,
1-(2-ethoxyethyl)-5-(*N*-ethyl-*N*-methylamino)-7-(4-fluoro-3-methylphenylamino)-1*H-*pyrazolo[4,3-*d*]pyrimidine-3-carboxylic acid,
7-(3,4-dimethylphenylamino)-1-(2-ethoxyethyl)-5-(*N*-ethyl-*N*-methylamino)-1*H-*pyrazolo[4,3-*d*]pyrimidine-3-carboxylic acid,
1-(2-(cyclopropylmethoxy)ethyl)-5-(*N*-isopropyl-*N*-methylamino)-7-(4-methyl-pyridin-2-ylamino)-1*H*-pyrazolo[4,3-*d*]pyrimidine-3-carboxylic acid,
1-(2-(cyclopropylmethoxy)ethyl)-5-(*N*-ethyl-*N*-methylamino)-7-(4-methyl-pyridin-2-ylamino)-1*H*-pyrazolo[4,3-*d*]pyrimidine-3-carboxylic acid,
1-(2-ethoxyethyl)-5-(*N*-isopropyl-*N*-methylamino)-7-(4-methylpyridin-2-ylamino)-1*H-*pyrazolo[4,3-*d*]pyrimidine-3-carboxylic acid,
1-(2-isopropoxyethyl)-5-(*N*-isopropyl-*N*-methylamino)-7-(4-methylpyridin-2-ylamino)-1*H*-pyrazolo[4,3-*d*]pyrimidine-3-carboxylic acid,
*N*-[1-(2-ethoxyethyl)-5-(*N*-isopropyl-*N*-methylamino)-7-(4-methylpyridin-2-ylamino)-1*H*-pyrazolo[4,3-*d*]pyrimidine-3-carbonyl]methanesulfonamide, and
*N*-[1-(2-ethoxyethyl)-5-(*N*-ethyl-*N*-methylamino)-7-(4-methylpyridin-2-ylamino)-1*H-*pyrazolo[4,3-*d*]pyrimidine-3-carbonyl]methanesulfonamide
and tautomers thereof and pharmaceutically acceptable salts or solvates of said compounds or tautomers.

Pharmaceutically acceptable salts of the compounds of formula (I) include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, Weinheim, Germany, 2002).

A pharmaceutically acceptable salt of a compound of formula (I) may be readily prepared by mixing together solutions of the compound of formula (I) and the desired acid or base, as appropriate. The salt may precipitate from solution and be collected by filtration or may be recovered by evaporation of the solvent. The degree of ionisation in the salt may vary from completely ionised to almost non-ionised.

The compounds of the invention may exist in both unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8),1269-1288 by Haleblian (August 1975).

Hereinafter all references to compounds of formula (I) include references to salts, solvates and complexes thereof and to solvates and complexes of salts thereof.

The compounds of the invention include compounds of formula (I) as hereinbefore defined, polymorphs, prodrugs, and isomers thereof (including optical, geometric and tautomeric isomers) as hereinafter defined and isotopically-labeled compounds of formula (I).

Also within the scope of the invention are so-called 'prodrugs' of the compounds of formula (I). Thus certain derivatives of compounds of formula (I) which may have little or no pharmacological activity themselves can, when administered into or onto the body, be converted into compounds of formula (I) having the desired activity, for example, by hydrolytic cleavage. Such derivatives are referred to as 'prodrugs'. Further information on the use of prodrugs may be found in 'Pro-drugs as Novel Delivery Systems, Vol. 14, ACS Symposium Series (T Higuchi and W Stella) and 'Bioreversible Carriers in Drug Design', Pergamon Press, 1987 (ed. E B Roche, American Pharmaceutical Association).

Prodrugs in accordance with the invention can, for example, be produced by replacing appropriate functionalities present in the compounds of formula (I) with certain moieties known to those skilled in the art as 'pro-moieties' as described, for example, in "Design of Prodrugs" by H Bundgaard (Elsevier, 1985).

Some examples of prodrugs in accordance with the invention include:
(i) where the compound of formula (I) contains a carboxylic acid functionality (-COOH), an ester thereof, for example, replacement of the hydrogen with (C,-C₈)alkyl;
(ii) where the compound of formula (I) contains an alcohol functionality (-OH), an ether thereof, for example, replacement of the hydrogen with (C,-C₆)alkanoyloxymethyl; and
(iii) where the compound of formula (I) contains a primary or secondary amino functionality (-NH₂ or -NHR where R ≠ H), an amide thereof, for example, replacement of one or both hydrogens with (C₁-C₁₀)alkanoyl.

Further examples of replacement groups in accordance with the foregoing examples and examples of other prodrug types may be found in the aforementioned references.

Finally, certain compounds of formula (I) may themselves act as prodrugs of other compounds of formula (I).

Compounds of formula (I) containing one or more asymmetric carbon atoms can exist as two or more stereoisomers. Where a compound of formula (I) contains an alkenyl or alkenylene group, geometric *cis*/*trans* (or Z/E) isomers are possible. Where the compound contains, for example, a keto or oxime group or an aromatic moiety, tautomeric isomerism ('tautomerism') can occur. It follows that a single compound may exhibit more than one type of isomerism.

Included within the scope of the present invention are all stereoisomers, geometric isomers and tautomeric forms of the compounds of formula (I), including compounds exhibiting more than one type of isomerism, and mixtures of one or more thereof. Also included are acid addition or base salts wherein the counterion is optically active, for example, D-lactate or L-lysine, or racemic, for example, DL-tartrate or DL-arginine.

*Cis*/*trans* isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1% diethylamine. Concentration of the eluate affords the enriched mixture.

Stereoisomeric conglomerates may be separated by conventional techniques known to those skilled in the art - see, for example, "Stereochemistry of Organic Compounds" by E L Eliel (Wiley, New York; 1994).

The present invention includes all pharmaceutically acceptable isotopically-labelled compounds of formula (I) wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula (I), for example, those incorporating a radioactive isotope, are useful in drug and/or substrate tissue distribution studies. The radioactive isotopes tritium, *i*.*e*. ³H, and carbon-14, *i*.*e*. ¹⁴C, are particularly useful for this purpose in view of their ease of incorporation and ready means of detection.

Substitution with heavier isotopes such as deuterium, *i*.*e*. ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

Pharmaceutically acceptable solvates in accordance with the invention include those wherein the solvent of crystallization may be isotopically substituted, *e.g.* D₂O, d₆-acetone, d₆-DMSO.

Compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

The compounds of formula (I) are inhibitors of PDE5. Accordingly, in a further aspect the present invention provides for the use of a compound of formula (I), or a tautomer, salt or solvate thereof, as a pharmaceutical agent, and particularly as a therapeutic agent for the treatment of a condition where inhibition of PDE5 is known, or can be shown, to produce a beneficial effect.

The term "treatment" includes palliative, curative and prophylactic treatment.

Conditions suitable for treatment with the compounds of the invention include hypertension (including essential hypertension, pulmonary hypertension, secondary hypertension, isolated systolic hypertension, hypertension associated with diabetes, hypertension associated with atherosclerosis, and renovascular hypertension), congestive heart failure, angina (including stable, unstable and variant (Prinzmetal) angina), stroke, coronary artery disease, congestive heart failure, conditions of reduced blood vessel patency (such as post-percutaneous coronary angioplasty), peripheral vascular disease, atherosclerosis, nitrate-induced tolerance, nitrate tolerance, diabetes, impaired glucose tolerance, metabolic syndrome, obesity, sexual dysfunction (including male erectile disorder, impotence, female sexual arousal disorder, clitoral dysfunction, female hypoactive sexual desire disorder, female sexual pain disorder, female sexual orgasmic dysfunction and sexual dysfunction due to spinal cord injury), premature labour, pre-eclampsia, dysmenorrhea, polycystic ovary syndrome, benign prostatic hyperplasia, bladder outlet obstruction, incontinence, chronic obstructive pulmonary disease, acute respiratory failure, bronchitis, chronic asthma, allergic asthma, allergic rhinitis, gut motility disorders (including irritable bowel syndrome), Kawasaki's syndrome, multiple sclerosis, Alzheimer's disease, psoriasis, skin necrosis, scarring, fibrosis, pain (particularly neuropathic pain), cancer, metastasis, baldness, nutcracker oesophagus, anal fissure and haemorrhoids.

In a further aspect, the present invention provides for the use of a compound of formula (I), or a tautomer, salt or solvate thereof, for the manufacture of a medicament for the treatment of such a condition.

The compounds of the present invention may be used alone or in combination with other therapeutic agents. When used in combination with another therapeutic agent the administration of the two agents may be simultaneous or sequential. Simultaneous administration includes the administration of a single dosage form that comprises both agents and the administration of the two agents in separate dosage forms at substantially the same time. Sequential administration includes the administration of the two agents according to different schedules provided that there is an overlap in the periods during which the treatment is provided. Suitable agents with which the compounds of formula (I) can be co-administered include aspirin, angiotensin II receptor antagonists (such as losartan, candesartan, telmisartan, valsartan, irbesartan and eprosartan), calcium channel blockers (such as amlodipine), beta-blockers (i.e. beta-adrenergic receptor antagonists such as sotalol, proporanolol, timolol, antenolol, carvedilol and metoprolol), CI1027, CCR5 receptor antagonists, imidazolines, sGCa's (soluble guanylate cyclase activators) antihypertensive agents, diuretics (such as hydrochlorothiazide, torsemide, chlorothiazide, chlorthalidone and amiloride), alpha adrenergic antagonists (such as doxazosin), ACE (angiotensin converting enzyme) inhibitors (such as quinapril, enalapril, ramipril and lisinopril), aldosterone receptor antagonists (such as eplerenone and spironolactone), neutral endopeptidase inhibitors, antidiabetic agents (such as insulin, sulfonylureas (such as glyburide, glipizide and glimepiride), glitazones (such as rosiglitazone and pioglitazone) and metformin), cholesterol lowering agents (such as atorvastatin, pravastatin, lovastatin, simvastatin, clofibrate and rosuvastatin), and alpha-2-delta ligands (such as gabapentin, pregabalin, [(1R,5R,6S)-6-(aminomethyl)bicyclo[3.2.0]hept-6-yl]acetic acid, 3-(1-aminomethyl-cyclohexylmethyl)-4H-[1,2,4]oxadiazol-5-one, C-[1-(1H-tetrazol-5-ylmethyl)-cycloheptyl]-methylamine, (3S,4S)-(1-aminomethyl-3,4-dimethyl-cyclopentyl)-acetic acid, (1α,3α,5α)-(3-amino-methyl-bicyclo[3.2.0]hept-3-yl)-acetic acid, (3S,5R)-3-aminomethyl-5-methyl-octanoic acid, (3S,5R)-3-amino-5-methyl-heptanoic acid, (3S,5R)-3-amino-5-methyl-nonanoic acid and (3S,5R)-3-amino-5-methyl-octanoic acid).

The compounds of formula (I) may be administered alone or in combination with one or more other compounds of the invention or in combination with one or more other drugs (or as any combination thereof). Generally, they will be administered as a formulation in association with one or more pharmaceutically acceptable excipients. The term "excipient" is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films (including muco-adhesive), ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001).

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 wt% to 5 wt% of the tablet, and glidants may comprise from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 wt% to 10 wt%, preferably from 0.5 wt% to 3 wt% of the tablet.

Other possible ingredients include anti-oxidants, colourants, flavouring agents, preservatives and taste-masking agents.

Exemplary tablets contain up to about 80% drug, from about 10 wt% to about 90 wt% binder, from about 0 wt% to about 85 wt% diluent, from about 2 wt% to about 10 wt% disintegrant, and from about 0.25 wt% to about 10 wt% lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in "Pharmaceutical Dosage Forms: Tablets, Vol. 1", by H. Lieberman and L. Lachman, Marcel Dekker, N.Y., N.Y., 1980 (ISBN 0-8247-6918-X).

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Verma et al, Pharmaceutical Technology On-line, 25(2), 1-14 (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (*e.g.* Powderject™, Bioject™, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or HPMC), blisters and cartridges for use in an inhaler or insufflator may be formulated to contain a powder mix of the compound of the invention, a suitable powder base such as lactose or starch and a performance modifier such as *l*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 10mg of the compound of the invention per actuation and the actuation volume may vary from 1µl to 100µl. A typical formulation may comprise a compound of formula (I), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, poly(DL-lactic-coglycolic acid (PGLA). Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 1µg to 20mg of the compound of formula (I). The overall daily dose will typically be in the range 1µg to 80mg which may be administered in a single dose or, more usually, as divided doses throughout the day.

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (*e*.*g*. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i*.*e*. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula ... in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the range 0.1mg to 500 mg depending, of course, on the mode of administration. For example, oral administration may require a total daily dose of from 0.1 mg to 500 mg, while an intravenous dose may only require from 0.01 mg to 50mg. The total daily dose may be administered in single or divided doses.

These dosages are based on an average human subject having a weight of about 65kg to 70kg. The physician will readily be able to determine doses for subjects whose weight falls outside this range, such as infants and the elderly.

Compounds of the invention may be prepared, in known manner in a variety of ways. In the following reaction schemes and hereafter, unless otherwise stated R¹ to R⁶ are as defined in the first aspect. These processes form further aspects of the invention.
a) Compounds of formula (I^{C}), i.e. compounds of formula (I) wherein R⁵ is -Y-CO₂R¹⁵ and R¹⁵ is H, may generally be prepared from the corresponding esters of formula (II) wherein R^{A} is an alkyl group (particularly a methyl, ethyl, or *tert*-butyl group) or a benzyl group, as illustrated in Scheme 1.

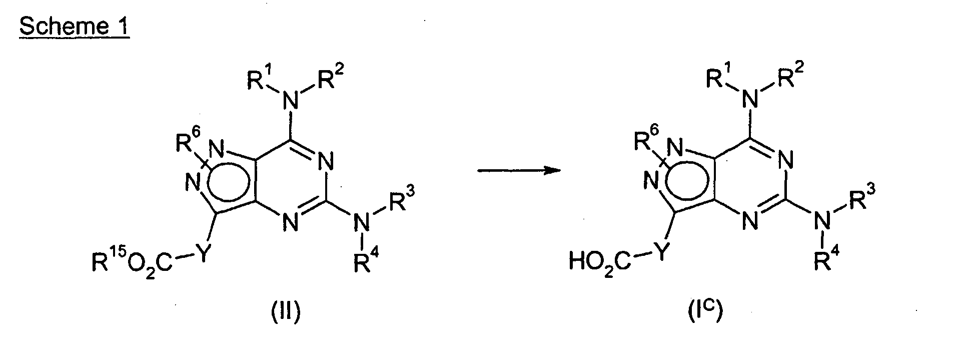

When R¹⁵ is methyl or ethyl the conversion may conveniently be accomplished by treating the compound of formula (II) with an alkaline metal hydroxide such as lithium, sodium or potassium hydroxide in a suitable solvent at a temperature of between about 10°C and the boiling point of the solvent. Suitable solvents include water, methanol, ethanol and mixtures of water with methanol, ethanol, tetrahydrofuran and dioxan. When R¹⁵ is *tert*-butyl the conversion may be accomplished by treating the compound of formula (II) with an acid such as hydrogen chloride or trifluoroacetic acid in a suitable solvent at a temperature of between 0°C and ambient temperature. Suitable solvents include dioxan and dichloromethane. When R¹⁵ is benzyl the conversion may conveniently be accomplished by treating the compound of formula (II) with an alkaline metal hydroxide as discussed above, or by hydrogenolysis using molecular hydrogen or a suitable hydrogen donor such as ammonium formate in the presence of a transition metal or transition metal salt catalyst such as palladium-on-carbon, in a suitable solvent, such as methanol.

When there is a functional group in another part of the structure of (I^{C}) that is protected, such as an amino group in R¹ or R³, it may be convenient to select R¹⁵ and the protecting group such that they may both be removed in a single operation. For example, if there is an amine group protected by a BOC group, then selecting R¹⁵ to be *tert*-butyl will allow both unmasking oprerations to be achieved with a single acid treatment. Similarly, if benzyloxycarbonyl is the preferred amine protecting group, the use of benzyl for R¹⁵ permits simultaneous unmasking in a single hydrogenolysis step. Alternatively, the protecting group and R¹⁵ may be chosen so as to be 'orthogonal', i.e. each is stable to the conditions used to cleave the other. Unmasking is then a two stage process, but the intermediate can be subject to a purification step.
b) Compounds of formula (I^{D}), i.e. compounds of formula (I) wherein R⁵ is -Y-CO₂R¹⁵ and R¹⁵ is not hydrogen may be prepared by esterification of the corresponding acid of formula (I^{C}), as illustrated in Scheme 2, but this step is only necessary if the nature of R¹⁵ is such that the ester group -CO₂R¹⁵ is not compatible with one or more of the synthetic steps used.

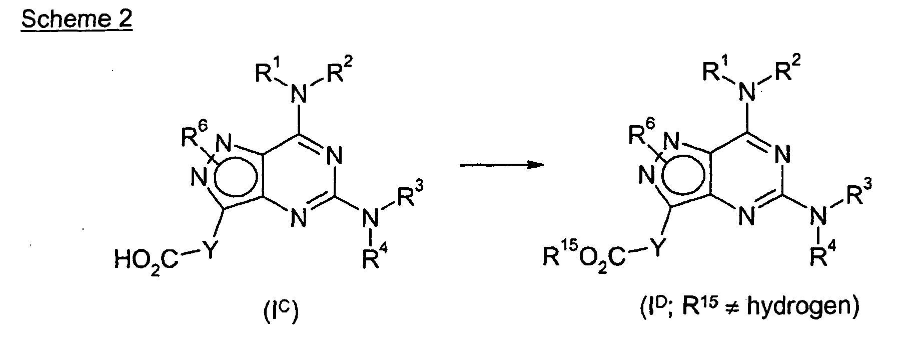

The conversion may conveniently be accomplished by treating a mixture of the acid of formula (I^{C}) and an alcohol R¹⁵-OH in a suitable solvent with a condensing agent such as a carbodiimide, e.g. dicyclohexylcarbodiimide or N-(3-dimethylaminopropyl)-N'-ethylcarbodiimide, optionally in the presence of 4-dimethylaminopyridine, at a temperature of between 0°C and the boiling point of the solvent. Suitable solvents include dichloromethane and dimethylformamide. Alternatively, the acid of formula (I^{C}) may be converted to the corresponding acid chloride using thionyl chloride or oxalyl chloride and then treated with the alcohol R¹⁵-OH.
c) Compounds of formula (II^{A}) and (II^{B}), wherein R^{6A} is as defined for R⁶ except that it cannot be hydrogen, i.e. compounds of formula (II) wherein R⁶ is other than H, can be prepared from compounds of formula (II^{C}), i.e. compounds of formula (II) wherein R⁶ is H, as illustrated in Scheme 3.

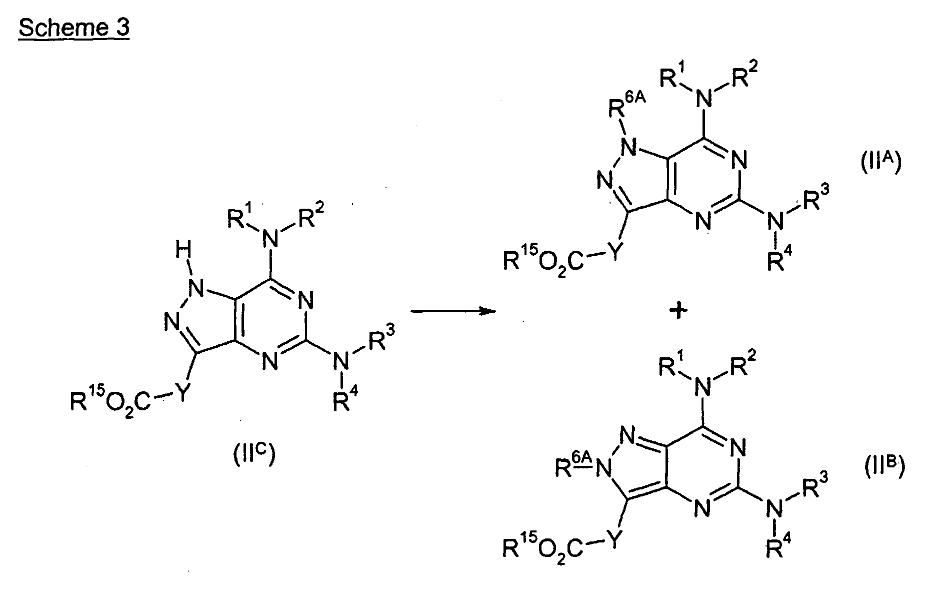

The compound of formula (II^{C}) is treated with a base such as an alkaline metal carbonate or bicarbonate, for example potassium carbonate or caesium carbonate, or a tertiary amine, for example triethylamine, diisopropylethylamine or pyridine, and the appropriate chloride (R^{6A}-Cl), bromide (R^{6A}-Br), iodide (R^{6A}-I), mesylate (R^{6A}-OSO₂CH₃) or tosylate (R^{6A}-OSO₂Tol) in a suitable solvent at a temperature of between -70°C and 100°C. Suitable solvents include ethers such as tetrahydrofuran and dioxan, dimethylformamide and acetonitrile. Stronger bases such as sodium hydride, potassium *tert*-butoxide and sodium or potassium hexamethyldisilazide may also be used. Alternatively, the transformation may be achieved using the Mitsunobu reaction, in which a solution of the compound of formula (II^{C}) and the appropriate alcohol R^{6A}-OH in a suitable solvent is treated with triphenylphosphine and a dialkylazodicarboxylate such as diethyl azodicarboxylate or diisopropyl azodicarboxylate. A preferred solvent is tetrahydrofuran. The reaction is preferably performed at a temperature of between -10°C and ambient temperature.

When the reaction gives a mixture of the two products (II^{A}) and (II^{B}), these can be separated using standard techniques.

The introduction of R⁶ at this stage of the synthetic sequence is not always necessary. It is often more convenient to introduce R⁶ at an early stage and carry it through to the final product.
d) Compounds of formula (II) can be prepared from the corresponding monochlorides of formula (III) by reaction with HNR³R⁴ as illustrated in Scheme 4.

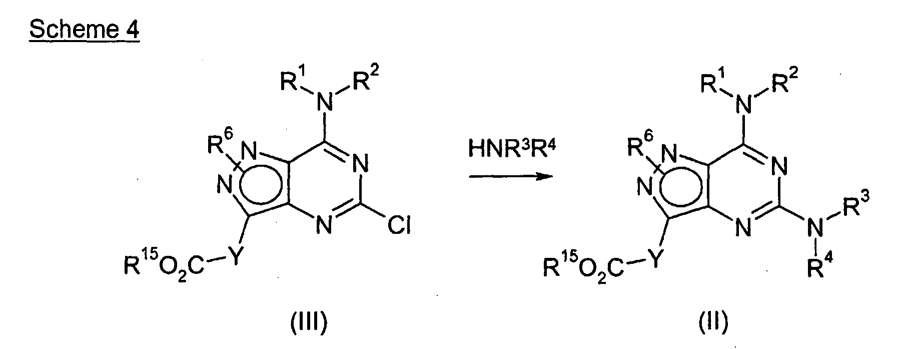

A solution of the monochloride (III) and the amine HNR³R⁴ in a suitable dipolar aprotic solvent are stirred at elevated temperature for between 1 and 24 hours. Suitable solvents include dimethylsulfoxide, dimethylformamide and N-methylpyrrolidinone. An excess of a tertiary amine such as N-ethyldiisopropylamine, N-methylmorpholine or triethylamine and/or a fluoride source such as caesium fluoride or tetraethylammonium fluoride may optionally be included. It is sometimes necessary to perform the reaction at elevated pressure in a closed vessel, particularly when the amine HNR³R⁴ or the solvent is volatile. It will be appreciated that any functional groups in HNR³R⁴, and particularly any primary or secondary amine groups, may need to be protected in order to allow this reaction to proceed successfully.

Preferably, the monochloride is treated with 3-5 equivalents of the amine HNR³R⁴ and optionally 2-5 equivalents of N-ethyldiisopropylamine in dimethylsulfoxide or N-methylpyrrolidinone, optionally in the presence of caesium fluoride or tetraethylammonium fluoride, at 80-125°C for 12-18 hours, optionally in a closed vessel.

Alternatively, the compounds of formula (III) may be hydrolysed as described in part a) above to provide the corresponding carboxylic acid of formula (IV) which is then treated with amine HNR³R⁴ to provide compounds of formula (I^{C}), as illustrated in scheme 4a.

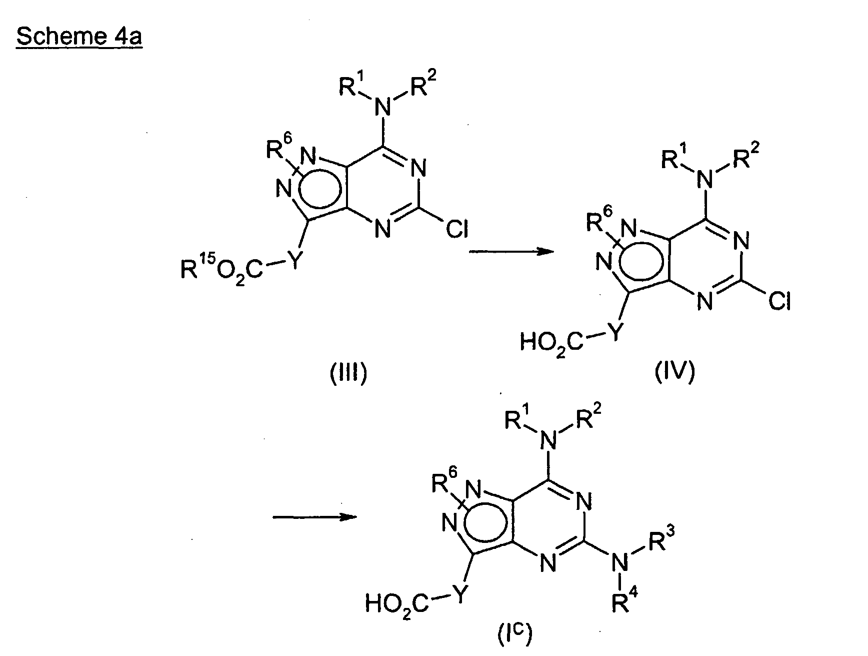

Preferably, the monochloride (IV) is treated with 3-5 equivalents of the amine HNR³R⁴ and optionally 2-5 equivalents of N-ethyldiisopropylamine in dimethylsulfoxide or N-methylpyrrolidinone, optionally in the presence of caesium fluoride or tetraethylammonium fluoride, at 80-125°C for 12-18 hours, optionally in a closed vessel.
e) Compounds of formula (III) can be prepared from the corresponding dichlorides of formula (V) by reaction with HNR¹R² as illustrated in Scheme 5.

Preferably, the monochloride is treated with 3-5 equivalents of the amine HNR³R⁴ and optionally 2-5 equivalents of N-ethyldiisopropylamine in dimethylsulfoxide or N-methylpyrrolidinone, optionally in the presence of caesium fluoride or tetraethylammonium fluoride, at 80-125°C for 12-18 hours, optionally in a closed vessel.

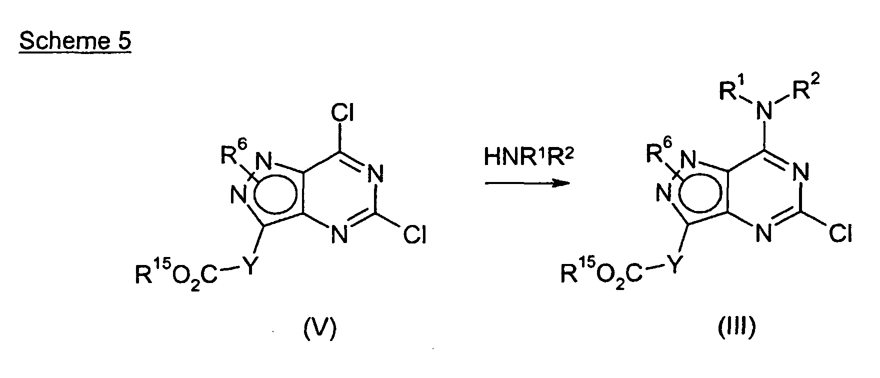

A solution of the dichloride (V), the amine HNR¹R² and optionally an excess of a tertiary amine such as N-ethyldiisopropylamine, N-methylmorpholine or triethylamine in a suitable solvent are stirred at ambient or elevated temperature for between 1 and 24 hours. Suitable solvents include dichloromethane, dimethylsulfoxide, dimethylformamide, acetonitrile, tetrahydrofuran and N-methylpyrrolidinone. It will be appreciated that any functional groups in HNR¹R², and particularly any primary or secondary amine groups, may need to be protected in order to allow this reaction to proceed successfully. Preferably, the monochloride is treated with 3-5 equivalents of the amine HNR¹R² and optionally 3-5 equivalents of N-ethyldiisopropylamine in dichloromethane, dimethylsulfoxide or a mixture of dimethylsulfoxide and N-methylpyrrolidinone at 25-90°C for 1-18 hours.

Alternatively, a solution of the amine HNR¹R² in a suitable solvent is treated with butyllithium or sodium hexamethyldisilazide at low temperature, and the dichloride is added to the resulting solution. Suitable solvents include tetrahydrofuran, dioxan and N-methylpyrrolidinone.

In certain cases, particularly when Y is a covalent bond and the amine HNR¹R² is only weakly nucleophilic, the direct transformation of compounds of formula (V) into compounds of formula (III) gives unsatisfactory results and a more indirect alternative route may be employed. This route is described in part w) below.
f) Compounds of formula (V) can be prepared from the corresponding pyrazolopyrimidinediones of formula (VI) as illustrated in Scheme 6.

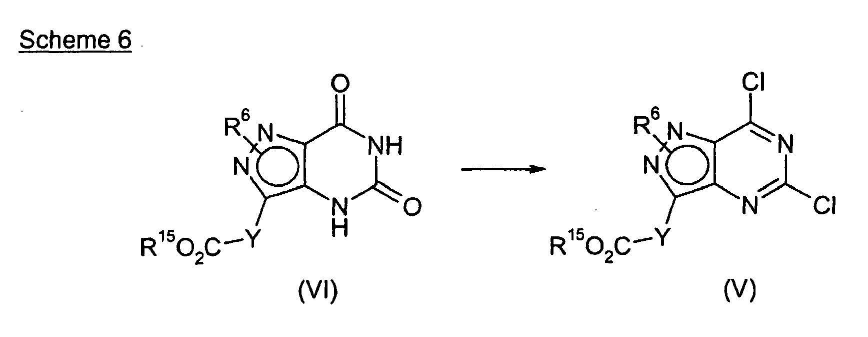

The dione is treated with a large excess of a suitable chlorinating reagent such as phosphorus oxychloride (POCl₃) or phenylphosphonyl dichloride (PhP(O)Cl₂) in the presence of a tertiary amine such as N-ethyldiisopropylamine, N-methylmorpholine, triethylamine or N,N-dimethylaniline at elevated temperature for 8-48 hours. Dimethylformamide can optionally be added as a catalyst. Alternatively, the dione is treated with POCl₃ or PhP(O)Cl₂ in a suitable solvent in the presence of a tetraalkylammonium chloride, such as tetraethylammonium chloride, and optionally in the presence of a tertiary amine such as N-ethyldiisopropylamine at elevated temperature. Suitable solvents include acetonitrile and propionitrile. Preferably, the dione is treated with 10-30 equivalents of POCl₃ and 3-5 equivalents of tetraethylammonium chloride in propionitrile or acetonitrile at reflux for 4-24 hours.
g) Compounds of formula (VI) can be prepared from the corresponding aminoamides of formula (VII) as illustrated in Scheme 7.

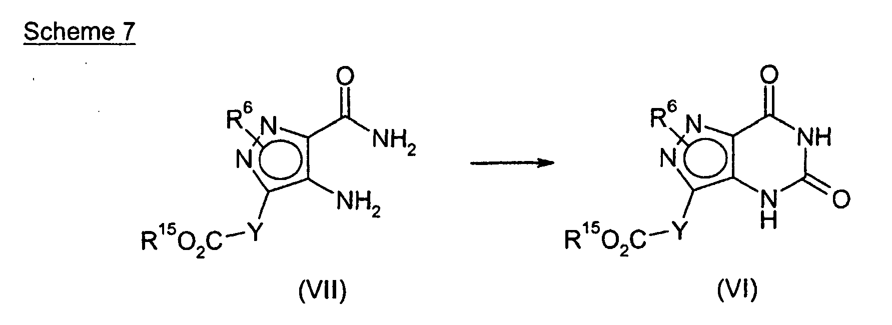

A solution of the pyrazolecarboxamide (VII) and phosgene or an equivalent thereof, such as 1,1'-carbonyldiimidazole, trichloromethyl chloroformate or bis(trichloromethyl) carbonate, in a suitable solvent is stirred at a temperature of between ambient temperature and the boiling point of the solvent, optionally at elevated pressure, for between 2 and 18 hours. Suitable solvents include acetonitrile, dichloromethane and dimethylformamide. Preferably, a solution of the amine of formula (VII) and 1-2.5 equivalent of 1,1'-carbonyldiimidazole in N,N-dimethylformamide, acetonitrile or dichloromethane is heated at between room temperature and the reflux temperature of the reaction for 1-18 hours.
h) Compounds of formula (VII) can be prepared from the corresponding nitroamides of formula (VIII) as illustrated in Scheme 8.

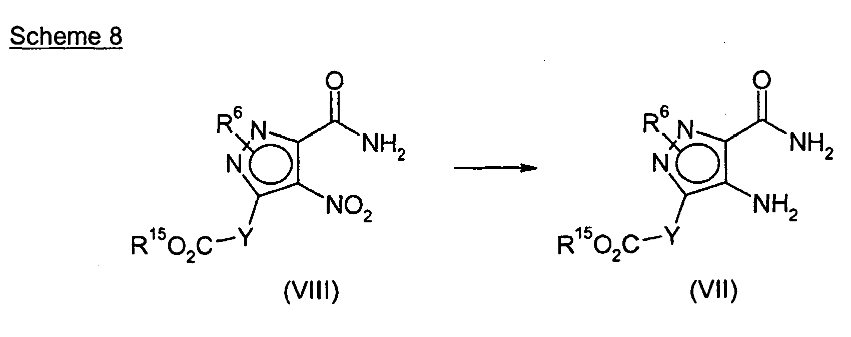

Reduction of the nitro group can be achieved by, for example, by transfer or catalytic hydrogenation, or by a dissolving metal reduction.

For transfer hydrogenation, the nitro compound is reacted with a suitable hydrogen donor, such as ammonium formate or cyclohexene, in a polar solvent, such as tetrahydrofuran, methanol or ethanol, in the presence of a transition metal or transition metal salt catalyst, such as palladium or palladium(II) hydroxide, optionally at elevated temperature and pressure.

For catalytic hydrogenation, a solution of the nitro compound in a polar solvent, such as tetrahydrofuran, methanol or ethanol, is stirred under a hydrogen atmosphere in the presence of a transition metal or transition metal salt catalyst, such as palladium or palladium(II) hydroxide, optionally at elevated pressure and temperature. The catalyst may be in solution (homogeneous catalysis) or in suspension (heterogeneous catalysis).

For dissolving metal reduction, the nitro compound is treated with a suitable reactive metal, such as zinc or tin, in the presence of an acid such as acetic acid or hydrochloric acid. Other reducing agents, such as tin(II) chloride, may also be used.
i) Compounds of formula (VIII) can be prepared from the corresponding nitroesters of formula (IX) as illustrated in Scheme 9.

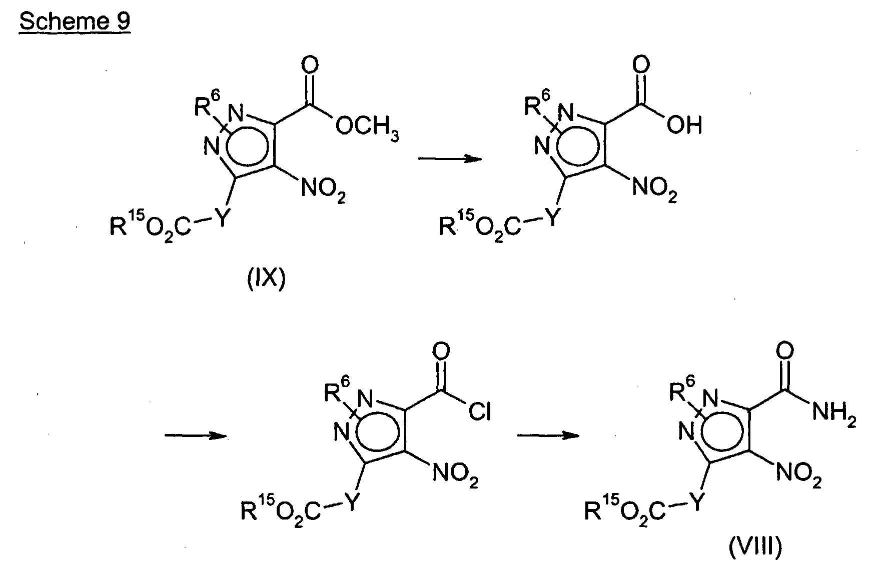

The methyl ester of the compounds of formula (IX) can be hydrolysed as described in part a) above. The acid is then converted to the corresponding acid chloride by treatment with oxalyl chloride and dimethylformamide in a suitable solvent such as dichloromethane, or with thionyl chloride. Finally, a solution of the acid chloride in a suitable solvent such as dichloromethane, tetrahydrofuran or dioxan is treated with gaseous ammonia or aqueous ammonia at between -78°C and room temperature to provide the amide of formula (VIII).

In the embodiments (IX^{A}) in which Y is a covalent bond and R¹⁵ is a methyl group, the use of one equivalent of metal hydroxide leads to the chemoselective hydrolysis of the ester group adjacent to the R⁶ substituent (Chambers, D. et al., J. Org. Chem. 50, 4736-4738, 1985), as illustrated in scheme 9A. 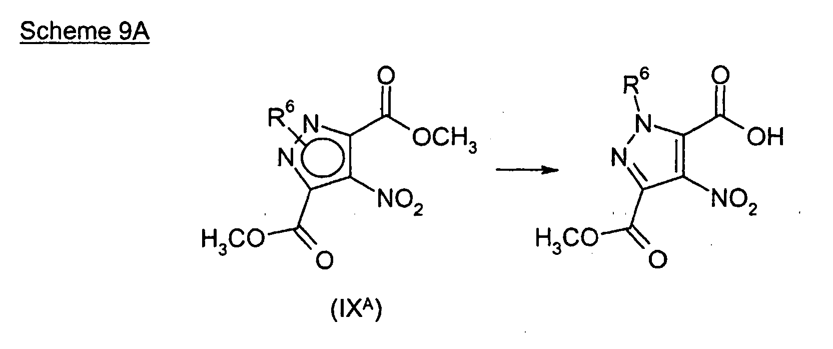
j) Compounds of formula (IX^{B}), wherein R^{6A} is any group according to R⁶ except hydrogen, i.e. compounds of formula (IX) except those wherein R⁶ is hydrogen, can be prepared from the corresponding esters of formula (IX^{C}) as illustrated in Scheme 10.

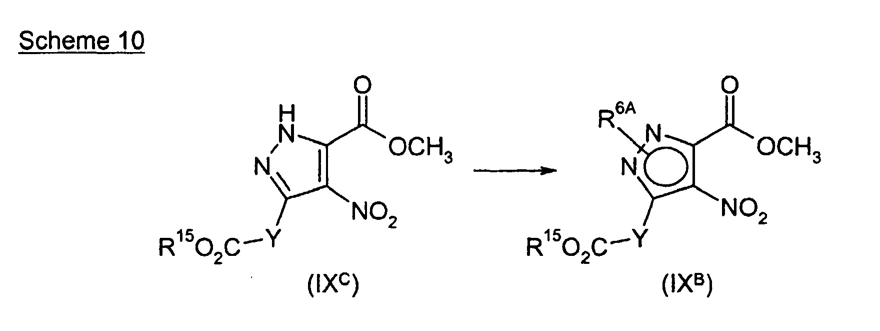

The compounds of formula (IX^{C}) are treated with a combination of an alkylating agent and a base, or with an alcohol, triphenylphosphine and a dialkyl azodicarboxylate, as described in part c) above.
k) The compound of formula (IX^{C}) wherein R¹⁵ is methyl and Y is a covalent bond is described in published international patent application WO00/24745 (see preparation 2, page 48). Other compounds of formula (IX), and particularly compounds of formula (IX^{C}), can be prepared in two steps from the diacids of formula (X), as illustrated in scheme 11.

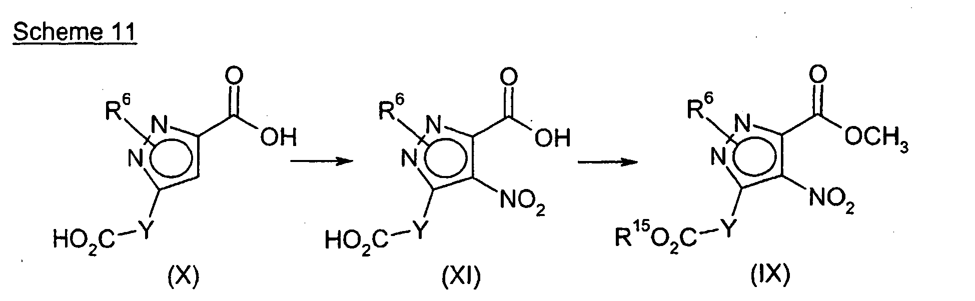

In the first step, the compounds of formula (X) are treated with a nitrating agent such as nitric acid or a mixture of nitric acid and sulphuric acid to provide the compounds of formula (XI). In the second step, the two carboxylic acid groups are esterified. When R¹⁵ is methyl, this is conveniently achieved in a single operation. When R¹⁵ is other than methyl, two sub-steps are necessary, and the order in which the two groups are esterified will depend on the nature of Y and R⁶. Suitable conditions for forming esters are well known in the art. When R¹⁵ is methyl, a preferred method is to treat the diacid with thionyl chloride so as to form the bis-chloride and then react this with methanol.
I) Certain compounds of formula (X) are commercially available or are described in the literature, in particular those wherein Y is a covalent bond. Compounds of formula (X) that are not items of commerce can be prepared as illustrated in Schemes 12, 13 and 14.

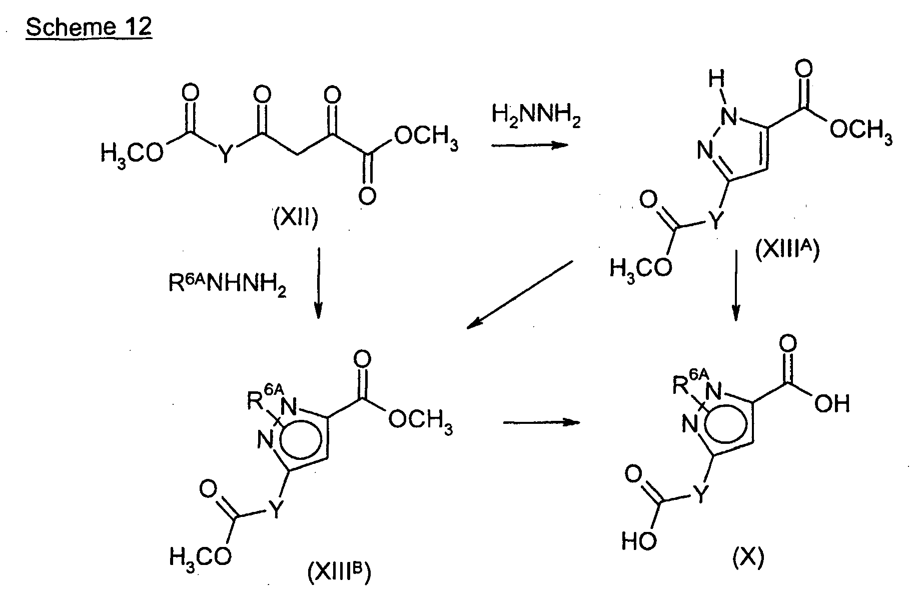

The method illustrated in Scheme 12 is the Knorr pyrazole synthesis. A 1,3-diketone of formula (XII) is reacted with hydrazine to give a pyrazole of formula (XIII^{A}), or with a substituted hydrazine R^{6A}-NHNH2, wherein R^{6A} is as defined in part c) above, to give a pyrazole of formula (XIII^{B}).

Pyrazoles of formula (XIII^{B}) may also be obtained by N-alkylation of the corresponding pyrazoles of formula (XIII^{A}) following the method described in part c) above. Hydrolysis of the ester groups as described in part a) above then provides the compounds of formula (X).

Compounds of formula (XII) can be prepared from the corresponding methyl ketones of formula (XIV) using a crossed Claisen condensation as illustrated in Scheme 13. 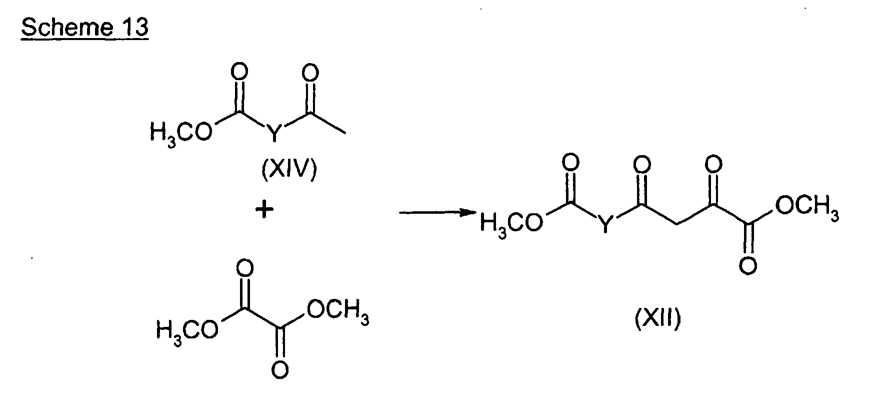 A methyl ketone of formula (XIV) is reacted with dimethyl oxalate in a suitable solvent in the presence of a suitable base. Suitable solvents include ethers, such as tetrahydrofuran. Suitable bases include sodium hydride, potassium *tert*-butoxide and lithium diisopropylamide. Alternatively, sodium methoxide may be used as the base and methanol as the solvent.

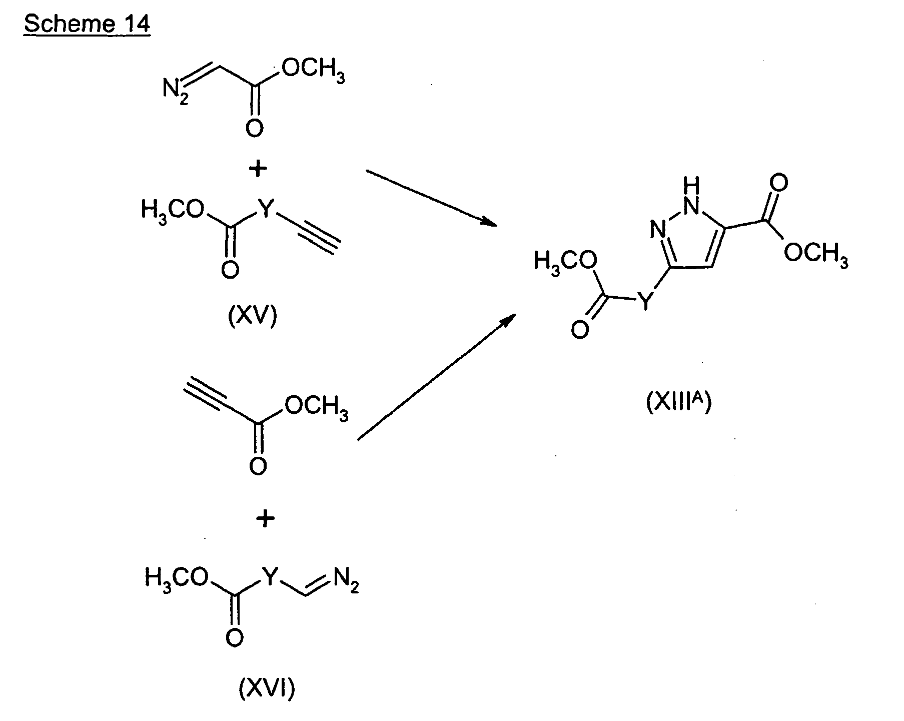

The method illustrated in scheme 14 is the Pechmann pyrazole synthesis. A diazo compound and an acetylene are combined to produce a pyrazole of formula (XIII^{A}). When Y is othe rthan a covalent bond two variants of the method can be considered. An acetylene of formula (XV) can be combined with methyl diazoacetate, or a diazo compound of formula (XVI) can be combined with methyl propiolate. The product of formula (XIII^{A}) may be carried forward as described above.

In addition to the methods described above, certain compounds of general formulae (III) and (IV) may be prepared by modification of the substituent at the C-3 position of the pyrazolopyrimidine, as further illustrated below. It will be appreciated that the synthetic transformations discussed may also be used in the elaboration of precursor compounds such as the pyrazoles of formula (IX).
m) Compounds of formula (III^{A}), i.e compounds of formula (III) wherein Y is CH₂, may be prepared from the corresponding compounds of formula (IV^{A}), i.e. compounds of formula (IV) wherein Y is a covalent bond, by a one-carbon homologation method such as the Arndt-Eistert reaction illustrated in Scheme 15.

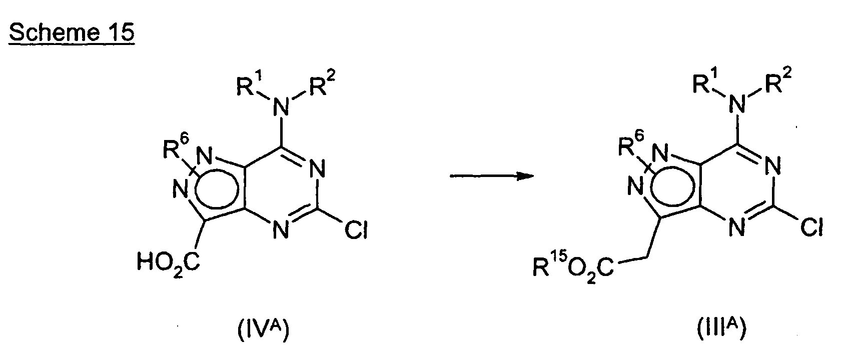

The carboxylic acid is converted to a reactive intermediate such as the acid chloride (by reaction with oxalyl chloride) or a mixed anhydride (by reaction with isobutyl chloroformate). The intermediate is reacted with diazomethane to provide an α-diazoketone. This is treated with silver oxide in the presence of R¹⁵-OH to give the homologated ester of formula (III^{A}).
n) Compounds of formula (IV^{B}), i.e. compounds of formula (IV) wherein Y is CH₂, may be prepared from the corresponding nitriles of formula (XVII) by the method illustrated in Scheme 16.

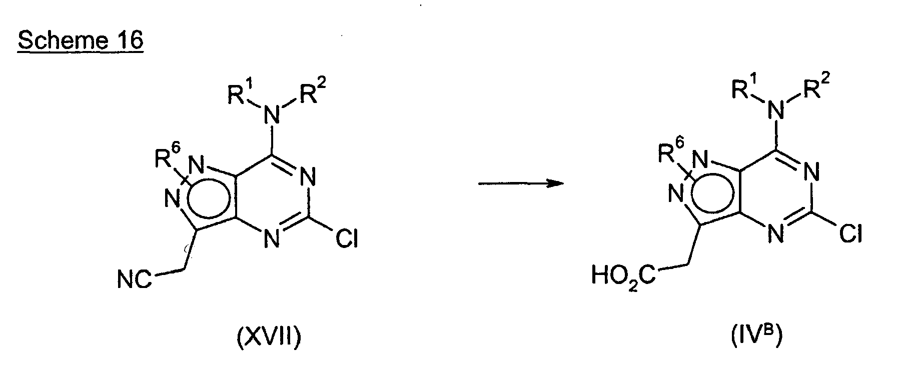

The nitrile can be hydrolysed, e.g. by treatment with aqueous mineral acids, such as hydrochloric acid.
o) Compounds of formula (XVII) can be prepared from the corresponding chlorides of formula (XVIII) by the method illustrated in Scheme 17.

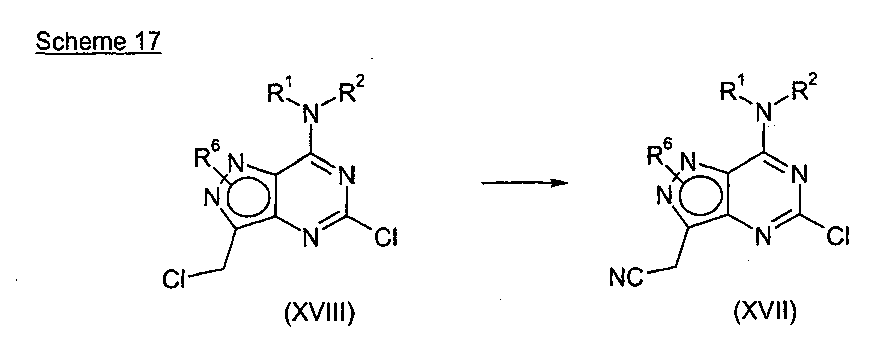

The chloride is treated with a metal cyanide, such as sodium cyanide or potassium cyanide in a suitable solvent, such as dimethylsulfoxide, dimethylformamide or ethanol.
p) Compounds of formula (XVIII) can be prepared from the corresponding alcohols of formula (XIX) by the method illustrated in Scheme 18.

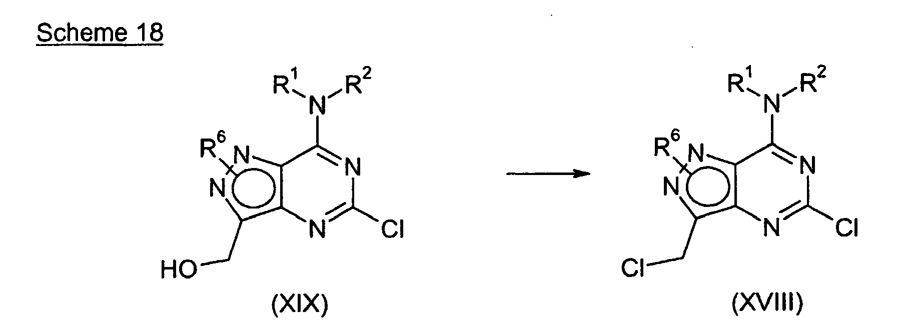

The alcohol is treated with a mixture of triphenylphosphine and N-chlorosuccinimide or tetrachloromethane, or with thionyl chloride.
q) Compounds of formula (XIX) can be prepared from the corresponding esters of formula (III^{B}), i.e. compounds according to formula (III) wherein Y is a covalent bond, or from the corresponding acids of formula (IV^{A}) by the method illustrated in Scheme 19.

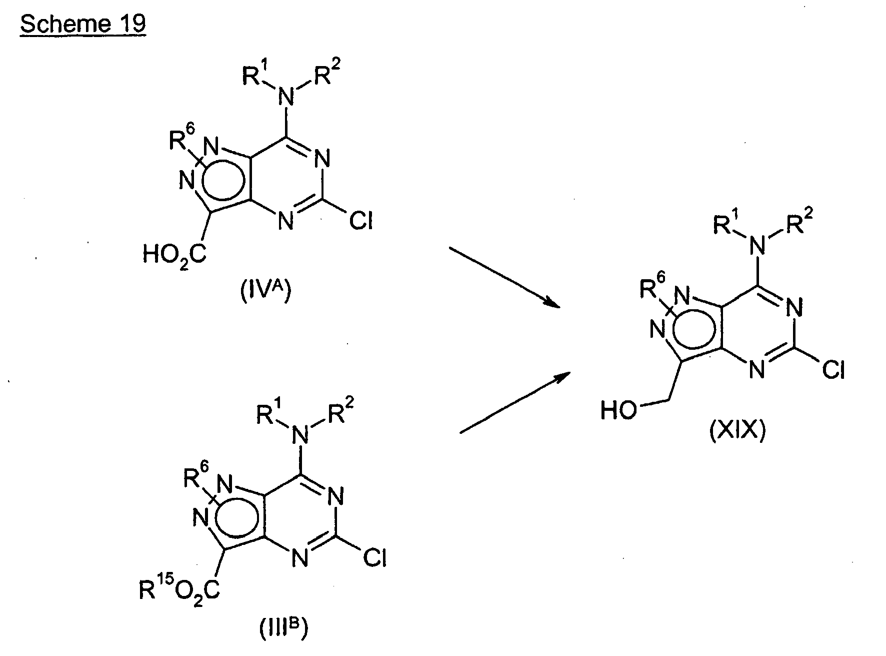

The acids of formula (IV^{A}) and the esters of formula (III^{B}) can be reduced to the alcohols of formula (XIX) by treatment with lithium aluminium hydride in a suitable solvent at a temperature of between 0° and the boiling point of the solvent. Suitable solvents include ethers such as tetrahydrofuran. The acids can also be reduced by treatment with isobutyl chloroformate and a tertiary amine base to provide a mixed anhydride, followed by reaction with sodium borohydride. The esters can also be reduced by treatment with disobutylaluminium hydride or lithium borohydride.
r) Compounds of formula (III^{C}), i.e. compounds of formula (III) wherein Y is CH₂CH₂ can be prepared from the corresponding acrylate ester of formula (XX) by the method illustrated in Scheme 20.

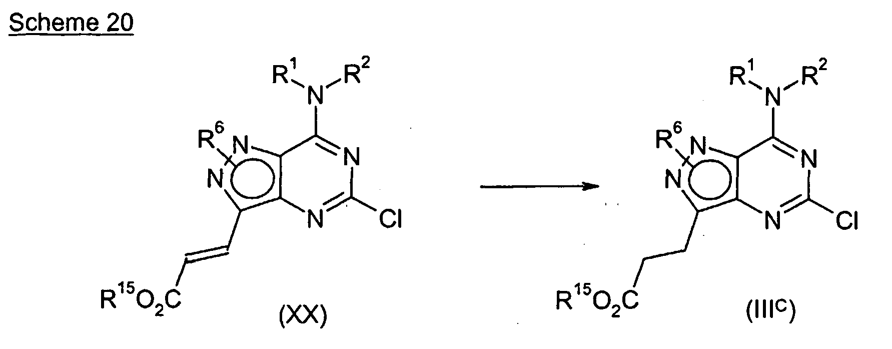

The reduction of the carbon-carbon double bond of (XX) to give the compounds of formula (III^{C}) can be accomplished by catalytic hydrogenation using molecular hydrogen in the presence of a transition metal catalyst such as palladium, platinum or nickel. When R¹⁵ is benzyl the conditions can be chosen such that only the double bond is reduced or reduction is accompanied by hydrogenolytic cleavage of the ester to give the carboxylic acid.

The acrylates of formula (XX) can also be treated with alkylcopper reagents to give analogues of the compounds of formula (III^{C}) in which an alkyl substituent is introduced on the carbon atom adjacent to the pyrazolopyrimidine ring system, or with a sulphonium ylid or a carbene equivalent to give a 2-(pyrazolopyrimidinyl)-cyclopropane-1-carboxylate derivative.
s) Compounds of formula (XX) can be prepared from the corresponding aldehydes of formula (XXI) by the method illustrated in Scheme 21.

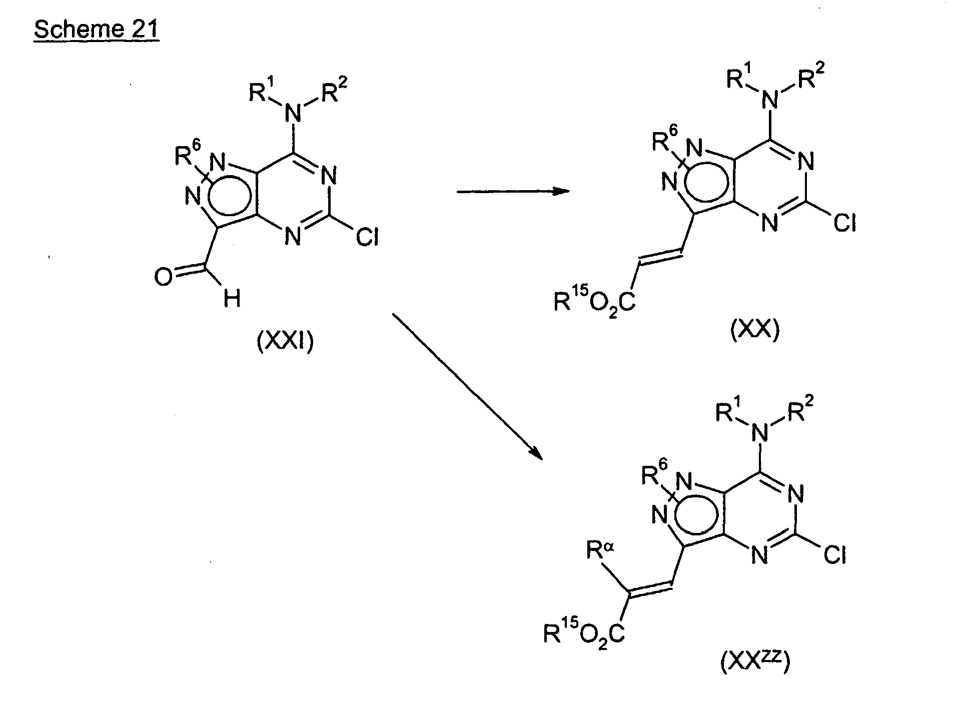

The aldehyde of formula (XXI) can be converted to the acrylate ester of formula (XX) by reaction with a phosphorus reagent following the protocols of the Wittig, Horner or Wadsworth-Horner-Emmons reactions. The reagent is prepared by treating a triphenylphosphonium salt Ph₃P⁺CH₂CO₂R¹⁵.X⁻ (Wittig), a phosphine oxide Ph₂P(O)CH₂CO₂R¹⁵ (Horner), or a phosphonate (EtO)₂P(O)CH₂CO₂R¹⁵ (Wadsworth-Horner-Emmons), with a base such as butyllithium, a lithium dialkylamide or an alkaline metal alkoxide, in a suitable solvent such as tetrahydrofuran, wherein X⁻ is a suitable anion such as a halide, for example chloride, bromide or iodide.

The method is not limited to the preparation of α-unsubstituted acrylate esters. The use of an alkyl-substituted phosphorus reagent such as Ph₃P⁺CH(R^{α})CO₂R¹⁵.X⁻ or the equivalent phosphine oxide or phosphonate, wherein R^{α} is alkyl, and further wherein X⁻ is a suitable anion such as a halide, for example chloride, bromide or iodide, gives access to the corresponding α-alkyl acrylate derivative (XX^{ZZ}).

The conversion of the aldehydes of formula (XXI) to acrylate esters of formula (XX) can also be achieved by reaction with a malonate derivative following the method of the Knoevenagel condensation.
t) Compounds of formula (XXI) can be prepared from the esters of formula (III^{B}) or more preferably from the corresponding alcohols of formula (XIX) by the methods illustrated in Scheme 22.

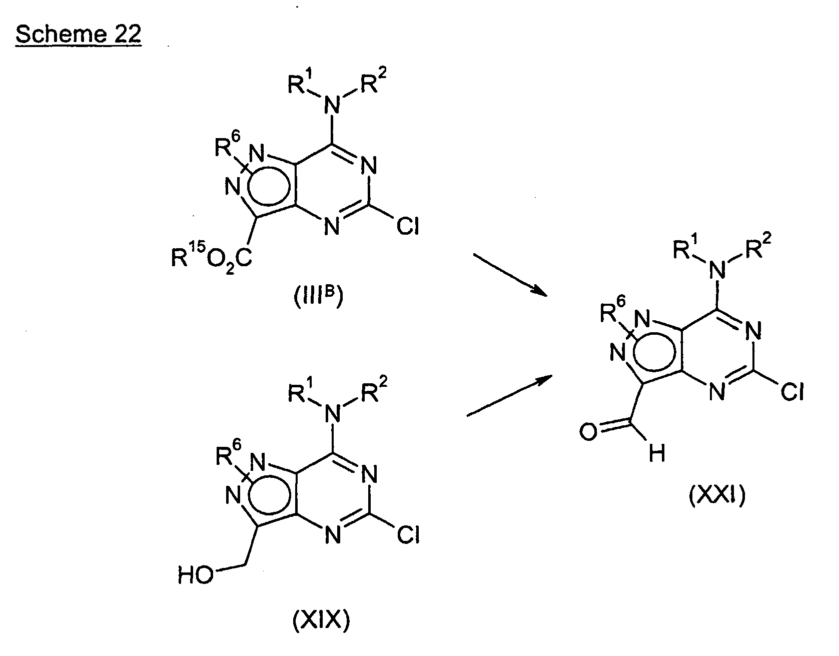

The reduction of the esters of formula (III^{B}) can be achieved using diisobutylaluminium hydride (DIBAL) in a suitable solvent at a temperature of less than 0°C, preferably less than -60°C. Suitable solvents include hydrocarbons such as pentane, hexane and toluene, ethers such as tetrahydrofuran, and mixtures thereof.

The oxidation of the alcohols of formula (XIX) can be achieved using a chromium(VI) reagent such as pyridinium chlorochromate, a hypervalent iodine reagent such as the Dess-Martin periodinane, or a combination of tetra-n-propylammonium perruthenate and N-methylmorpholine-N-oxide in a suitable solvent at a temperature of between 0°C and ambient temperature. Suitable solvents include dichloromethane.
u) The aldehydes of formula (XXI) may be converted to esters of formula (III^{A}) as illustrated in Scheme 23

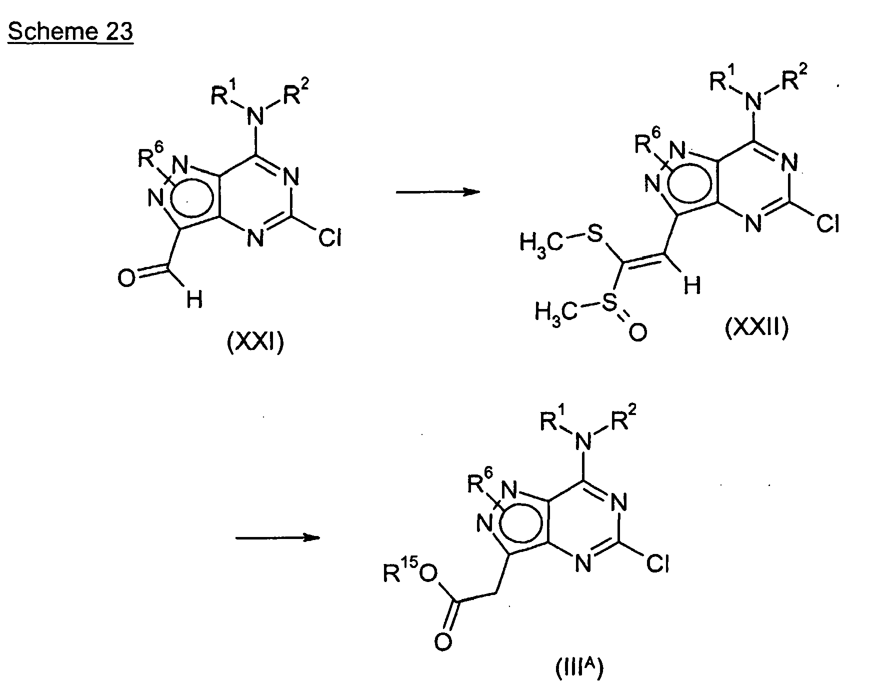

The aldehyde is treated with methyl methylmercaptomethyl sulfoxide (CH₃SCH₂S(O)CH₃) and triton B in tetrahydrofuran to give intermediate (XXII) which is treated with the appropriate alcohol R¹⁵OH and acetyl chloride to provide the ester of formula (III^{A}). This method is particularly useful when R¹⁵ is methyl.
v) Compounds of formula (III^{C}) can also be prepared from the corresponding chlorides of formula (XVIII) by the method illustrated in Scheme 24.

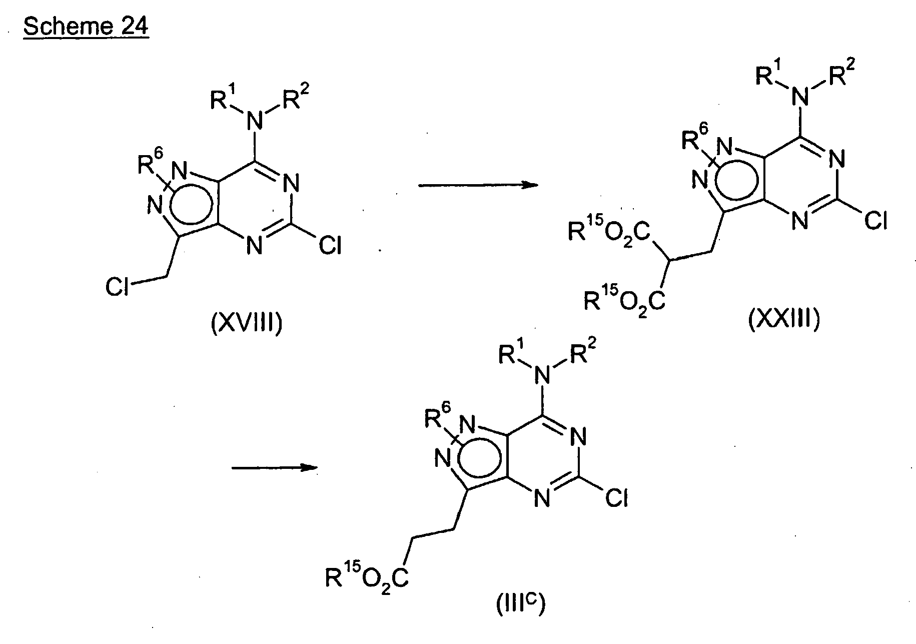

The chloride of formula (XVIII) is reacted with a dialkyl malonate (R¹⁵O₂C)₂CH₂ and a base in a suitable solvent. Typically, the base is an alkaline metal alkoxide such as sodium ethoxide or potassium *tert*-butoxide, and the solvent is an alcohol such as ethanol or an ether such as tetrahydrofuran. Preferably the base and the solvent are chosen such as to minimise transesterification with the malonate reagent and the intermediate (XXIII). For example, when the reagent is diethyl malonate the base is preferably sodium ethoxide and the solvent is ethanol. The intermediate (XXIII) is then decarboxylated to give the product (III^{C}). This can be achieved by selective hydrolysis using one equivalent of an alkaline metal hydroxide, such as sodium hydroxide, followed by acidification, or by any other method known in the art.

The method is not limited to symmetrical malonates. For example, the use of *tert-*butyl methyl malonate would give an intermediate (XXIII) in which one R¹⁵ is methyl and the other is *tert*-butyl. By choosing the appropriate conditions, decarboxylation could then be controlled to give a product (III^{C}) in which R¹⁵ was either *tert*-butyl or methyl.

The method can be extended to substituted malonates (R¹⁵O₂C)₂CHR, where R is an alkyl group. This gives access to compounds analogous to (IIF) in which the group R is a substituent on the carbon atom adjacent to the R¹⁵O₂C group. These compounds can also be prepared by alkylating the intermediate (XXIII) with R-Br or R-I in the presence of an alkaline metal alkoxide base.
w) As mentioned in part e) above, the reaction of compounds of formula (V^{A}), i.e. compounds of formula (V) wherein Y is a covalent bond, with weakly nucleophilic amines HNR¹R² is sometimes not high yielding. An alternative route is illustrated in Schemes 25A and 25B.

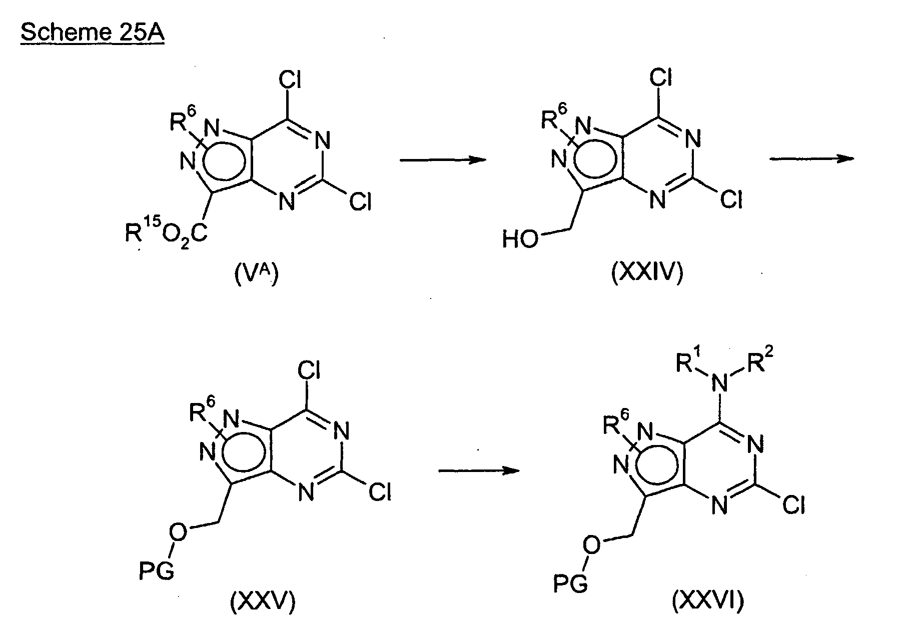

The esters of formula (V^{A}) can be reduced to the alcohols of formula (XXIV) according to the methods described in part q) above. A preferred method is reduction with diisobutylaluminium hydride at a temperature of between -20°C and 0°C. The primary alcohol is then protected to give compounds of formula (XXV), wherein PG is an alcohol protecting group. A preferred protecting group is a trialkylsilyl group, particularly a *tert*-butyldimethylsilyl group. The compounds of formula (XXV) are then reacted with an amine HNR¹R² according to the methods described in part e) above to give compounds of formula (XXVI).

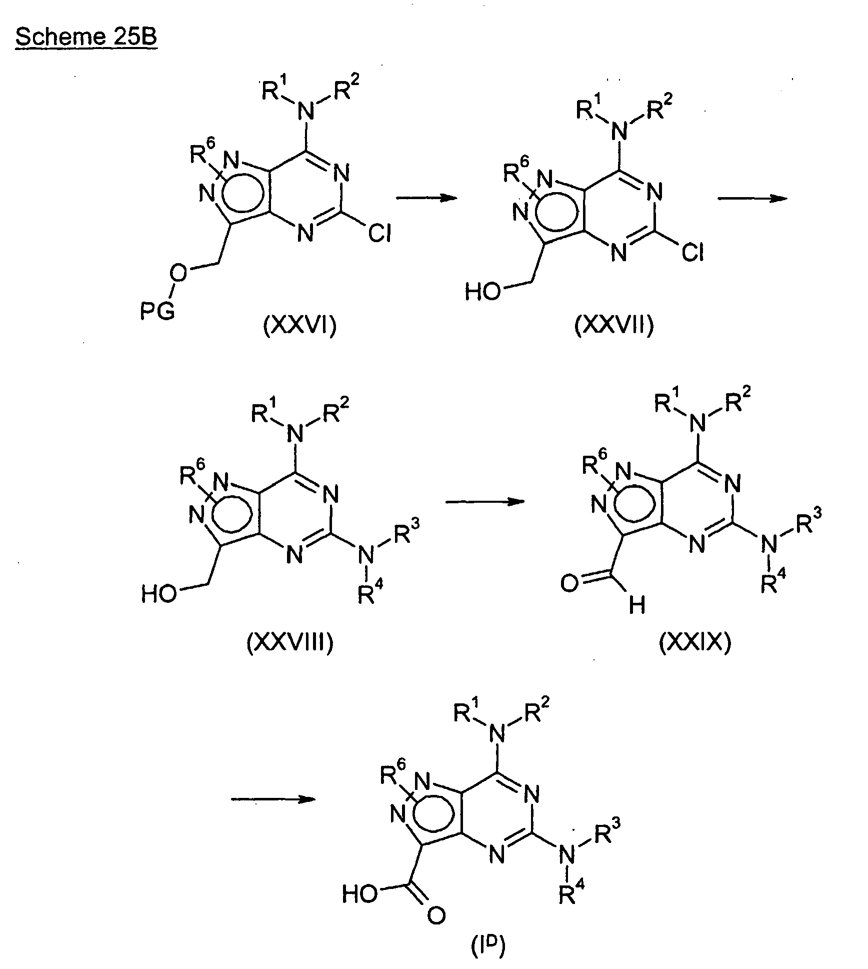

The compounds of formula (XXVI) are deprotected to provide the primary alcohols of formula (XXVII) using appropriate conditions. When PG is a trialkylsilyl group it may be removed by treatment with a fluoride salt, such as tetrabutylammonium fluoride, or with hydrochloric acid. The -NR³R⁴ group is then introduced according to the methods described in part d) above to provide compounds of formula (XXVIII). The primary alcohol is oxidised as described in part t) above to provide the aldehydes of formula (XXIX). A preferred oxidising agent is the Dess-Martin periodinane. Finally the aldehydes of formula (XXIX) are oxidised to provide the acids of formula (I^{D}), i.e. compounds of formula (I^{C}) wherein Y is a covalent bond. Suitable oxidising agents include potassium permanganate, Jones' reagent and sodium chlorite. A preferred method is to treat the aldehydes with sodium chlorite, sodium dihydrogenphosphate and 2-methyl-2-butene in *tert*-butanol at room temperature for about 1 hour.

Alternatively, it may be preferred to perform the oxidation of the alcohol of formula (XXVII) to the corresponding acid (via the corresponding aldehyde), using the methods previously described, prior to reaction with HNR³R⁴, to provide the compound of formula (I^{D}).
x) Compounds of formula (I^{E}), i.e. compounds of formula (I) wherein R⁵ is -Y-R¹⁶ can be prepared from the corresponding monochlorides of formula (XXX) as illustrated in Scheme 26.

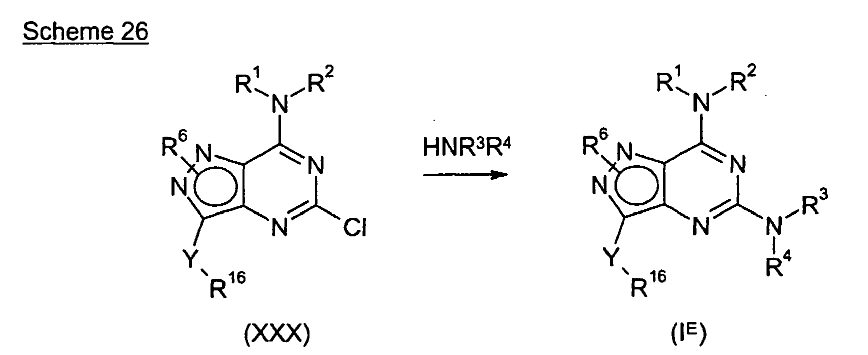

The monochlorides of formula (XXX) are reacted with amines HNR³R⁴ as described in part d) above.

Alternatively, the -NR³R⁴ group may be introduced into a suitable precursor and the -Y-R¹⁶ group elaborated subsequently.
y) Compounds of formula (XXX^{A}), i.e. compounds of formula (XXX) wherein R¹⁶ is -CONHR¹⁸ can be prepared from the corresponding compounds of formula (IV) as illustrated in Scheme 27.

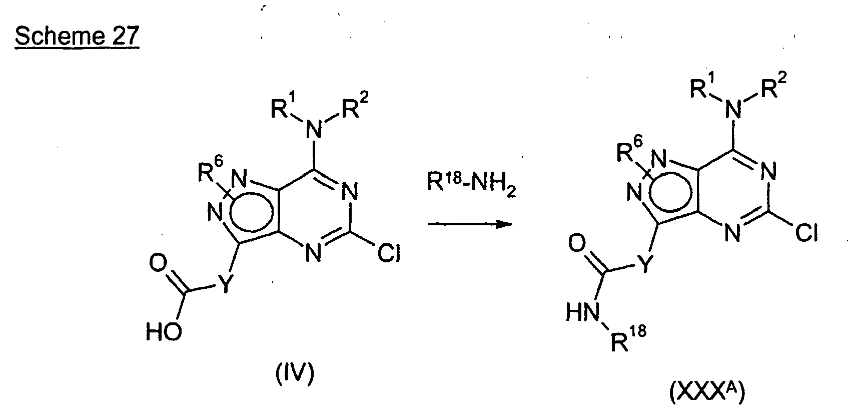

The acid of formula (IV) is treated with the appropriate sulfonamide R¹⁸-NH₂ and a carbodiimide in a suitable solvent in the presence of 4-(dimethylamino)pyridine. A suitable solvent is dimethylformamide or dichloromethane. It is sometimes preferred to introduce the R¹⁸-NH₂ group in the final step, i.e. after elaboration of the -NR³R⁴ group.

Preferably, the acid is treated with 1.3 equivalents of 1-(3-dimethylaminopropyl)-3-ethylcarbodiimide hydrochloride, 1.3 equivalents of 4-dimethylaminopyridine and 1.2-1.3 equivalents of the sulphonamide R¹⁸NH₂, in dichloromethane at about room temperature for upto 18 hours.
z) Compounds of formula (XXX) wherein R¹⁶ is a heterocyclic carboxylic isostere such as tetrazol-5-yl (compounds of formula (XXX^{B})), 5-trifluoromethyl-1,2,4-triazol-3-yl (compounds of formula (XXX^{C})) and 2,5-dihydro-5-oxo-1,2,4-oxadiazol-3-yl (compounds of formula (XXX^{D})) can be prepared from compounds of formula (XXXI) using standard methods such as those illustrated in Scheme 28A, 28B and 28C.

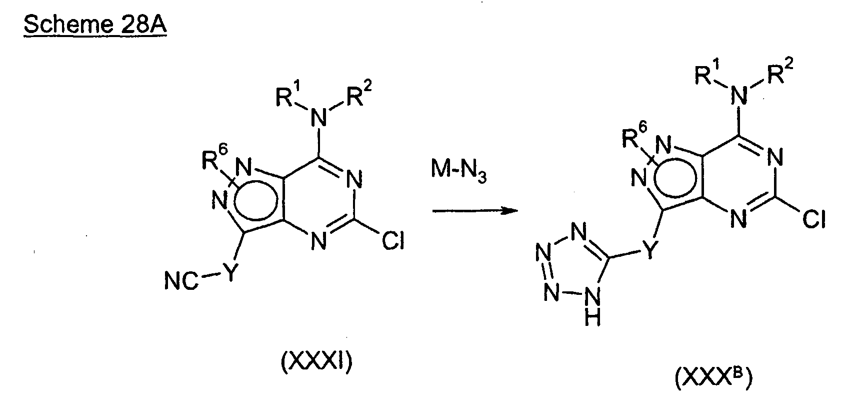

The nitrile of formula (XXXI) is treated with an azide, such as an alkaline metal azide (M = Na, K), a trialkylsilyl azide (M = alkyl₃Si) or a trialkyltin azide (M = alkyl₃Sn), in a suitable solvent at a temperature of between ambient temperature and the boiling point of the solvent. A preferred azide is tributyltin azide. A preferred solvent is dioxan.

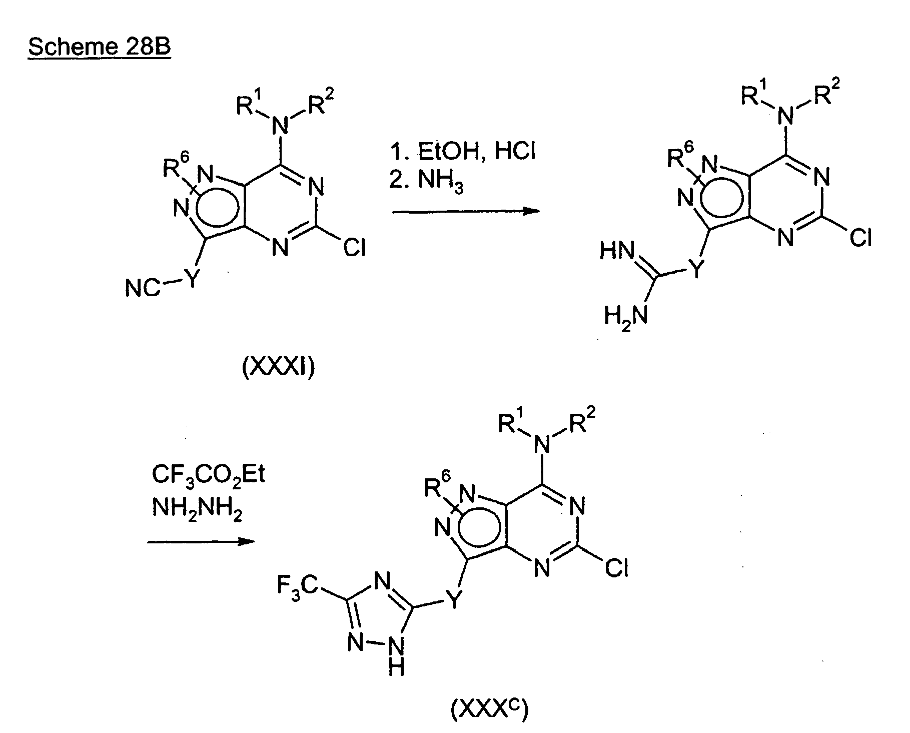

The nitrile of formula (XXXI) is treated with ethanol and hydrogen chloride to form an imidate, which is then treated with ammonia to form an amidine. The amidine is treated with ethyl trifluoroacetate and hydrazine to provide the triazole of formula (XXX^{C}). The 5-(methylsulfonyl)-substituted triazole can be prepared in an analogous manner.

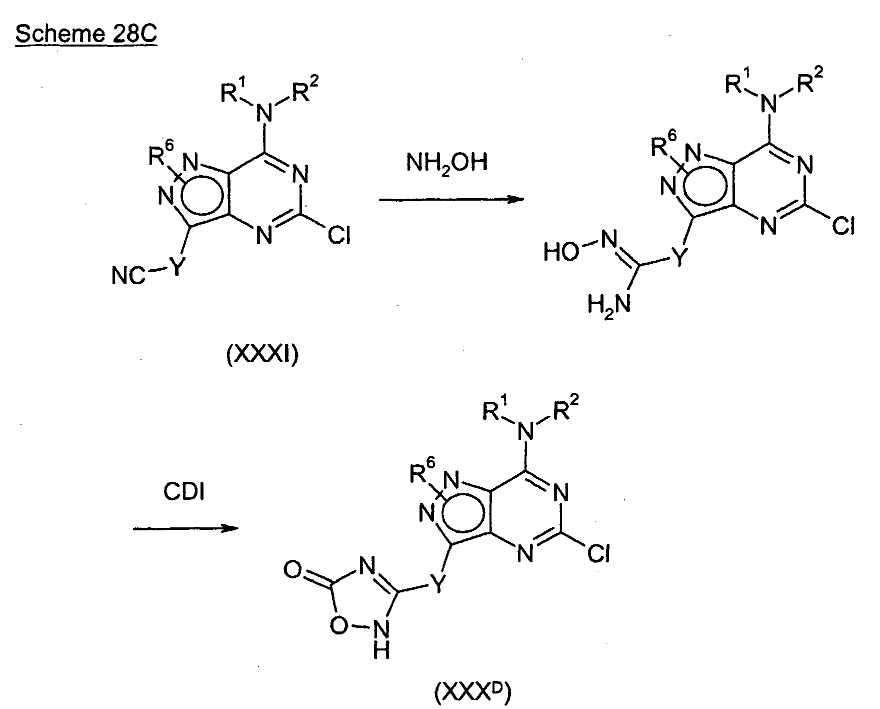

The nitrile of formula (XXXI) is treated with hydroxylamine to form an N-hydroxyamidine, which is then treated with 1,1'-carbonyldiimidazole to provide the oxadiazolone of formula (XXX^{C}).
aa) Compounds of formula (XXXI) can be prepared using the methods described in part o) above, or from compounds of formula (IV) using the method illustrated in Scheme 29.

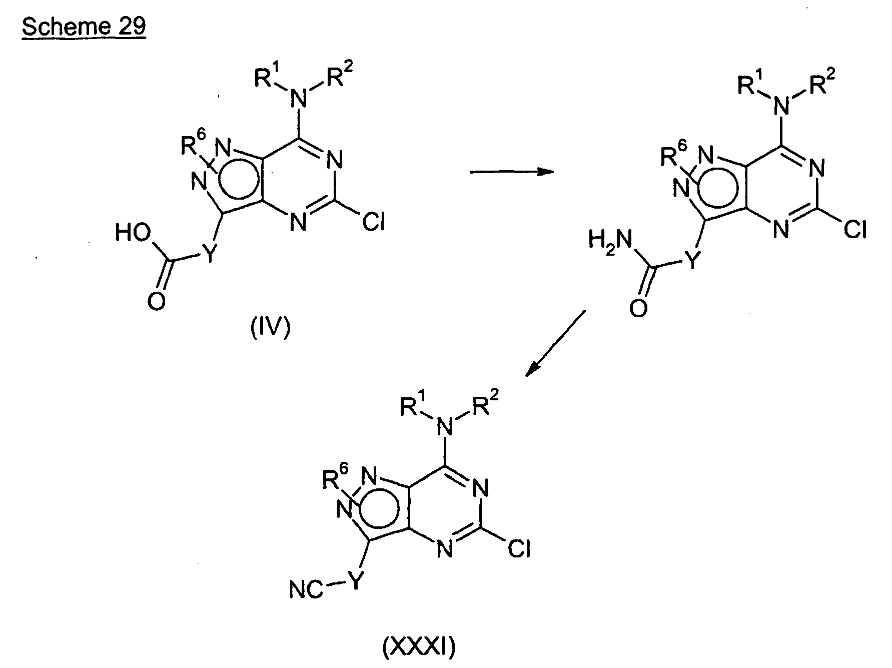

The acid of formula (IV) is converted into the corresponding primary amide following the method described in part i) above. The amide is then dehydrated using trifluoroacetic anhydride.
bb) Compounds of formula (III) or (XXXI) wherein Y is -CH₂-O-CH₂- may be prepared from the alcohols of formula (XIX) by alkylation with an alkyl α-haloacetate or an α-haloacetonitrile derivative, as illustrated in scheme 29.

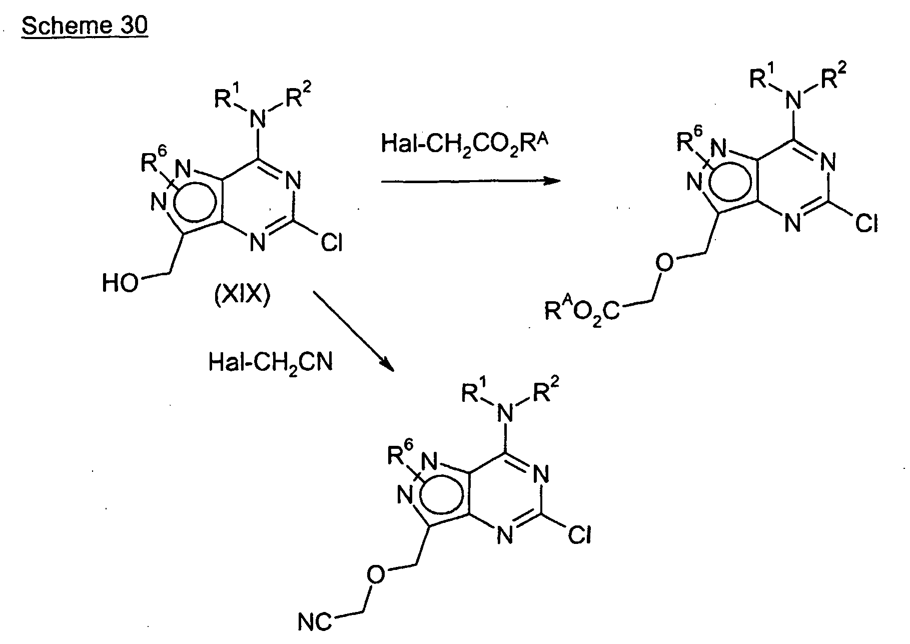

Hal is chlorine, bromine or iodine, preferably chlorine or bromine. The alcohol (XIX) and alkylating agent are combined in a suitable solvent in the presence of a base such as potassium carbonate or sodium hydride. Suitable solvents include tetrahydrofuran and dimethylformamide.

It will be appreciated by those skilled in the art that certain compounds of formula (I) may undergo standard chemical transformations to provide alternative compounds of formula (I), for example the preparation of example 184, by dealkylation of an alkyl ether.

For some of the steps of the here above described process of preparation of the compounds of formula (I), it may be necessary to protect potential reactive functions that are not wished to react, and to cleave said protecting groups in consequence. In such a case, any compatible protecting radical can be used. In particular methods of protection and deprotection such as those described by T.W. GREENE (Protective Groups in Organic Synthesis, A. Wiley-Interscience Publication, 1981) or by P. J. Kocienski (Protecting groups, Georg Thieme Verlag, 1994), can be used.

The following compounds form further aspects of the present invention:

A compound of formula (III) 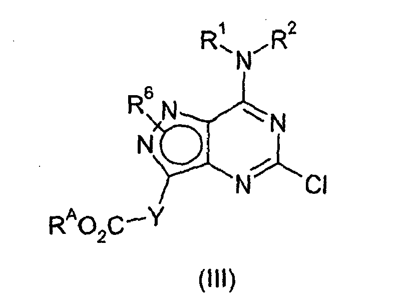 wherein R¹, R², R⁶ , R^{A} and Y are as defined above, with the proviso as defined in claim 71.

Preferred is a compound of formula (III^{D}) 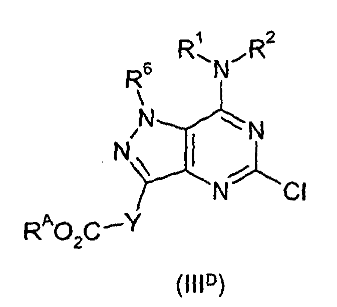 wherein R¹, R², R⁶ R^{A} and Y are as defined above.

A compound of formula (V) 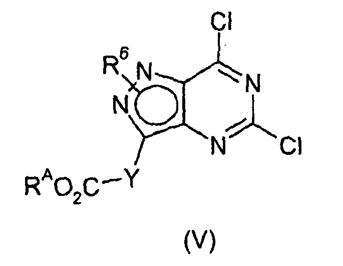 wherein R⁶, R^{A} and Y are as defined above, with the proviso as defined in claim 73.

Preferred is a compound of formula (V^{B}) 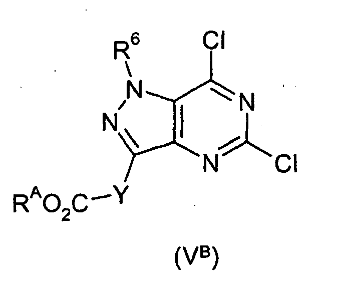 wherein R⁶, R^{A} and Y are as defined above.

The invention is further illustrated by the following, non-limiting examples.

Melting points were determined on a Gallenkamp melting point apparatus using glass capillary tubes and are uncorrected. Unless otherwise indicated all reactions were carried out under a nitrogen atmosphere, using commercially available anhydrous solvents. '0.88 Ammonia' refers to commercially-available aqueous ammonia solution of about 0.88 specific gravity. Thin-layer chromatography was performed on glass-backed pre-coated Merck silica gel (60 F254) plates, and silica gel column chromatography was carried out using 40-63µm silica gel (Merck silica gel 60). Ion exchange chromatography was performed using with the specified ion exchange resin which had been pre-washed with deionised water. Proton NMR spectra were measured on a Varian Inova 300, Varian Inova 400, or Varian Mercury 400 spectrometer in the solvents specified. In the NMR spectra, only non-exchangeable protons which appeared distinct from the solvent peaks are reported. Low resolution mass spectra were recorded on either a Fisons Trio 1000, using thermospray positive ionisation, or a Finnigan Navigator, using electrospray positive or negative ionisation. High resolution mass spectra were recorded on a Bruker Apex II FT-MS using electrospray positive ionisation. Combustion analyses were conducted by Exeter Analytical UK. Ltd., Uxbridge, Middlesex. Optical rotations were determined at 25°C using a Perkin Elmer 341 polarimeter using the solvents and concentrations specified. Example compounds designated as (+) or (-) optical isomers are assigned based on the sign of optical rotation when determined in a suitable solvent.

### Abbreviations, Definitions and Glossary

- AcOH: acetic acid
- Amberlyst^{®} 15: Ion exchange resin, available from Aldrich Chemical Company
- APCI: Atmospheric Pressure Chemical Ionisation
- Arbocel™: Filtration agent, from J. Rettenmaier & Sohne, Germany
- atm: Pressure in atmospheres (1 atm = 760 Torr = 101.3 kPa)
- Biotage™: Chromatography performed using Flash 75 silica gel cartridge, from Biotage, UK
- BOC: *tert*-butoxycarbonyl
- br: Broad
- *c*: Concentration used for optical rotation measurements in g per 100 ml (1 mg/ml is *c* 0.10)
- cat: Catalytic
- CBz: benzyloxycarbonyl
- CDI: N,N'-carbonyldiimidazole
- *d*: Doublet
- DCC: N,N'-dicyclohexylcarbodiimide
- DCM: dichloromethane
- dd: Doublet of doublets
- DEAD: diethyl azodicarboxylate
- Degussa^{®} 101: 10 wt% palladium on activated carbon, Degussa type E101 available from Aldrich Chemical Company
- Dess-Martin: 1,1,1-triacetoxy-1,1-dihydro-1,2-benziodoxol-3(1H)-one
- periodinane Develosil: Supplied by Phenomenex - manufactured by Nomura Chemical
- Combi-RP C₃₀: Co. Composed of spherical silica particles ( size 3 µm or 5 µm)
- hplc column: which have a chemically bonded surface of C30 chains. These particles are packed into stainless steel columns of dimensions 2 cm internal diameter and 25 cm long.
- DIAD: diisopropyl azodicarboxylate
- DIBAL: diisobutylaluminium hydride
- DMAP: 4-dimethylaminopyridine
- DMF: N,N-dimethylformamide
- DMSO: dimethyl sulphoxide
- Dowex^{®}: Ion exchange resin, from Aldrich Chemical Company
- ee: Enantiomeric excess
- Et₃N: triethylamine
- EtOAc: ethyl acetate
- EtOH: ethanol
- HOAT: 1-hydroxy-7-azabenzotriazole
- HOBT: 1-hydroxybenzotriazole hydrate
- HRMS: High Resolution Mass Spectrocopy (electrospray ionisation positive scan)
- Hünig's base: N-ethyldiisopropylamine
- Hyflo™: Hyflo supercel®, from Aldrich Chemical Company
- KHMDS: potassium bis(trimethylsilyl)amide
- liq: Liquid
- LRMS: Low Resolution Mass Spectroscopy (electrospray or thermospray ionisation positive scan)
- LRMS (ES⁻): Low Resolution Mass Spectroscopy (electrospray ionisation negative scan)
- m: Multiplet
- m/z: Mass spectrum peak
- MCI™ gel: High porous polymer, CHP20P 75-150µm, from Mitsubishi Chemical Corporation
- MeOH: methanol
- Mukaiyama's: 2-chloro-1-methylpyridinium iodide
- reagent NaHMDS: sodium bis(trimethylsilyl)amide
- NMM: N-methylmorpholine
- NMO: 4-methylmorpholine *N*-oxide
- NMP: 1-methyl-2-pyrrolidinone
- Phenomenex: Supplied by Phenomenex. Composed of spherical silica particles
- Luna C18 hplc: (size 5 µm or 10 µm) which have a chemically bonded surface of
- column: C18 chains. These particles are packed into a stainless steel column of dimensions 2.1cm internal diameter and 25 cm long.
- psi: Pounds per square inch (1 psi = 6.9 kPa)
- PyBOP®: Benzotriazol-1-yloxytris(pyrrolidino)phosphonium hexafluorophosphate
- PyBrOP®: bromo-tris-pyrrolidino-phosphonium hexafluorophosphate
- q: Quartet
- R_{f}: Retention factor on TLC
- s: Singlet
- Sep-Pak^{®}: Reverse phase C₁₈ silica gel cartridge, Waters Corporation
- t: Triplet
- TBDMS-CI: *tert*-butyldimethylchlorosilane
- TFA: trifluoroacetic acid
- THF: tetrahydrofuran
- TLC: Thin Layer Chromatography
- TMS-CI: chlorotrimethylsilane
- WSCDI: 1-(3-dimethylaminopropyl)-3-ethylcarbodiimide hydrochloride
- δ: Chemical shift

The following Examples illustrate the preparation of the compounds of the formula (I):-

### Preparation 1

### tert-Butyl (3R)-3-methoxypyrrolidine-1-carboxylate

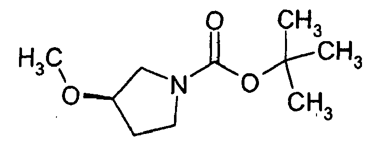

*tert*-Butyl (3*R*)-3-hydroxypyrrolidine-1-carboxylate (12.5g, 66.70mmol) was dissolved in tetrahydrofuran (334mL) and the reaction mixture cooled to 0°C in an ice bath. The reaction mixture was treated with 80% sodium hydride in mineral oil (2.20g, 73.3mmol) and stirred until back at room temperature. The reaction mixture was then treated with methyl iodide (14.5g, 100.0mmol) and stirred at room temperature for 18 hours. The reaction mixture was diluted with water (100mL) and concentrated *in vacuo* until just the aqueous remained. The aqueous solution was treated with ethyl acetate (750mL), the organic layer separated, dried over magnesium sulphate and concentrated *in vacuo* to yield the title product as a brown oil, 12.48g.
¹H NMR (CDCl₃, 400MHz) δ: 1.41 (s, 9H), 1.95 (m, 2H), 3.30 (s, 3H), 3.40 (m, 4H), 3.86 (m, 1H)

### Preparation 2

### tert-Butyl (3S)-3-methoxypyrrolidine-1-carboxylate

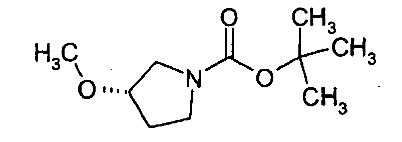

The title product was prepared by a method similar to that described for preparation 1 using *tert*-butyl (3*S*)-3-hydroxypyrrolidine-1-carboxylate.
¹H NMR (CDCl₃, 400MHz) δ: 1.41 (s, 9H), 1.95 (m, 2H), 3.30 (s, 3H), 3.40 (m, 4H), 3.86 (m, 1H)

### Preparation 3

### (3R)-3-Methoxy-pyrrolidine hydrochloride

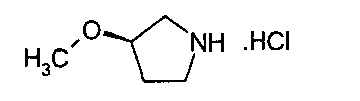

Hydrogen chloride gas was bubbled through an ice-cooled solution of the compound from preparation 1 (6.02g, 30.0mmol) in dichloromethane (30mL), and the reaction then allowed to warm to room temperature and stirred for 48 hours. The solution was concentrated under reduced pressure and the residue triturated with ether. The resulting crystals were filtered off and dried *in vacuo* to afford the title compound.
¹H NMR (CD₃OD, 400MHz) δ: 2.06 (m, 1H), 2.20 (m, 1H), 3.26-3.42 (m, 7H), 4.17 (m, 1H).

### Preparation 4

### (3S)-3-Methoxy-pyrrolidine hydrochloride

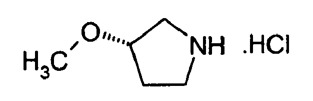

The title compound was obtained from the compound from preparation 2, following a similar method to that described in preparation 3.
¹H NMR (CD₃OD, 400MHz) δ: 2.14 (m, 1H), 2.20 (m, 1H), 3.24-3.44 (m, 7H), 4.18 (m, 1H).

### Preparation 5

### 2-Chloropyrimidin-4-ylamine

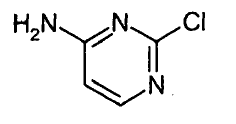

2,4-Dichloropyrimidine (625mg, 4.23mmol) was dissolved in n-butanol (3mL) and the solution treated with ammonia (620µL). The reaction mixture was heated to 100°C for 20 minutes before being allowed to cool to room temperature. Methanol was added to help dissolved the precipitate formed on cooling and the solution was concentrated *in vacuo.* The residue was purified by column chromatography on silica gel eluting with dichloromethane:methanol 100:0 to 96:4.
¹H NMR (CD₃OD, 400MHz) δ: 6.41 (d, 1H), 7.90 (d, 1H)

### Preparation 6

### 2-Methoxypyrimidin-4-ylamine

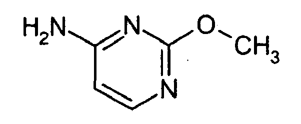

The chloro compound of preparation 5 (1.52g, 11.8mmol) was dissolved in methanol (17mL) and the solution treated with a 4.62M solution of sodium methoxide in methanol (2.8mL, 12.9mmol). The reaction mixture was then refluxed under nitrogen for 6 hours. The reaction mixture was filtered whilst hot and concentrated *in vacuo* to a volume of 2mL and the solid allowed to crystallise out. The crude product was recrystallised from methanol and dried in an oven to yield the title product, 390mg. ¹H NMR (DMSO-D₆, 400MHz) δ: 3.75 (s, 3H), 6.05 (d, 1H), 6.80 (m, 2H), 7.80 (d, 1H)

### Preparation 7

### Dimethyl 4-nitro-1-(2-propoxyethyl)-1H-pyrazole-3,5-dicarboxylate

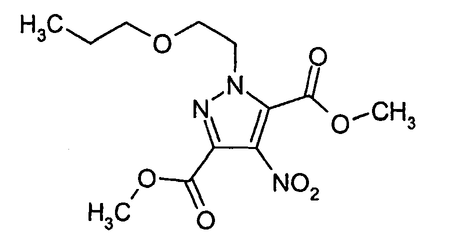

Dimethyl 4-nitro-1*H*-pyrazole-3,5-dicarboxylate (WO00/24745, page 48, preparation 2) (15g, 60mmol), 2-propoxyethanol (8.2mL, 70mmol) and triphenylphosphine (18.9g, 70mmol) were dissolved in tetrahydrofuran (150mL) and the solution cooled to 0°C. The solution was treated with diisopropyl azodicarboxylate (14.2mL, 70mmol) and the reaction mixture stirred at 0°C for 3 hours before being allowed to warm to room temperature. The reaction mixture was concentrated *in vacuo* and the residue purified by column chromatography on silica gel eluting with ethyl acetate:pentane 15:85 and then again eluting with dichloromethane to yield the title product.
¹H NMR (CD₃OD, 400MHz) δ: 0.82 (t, 3H), 1.47 (q, 2H), 3.34 (t, 2H), 3.78 (t, 2H), 3.91 (s, 6H), 4.76 (t, 2H). MS APCI+ m/z 316 [MH]⁺

### Preparation 8

### Dimethyl (2'R)-1-(2'-methoxypropyl)-4-nitro-1H-pyrazole-3,5-dicarboxylate

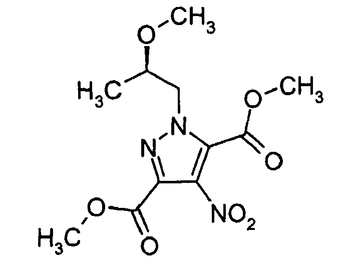

The title compound was prepared by a method similar to that described for preparation 7 using (2*R*)-2-methoxypropanol (Chem. Eur. J., 1997, 3 (12), 2063-2070). The title product was purified by column chromatography on silica gel eluting with pentane:dichloromethane 20:80.
¹H NMR (CDCl₃, 400MHz) δ: 1.18 (d, 3H), 3.20 (s, 3H), 3.70 (m, 1H), 3.92 (s, 3H), 3.94 (s, 3H), 4.42 (m, 1H), 4.74 (m, 1H). MS APCI+ m/z 302 [MH]⁺

### Preparation 9

### Dimethyl 1-(2-isopropoxyethyl)-4-nitro-1H-pyrazole-3,5-dicarboxylate

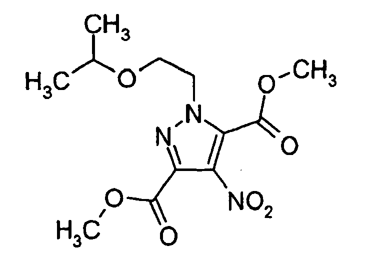

Dimethyl 4-nitro-1*H*-pyrazole-3,5-dicarboxylate (11.4g, 50mmol) was dissolved in tetrahydrofuran (200mL) and the solution treated with triphenylphosphine (14.4g, 55mmol) and 2-isopropoxyethanol (6.36mL, 55mmol). The mixture was cooled on an ice bath to 0°C and diisopropyl azodicarboxylate (10.8mL, 55mmol) added dropwise over 10 minutes, keeping the temperature between 20°C and 30°C. The reaction mixture was then stirred at room temperature for 30 minutes. The reaction mixture was concentrated *in vacuo* and the crude product azeotroped with dichloromethane to yield the title product.
¹H NMR (CDCl₃, 400MHz) δ: 1.02 (d, 6H), 3.45 (m, 1H), 3.72 (t, 2H), 3.90 (s, 3H), 3.94 (s, 3H), 4.74 (t, 2H). MS ES+ m/z 216 [MH]⁺

The following compounds, of the general formula shown below, were prepared by a method similar to that described for preparation 9, using the appropriate R⁶OH alcohol.

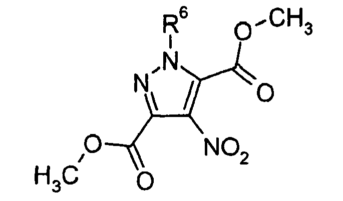

| No | R⁶ | Data |
|---|---|---|
| 10 | -(CH₂)₂CH(CH₃)OCH₃ | ¹H NMR (CDCl₃, 400MHz) δ: 1.10 (d, 3H), 2.00 (m, 2H), 3.20 (s, 3H), 3.30 (m, 1H), 3.86 (m, 6H), 4.62 (m, 2H) |
| 11 | 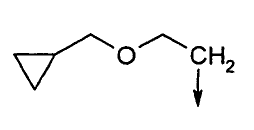 | ¹H NMR (CDCl₃, 400MHz) δ: 0.11 (m, 2H), 0.48 (m, 2H), 0.92 (m, 1H), 3.22 (d, 2H), 3.90 (m, 2H), 3.97 (m, 6H), 4.81 (m, 2H). MS ES+ m/z 350 [MNa]⁺ |
| 12 | 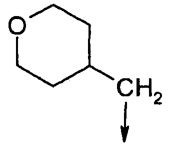 | ¹H NMR (CDCl₃, 400MHz) δ: 1.47 (m, 2H), 1.86 (m, 2H), 2.24 (m, 1H), 3.36 (m, 2H), 3.74 (m, 2H), 3.93 (s, 3H), 3.97 (s, 3H), 4.52 (d, 2H). MS ES+ m/z 328 [MH]⁺ |
| 13 | 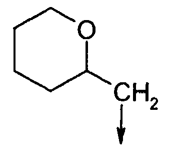 | ¹H NMR (CDCl₃, 400MHz) δ: 1.50 (m, 4H), 1.84 (m, 2H), 3.27 (m, 1H), 3.60-3.90 (m, 2H), 3.92 (s, 3H), 3.94 (s, 3H), 4.45 (m, 1H), 4.73 (m, 1H). MS APCI+ m/z 328 [MH]⁺ |

- Preparation 11 was prepared using 2-(cyclopropylmethoxy)ethanol (FR 2248255, Pg. 2, example 1) as the R⁶OH alcohol.
- Preparation 12 was prepared using tetrahydro-2*H*-pyran-4-methanol (DE 4233431, Pg. 4, example 1) as the R⁶OH alcohol.

### Preparation 14

### Dimethyl 1-(2-ethoxyethyl)-4-nitro-1H-pyrazole-3,5-dicarboxylate

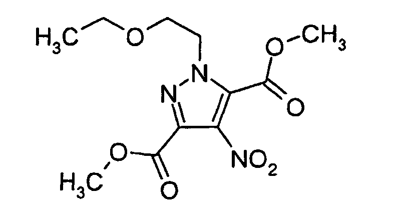

Dimethyl 4-nitro-1*H*-pyrazole-3,5-dicarboxylate (2.0g, 8.83mmol) was added to a solution of 2-ethoxyethyl bromide (1.18mL, 10.45mmol) and potassium carbonate (1.32g, 9.56mmol) in N,N-dimethylformamide (35mL) and the reaction mixture stirred for 48 hours at room temperature. The reaction mixture was concentrated *in vacuo* and the residue partitioned between ethyl acetate (200mL) and water (100mL). The organic layer was separated, dried over magnesium sulphate and concentrated in *vacuo.* The crude product was purified by column chromatography on silica gel eluting with pentane:ethyl acetate 100:0 to 70:30 to yield the title product, 1.63g.
¹H NMR (CDCl₃, 400MHz) δ: 1.07 (s, 3H), 3.41 (q, 2H), 3.73 (t, 2H), 3.89 (s, 3H), 3.94 (s, 3H), 4.76 (t, 2H). MS APCI+ m/z 302, [MH]⁺

### Preparation 15

### Dimethyl 1-[2-(2-methoxyethoxy)ethyl]-4-nitro-1H-pyrazole-3,5-dicarboxylate

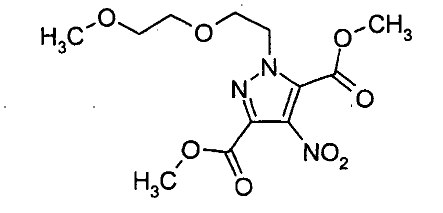

Dimethyl 4-nitro-1*H*-pyrazole-3,5-dicarboxylate (9.53g, 41.6mmol) and potassium carbonate (3.44g, 25mmol) were dissolved in N,N-dimethylformamide (140mL) under nitrogen. The mixture was then treated with a solution of 1-bromo-2-(2-methoxyethoxy)ethane (9.90g, 54mmol) in N,N-dimethylformamide (10mL). The reaction mixture was stirred at 30°C for 18 hours and then allowed to cool to room temperature. Additional 1-bromo-2-(2-methoxyethoxy)ethane (9.90g, 54mmol) and potassium carbonate (3.44g, 25mmol) were added and the reaction mixture allowed to stir at 30°C for 4 hours. The reaction mixture was concentrated *in vacuo* and the residue taken up in ethyl acetate (200mL) and water (200mL). The aqueous was separated and washed with ethyl acetate (200mL), the organics were combined and washed with water. The organic layer was dried over magnesium sulphate and concentrated *in vacuo* to yield the title product.
¹H NMR (CDCl₃, 400MHz) δ: 3.25 (s, 3H), 3.38 (m, 2H), 3.50 (m, 2H), 3.80 (t, 2H), 3.92 (s, 3H), 3.93 (s, 3H), 4.77 (t, 2H). MS APCI+ m/z 333 [MH]⁺

### Preparation 16

### Dimethyl 1-(2-methoxyethyl)-4-nitro-1H-pyrazole-3,5-dicarboxylate

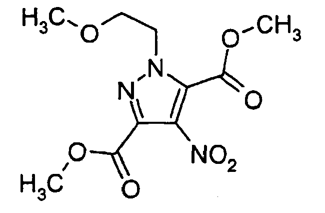

The title compound was prepared by a method similar to that described for preparation 15 using 1-bromo-2-methoxyethane.
¹H NMR (CDCl₃, 400MHz) δ: 3.22 (s, 3H), 3.67 (m, 2H), 3.89 (m, 6H), 4.77 (m, 2H) MS ES+ m/z 288 [MH]⁺

### Preparation 17

### 4-Nitro-1-(2-propoxyethyl)-1H-pyrazole-3,5-dicarboxylic acid 3-methyl ester

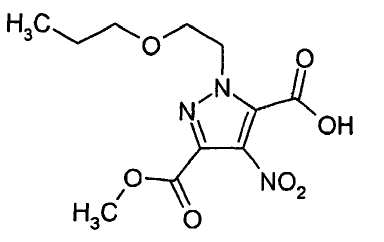

The ester of preparation 7 (150mg, 0.5mmol) and potassium hydroxide (29mg, 0.55mmol) were dissolved in methanol (2mL) and the reaction mixture stirred at room temperature for 48 hours. The reaction mixture was concentrated *in vacuo* and the residue taken up in water. The aqueous was washed with ether (x2) and extracted with dichloromethane. The organic phase was then washed with 2M hydrochloric acid (x2) and water (x2), dried over magnesium sulphate and concentrated *in vacuo* to yield the title product.
¹H NMR (CD₃OD, 400MHz) δ: 0.83 (t, 3H), 1.49 (q, 2H), 3.36 (t, 2H), 3.80 (t, 2H), 3.90 (s, 3H), 4.78 (t, 2H). MS APCI+ m/z 302 [MH]⁺

### Preparation 18

### 4-Nitro-1-(2-ethoxyethyl)-1H-pyrazole-3,5-dicarboxylic acid 3-methyl ester

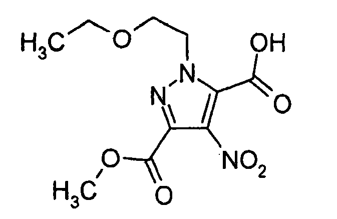

The ester of preparation 14 (1.63g, 5.4mmol) was added to a solution of potassium hydroxide (330mg, 5.9mmol) in methanol (20mL) and the reaction mixture stirred at room temperature for 18 hours. The reaction mixture was concentrated *in vacuo* and the crude product dissolved in water and washed with ether. The aqueous phase was acidified with 2M hydrochloric acid and extracted into dichloromethane (3x100mL). The organics were combined, dried over magnesium sulphate and concentrated *in vacuo* to yield the title product.
¹H NMR (CD₃OD, 400MHz) δ: 1.07 (s, 3H), 3.47 (q, 2H), 3.80 (t, 2H), 3.88 (s, 3H), 4.77 (t, 2H). MS APCI+ m/z 288 [MH]⁺

### Preparation 19

### 1-(2-Isopropoxyethyl)-4-nitro-1H-pyrazole-3,5-dicarboxylic acid 3-methyl ester

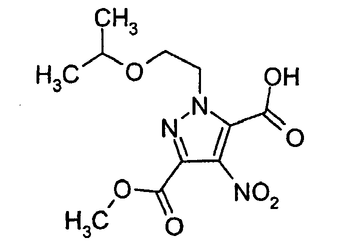

The ester of preparation 9 (15.8g, 50mmol) was dissolved in methanol (200mL) and the solution cooled in an ice bath before being treated with potassium hydroxide (2.8g, 50mmol). The reaction mixture was then stirred at room temperature for 18 hours. The reaction mixture was concentrated *in vacuo* and the residue partitioned between dichloromethane (500mL) and water (250mL). The aqueous phase was separated, acidified with hydrochloric acid and then extracted with dichloromethane (2x500mL). The combined dichloromethane extracts were dried over magnesium sulphate and concentrated *in vacuo* to yield the title product as a white solid, 11.4g. ¹H NMR (DMSO-D₆, 400MHz) δ: 0.92 (d, 6H), 3.45 (m, 1H), 3.67 (t, 2H), 3.82 (s, 3H), 4.66 (t, 2H). MS ES+ m/z 302 [MH]⁺

The following compounds, of the general formula shown below, were prepared by a method similar to that described for preparation 19 using the appropriate ester of preparations 8, 10, 11, 13, 15 and 16

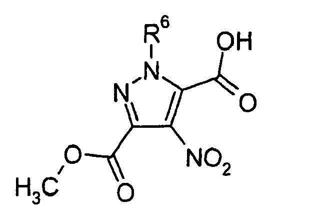

| No | R⁶ | Data |
|---|---|---|
| 20 | -(CH₂)₂CH(CH₃)OCH₃ | ¹H NMR (DMSO-D₆, 400MHz) δ: 1.02 (d, 3H), 1.90 (m, 2H), 3.18 (s, 3H), 3.28 (m, 3H), 3.37 (m, 1H), 4.58 (m, 2H). |
| 21 | -(CH₂)₂O(CH₂)₂OCH₃ | ¹H NMR (CDCl₃, 400MHz) δ: 3.30 (s, 3H), 3.50 (m, 2H), 3.58 (m, 2H), 3.90 (m, 5H), 4.80 (t, 2H). MS APCI+ m/z 318 [MH]⁺ |
| 22 | 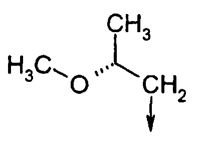 | ¹H NMR (DMSO-D₆, 400MHz) δ: 1.05 (d, 3H), 3.14 (s, 3H), 3.72 (m, 1H), 3.84 (s, 3H), 4.48 (m, 1H), 4.60 (m, 1H). MS APCI+ m/z 288 [MH]⁺ |
| 23 | 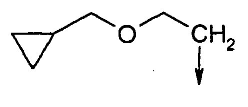 | ¹H NMR (CDCl₃, 400MHz) δ: 0.12 (m, 2H), 0.48 (m, 2H), 0.95 (m, 1H), 3.32 (d, 2H), 3.91 (m, 5H), 4.83 (t, 2H). MS ES- m/z 312 [M-H]⁻ |
| 24 | -(CH₂)₂OCH₃ | ¹H NMR (DMSO-D₆, 400MHz) δ: 3.22 (s, 3H), 3.71 (m, 2H), 3.83 (s, 3H), 4.77 (m, 2H), 9.95 (m, 1H). MS ES+ m/z 274 [MH]⁺ |
| 25 | 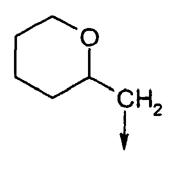 | ¹H NMR (DMSO-D₆, 400MHz) δ: 1.19 (m, 1H), 1.36 (m, 3H), 1.58 (m, 1H), 1.73 (m, 1H), 3.22 (m, 1H), 3.66 (m, 1H), 3.75 (m, 1H), 3.80 (s, 3H), 4.47 (m, 1H), 4.60 (m, 1H). MS APCI+ m/z 314 [MH]⁺ |

### Preparation 26

### 4-Nitro-1-(tetrahydropyran-4-ylmethyl)-1H-pyrazole-3,5-dicarboxylic acid 3-methyl ester

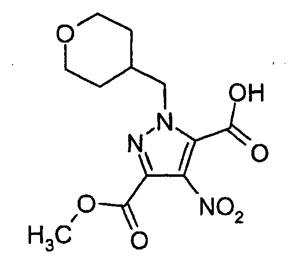

The ester of preparation 12 (13.7g, 42mmol) was added to a solution of potassium hydroxide (2.59g, 46.2mmol) in methanol (200mL) and the reaction mixture stirred at room temperature for 18 hours. The reaction mixture was concentrated *in vacuo* and the residue partitioned between dichloromethane (300mL) and water (200mL). The dichloromethane layer was concentrated *in vacuo* and the residue partitioned between ether (200mL) and water (200mL). The aqueous was added to the first aqueous extract, washed with ether (2x200mL) and acidified with hydrochloric acid. The solution was extracted with dichloromethane (3x400mL), dried over magnesium sulphate and concentrated *in vacuo* to yield the title product.
¹H NMR (DMSO-D₆, 400MHz) δ: 1.24 (m, 2H), 1.36 (m, 2H), 2.10 (m, 1H), 3.20 (m, 2H), 3.78 (m, 2H), 3.84 (s, 3H), 4.43 (d, 2H),
MS APCI+ m/z 314 [MH]⁺

### Preparation 27

### Methyl 5-carbamoyl-4-nitro-1-(2-propoxyethyl)-1H-pyrazole-3-carboxylate

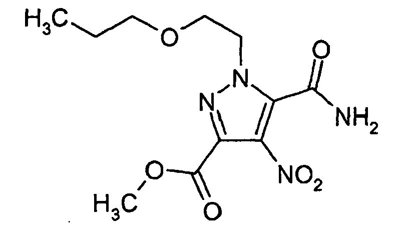

The carboxylic acid of preparation 17 (13.2g, 44mmol) was dissolved in dichloromethane (140mL) and the solution treated with N,N-dimethylformamide (150µL). The mixture was cooled in an ice bath with acetone to -5°C and oxalyl chloride (11.48mL, 132mmol) added dropwise over 30 minutes. The reaction mixture was stirred at -5°C for 1 hour and then allowed to warm to room temperature and stirred for a further 90 minutes. The reaction mixture was concentrated *in vacuo* and the residue azeotroped with dichloromethane (x2). The crude product was dissolved in tetrahydrofuran and cooled in an ice bath. 0.88 Ammonia (60mL) was added to the reaction mixture over 10 minutes, the ice bath removed and the reaction mixture stirred for 1 hour until at room temperature. The reaction mixture was concentrated *in vacuo* and the residue taken up in water. The precipitate formed was filtered off and dried for 18 hours in an oven at 70°C to yield the title product, 10.22g.
¹H NMR (DMSO-D₆, 400MHz) δ: 0.81 (t, 3H), 1.45 (q, 2H), 3.32 (t, 2H), 3.74 (t, 2H), 3.90 (s, 3H), 4.40 (t, 2H), 8.33 (s, 1H), 8.48 (s, 1H)

### Preparation 28

### Methyl 5-carbamoyl-1-(2-methoxyethyl)-4-nitro-1H-pyrazole-3-carboxylate

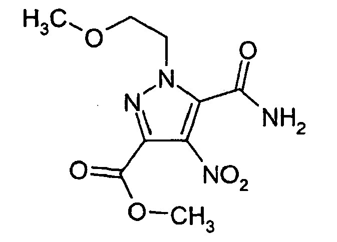

The title compound was prepared by a method similar to that described for preparation 27 using the carboxylic acid of preparation 24.
¹H NMR (DMSO-D₆, 400MHz) δ: 3.18 (s, 3H), 3.65 (m, 2H), 4.82 (s, 3H), 4.38 (m, 2H), 8.33 (m, 1H), 8.47 (m, 1H). MS ES+ m/z 273 [MH]⁺

### Preparation 29

### Methyl 5-carbamoyl-1-(2-ethoxyethyl)-4-nitro-1H-pyrazole-3-carboxylate

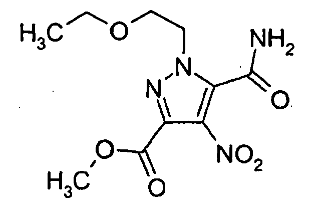

Oxalyl chloride (1.2mL, 13.76mmol) and N,N-dimethylformamide (39µL) were added to a solution of the carboxylic acid of preparation 18 (1.33g, 4.63mmol) in dichloromethane (20mL) and the reaction mixture stirred at room temperature for 2 hours. The reaction mixture was concentrated *in vacuo* and azeotroped from dichloromethane (3x50mL). The product was dissolved in tetrahydrofuran (50mL), cooled in an ice bath, treated with 0.88 ammonia solution (10mL) and stirred for 18 hours at room temperature. The mixture was concentrated *in vacuo* and the residue partitioned between dichloromethane (200mL) and water (50mL). The organics phase was dried over magnesium sulphate and concentrated *in vacuo* to yield the title product.
¹H NMR (DMSO-D₆, 400MHz) δ: 1.06 (t, 3H), 2.48 (m, 2H), 3.77 (m, 2H), 3.84 (s, 3H), 4.38 (m, 2H), 8.35 (m, 1H), 8.46 (m, 1H). MS APCI+ m/z 287 [MH]⁺

### Preparation 30

### Methyl 5-carbamoyl-1-[2-(2-methoxy-ethoxy)-ethyl]-4-nitro-1H-pyrazole-3-carboxylate

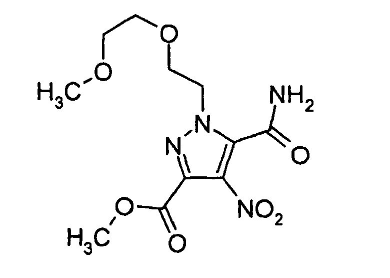

The title product was prepared by a method similar to that described for preparation 29 using the carboxylic acid of preparation 21.
¹H NMR (CDCl₃, 400MHz) δ: 3.30 (s, 3H), 3.50 (m, 2H), 3.58 (m, 2H), 3.90 (m, 2H), 3.99 (s, 3H), 4.50 (t, 2H), 6.25 (m, 1H), 7.80 (m, 1H). MS APCI+ m/z 317 [MH]⁺

### Preparation 31

### Methyl 5-carbamoyl-1-(2-cyclopropylmethoxy-ethyl)-4-nitro-1H-pyrazole-3-carboxylate

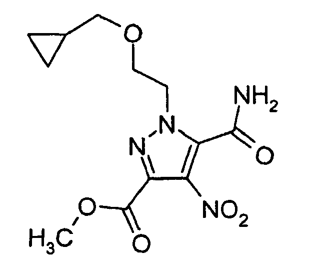

The title compound was prepared by a method similar to that described for preparation 29 using the carboxylic acid of preparation 23.
¹H NMR (CDCl₃, 400MHz) δ: 0.12 (m, 2H), 0.52 (m, 2H), 0.95 (m, 1H), 3.27 (m, 2H), 3.87 (t, 2H), 3.96 (s, 3H), 4.61 (t, 2H), 6.09 (m, 1H), 7.72 (m, 1H)
MS ES+ m/z 335 [MNa]⁺

### Preparation 32

### Methyl 5-carbamoyl-1-(2-isopropoxyethyl)-4-nitro-1H-pyrazole-3-carboxylate

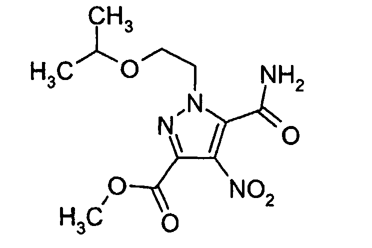

The carboxylic acid of preparation 19 (11.9, 37.8mmol) was dissolved in dichloromethane (140mL) and the solution treated with oxalyl chloride (4.0mL, 45.4mmol) and N,N-dimethylformamide (310µL, 4mmol). The reaction mixture was stirred at room temperature for 18 hours, then concentrated *in vacuo* and the residue azeotroped with dichloromethane (2x100mL). The product was dissolved in tetrahydrofuran (200mL) and the solution cooled in an ice bath and then treated with 0.88 ammonia (50mL). The reaction mixture was stirred for 15 minutes before being concentrated *in vacuo* and partitioned between dichloromethane (1000mL) and water (500mL). The aqueous was separated and extracted with dichloromethane (3x300mL), the organics were combined, dried over magnesium sulphate and concentrated *in vacuo* to yield the title product, 10.4g.
¹H NMR (DMSO-D₆, 400MHz) δ: 0.95 (d, 6H), 3.44 (m, 1H), 3.68 (t, 2H), 3.83 (s, 3H), 4.66 (t, 2H). MS APCI+ m/z 301 [MH]⁺

The following compounds, of the general formula shown below, were prepared by a method similar to that described for preparation 32, using the appropriate carboxylic acid of preparations 20 and 22.

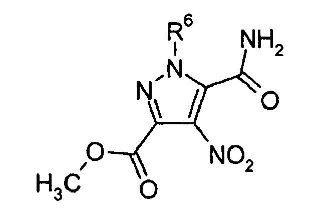

| No | R⁶ | Data |
|---|---|---|
| 33 | -(CH₂)₂CH(CH₃)OCH₃ | ¹H NMR (CDCl₃, 400MHz) δ: 1.16 (d, 3H), 2.08 (m, 2H), 3.25 (s, 3H), 3.38 (m, 1H), 3.97 (s, 3H), 4.59 (t, 2H). MS ES- m/z 299 [M-H]⁻ |
| 34 | 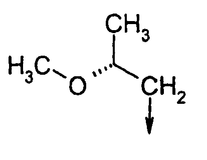 | ¹H NMR (DMSO-D₆, 400MHz) δ: 1.08 (d, 3H), 3.04 (s, 3H), 3.73 (m, 1H), 3.84 (s, 3H), 4.25 (m, 2H), 8.30 (s, 1H), 8.48 (s, 1H). MS ES+ m/z 309 [MNa]⁺ |

### Preparation 35

### Methyl 5-carbamoyl-4-nitro-1-(tetrahydropyran-4-ylmethyl)-1H-pyrazole-3-carboxylate

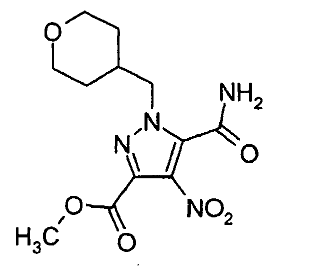

The carboxylic acid of preparation 26 (11.3g, 36mmol) was dissolved in dichloromethane (150mL) and the solution treated with oxalyl chloride (38mL, 43.2mmol) and N,N-dimethylformamide (280µL, 3.6mmol). The reaction mixture was stirred at room temperature for 18 hours and then concentrated *in vacuo.* The residue was azeotroped from dichloromethane (2x200mL) and the resulting solid dissolved in tetrahydrofuran and cooled to -30°C. The solution was treated with 0.88 ammonia (3.85mL, 79.2mmol) and stirred at -30°C for 1 hour. The reaction mixture was concentrated *in vacuo,* diluted with water (100mL) and extracted with ethyl acetate (2x400mL). The combined organics were dried over magnesium sulphate and concentrated *in vacuo.* The residue was triturated with methanol and ether and dried *in vacuo* to yield the title product.
¹H NMR (DMSO-D₆, 400MHz) δ: 1.20 (m, 2H), 1.40 (m, 2H), 2.10 (m, 1H), 3.22 (m, 2H), 3.81 (m, 2H), 3.86 (s, 3H), 4.19 (d, 2H), 8.37 (m, 1H), 8.53 (m, 1H),
MS APCI+ m/z 313 [MH]⁺

### Preparation 36

### Methyl 5-carbamoyl-4-nitro-1-(tetrahydropyran-2-ylmethyl)-1H-pyrazole-3-carboxylate

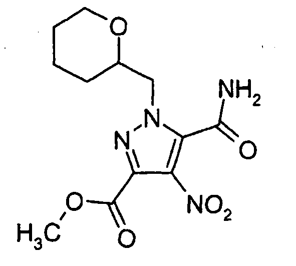

The title compound was prepared by a method similar to that described for preparation 35 using the carboxylic acid of preparation 25.
¹H NMR (DMSO-D₆, 400MHz) δ: 1.18 (m, 1H), 1.40 (m, 3H), 1.58 (m, 1H), 1.78 (m, 1H), 3.22 (m, 1H), 3.65 (m, 1H), 3.78 (m, 1H), 3.85 (s, 3H), 4.22 (m, 2H), 8.27 (m, 1H), 8.46 (m, 1H). MS APCI+ m/z 313 [MH]⁺

### Preparation 37

### Methyl 4-amino-5-carbamoyl-1-(2-propoxyethyl)-1H-pyrazole-3-carboxylate

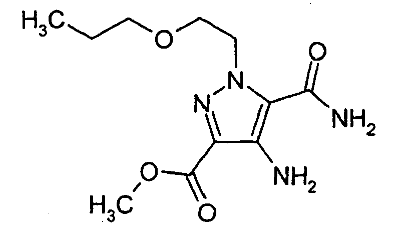

The nitro compound of preparation 27 (10g, 33mmol) was dissolved in ethanol (180mL) and the solution treated with palladium(II) hydroxide (933mg, 6.7mmol) and heated to 75°C. Ammonium formate (21g, 330mmol) was added and the reaction mixture was stirred at 75°C for 3 hours. The reaction mixture was filtered through Arbocel® under nitrogen washing through with ethanol. The filtrate was concentrated *in vacuo* to yield the title product as a pale pink solid, 9.1g.
¹H NMR (CD₃OD, 400MHz) δ: 0.84 (t, 3H), 1.51 (q, 2H), 3.40 (t, 2H), 3.83 (t, 2H), 3.89 (s, 3H), 4.56 (t, 2H). MS APCI+ m/z 271 [MH]⁺

### Preparation 38

### Methyl 4-amino-5-carbamoyl-1-(2-ethoxyethyl)-1H-pyrazole-3-carboxylate

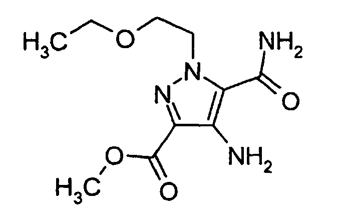

Palladium(II) hydroxide (100mg) was added to a solution of the nitro compound of preparation 29 (970mg, 3.39mmol) in methanol (20mL) and the mixture warmed to reflux. Ammonium formate (1.07g, 16.97mmol) was added and the reaction mixture stirred at reflux for 2 hours. The catalyst was removed by filtration through Arbocel® and the reaction mixture concentrated *in vacuo* to yield the title product.
¹H NMR (DMSO-D₆, 400MHz) δ: 1.02 (t, 3H), 3.33 (m, 2H), 3.66 (m, 2H), 4.80 (s, 3H), 4.57 (m, 2H), 5.11 (m, 2H), 7.49 (m, 2H), MS APCI+ m/z 257 [MH]⁺

The following compounds, of the general formula shown below, were prepared by a method similar to that described for preparation 38 using the appropriate nitro-pyrazoles of preparations 30, 31, 32, 33, 34, 35 and 36.

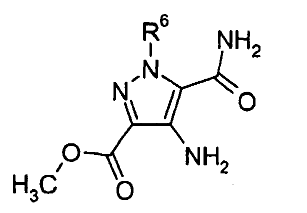

| No | R⁶ | Data |
|---|---|---|
| 39 | -(CH₂)₂OCH(CH₃)₂ | ¹H NMR (DMSO-D₆, 400MHz) δ: 0.98 (d, 6H), 3.48 (m, 1H), 3.64 (m, 2H), 3.76 (s, 3H), 4.45 (t, 2H), 5.14 (m, 2H), 7.50 (m, 2H). MS ES+ m/z 293 [MNa]⁺ |
| 40 | -(CH₂)₂CH(CH₃)OCH₃ | ¹H NMR (CDCl₃, 400MHz) δ: 1.10 (d, 3H), 1.90 (m, 2H), 3.25 (s, 3H), 3.30 (m, 1H), 3.90 (s, 3H), 4.50 (m, 2H), 4.92 (m, 2H), 6.50 (m, 2H). MS APCI+ m/z 271 [MH]⁺ |
| 41 | -(CH₂)₂O(CH₂)₂OCH₃ | ¹H NMR (CDCl₃, 400MHz) δ: 3.30 (s, 3H), 3.50 (m, 2H), 3.58 (m, 2H), 3.90 (s, 3H), 3.99 (t, 2H), 4.50 (t, 2H). MS APCI+ m/z 309 [MNa]⁺ |
| 42 | 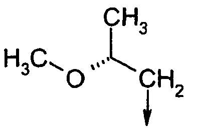 | ¹H NMR (DMSO-D₆, 400MHz) δ: 1.04 (d, 3H), 3.12 (s, 3H), 3.65 (m, 1H), 3.78 (s, 3H), 4.30 (m, 1H), 4.44 (m, 1H), 5.10 (m, 2H), 7.48 (m, 2H). MS APCI+ m/z 257 [MH]⁺ |
| 43 | 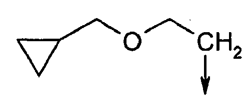 | ¹H NMR (CDCl₃, 400MHz) δ: 0.12 (m, 2H), 0.50 (m, 2H), 0.97 (m, 1H), 3.30 (d, 2H), 3.92 (m, 5H), 4.53 (t, 2H). MS ES+ m/z 305 [MNa]⁺ |
| 44 | 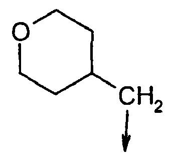 | ¹H NMR (DMSO-D₆, 400MHz) δ: 1.19 (m, 2H), 1.30 (m, 2H), 1.96 (m, 1H), 3.20 (m, 2H), 3.76 (m, 5H), 4.28 (d, 2H), 5.10 (m, 2H), 7.44 (m, 2H). MS APCI+ m/z 283 [MH]⁺ |
| 45 | 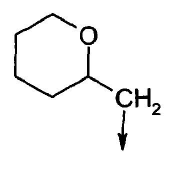 | ¹H NMR (CD₃OD, 400MHz) δ: 1.27 (m, 1H), 1.52 (m, 3H), 1.67 (m, 1H), 1.87 (m, 1H), 3.38 (m, 1H), 3.78 (m, 1H), 3.88 (s, 3H), 3.94 (m, 1H), 4.30 (m, 1H), 4.45 (m, 1H). MS APCI+ m/z 283 [MH]⁺ |

### Preparation 46

### Methyl 4-amino-5-carbamoyl-1-(2-methoxyethyl)-1H-pyrazole-3-carboxylate

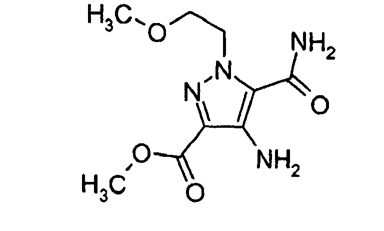

The nitro compound of preparation 28 (1.00g, 3.7mmol) was dissolved in ethyl acetate (15mL) and treated with 10% Pd/C (100mg). The reaction mixture was stirred at room temperature under 15psi of hydrogen for 18 hours. The reaction mixture was filtered through Arbocel®, washing with ethyl acetate and the filtrate concentrated *in vacuo.* The residue was purified by column chromatography on silica gel eluting with pentane:ethyl acetate 50:50 to 34:66 to 0:100 to yield the title product.
¹H NMR (DMSO-D₆, 400MHz) δ: 3.16 (s, 3H), 3.60 (m, 2H), 3.76 (s, 3H), 4.45 (m, 2H), 5.07 (m, 2H), 7.42 (m, 2H). MS ES+ m/z 244 [MH]⁺

### Preparation 47

### Methyl 5,7-dioxo-1-(2-propoxyethyl)-4,5,6,7-tetrahydro-1H-pyrazolo[4,3-d]pyrimidine-3-carboxylate

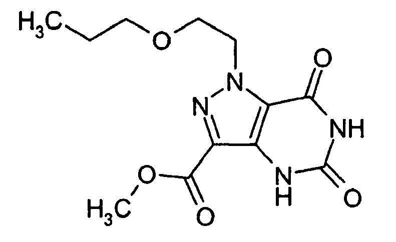

The amide of preparation 37 (9g, 33mmol) and N,N'-carbonyldiimidazole (5.4g, 33mmol) were dissolved in N,N-dimethylformamide (400mL) and the reaction mixture stirred at room temperature for 30 minutes and then at 75°C for 18 hours. Addditional N,N'-carbonyldiimidazole (400mg, 2.69mmol) was added and the reaction mixture stirred for a further 90 minutes. The reaction mixture was concentrated *in vacuo* and the residue taken up in water and stirred for 30 minutes. The precipitate formed was filtered off to yield the title product as a pale pink solid, 6.05g.
¹H NMR (DMSO-D₆, 400MHz) δ: 0.72 (t, 3H), 1.37 (q, 2H), 3.28 (t, 2H), 3.76 (t, 2H), 3.82 (s, 3H), 4.64 (t, 2H), 10.77 (s, 1H), 11.37 (s, 1H). MS APCI- m/z 295, [M-H]⁻

### Preparation 48

### Methyl 1-(2-ethoxyethyl)-5,7-dioxo-4,5,6,7-tetrahydro-1H-pyrazolo[4,3-d]pyrimidine-3-carboxylate

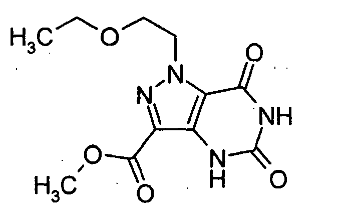

A solution of the amide of preparation 38 (570mg, 3.38mmol) in N,N-dimethylformamide (30mL) was treated with N,N'-carbonyldiimidazole (658mg, 4.06mmol) and the reaction mixture stirred at room temperature for 1 hour and then at 90°C for 18 hours. The reaction mixture was concentrated *in vacuo* and the crude product suspended in acetone and sonicated for 30 minutes. The solid product was filtered off and dried *in vacuo* to yield the title product.
¹H NMR (DMSO-D₆, 400MHz) δ: 1.02 (t, 3H), 3.37 (m, 2H), 3.77 (m, 2H), 4.83 (s, 3H), 4.63 (m, 2H), 10.75 (s, 1H), 11.40 (s, 1H). MS ES- m/z 281 [M-H]⁻

The following compounds, of the general formula shown below, were prepared by a method similar to that described for preparation 48, using the appropriate amide of preparations 39, 40, 41, 42, 43 and 46.

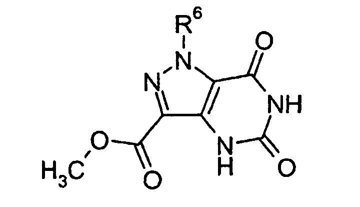

| No. | R⁶ | Data |
|---|---|---|
| 49 | -(CH₂)₂OCH(CH₃)₂ | ¹H NMR (DMSO-D₆, 400MHz) δ: 0.95 (d, 6H), 3.47 (m, 1H), 3.73 (t, 2H), 3.80 (s, 3H), 4.58 (t, 2H), 10.78 (m, 1H), 11.47 (m, 1H). MS ES+ m/z 319 [MNa]⁺ |
| 50 | -(CH₂)₂CH(CH₃)OCH₃ | ¹H NMR (DMSO-D₆, 400MHz) δ: 1.02 (d, 3H), 1.90 (m, 2H), 3.17 (s, 3H), 3.30 (m, 1H), 3.80 (s, 3H), 4.50 (t, 2H), 7.00 (m, 1H), 7.60 (m, 1H). MS APCI- m/z 295 [M-H]⁻ |
| 51 | -(CH₂)O(CH₂)₂OCH₃ | ¹H NMR (DMSO-D₆, 400MHz) δ: 3.15 (s, 3H), 3.30 (t, 2H), 3.45 (t, 2H), 3.80 (t, 5H), 4.60 (t, 2H). MS APCI+ m/z 311 [M-H]⁻ |
| 52 | 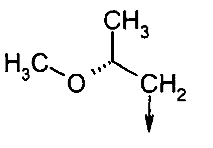 | ¹H NMR (DMSO-D₆, 400MHz) δ: 1.07 (d, 3H), 3.14 (s, 3H), 3.74 (m, 1H), 3.82 (s, 3H), 4.40 (m, 1H), 4.60 (m, 1H), 10.76 (m, 1H), 11.37 (m, 1H). MS APCI+ m/z 283 [MH]⁺ |
| 53 | 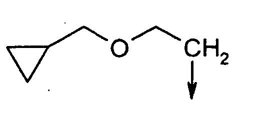 | ¹H NMR (DMSO-D₆, 400MHz) δ: 0.06 (m, 2H), 0.35 (m, 2H), 0.83 (m, 1H), 3.16 (d, 2H), 3.78 (t, 2H), 3.81 (s, 3H), 4.61 (t, 2H), 10.77 (m, 1H), 11.37 (m, 1H). MS ES+ m/z 331 [MNa]⁺ |
| 54 | -(CH₂)₂OCH₃ | ¹H NMR (DMSO-D₆, 400MHz) δ: 3.17 (s, 3H), 3.69 (m, 2H), 3.80 (s, 3H), 4.61 (m, 2H), 10.74 (m, 1H), 11.37 (m, 1H). MS ES+ m/z 269 [MH]⁺ |

### Preparation 55

### Methyl 5,7-dioxo-1-(tetrahydropyran-4-ylmethyl)-4,5,6,7-tetrahydro-1H-pyrazolo[4,3-d]pyrimidine-3-carboxylate

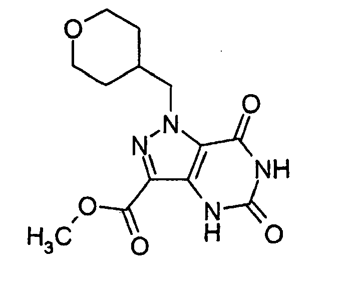

The amide of preparation 44 (9.8g, 34.9mmol) was dissolved in acetonitrile (100mL) and the solution treated with N,N'-carbonyldiimidazole (6.8g, 42mmol). The reaction mixture was heated to reflux for 18 hours before being allowed to return to room temperature. The white precipitate formed was removed by filtration, washed with acetonitrile and dried *in vacuo* to yield the title product.
¹H NMR (DMSO-D₆, 400MHz) δ: 1.24 (m, 2H), 1.36 (m, 2H), 2.08 (m, 1H), 3.21 (m, 2H), 3.80 (m, 2H), 3.83 (s, 3H), 4.40 (d, 2H), 10.78 (m, 1H), 11.37 (m, 1H)
MS APCI- m/z 307 [M-H]⁻

### Preparation 56

### Methyl 5,7-dioxo-1-(tetrahydropyran-2-ylmethyl)-4,5,6,7-tetrahydro-1H-pyrazolo[4,3-d]pyrimidine-3-carboxylate

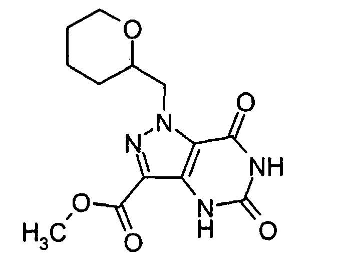

The title compound was prepared by a method similar to that described for preparation 55 using the amide of preparation 45.
¹H NMR (DMSO-D₆, 400MHz) δ: 1.20 (m, 1H), 1.40 (m, 3H), 1.52 (d, 1H), 1.75 (m, 1H), 3.22 (m, 1H), 3.74 (m, 2H), 3.80 (s, 3H), 4.40 (m, 1H), 4.58 (m, 1H), 10.75 (m, 1H), 11.35 (m, 1H). MS APCI+ m/z 309 [MH]⁺

### Preparation 57

### Methyl 5,7-dichloro-1-(2-propoxyethyl)-1H-pryazolo[4,3-d]pyrimidine-3-carboxylate

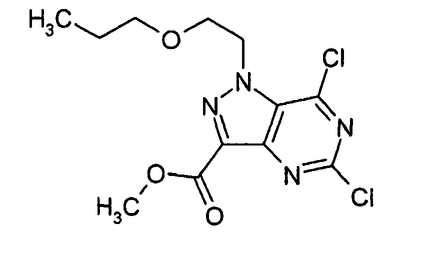

The dione of preparation 47 (3g, 10mmol), phosphorous oxychloride (14.2mL, 152mmol) and tetraethylammonium chloride (3.95g, 30mmol) were dissolved in propionitrile (80mL) and the reaction mixture heated at 115°C for 18 hours. The reaction mixture was concentrated *in vacuo* and the residue dissolved in additional propionitrile (80mL) and treated with additional phosphorous oxychloride (15mL, 145mmol). The reaction mixture was then heated to 115°C for a further 18 hours. The reaction mixture was concentrated *in vacuo* and the residue azeotroped with toluene. The crude product was taken up in ethyl acetate and cautiously treated with water. The two layers were separated and the aqueous layer re-extracted with ethyl acetate (x3). The combined organics were washed with brine, dried over magnesium sulphate and concentrated *in vacuo.* The crude product was purified by column chromatography on silica gel eluting with pentane:ethyl acetate 75:25 to yield the title product, 3.1 g.
¹H NMR (DMSO-D₆, 400MHz) δ: 0.65 (t, 3H), 1.33 (q, 2H), 3.26 (t, 2H), 3.82 (t, 2H), 3.93 (s, 3H), 4.94 (t, 2H). MS APCI+ m/z 333, [MH]⁺

### Preparation 58

### Methyl 5,7-dichloro-1-(2-ethoxyethyl)-1H-pyrazolo[4,3-d]pyrimidine-3-carboxylate

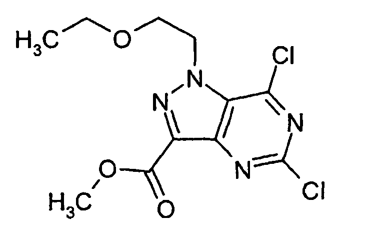

Phosphorous oxychloride (934µL, 10.0mmol) and tetraethylammonium chloride (195mg, 1.50mmol) were added to a solution of the dione of preparation 48 (140mg, 0.50mmol) in propionitrile (5mL) and the reaction mixture refluxed for 18 hours. The reaction mixture was concentrated *in vacuo* and the crude product partitioned between ethyl acetate (50mL) and water (50mL). The organic layer was dried over magnesium sulphate and concentrated *in vacuo*. The crude product was purified by column chromatography on silica gel eluting with pentane:ethyl acetate 100:0 to 75:25 to yield the title product.
¹H NMR (CDCl₃, 400MHz) δ: 1.05 (t, 3H), 3.41 (m, 2H), 3.84 (m, 2H), 4.06 (s, 3H), 5.00 (m, 2H). MS APCI+ m/z 319 [MH]⁺

### Preparation 59

### Methyl 5,7-dichloro-1-(2-isopropoxyethyl)-1H-pyrazolo[4,3-d]pyrimidine-3-carboxylate

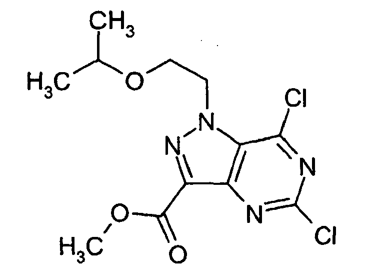

The dione of preparation 49 (2.37g, 8.00mmol) was suspended in acetonitrile (30mL) and the solution treated with phosphorous oxychloride (15mL, 160mmol) and tetraethyl ammonium chloride (3.97g, 24mmol). The reaction mixture was stirred at reflux for 18 hours. The reaction mixture was allowed to cool and then concentrated *in vacuo* before being partitioned between dichloromethane (300mL) and water (200mL). The dichloromethane layer was separated, dried over magnesium sulphate and concentrated *in vacuo.* The crude product was purified by column chromatography on silica gel eluting with pentane:ethyl acetate 100:0 to 75:25 to yield the title product as a white solid, 1.54g.
¹H NMR (CDCl₃, 400MHz) δ 0.96 (d, 6H), 3.43 (m, 1H), 3.86 (t, 2H), 4.08 (s, 3H), 4.96 (t, 2H). MS ES+ m/z 355 [MNa]⁺

The following compounds, of the general formula shown below, were prepared by a method similar to that described for preparation 59 using the appropriate dione of preparations 50, 51, 52, 54, 55 and 56.

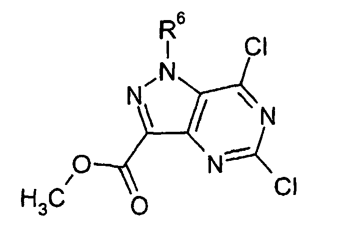

| No. | R⁶ | Data |
|---|---|---|
| 60 | -(CH₂)₂CH(CH₃)OCH₃ | ¹H NMR (CDCl₃, 400MHz) δ: 1.18 (d, 3H), 2.00-2.15 (m, 2H), 3.20 (s, 3H), 3.30 (m, 1H), 4.01 (s, 3H), 4.90 (t, 2H). MS APCI+ m/z 333 [MH]⁺ |
| 61 | -(CH₂)₂O(CH₂)₂OCH₃ | ¹H NMR (CDCl₃, 400MHz) δ: 3.20 (s, 3H), 3.30 (t, 2H), 3.45 (t, 2H), 3.99 (t, 2H), 4.10 (s, 3H), 5.00 (t, 2H) |
| 62 | 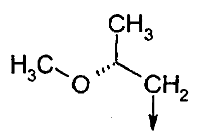 | ¹H NMR (CDCl₃, 400MHz) δ: 1.24 (d, 3H), 3.12 (s, 3H), 3.84 (m, 1H), 4.08 (s, 3H), 4.65 (m, 1H), 4.94 (m, 1H). MS APCI+ m/z 319 [MH]⁺ |
| 63 | -(CH₂)₂OCH₃ | ¹H NMR (CDCl₃, 400MHz) δ: 3.25 (s, 3H), 3.84 (m, 2H), 4.09 (s, 3H), 4.98 (m, 2H). MS APCI+ m/z 305 [MH]⁺ |
| 64 | 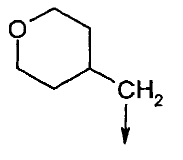 | ¹H NMR (CDCl₃, 400MHz) δ: 1.45 (m, 2H), 1.54 (m, 2H), 2.30 (m, 1H), 3.32 (m, 2H), 3.98 (m, 2H), 4.07 (s, 3H), 4.73 (s, 2H). MS APCI+ m/z 345 [MH]⁺ |
| 65 | 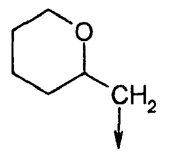 | ¹H NMR (CDCl₃, 400MHz) δ: 1.34-1.60 (m, 4H), 1.66 (m, 1H), 1.89 (m, 1H), 3.23 (m, 1H), 3.81 (m, 2H), 4.07 (s, 3H), 4.67 (m, 1H), 4.96 (m, 1H). MS APCI+ m/z 345 [MH]⁺ |

### Preparation 66

### Methyl 5,7-dichloro-1-(2-(cyclopropylmethoxy)ethyl)-1H-pyrazolo[4,3-d]pyrimidine-3-carboxylate

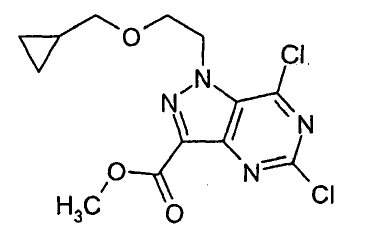

The dione of preparation 53 (2.52g, 8.17mmol) was suspended in acetonitrile (40mL) and the suspension treated with phosphorous oxychloride (15mL, 163.4mmol) and tetraethylammonium chloride (4.08g, 24.51 mmol). The reaction mixture was heated at reflux for 24 hours. The reaction mixture was concentrated *in vacuo* and the residue triturated with ether. The filtrate was concentrated *in vacuo* and purified by column chromatography on silica gel eluting with dichloromethane:ethyl acetate 50:50 to yield the title product as a colourless oil, 907mg.
¹H NMR (CDCl₃, 400MHz) δ: 0.03 (m, 2H), 0.40 (m, 2H), 0.82 (m, 1H), 3.18 (d, 2H), 3.92 (t, 2H), 4.07 (s, 3H), 4.99 (t, 2H). MS ES+ m/z 345 [MH]⁺

### Preparation 67

### Methyl 5-chloro-7-(4-methylpyridin-2-ylamino)-1-(2-propoxyethyl)-1H-pyrazolo[4,3-d]pyrimidine-3-carboxylate

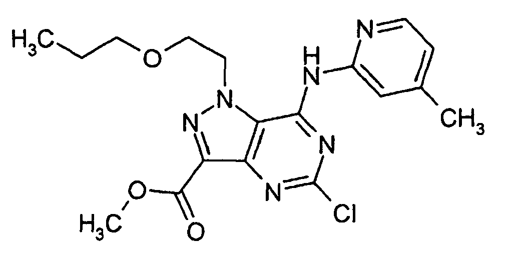

The dichloro compound of preparation 57 (400mg, 1.2mmol) and 2-amino-4-methylpyridine (649mg, 6.0mmol) were dissolved in dimethyl sulphoxide (5mL) and the reaction mixture stirred at 30°C for 1 hour. The reaction mixture was partitioned between dichloromethane and water and the aqueous layer extracted with dichloromethane (x2). The combined organics were washed with water (x2), aqueous citric acid and brine before being dried over magnesium sulphate and concentrated *in vacuo* to yield the title product as a yellow solid, 800mg.
MS APCI+ m/z 405 [MH]⁺

### Preparations 68 to 71

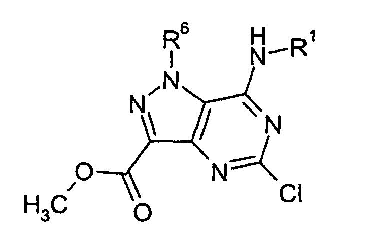

The following compounds of the general formula above were prepared by a method similar to that described for preparation 67 using the appropriate dichloro starting material of preparations 58 and 63, and the appropriate HNR¹R² amine.

| No. | | | |
|---|---|---|---|
| 68 | 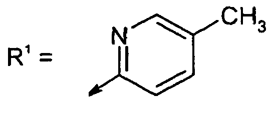 | ; R⁶ = -(CH₂)₂OCH₂CH₃ | |
| | ¹H NMR (DMSO-D₆, 400MHz) δ: 1.01 (t, 3H), 2.26 (s, 3H), 3.52 (m, 2H), 3.88 (m, 5H), 4.96 (m, 2H), 7.76 (m, 1H), 8.03 (m, 1H), 8.20 (m, 1H). MS APCI+ m/z 391 [MH]⁺ | | |
| 69 | 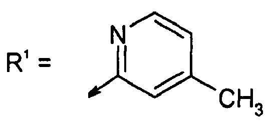 | ; R⁶ = -(CH₂)₂OCH₃ | |
| | ¹H NMR (CDCl₃, 400MHz) δ: 2.46 (s, 3H), 3.47 (m, 3H), 3.95 (m, 2H), 4.04 (s, 3H), 5.01 (m, 2H), 6.92 (m, 2H), 8.16 (m, 1H). MS APCI+ m/z 377 [MH]⁺ | | |
| 70 | 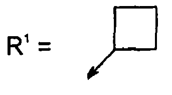 | ; R⁶ = -(CH₂)₂OCH₂CH₃ | |
| | ¹H NMR (CD₃OD, 400MHz) δ: 1.16 (t, 3H), 1.85 (m, 2H), 2.16 (m, 2H), 2.48 (m, 2H), 3.58 (q, 2H), 3.90 (t, 2H), 3.98 (s, 3H), 4.64 (m, 1H), 4.79 (t, 2H). MS APCI+ m/z 354 [MH]⁺ | | |
| 71 | 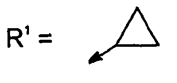 | ; R⁶ = -(CH₂)₂OCH₂CH₃ | |
| | ¹H NMR (CD₃OD, 400MHz) δ: 0.73 (m, 2H), 0.92 (m, 2H), 1.19 (t, 3H), 2.99 (m, 1H), 3.53 (q, 2H), 3.88 (t, 2H), 4.00 (s, 3H), 4.74 (t, 2H). MS APCI+ m/z 340 [MH]⁺ | | |

### Preparation 72

### Methyl 5-chloro-1-(2-ethoxyethyl)-7-(4-methylpyridin-2-ylamino)-1H-pyrazolo[4,3-d]pyrimidine-3-carboxylate

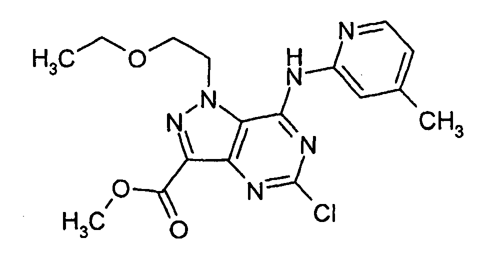

The dichloro compound of preparation 58 (1.98g, 6.20mmol) was dissolved in dimethyl sulphoxide (10mL) and the solution treated with 2-amino-4-methylpyridine (1.34g, 12.4mmol). The reaction mixture was stirred at room temperature for 18 hours. The reaction mixture was partitioned between dichloromethane (300mL) and water (500mL) and the dichloromethane layer separated. The organic phase was washed with water (3x100mL), dried over magnesium sulphate and concentrated *in vacuo.* The residue was purified by column chromatography on silica gel eluting with dichloromethane:methanol 100:0 to 98:2. The crude product was triturated with ether (50mL), filtered and concentrated *in vacuo* to yield the title product, 1.2g.
¹H-NMR (CDCl₃, 400MHz) δ: 1.06 (t, 3H), 2.49 (s, 3H), 3.62 (m, 2H), 4.00 (t, 2H), 4.06 (s, 3H), 5.05 (m, 2H), 6.98 (m, 1H), 8.16 (m, 1H), 8.50 (m, 1H). MS APCI+ m/z 391 [MH]⁺

### Preparations 73 to 85

The following compounds, of the general formula shown below, were prepared by a method similar to that described for preparation 72 using the appropriate HNR1R2 amine and the appropriate dichloro compound of preparations 58 and 61.

| | | |
|---|---|---|
| 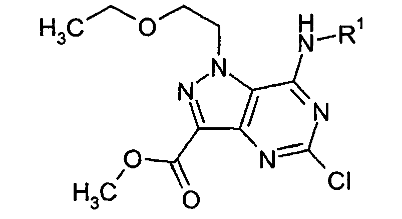 | | |

| No. | R¹ | Data |
|---|---|---|
| 73 | 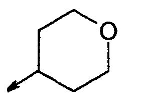 | ¹H NMR (CD₃OD, 400MHz) δ: 1.14 (t, 3H), 1.72 (m, 2H), 2.08 (m, 2H), 3.57 (m, 4H), 3.91 (t, 2H), 3.97 (t, 2H), 4.02 (m, 2H), 4.40 (s, 3H), 4.79 (t, 2H) |
| 74 | 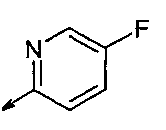 | ¹H NMR (CD₃OD, 400MHz) δ: 1.21 (t, 3H), 3.68 (q, 2H), 4.03 (t, 2H), 4.08 (s, 3H), 4.89 (t, 2H), 7.55 (m, 1H), 8.20 (d, 1H), 8.50 (m, 1H). MS APCI+ m/z 395 [MH]⁺ |
| 75 | 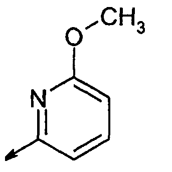 | ¹H NMR (CDCl₃, 400MHz) δ: 1.24 (t, 3H), 3.71 (q, 2H), 3.92 (s, 3H), 4.02 (t, 2H), 4.07 (s, 3H), 4.90 (t, 2H), 6.54 (d, 1H), 7.67 (t, 1H), 7.95 (d, 1H). MS APCI+ m/z 407 [MH]⁺ |
| 76 | 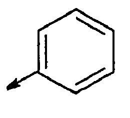 | ¹H NMR (CDCl₃, 400MHz) δ: 1.21 (t, 3H), 3.67 (q, 2H), 4.06 (t, 2H), 4.07 (s, 3H), 4.86 (t, 2H), 7.17 (t, 1H), 7.41 (m, 2H), 7.71 (d, 2H). MS APCI+ m/z 376 [MH]⁺ |
| 77 | 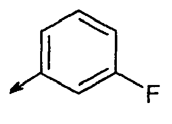 | ¹H NMR (CDCl₃, 400MHz) δ: 1.21 (t, 3H), 3.68 (q, 2H), 4.06 (t, 2H), 4.08 (s, 3H), 4.87 (t, 2H), 6.86 (m, 1H), 7.08 (m, 1H), 7.38 (m, 1H), 7.66 (m, 1H). MS APCI+ m/z 394 [MH]⁺ |
| 78 | 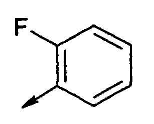 | ¹H NMR (CDCl₃, 400MHz) δ: 1.11 (t, 3H), 3.62 (q, 2H), 4.00 (t, 2H), 4.08 (s, 3H), 4.88 (t, 2H), 7.14 (m, 2H), 7.23 (m, 1H), 8.42 (t, 1H), 9.49 (m, 1H). MS APCI+ m/z 394 [MH]⁺ |
| 79 | 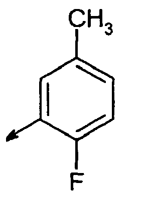 | ¹H NMR (CDCl₃, 400MHz) δ: 1.09 (t, 3H), 2.39 (s, 3H), 3.60 (q, 2H), 3.98 (t, 2H), 4.07 (s, 3H), 4.86 (t, 2H), 6.94 (m, 1H), 7.04 (t, 1H), 8.21 (d, 1H), 9.42 (m, 1H). MS APCI+ m/z 408 [MH]⁺ |
| 80 | 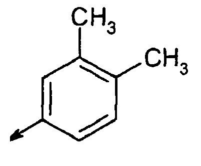 | ¹H NMR (CDCl₃, 400MHz) δ: 1.20 (t, 3H), 2.27 (s, 3H), 2.31 (s, 3H), 3.66 (q, 2H), 4.04 (t, 2H), 4.07 (s, 3H), 4.84 (t, 2H), 7.16 (d, 1H), 7.41 (s, 1H), 7.47 (d, 1H), 9.31 (s, 1H). MS APCI+ m/z 404 [MH]⁺ |
| 81 | 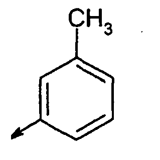 | ¹H NMR (CDCl₃, 400MHz) δ: 1.13 (t, 3H), 2.39 (s, 3H), 3.62 (q, 2H), 4.00 (s, 3H), 4.02 (t, 2H), 4.93 (t, 2H), 7.02 (d, 1H), 7.28 (t, 1H), 7.54 (s, 1H), 7.61 (d, 1H). MS APCI+ m/z 390 [MH]⁺ |
| 82 | 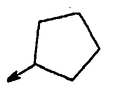 | ¹H NMR (CDCl₃, 400MHz) δ: 1.20 (t, 3H), 1.50 (m, 2H), 1.71 (m, 4H), 2.21 (m, 2H), 3.56 (q, 2H), 3.93 (m, 2H), 4.02 (s, 3H), 4.47 (m, 1H), 4.67 (t, 2H), 7.35 (d, 1H). MS ES+ m/z 368 [MH]⁺ |
| 83 | 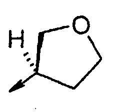 | ¹H NMR (CDCl₃, 400MHz) δ: 1.13 (t, 3H), 2.04 (m, 1H), 2.45 (m, 1H), 3.56 (q, 2H), 3.83 (m, 2H), 3.91 (t, 2H), 3.97 (s, 3H), 4.02 (m, 2H), 4.76 (m, 1H), 4.79 (m, 2H). MS ES+ m/z 356 [MH]⁺ |
| 84 | 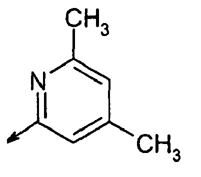 | ¹H NMR (DMSO-D₆, 400MHz) δ: 1.03 (m, 3H), 2.35 (s, 3H), 2.43 (m, 3H), 3.54 (m, 2H), 3.87 (m, 5H), 4.96 (m, 2H), 6.92 (m, 1H), 7.65 (m, 1H). MS APCI+ m/z 405 [MH]⁺ |
| 85 | 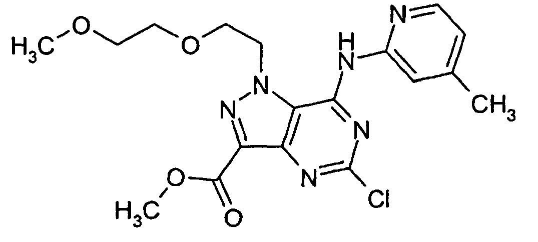 | |
| | ¹H NMR (CDCl₃, 400MHz) δ: 2.50 (m, 3H), 3.40 (m, 3H), 3.70 (m, 2H), 4.10 (m, 7H), 5.10 (m, 2H), 7.02 (m, 2H), 8.18 (m, 1H). MS APCI- m/z 419 [M-H]⁻ | |

- Preparation 73 used tetrahydropyran-4-ylamine (WO 98/08855, Pg. 17, e.g. 3) as the HNR¹R² amine
- Preparation 75 used 6-methoxy-pyridin-2-ylamine (US 01/0047013, pg. 3, example 2) as the HNR¹R² amine
- Preparation 83 used (3*R*)-tetrahydrofuran-3-ylamine tosylate as the HNR¹R² amine with 1 eq of N-ethyldiisopropylamine.

### Preparation 86

### Methyl 5-chloro-1-(3-methoxybutyl)-7-(4-methylpyridin-2-ylamino)-1H-pyrazolo[4,3-d]pyrimidine-3-carboxylate

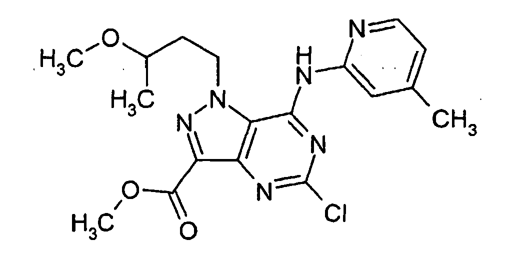

The dichloro compound of preparation 60 (700mg, 2.11mmol) and 4-methylpyridin-2-ylamine (1.14g, 10.54mmol) were dissolved in dimethyl sulphoxide (10mL) and the reaction mixture heated to 30°C under nitrogen for 3 hours. The reaction mixture was concentrated *in vacuo* and the residue taken up in dichloromethane (100mL) and water (150mL). The layers were separated and the aqueous layer washed with dichloromethane (50mL). The organics were combined, washed with water (100mL) and citric acid (50mL) solution, dried over magnesium sulphate and concentrated in *vacuo.* The crude product was purified by column chromatography on silica gel eluting with dichloromethane:methanol 100:0 to 99:1 to yield the title product as a yellow solid, 330mg.
¹H NMR (CD₃OD, 400MHz) δ: 1.20 (m, 3H), 2.10 (m, 2H), 2.45 (s, 3H), 3.30 (s, 3H), 3.40 (m, 1H), 3.98 (s, 3H), 5.00 (m, 2H), 6.90 (m,1 H), 7.30 (m, 1H), 8.00 (m, 1H) MS ES+ m/z 405 [MH]⁺

The following compounds, of the general formula shown below, were prepared by a method similar to that described for preparation 86 using the appropriate HNR¹R² amine and dichloro compound of preparations 58, 59, 62, 64 and 65.

| | | |
|---|---|---|
| | 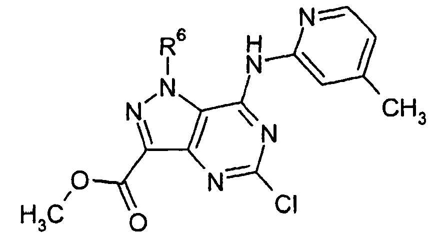 | |

| No. | R⁶ | Data |
|---|---|---|
| 87 | -(CH₂)₂OCH(CH₃)₂ | ¹H NMR (CDCl₃, 400MHz) δ: 0.94 (d, 6H), 2.62 (s, 3H), 3.70 (m, 1H), 3.95 (t, 2H), 4.07 (s, 3H), 5.24 (m, 2H), 7.16 (d, 1H), 8.17 (d, 1H), 8.84 (m, 1H). MS ES+ m/z 427 [MNa]⁺ |
| 88 | 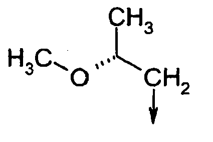 | ¹H NMR (DMSO-D₆, 400MHz) δ: 1.12 (d, 3H), 2.39 (s, 3H), 3.20 (s, 3H), 3.85 (s, 3H), 3.85 (m, 1H), 4.82 (d, 2H), 7.05 (d, 1H), 7.78 (s, 1H), 8.25 (d, 1H). MS ES+ m/z 391 [MH]⁺ |
| 89 | 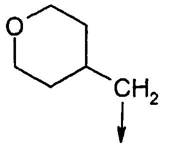 | ¹H NMR (DMSO-D₆, 400MHz) δ: 1.36 (m, 4H), 2.14 (m, 1H), 2.42 (s, 3H), 3.18 (m, 2H), 3.77 (m, 2H), 3.84 (s, 3H), 4.79 (d, 2H), 7.03 (d, 1H), 7.67 (s, 1H), 8.20 (d, 1H). MS APCI+ m/z 417 [MH]⁺ |
| 90 | 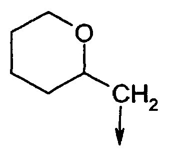 | ¹H NMR (DMSO-D₆, 400MHz) δ: 1.24 (m, 1H), 1.45 (m, 3H), 1.72 (m, 1H), 1.79 (m, 1H), 2.40 (s, 3H), 3.39 (m, 1H), 3.85 (m, 1H), 3.90 (s, 3H), 3.96 (m, 1H), 4.83 (m, 2H), 7.08 (m, 1H), 7.82 (s, 1H), 8.25 (m, 1H). MS APCI+ m/z 417 [MH]⁺ |
| | 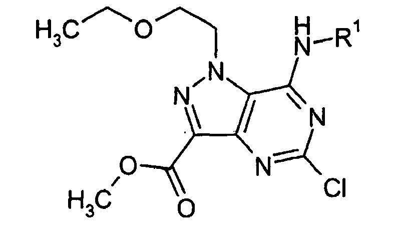 | |

| No. | R¹ | Data |
|---|---|---|
| 91 | 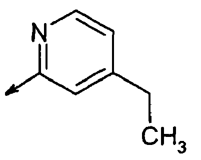 | ¹H NMR (DMSO-D₆, 400MHz) δ: 1.10 (t, 3H), 1.30 (t, 3H), 2.81 (q, 2H), 3.50 (q, 2H), 3.90 (m, 5H), 4.98 (t, 2H), 7.05 (d, 1H), 7.90 (m, 2H), 13.50 (m, 1H). MS APCI- m/z 403 [M-H]⁻ |
| 92 | 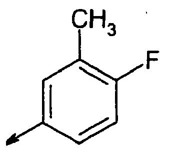 | ¹H NMR (DMSO-D₆, 400MHz) δ: 0.94 (t, 3H), 2.06 (s, 3H), 3.42 (q, 2H), 3.80 (t, 2H), 3.88 (s, 3H), 4.97 (t, 2H), 6.73 (t, 1H), 7.20 (t, 1H), 7.46 (m, 1H). MS APCI- m/z 408 [M-H]⁻ |
| 93 | 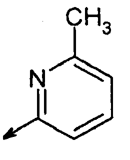 | ¹H NMR (CDCl₃, 400MHz) δ: 1.26 (t, 3H), 2.48 (s, 3H), 3.67 (q, 2H), 4.05 (t, 2H), 4.07 (s, 3H), 4.89 (t, 2H), 6.93 (d, 1H), 7.67 (t, 1H), 8.20 (d, 1H), 10.19 (s, 1H). MS APCI+ m/z 391 [MH]⁺ |
| 94 | 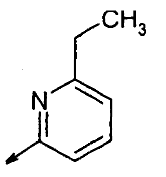 | ¹H NMR (DMSO-D₆, 400MHz) δ: 1.10 (t, 3H), 1.30 (t, 3H), 2.81 (q, 2H), 3.50 (q, 2H), 3.90 (m, 5H), 4.98 (t, 2H), 7.05 (d, 1H), 7.90 (m, 2H), 13.50 (m, 1H). MS APCI1 m/z 403 [M-H]⁻ |
| 95 | 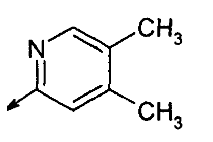 | ¹H NMR (CDCl₃, 400MHz) δ: 1.16 (t, 3H), 2.24 (s, 3H), 2.36 (s, 3H), 3.62 (q, 2H), 4.00 (t, 2H), 4.06 (s, 3H), 4.91 (t, 2H), 8.04 (m, 1H), 8.27 (m, 1H), 10.05 (m, 1H). MS APCI+ m/z 405 [MH]⁺ |
| 96 | 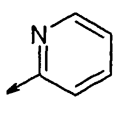 | ¹H NMR (CDCl₃, 400MHz) δ: 1.22 (t, 3H), 3.70 (q, 2H), 4.03 (t, 2H), 4.08 (s, 3H), 4.90 (t, 2H), 7.08 (t, 1H), 7.79 (t, 1H), 8.35 (d, 1H), 8.48 (d, 1H), 10.22 (m, 1H). MS APCI+ m/z 377 [MH]⁺ |
| 97 | 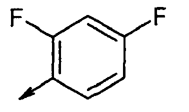 | ¹H NMR (CDCl₃, 400MHz) δ: 1.10 (t, 3H), 3.61 (q, 2H), 4.00 (t, 2H), 4.05 (s, 3H), 4.89 (m, 2H), 6.98 (m, 2H), 8.38 (s, 1H), 9.40 (m, 1H) |
| 98 | 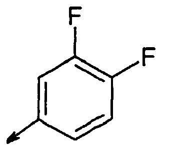 | ¹H NMR (CDCl₃, 400MHz) δ: 1.20 (t, 3H), 3.67 (q, 2H), 4.05 (m, 5H), 4.83 (m, 2H), 7.30 (m, 2H), 7.80 (m, 1H), 9.50 (s, 1H) |
| 99 | 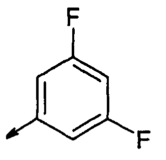 | ¹H NMR (CDCl₃, 400MHz) δ: 1.11 (t, 3H), 3.70 (q, 2H), 4.10 (m, 5H), 4.85 (m, 2H), 6.61 (m, 1H), 7.37 (m, 2H), 9.65 (s, 1H) |
| 100 | 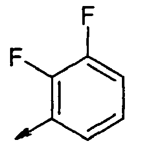 | ¹H NMR (CDCl₃, 400MHz) δ: 1.10 (t, 3H), 3.62 (q, 2H), 4.01 (m, 2H), 4.10 (s, 3H), 4.90 (m, 2H), 6.99 (m, 1H), 7.18 (m, 1H), 8.18 (m, 1H), 9.58 (s, 1H). MS APCI+ m/z 412 [MH]⁺ |
| 101 | 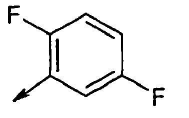 | ¹H NMR (CDCl₃, 400MHz) δ: 1.10 (t, 3H), 3.61 (q, 2H), 4.00 (t, 2H), 4.10 (s, 3H), 4.88 (m, 2H), 6.80 (m, 1H), 7.12 (m, 1H), 8.38 (m, 1H), 9.60 (s, 1H) |

- Preparation 95 was prepared using 2-amino-4,5-dimethylpyridine (J. Het. Chem., 1981, 18 (8), 1613-1618, page 1616 as the HNR¹R² amine.

### Preparation 102

### Methyl 5-chloro-1-(2-(cyclopropylmethoxy)ethyl)-7-(4-methylpyridin-2-ylamino)-1H-pyrazolo[4,3-d]pyrimidine-3-carboxylate

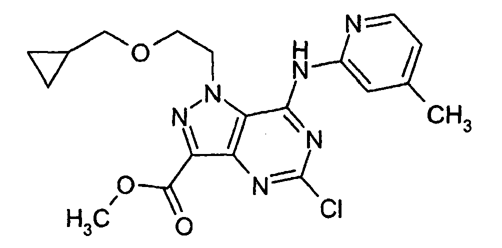

The dichloro compound of preparation 66 (900mg, 2.61 mmol) was dissolved in dimethyl sulphoxide (10mL) and the solution treated with 4-methylpyridin-2-ylamine (1.13g, 10.46mmol). The reaction mixture was then stirred at 35°C in an oil bath for 1 hour. The reaction mixture was allowed to cool and treated with water to induce precipitation of a solid. The crude product was filtered off and dried *in vacuo* at 50°C for 18 hours. The mother liquors were extracted with dichloromethane (2x50mL) and then concentrated *in vacuo.* The combined solids were purified by column chromatography on silica gel eluting with dichloromethane:methanol 100:0 to 98:2. The crude product was then re-purified by column chromatography on silica gel eluting with dichloromethane:ethyl acetate 70:30 to yield the title product, 160mg. ¹H NMR (CDCl₃, 400MHz) δ: 0.05 (d, 2H), 0.27 (m, 2H), 0.92 (m, 1H), 2.48 (s, 3H), 3.38 (d, 2H), 4.02 (m, 2H), 4.03 (s, 3H), 5.08 (m, 2H), 6.80 (m, 1H), 7.00 (m, 1H), 7.80 (m, 1H), 8.18 (m, 1H). MS ES+ m/z 439 [MNa]⁺

### Preparation 103

### Methyl 5-chloro-7-(cyclohexyl)amino-1-(2-ethoxyethyl)-1H-pyrazolo[4,3-d]pyrimidine-3-carboxylate

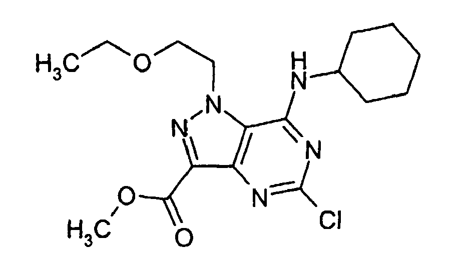

The dichloro compound of preparation 58 (2.50g, 7.84mmol) was dissolved in tetrahydrofuran (10mL) and the solution treated dropwise with a solution of cyclohexylamine (4.48mL, 39.20mmol) whilst being cooled on an ice bath. The reaction mixture was stirred for 15 minutes at room temperature. The reaction mixture was diluted with water (50mL) and ethyl acetate (50mL) and the reaction mixture stirred for 1 hour. The solid present was collected by filtration, washed with water and dried *in vacuo.* The ethyl acetate layer was separated and washed with water, dried over magnesium sulphate and concentrated *in vacuo.* The residue was triturated with ether to yield further solid. A total of 2.25g of the desired product was collected.
¹H NMR (CDCl₃, 400MHz) δ: 1.18 (t, 3H), 1.27 (m, 2H), 1.47 (m, 2H), 1.53-1.75 (m, 2H), 1.78 (m, 2H), 2.12 (m, 2H), 3.76 (q, 2H), 3.92 (t, 2H), 4.00 (s, 3H), 4.12 (m, 1H), 4.70 (t, 2H), 7.20 (d, 1H). MS ES+ m/z 382 [MH]⁺

### Preparations 104 to 117

The appropriate monochloro compound (1eq), the appropriate HNR³R⁴ amine (3-5eq), N-ethyldiisopropylamine (5eq) and tetraethylammonium fluoride hydrate (1 eq) were dissolved in 1-methyl-2-pyrrolidinone (5.3mL.mmol⁻¹) and the reaction vessel sealed and heated in a microwave oven for 45 minutes. The reaction mixture was allowed cool to room temperature before being partitioned between ethyl acetate (50mL) and water (50mL). The organic layer was washed with water (25mL), dried over magnesium sulphate and concentrated *in vacuo*. The residue was purified by column chromatography on silica gel eluting with dichloromethane:ethyl acetate 50:50 to yield the desired product.

The monochloro compounds of preparations 73, 74, 75, 76, 77, 81, 86, 87, 88, 92, 97 and 102 were used.

| | | |
|---|---|---|
| | 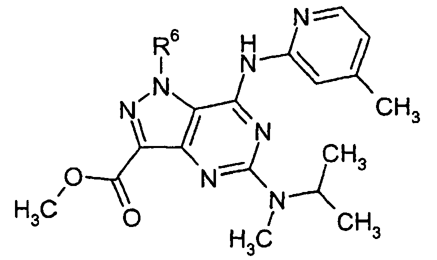 | |

| No. | R⁶ | Data |
|---|---|---|
| 104 | 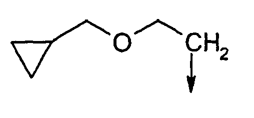 | ¹H NMR (CD₃OD, 400MHz) δ: 0.03 (m, 2H), 0.24 (m, 2H), 0.96 (m, 1H), 1.25 (d, 6H), 2.40 (s, 3H), 3.09 (s, 3H), 3.38 (d, 2H), 3.94 (s, 3H), 3.98 (m, 2H), 4.81 (m, 2H), 5.15 (m, 1H), 6.93 (d, 1H), 8.15 (d, 1H), 8.31 (s, 1H). MS ES+ m/z 454 [MH]⁺ |
| 105 | 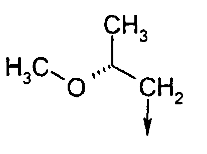 | ¹H NMR (CD₃OD, 400MHz) δ: 1.24 (m, 6H), 1.32 (m, 3H), 2.38 (s, 3H), 3.07 (s, 3H), 3.41 (s, 3H), 3.92 (m, 1H), 3.94 (s, 3H), 4.58 (m, 1H), 4.68 (m, 1H), 5.14 (m, 1H), 6.93 (d, 1H), 8.17 (d, 1H), 8.22 (s, 1H). MS APCI+ m/z 428 [MH]⁺ |
| 106 | -(CH₂)₂OCH(CH₃)₂ | ¹H NMR (CD₃OD, 400MHz) δ: 1.07 (d, 6H), 1.25 (d, 6H), 2.37 (s, 3H), 3.10 (s, 3H), 3.66 (m, 1H), 3.94 (s, 3H), 3.94 (m, 2H), 4.76 (t, 2H), 5.16 (m, 1H), 6.93 (d, 1H), 8.17 (d, 1H), 8.32 (s, 1H). MS ES+ m/z 442 [MH]⁺ |
| 107 | -(CH₂)₂CH(CH₃)OCH₃ | ¹H NMR (DMSO-D₆, 400MHz) δ: 1.05 (m, 3H), 1.18-1.25 (m, 8H), 2.35 (s, 3H), 2.98 (s, 3H), 3.15 (s, 3H), 3.30 (m, 1H) 3.80 (s, 3H), 4.65 (m, 2H), 5.02 (m, 1H), 6.95 (m, 1H), 8.00 (m, 1H), 8.20 (m, 1H), 9.20 (m, 1H). MS ES+ m/z 442 [MH]⁺ |

| | | | |
|---|---|---|---|
| | 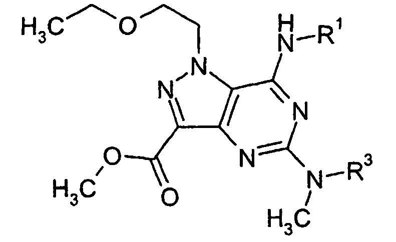 | | |

| No. | R¹ | R³ | Data |
|---|---|---|---|
| 108 | 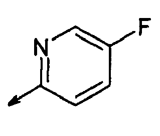 | -CH(CH₃)₂ | ¹H NMR (CD₃OD, 400MHz) δ: 1.20 (t, 3H), 1.23 (d, 6H), 3.07 (s, 3H), 3.66 (q, 2H), 3.96 (s, 3H), 4.00 (t, 2H), 4.80 (t, 2H), 5.10 (m, 1H), 7.64 (t, 1H), 8.21 (s, 1H), 8.32 (d, 1H). MS APCI- m/z 430 [M-H]⁻ |
| 109 | 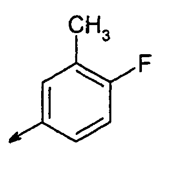 | -CH(CH₃)₂ | ¹H NMR (CD₃OD, 400MHz) δ: 1.14 (t, 3H), 1.22 (d, 6H), 2.30 (s, 3H), 3.04 (s, 3H), 3.63 (q, 2H), 3.96 (s, 3H), 3.98 (t, 2H), 4.79 (t, 2H), 5.08 (m, 1H), 7.02 (t, 1H), 7.42 (m, 1H), 7.68 (m, 1H). MS ES+ m/z 445 [MH]⁺ |
| 110 | 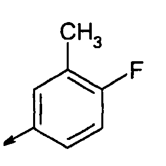 | -CH₂CH₃ | ¹H NMR (CD₃OD, 400MHz) δ: 1.15 (t, 3H), 1.20 (t, 3H), 2.30 (s, 3H), 3.19 (s, 3H), 3.60 (q, 2H), 3.70 (q, 2H), 3.96 (s, 3H), 3.98 (m, 2H), 4.80 (t, 2H), 7.01 (t, 1H), 7.42 (t, 1H), 7.67 (m, 1H). MS APCI+ m/z 431 [MH]⁺ |
| 111 | 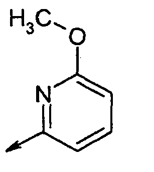 | -CH(CH₃)₂ | ¹H NMR (CD₃OD, 400MHz) δ: 1.15 (t, 3H), 1.23 (d, 6H), 3.05 (s, 3H), 3.65 (q, 2H), 3.95 (s, 3H), 3.98 (q, 2H), 4.02 (s, 3H), 4.78 (t, 2H), 5.01 (m, 1H), 6.49 (d, 1H), 7.66 (t, 1H), 7.82 (d, 1H). MS APCI+ m/z 444 [MH]⁺ |
| 112 | 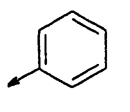 | -CH(CH₃)₂ | ¹H NMR (CD₃OD, 400MHz) δ: 1.15 (t, 3H), 1.23 (d, 6H), 3.05 (s, 3H), 3.65 (q, 2H), 3.96 (s, 3H), 4.00 (t, 2H), 4.79 (t, 2H), 5.11 (m, 1H), 7.09 (t, 1H), 7.40 (t, 2H), 7.71 (d, 2H). MS APCI+ m/z 413 [MH]⁺ |
| 113 | 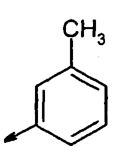 | -CH(CH₃)₂ | ¹H NMR (CD₃OD, 400MHz) δ: 1.16 (t, 3H), 1.22 (d, 6H), 2.37 (s, 3H), 3.06 (s, 3H), 3.45 (q, 2H), 3.98 (t, 3H), 4.00 (t, 2H), 4.79 (t, 2H), 5.10 (m, 1H), 6.93 (d, 1H), 7.23 (t, 1H), 7.43 (d, 1H), 7.65 (s, 1H). MS APCI+ m/z 427 [MH]⁺ |
| 114 | 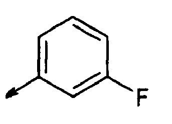 | -CH(CH₃)₂ | ¹H NMR (CD₃OD, 400MHz) δ: 1.15 (t, 3H), 1.25 (d, 6H), 3.07 (s, 3H), 3.64 (q, 2H), 3.96 (s, 3H), 4.00 (t, 3H), 4.80 (t, 2H), 5.10 (m, 1H), 6.80 (m, 1H), 7.33 (m, 1H), 7.44 (m, 1H), 7.78 (m, 1H). MS APCI+ m/z 431 [MH]⁺ |
| 115 | 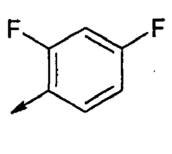 | -CH(CH₃)₂ | ¹H NMR (CDCl₃, 400MHz) δ: 1.10 (t, 3H), 1.18 (d, 6H), 3.05 (s, 3H), 3.60 (q, 2H), 3.97 (t, 2H), 4.02 (s, 3H), 4.78 (m, 2H), 5.00 (m, 1H), 6.90 (m, 2H), 8.18 (m, 1H), 8.90 (s, 1H). MS APCI+ m/z 449 [MH]⁺ |
| 116 | 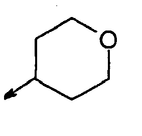 | -CH(CH₃)₂ | ¹H NMR (CD₃OD, 400MHz) δ: 1.15 (t, 3H), 1.20 (d, 6H), 1.69 (m, 2H), 2.15 (m, 2H), 3.02 (s, 3H), 3.56 (m, 4H), 3.88 (m, 2H), 3.95 (s, 3H), 4.02 (m, 2H), 4.25 (m, 1H), 4.66 (t, 2H), 5.12 (m, 1H). MS ES+ m/z 421 [MH]⁺ |
| 117 | 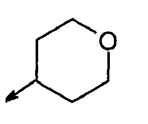 | -CH₃ | ¹H NMR (CD₃OD, 400MHz) δ: 1.15 (t, 3H), 1.70 (m, 2H), 2.13 (m, 2H), 3.19 (s, 6H), 3.54 (m, 4H), 3.88 (t, 2H), 3.94 (s, 3H), 4.00 (m, 2H), 4.30 (m, 1H), 4.65 (t, 2H). MS ES+ m/z 393 [MS]⁺ |

### Preparation 118

### Methyl 5-(N-isopropyl-N-methylamino)-1-[2-(2-methoxyethoxy)ethyl]-7-(4-methyl-pyridin-2-ylamino)-1H-pyrazolo[4,3-d]pyrimidine-3-carboxylate

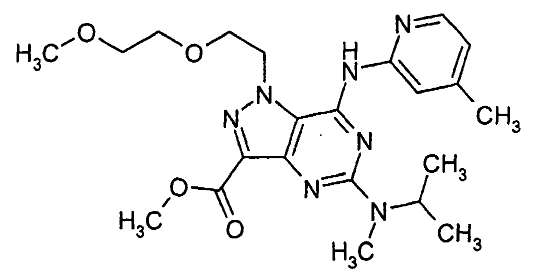

The monochloro compound of preparation 85 (150mg, 0.36mmol), N-ethyldiisopropylamine (186µL, 1.07mmol) and N-methyl-isopropylamine (50µL, 0.43mmol) were dissolved in dimethyl sulphoxide (1.5mL) and the reaction mixture stirred at 120°C for 18 hours. Additional N-methyl-isopropylamine (62µL, 0.36mmol) was added and the reaction stirred at 120°C for a further 4 hours. The reaction mixture was concentrated *in vacuo* and the residue taken up in a mixture of dichloromethane (50mL) and water (100mL). The two layers were separated and the aqueous layer washed with dichloromethane (50mL). The organics were combined and washed with water (2x50mL) before being dried over magnesium sulphate and concentrated *in vacuo.* The crude product was purified by column chromatography on silica gel eluting with dichloromethane:methanol 95:5 to yield the title product as a yellow oil, 65mg.
¹H NMR (CD₃OD, 400MHz) δ: 1.25 (d, 6H), 2.39 (s, 3H), 2.93 (m, 3H), 3.05 (s, 3H), 3.45 (t, 2H), 3.62 (m, 2H), 3.95 (s, 3H), 4.00 (t, 2H), 4.78 (m, 2H), 5.10 (m, 1H), 6.90 (m, 1H), 8.15 (d, 1H), 8.25 (m, 1H). MS APCI+ m/z 458 [MH]⁺

### Preparation 119

### Methyl 5-(dimethylamino)-1-(2-ethoxyethyl)-7-(6-ethylpyridin-2-ylamino)-1H-pyrazolo[4,3-d]pyrimidine-3-carboxylate

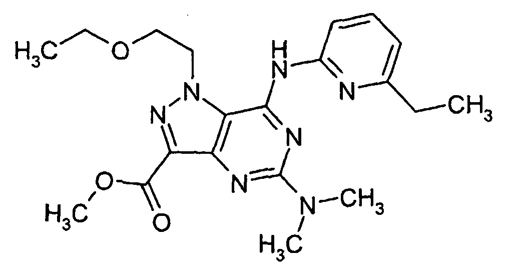

A solution of the monochloro compound of preparation 94 (200mg, 0.50mmol) and N-ethyldiisopropylamine (172µL, 0.99mmol) in dimethyl sulphoxide (2mL) was treated with a 5.6M solution of dimethylamine in ethanol (180µL, 1.0mmol) and the reaction mixture stirred at 120°C for 18 hours. The reaction mixture was concentrated *in vacuo* and the residue taken up in ether (100mL) and washed with water (50mL). The aqueous was extracted with ether (25mL) and the combined organics washed with water (2x100mL) and brine (50mL), dried over magnesium sulphate and concentrated *in vacuo*. The residue was purified by column chromatography on silica gel eluting with dichloromethane:methanol 100:0 to 95:5. The crude product was recrystallised from ethanol to yield the title product.
¹H NMR (CDCl₃, 400MHz) δ: 1.30 (m, 6H), 2.76 (q, 2H), 3.30 (s, 6H), 3.70 (q, 2H), 4.01 (m, 2H), 4.02 (s, 3H), 4.80 (t, 2H), 6.83 (d, 1H), 7.60 (t, 1H), 8.10 (d, 1H), 9.80 (s, 1H). MS APCI- m/z 412 [M-H]⁻

The following compounds, of the general formula shown below, were prepared by a method similar to that described for preparation 119 using the appropriate HNR³R⁴ amine and monochloro compound of preparations 72, 78, 79, 80, 92, 94, 96, 97, 98, 99, 100 and 101.

| | |
|---|---|
| No. | 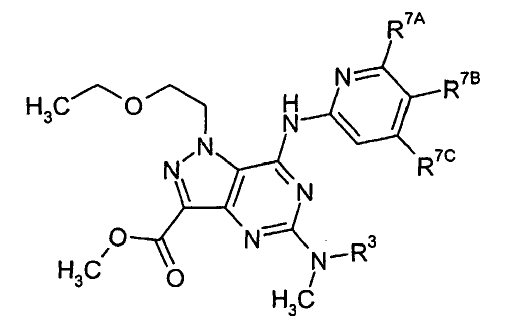 |
| 120 | R³ = -CH₂CH₃; R^{7A} = H; R^{7B} = H; R^{7C} = -CH₃ ¹H NMR (CD₃OD, 400MHz) δ: 1.10 (t, 3H), 1.22 (t, 3H), 2.40 (s, 3H), 3.21 (s, 3H), 3.61 (q, 2H), 3.79 (q, 2H), 3.96 (m, 5H), 4.79 (t, 2H), 6.98 (d, 1H), 8.17 (d, 1H), 8.37 (s, 1H). MS APCI+ m/z 414 [MH]⁺ |
| 121 | R³ = -CH(CH₃)₂; R^{7A} = H; R^{7B} = H; R^{7C} = -CH₃ ¹H NMR (CDCl₃, 400MHz) δ: 1.16 (t, 3H), 1.24 (s, 6H), 2.36 (s, 3H), 3.11 (s, 3H), 3.60 (q, 2H), 3.94 (t, 2H), 4.02 (s, 3H), 4.77 (m, 2H), 5.15 (m, 1H), 6.82 (m, 1H), 8.18 (q, 1H), 8.24 (s, 1H). MS APCI+ m/z 426 [MH]⁺ |
| 122 | R³ = -CH₂CH₃; R^{7A} = -CH₂CH₃; R^{7B} = H; R^{7C} = -CH₃ ¹H NMR (CD₃OD, 400MHz) δ: 1.20 (m, 6H), 1.30 (t, 3H), 2.78 (q, 2H), 3.21 (s, 3H), 3.65 (q, 2H), 3.78 (q, 2H), 3.97 (m, 5H), 4.81 (m, 2H), 6.98 (d, 1H), 7.70 (t, 1H), 8.20 (d, 1H). MS APCI- m/z 426 [M-H]⁻ |
| 123 | R³ = -CH(CH₃)₂; R^{7A} = H; R^{7B} = H; R^{7C} = H ¹H NMR (CDCl₃, 400MHz) δ: 1.20 (t, 3H), 1.25 (d, 6H), 3.12 (s, 3H), 3.64 (q, 2H), 3.98 (t, 2H), 4.03 (s, 3H), 4.79 (t, 2H), 5.15 (m, 1H), 6.98 (m, 1H), 7.68 (t, 1H), 8.33 (t, 2H), 9.81 (m, 1H). MS APCI+ m/z 414 [MH]⁺ |
| | 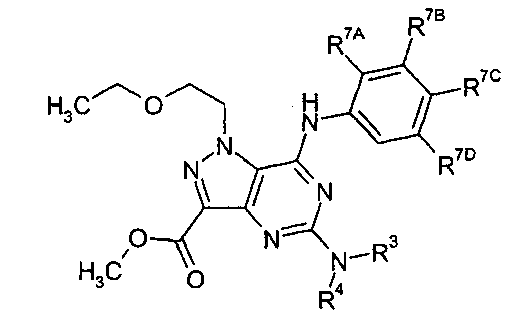 |
| 124 | R³ = -CH(CH₃)₂; R⁴ = -CH₃; R^{7A} = H; R^{7B} = F; R^{7C} = F; R^{7D} = H ¹H NMR (CDCl₃, 400MHz) δ: 1.20 (t, 3H), 1.22 (d, 6H), 3.10 (s, 3H), 3.62 (m, 2H), 4.00 (m, 5H), 4.78 (m, 2H), 5.10 (m, 1H), 7.10 (m, 2H), 7.80 (m, 1H), 9.10 (s, 1H). MS APCI+ m/z 449 [MH]⁺ |
| 125 | R³ = -CH(CH₃)₂; R⁴ = -CH₃; R^{7A} = H; R^{7B} = F; R^{7C} = H; R^{7D} = F ¹H NMR (CDCl₃, 400MHz) δ: 1.20 (t, 3H), 1.22 (d, 6H), 3.10 (s, 3H), 3.61 (q, 2H), 4.00 (m, 5H), 4.78 (m, 2H), 5.10 (m, 1H), 6.50 (t, 1H), 7.30 (m, 2H), 9.30 (s, 1H). MS APCI+ m/z 449 [MH]⁺ |
| 126 | R³ = -CH(CH₃)₂; R⁴ = -CH₃; R^{7A} = F; R^{7B} = H; R^{7C} = H; R^{7D} = F ¹H NMR (CDCl₃, 400MHz) δ: 1.10 (t, 3H), 1.20 (d, 6H), 3.12 (s, 3H), 3.58 (q, 2H), 3.95 (t, 2H), 4.02 (s, 3H), 4.78 (m, 2H), 5.10 (m, 1H), 6.70 (m, 1H), 7.05 (m, 1H), 8.30 (m, 1H), 9.20 (m, 1H). MS APCI-m/z 447 [M-H]⁻ |
| 127 | R³ = -CH(CH₃)₂; R⁴ = -CH₃; R^{7A} = F; R^{7B} = F; R^{7C} = H; R^{7D} = H ¹H NMR (CDCl₃, 400MHz) δ: 1.10 (t, 3H), 1.18 (d, 6H), 3.07 (s, 3H), 3.61 (q, 2H), 3.92 (t, 2H), 4.01 (s, 3H), 4.78 (m, 2H), 5.05 (m, 1H), 6.82 (m, 1H), 7.03 (m, 1H), 8.00 (m, 1H). MS APCI- m/z 447 [M-H]⁻ |
| 128 | R³ = -CH₂CH₃; R⁴ = -CH₂CH₃; R^{7A} = H; R^{7B} = -CH₃; R^{7C} = F; R^{7D} = H ¹H NMR (CDCl₃, 400MHz) δ: 1.20 (m, 9H), 2.30 (s, 3H), 3.65 (q, 2H), 3.70 (m, 4H), 4.00 (m, 5H), 4.75 (t, 2H), 6.95 (t, 1H), 7.35 (m, 1H), 7.60 (m, 1H). MS APCI- m/z 443 [M-H]⁻ |
| 129 | R³ = -CH₃; R⁴ = -CH₃; R^{7A} = F; R^{7B} = H; R^{7C} = H; R^{7D} = -CH₃ ¹H NMR (CDCl₃, 400MHz) δ: 1.10 (t, 3H), 2.35 (s, 3H), 3.26 (s, 6H), 3.59 (q, 2H), 3.95 (t, 2H), 4.03 (s, 3H), 4.77 (t, 2H), 6.84 (m, 1H), 7.01 (m, 1H), 8.25 (d, 1H), 9.00 (s, 1H). MS APCI+ m/z 417 [MH]⁺ |
| 130 | R³ = -CH₂CH₃; R⁴ = -CH₃; R^{7A} = F; R^{7B} = H; R^{7C} = H; R^{7D} = -CH₃ ¹H NMR (CDCl₃, 400MHz) δ: 1.10 (t, 3H), 1.22 (t, 3H), 2.35 (s, 3H), 3.25 (s, 3H), 3.59 (q, 2H), 3.63 (q, 2H), 3.95 (t, 2H), 4.03 (s, 3H), 4.76 (t, 2H), 6.82 (m, 1H), 7.02 (m, 1H), 8.23 (d, 1H), 8.98 (s, 1H). . MS APCI+ m/z 431 [MH]⁺ |
| 131 | R³ = -CH₃; R⁴ = -CH₃; R^{7A} = H; R^{7B} = -CH₃; R^{7C} = -CH₃; R^{7D} = H ¹H NMR (CDCl₃, 400MHz) δ: 1.19 (t, 3H), 2.27 (s, 3H), 2.29 (s, 3H), 3.26 (s, 6H), 3.62 (q, 2H), 4.00 (t, 2H), 4.03 (s, 3H), 4.74 (t, 2H), 7.10 (d, 1H), 7.40 (d, 1H), 7.52 (s, 1H), 8.90 (s, 1H). MS APCI+ m/z 413 [MH]⁺ |
| 132 | R³ = -CH₂CH₃; R⁴ = -CH₃; R^{7A} = H; R^{7B} = -CH₃; R^{7C} = -CH₃; R^{7D} = H ¹H NMR (CDCl₃, 400MHz) δ: 1.17 (t, 3H), 1.23 (t, 3H), 2.27 (s, 3H), 2.29 (s, 3H), 3.24 (s, 3H), 3.62 (q, 2H), 3.74 (q, 2H), 4.00 (t, 2H), 4.02 (s, 3H), 4.74 (t, 2H), 7.11 (d, 1H), 7.36 (d, 1H), 7.57 (s, 1H), 8.89 (s, 1H). MS APCI+ m/z 427 [MH]⁺ |
| 133 | R³ = -CH(CH₃)₂; R⁴ = -CH₃; R^{7A} = F; R^{7B} = H; R^{7C} = H; R^{7D} = H ¹H NMR (CDCl₃, 400MHz) δ: 1.10 (t, 3H), 1.21 (d, 6H), 3.08 (s, 3H), 3.61 (q, 2H), 3.96 (t, 2H), 4.03 (s, 3H), 4.78 (t, 2H), 5.01 (m, 1H), 7.05 (m, 1H), 7.14 (m, 2H), 8.29 (t, 1H), 9.01 (m, 1H). MS APCI+ m/z 431 [MH]⁺ |

### Preparation 134

### Methyl 1-(2-(cyclopropylmethoxy)ethyl)-5-(N-ethyl-N-methyl-amino)-7-(4-methyl-pyridin-2-ylamino)-1H-pyrazolo[4,3-d]pyrimidine-3-carboxylate

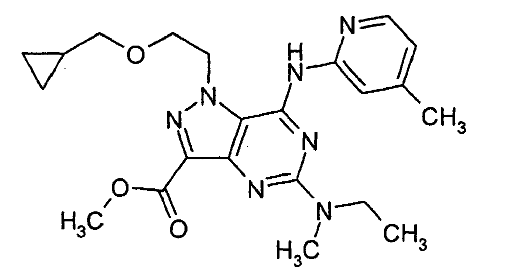

A solution of the chloro compound of preparation 102 (40mg, 0.096mmol) and N-ethyldiisopropylamine (83µL, 0.48mmol) in dimethyl sulphoxide (2mL) was treated with N-methylethylamine (41µL, 0.48mmol) and the reaction mixture stirred at 120°C for 18 hours. The reaction mixture was allowed to cool and partitioned between water (25mL) and ethyl acetate (25mL). The organic layer was washed with water (25mL), dried over magnesium sulphate and concentrated *in vacuo.* The residue was purified by column chromatography on silica gel eluting with ethyl acetate to yield the title product.
¹H NMR (CD₃OD, 400MHz) δ: 0.02 (m, 2H), 0.23 (m, 2H), 0.95 (m, 1H), 1.24 (t, 3H), 2.37 (s, 3H), 3.21 (s, 3H), 3.35 (d, 2H), 3.76 (m, 2H), 3.95 (s, 3H), 3.98 (t, 2H), 4.79 (m, 2H), 6.94 (m, 1H), 8.13 (d, 1H), 8.32 (s, 1H). MS ES+ m/z 462 [MNa]⁺

### Preparation 135

### 5-Chloro-7-(4-methylpyridin-2-ylamino)-1-(2-propoxyethyl)-1H-pyrazolo[4,3-d]pyrimidine-3-carboxylic acid

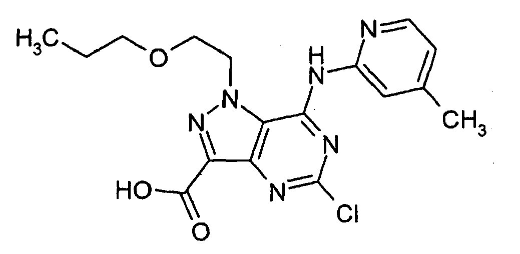

The ester of preparation 67 (500mg, 1.24mmol) was dissolved in dioxan (5mL) and the solution treated with a 1 M aqueous solution of sodium hydroxide (6.20mL, 6.2mmol). The reaction mixture was then stirred for 18 hours at room temperature. The reaction mixture was treated with 1 M citric acid solution (10mL) and a yellow precipitate formed. The mixture was stirred for 15 minutes before being filtered and the solid product dried *in vacuo* to yield the title product, 360mg.
¹H NMR (CDCl₃, 400MHz) δ: 0.73 (t, 3H), 1.52 (m, 2H), 2.51 (s, 3H), 3.51 (t, 2H), 4.01 (t, 2H), 5.05 (m, 2H), 6.98 (m, 1H), 7.24 (m, 1H), 8.14 (m, 1H). MS APCI+ m/z 391 [MH]⁺

The following compounds were prepared by a method similar to that described for preparation 135 using the appropriate ester of preparations 68, 69, 70, 71, 72, 80, 82, 83, 84, 87, 88, 89, 90, 91, 92, 93 and 103

| | | |
|---|---|---|
| | 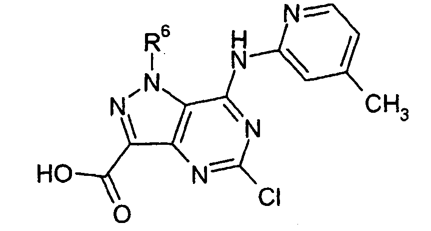 | |

| No. | R⁶ | Data |
|---|---|---|
| 137 | -(CH₂)₂OCH₂CH₃ | ¹H NMR (DMSO-D₆, 400MHz) δ: 1.00 (t, 3H), 2.34 (s, 3H), 3.45 (m, 2H), 3.81 (m, 2H), 4.84 (m, 2H), 6.93 (m, 1H), 7.89 (m, 1H), 8.16 (m, 1H). MS ES-m/z 375 [M-H]⁻ |
| 138 | 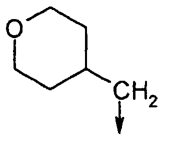 | ¹H NMR (DMSO-D₆, 400MHz) δ: 1.32 (m, 4H), 2.12 (m, 1H), 2.38 (s, 3H), 3.20 (m, 2H), 3.78 (m, 2H), 4.76 (d, 2H), 6.90 (d, 1H), 7.60 (s, 1H), 8.30 (s, 1H). MS APCI+ m/z 403 [MH]⁺ |
| 139 | 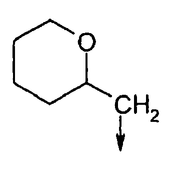 | ¹H NMR (DMSO-D₆, 400MHz) δ: 1.22 (m, 1H), 1.42 (m, 3H), 1.70 (m, 1H), 1.85 (m, 1H), 2.36 (s, 3H), 3.20-3.40 (m, 1H), 3.85 (m, 1H), 3.94 (m, 1H), 4.78 (m, 2H), 6.99 (d, 1H), 7,87 (m, 1H), 8.20 (m, 1H). MS APCI+ m/z 403 [MH]⁺ |
| 149 | -(CH₂)₂OCH₃ | ¹H NMR (DMSO-D₆, 400MHz) δ: 2.21 (s, 3H), 3.25 (m, 3H), 3.82 (m, 2H), 4.96 (m, 2H), 6.97 (m, 1H), 7.77 (m, 1H), 8.17 (m, 1H). MS ES- m/z 361 [M-H]⁻ |
| 150 | -(CH₂)₂OCH(CH₃)₂ | ¹H NMR (DMSO-D₆, 400MHz) δ: 0.93 (d, 6H), 2.38 (s, 3H), 3.54 (m, 1H), 3.84 (m, 2H), 4.89 (m, 2H), 7.04 (m, 1H), 7.90 (m, 1H), 8.23 (m, 1H). MS ES-m/z 389 [M-H]⁻ |
| 151 | 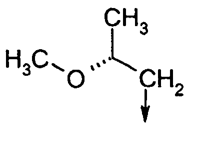 | ¹H NMR (DMSO-D₆, 400MHz) δ: 1.13 (d, 3H), 2.43 (s, 3H), 3.20 (s, 3H), 3.86 (m, 1H), 4.80 (d, 2H), 7.10 (d, 1H), 7.80 (s, 1H), 8.27 (d, 1H). MS ES+ m/z 377 [MH]⁺ |

| | | |
|---|---|---|
| | 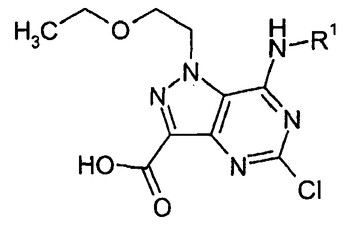 | |

| No. | R¹ | Data |
|---|---|---|
| 136 | 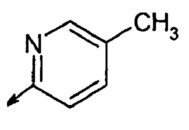 | ¹H NMR (DMSO-D₆, 400MHz) δ: 1.03 (t, 3H), 2.24 (s, 3H), 3.50 (m, 2H), 3.86 (m, 2H), 4.88 (m, 2H), 7.77 (m, 1H), 8.03 (m, 1H), 8.17 (m, 1H). MS ES- m/z 375 [M-H]⁻ |
| 140 | 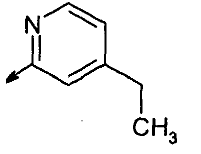 | ¹H NMR (DMSO-D₆, 400MHz) δ: 1.02 (t, 3H), 1.22 (t, 3H), 2.66 (q, 2H), 3.43 (m, 2H), 3.85 (m, 2H), 4.92 (m, 2H), 7.01 (m, 1H), 7.95 (m, 1H), 8.20 (m, 1H). MS ES- m/z 389 [M-H]⁻ |
| 141 | 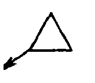 | ¹H NMR (DMSO-D₆, 400MHz) δ: 0.63 (m, 2H), 0.82 (m, 2H), 0.97 (t, 3H), 2.94 (m, 1H), 3.39 (m, 2H), 3.71 (m, 2H), 4.77 (m, 2H), 7.80 (m, 1H). MS ES- m/z 324 [M-H]⁻ |
| 142 | 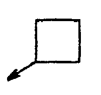 | ¹H NMR (DMSO-D₆, 400MHz) δ: 0.99 (t, 3H), 1.77 (m, 2H), 2.14 (m, 2H), 2.35 (m, 2H), 3.40 (m, 2H), 3.75 (m, 2H), 4.59 (m, 1H), 4.81 (m 2H), 6.72 (m, 1H). MS ES- m/z 338 [M-H]⁻ |
| 143 | 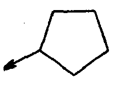 | ¹H NMR (DMSO-D₆, 400MHz) δ: 1.00 (t, 3H), 1.58 (m, 4H), 1.75 (m, 2H), 2.03 (m, 2H), 3.41 (m, 2H), 3.73 (m, 2H), 4.62 (m, 1H), 4.79 (m, 2H), 7.44 (m, 1H). MS ES- m/z 352 [M-H]⁻ |
| 144 | 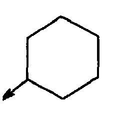 | ¹H NMR (CDCl₃, 400MHz) δ: 1.00 (t, 3H), 1.18 (m, 1H), 1.38 (m, 4H), 1.62 (m, 1H), 1.74 (m, 2H), 1.96 (m, 2H), 3.40 (t, 2H), 3.72 (m, 2H), 4.03 (m, 1H), 4.73 (m, 2H), 7.26 (d, 1H). MS ES- m/z 366 [M-H]⁻ |
| 145 | 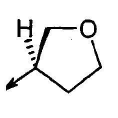 | ¹H NMR (DMSO-D₆, 400MHz) δ: 1.20 (t, 3H), 1.96 (m, 1H), 2.49 (m, 1H), 3.61 (q, 2H), 3.86 (m, 2H), 3.94 (t, 2H), 4.04 (m, 2H), 4.75 (t, 2H), 4.85 (m, 1H), 7.70 (m, 1H) |
| 146 | 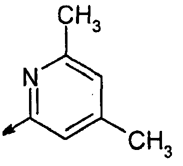 | ¹H NMR (CD₃OD, 400MHz) δ: 1.11 (t, 3H), 2.40 (s, 3H), 2.49 (s, 3H), 3.58 (m, 2H), 3.97 (m, 2H), 5.01 (m, 2H), 6.92 (m, 1H), 7.94 (m, 1H). . MS ES- m/z 389 [M-H]⁻ |
| 147 | 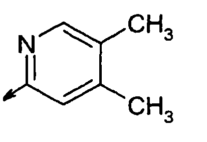 | ¹H NMR (CD₃OD, 400MHz) δ: 1.08 (t, 3H), 2.26 (s, 3H), 2.38 (s, 3H), 3.65 (q, 2H), 3.98 (t, 2H), 4.99 (t, 2H), 7.95 (m, 1H), 8.00 (s, 1H). MS APCI+ m/z 391 [MH]⁺ |
| 148 | 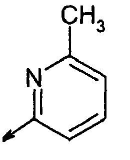 | ¹H NMR (CD₃OD, 400MHz) δ: 1.08 (t, 3H), 2.45 (s, 3H), 3.52 (q, 2H), 3.85 (t, 2H), 4.92 (t, 2H), 7.01 (d, 1H), 7.82 (t, 1H), 7.94 (d, 1H). MS APCI+ m/z 377 [MH]⁺ |
| 152 | 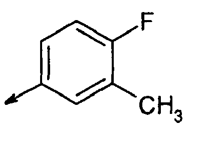 | ¹H NMR (CD₃OD, 400MHz) δ: 0.95 (t, 3H), 2.27 (s, 3H), 3.45 (q, 2H), 3.82 (t, 2H), 4.04 (t; 2H), 7.22 (t, 1H), 7.51 (m, 2H), 9.35 (s, 1H). MS APCI- m/z 392 [MNa]⁺ |

### Preparation 153

### [5,7-Dichloro-1-(2-ethoxyethyl)-1H-pyrazolo[4,3-d]pyrimidin-3-yl]methanol

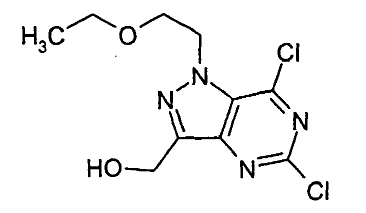

The dichloro compound of preparation 58 (2.4g, 7.52mmol) was dissolved in tetrahydrofuran (60mL) and the solution cooled to -78°C. Diisobutylaluminium hydride (37.6mL, 37.6mmol) in tetrahydrofuran (20mL) was added dropwise over 10 minutes and the reaction mixture stirred at -78°C for 10 minutes and then at -10°C for 1 hour. The reaction mixture was cooled to -78°C, quenched with ammonium chloride solution (25mL) and allowed to return to room temperature. The reaction mixture was diluted with dichloromethane (200mL) and water (100mL) and the solution filtered through Arbocel®, washing through with dichloromethane (3x100mL). The organic phase was separated, dried over magnesium sulphate and concentrated *in vacuo.* The crude product was purified by column chromatography on silica gel eluting with dichloromethane:methanol 99:1 to yield the title product, 1.67g.
¹HNMR (CDCl₃, 400MHz) δ: 1.08 (t, 3H), 3.42 (m, 2H), 3.80 (m, 2H), 4.90 (m, 2H), 5.10 (s, 2H). MS APCI+ m/z 291 [MH]⁺

### Preparation 154

### 3-(tert-Butyldimethylsilyloxymethyl)-5,7-dichloro-1-(2-ethoxyethyl)-1H-pyrazolo[4,3-d]pyrimidine

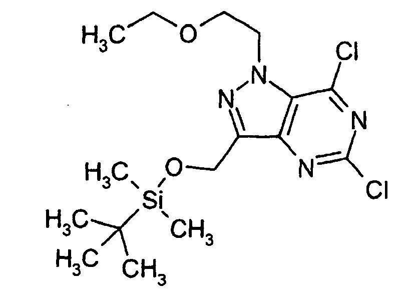

The alcohol of preparation 153 (1.32g, 4.53mmol) was dissolved in dichloromethane (25mL) and the solution treated with imidazole (339mg, 4.98mmol) and then *tert-*butyldimethylsilyl chloride (750mg, 4.98mmol). The reaction mixture was then stirred at room temperature for 18 hours. The reaction mixture was diluted with dichloromethane (200mL) and washed with 10% potassium carbonate solution (100mL). The organic phase was dried over sodium sulphate and concentrated *in vacuo*. The crude product was purified by column chromatography on silica gel eluting with dichloromethane:methanol 99:1 to yield the title product, 1.56g. ¹HNMR (CDCl₃, 400MHz) δ: 0.00 (s, 6H), 0.78 (s, 9H), 0.93 (t, 3H), 3.29 (m, 2H), 3.71 (t, 2H), 4.72 (m, 2H), 4.94 (s, 2H). MS APCI+ m/z 405 [MH]⁺

### Preparation 155

### N-[3-(tert-Butyldimethylsilyloxymethyl)-5-chloro-1-(2-ethoxyethyl)-1H-pyrazolo[4,3-d]pyrimidin-7-yl]eyrimidin-4-ylamine

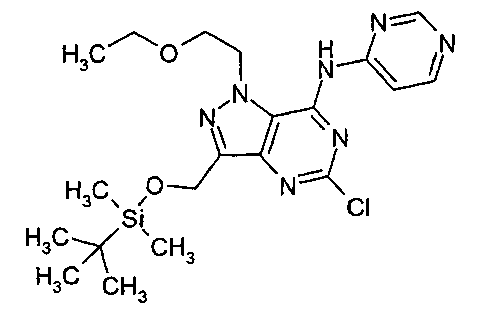

Pyrimidin-4-ylamine (1.10g, 11.55mmol) was dissolved in tetrahydrofuran (30mL) and the solution treated with sodium hexamethyldisilazide (2.12g, 11.55mmol) and stirred at room temperature for 20 minutes. The solution was then treated with a solution of the dichloro compound of preparation 154 (1.56g, 3.85mmol) in tetrahydrofuran (10mL) and the reaction mixture stirred for 90 minutes at room temperature. The reaction mixture was quenched with ammonium chloride solution (100mL) and extracted with dichloromethane (200mL). The organic phase was separated, dried over magnesium sulphate and concentrated *in vacuo.* The crude product was purified by column chromatography on silica gel eluting with dichloromethane:methanol 97:3 to yield the title product, 830mg.
¹HNMR (CDCl₃, 400MHz) δ: 0.00 (s, 6H), 0.77 (s, 9H), 1.08 (t, 3H), 3.54 (m, 4H), 4.63 (m, 2H), 4.90 (s, 2H), 8.33 (d, 1H), 8.51 (d, 1H), 8.77 (s, 1H)
MS APCI+ m/z 464 [MH]⁺

The following compounds, of the general formula shown below, were prepared by a method similar to that described for preparation 155 using the appropriate HNR¹R² amine.

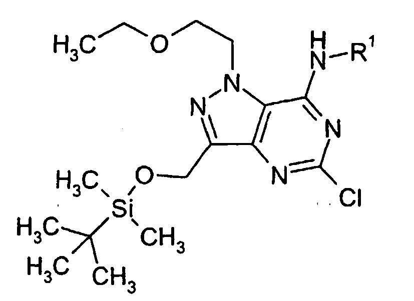

| No. | R¹ | Data |
|---|---|---|
| 156 | 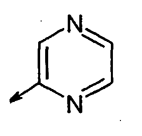 | ¹H NMR (CDCl₃, 400MHz) δ: 0.18 (s, 6H), 0.93 (s, 9H), 1.21 (t, 3H), 3.65 (q, 2H), 3.97 (m, 2H), 4.80 (m, 2H), 5.06 (m, 2H), 8.30 (m, 2H), 9.77 (m, 1H), 10.17 (m, 1H) |
| 157 | 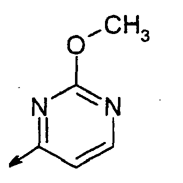 | ¹H NMR (CDCl₃, 400MHz) δ: 0.20 (s, 6H), 0.95 (s, 9H), 1.25 (q, 3H), 3.65 (m, 2H), 3.95 (t, 2H), 4.02 (s, 3H), 4.78 (t, 2H), 5.05 (s, 2H), 8.05 (d, 1H), 8.50 (d, 1H), 10.30 (s, 1H). MS APCI+ m/z 494 [MH]⁺ |
| 158 | 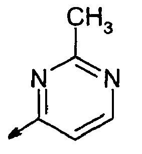 | ¹H NMR (CDCl₃, 400MHz) δ: 0.00 (s, 6H), 0.77 (s, 9H), 1.13 (t, 3H), 2.48 (s, 3H), 3.53 (q, 2H), 3.80 (t, 2H), 4.62 (t, 2H), 4.89 (s, 2H), 8.03 (d, 1H), 8.41 (d, 1H), 10.12 (s, 1H). MS ES+ m/z 478 [MH]⁺ |
| 159 | 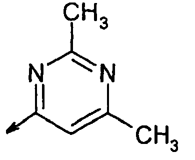 | ¹H NMR (CDCl₃, 400MHz) δ: 0.10 (s, 6H), 0.95 (s, 9H), 1.38 (t, 3H), 2.42 (s, 6H), 3.65 (q, 2H), 3.95 (t, 2H), 4.79 (t, 2h), 5.10 (s, 2H), 6.78 (s, 1H), 10.18 (s, 1H). MS APCI+ m/z 492 [MH]⁺ |

- Preparation 157 used the amine of preparation 6 as the HNR¹R² amine.
- Preparation 158 used 2-methylpyrimidin-4-ylamine (J. Het. Chem, 1987, 24, 1377-1380) as the HNR¹R² amine.

### Preparation 160

### [5-Chloro-1-(2-ethoxyethyl)-7-(pyrimidin-4-ylamino)-1H-pyrazolo[4,3-d]pyrimidin-3-yl]methanol

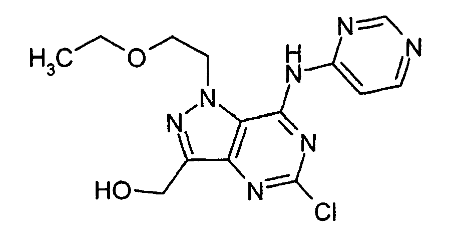

The protected alcohol of preparation 155 (815mg, 1.76mmol) was dissolved in tetrahydrofuran (40mL) and the solution treated with a 1 M solution of tetrabutylammonium fluoride in tetrahydrofuran (8.63mL, 8.63mmol). The reaction mixture was stirred for 90 minutes at room temperature and was then treated with additional tetrabutylammonium fluoride solution (4.32mL) and stirred for another hour. The reaction mixture was diluted with water (50mL) and the aqueous extracted with ethyl acetate (3x50mL). The combined organics were dried over magnesium sulphate and concentrated *in vacuo.* The crude product was purified by column chromatography on silica gel eluting with dichloromethane:methanol 99:1 to 95:5 to yield the title product, 1.25g.
¹HNMR (CDCl₃, 400MHz) δ: 1.26 (t, 3H), 3.70 (m, 2H), 3.97 (m, 2H), 4.76 (m, 2H), 5.10 (s, 2H), 8.51 (d, 1H), 8.72 (d, 1H), 8.99 (s, 1H). MS APCI+ m/z 350 [MH]⁺

The following compounds, of the general formula shown below, were prepared by a method similar to that described for preparation 160 using the appropriate protected alcohol of preparations 156, 157, 158 and 159.

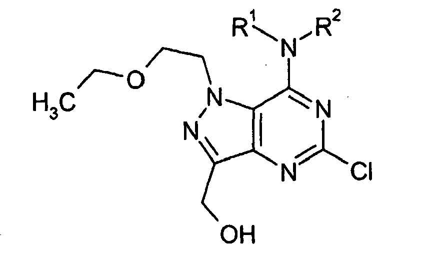

| No. | R¹ | Data |
|---|---|---|
| 161 | 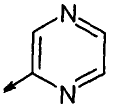 | ¹H NMR (CDCl₃, 400MHz) δ: 1.22 (t, 3H), 3.66 (m, 2H) 3.98 (m, 2H), 4.80 (m, 2H), 5.08 (s, 2H), 8.34 (m, 2H), 9.80 (m, 1H), 10.22 (m, 1H) |
| 162 | 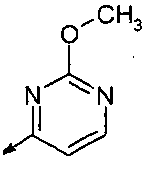 | ¹H NMR (DMSO-D₆, 400MHz) δ: 1.10 (t, 3H), 3.58 (q, 2H), 3.83 (t, 2H), 3.90 (s, 3H), 4.65 (t, 2H), 4.78 (t, 2H), 5.48 (t, 1H), 7.80 (d, 1H), 8.58 (d, 1H), 10.42 (s, 1H). MS APCI- m/z 378 [M-H]⁻ |
| 163 | 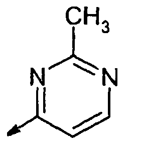 | ¹H NMR (DMSO-D₆, 400MHz) δ: 1.11 (t, 3H), 2.55 (s, 3H), 3.56 (q, 2H), 3.85, 4.69 (d, 2H), 4.79 (t, 2H), 5.33 (t, 1H), 7.99 (d, 1H), 8.60 (d, 1H). MS ES+ m/z 364 [MH]⁺ |
| 164 | 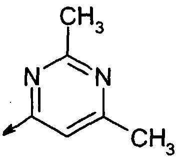 | ¹H NMR (DMSO-D₆, 400MHz) δ: 1.10 (m, 3H), 2.38 (s, 6H), 3.43 (q, 2H), 3.75 (m, 2H), 4.55 (m, 2H), 4.70 (t, 2H), 5.35 (t, 1H), 6.98 (s, 1H), 10.44 (s, 1H). MS APCI+ m/z 378 [MH]⁺ |

### Preparation 165

### 5-Chloro-1-(2-ethoxyethyl)-7-(pyrazin-2-ylamino)-1H-pyrazolo[4,3-d]pyrimidine-3-carbaldehyde

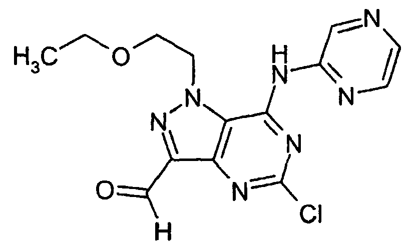

The alcohol of preparation 161 (251 mg, 0.72mmol) was dissolved in dichloromethane (12mL) and the solution cooled to 0°C in an ice bath. 1,1,1-Triacetoxy-1,1-dihydro-1,2-benziodoxol-3 (1H)-one (Dess-Martin periodinane, 456mg, 1.08mmol) was added and the reaction mixture stirred at room temperature for 2 hours. The reaction mixture was treated with a saturated solution of sodium thiosulphate in water (7.8mL) and then with saturated sodium hydrogencarbonate solution (7.8mL) and ether (7.8mL). The mixture was stirred at room temperature for 15 minutes, the organic phase separated and the aqueous extracted with dichloromethane (x3). The organics were combined, dried over sodium sulphate and concentrated *in vacuo.* The crude product was purified by column chromatography on silica gel eluting with dichloromethane:methanol 99:1 to yield the title product, 200mg.
¹HNMR (CDCl₃, 400MHz) δ: 1.22 (t, 3H), 3.69 (m, 2H), 4.06 (m, 2H), 4.92 (m, 2H), 7.22 (m, 1H), 8.32 (m, 1H), 8.40 (m, 1H), 9.77 (m, 1H), 10.35 (m, 1H)

The following compounds, of the general formula shown below, were prepared by a method similar to that described for preparation 165 using the appropriate alcohol of preparations 160, 162, 163, 164.

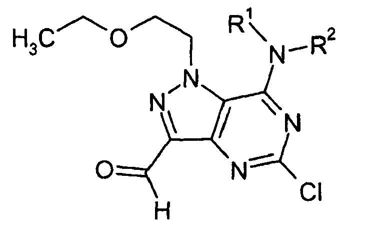

| No. | R¹ | Data |
|---|---|---|
| 166 | 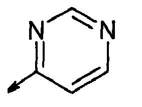 | ¹H NMR (CDCl₃, 400MHz) δ: 1.23 (t, 3H), 3.72 (q, 2H), 4.06 (t, 2H), 4.93 (m, 2H), 8.40 (d, 1H), 8.75 (d, 1H), 8.95 (s, 1H), 10.37 (s, 1H) |
| 167 | 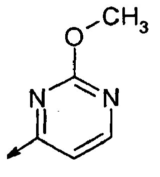 | ¹H NMR (CDCl₃, 400MHz) δ: 1.25 (t, 3H), 3.70 (q, 2H), 4.00 (m, 2H), 4.05 (s, 3H), 4.90 (t, 2H), 8.05 (d, 1H), 8.55 (d, 1H), 10.48 (m, 2H). MS APCI- m/z 376 [M-H]⁻ |
| 168 | 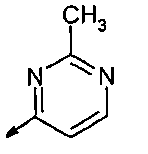 | ¹H NMR (DMSO-D₆, 400MHz) δ: 0.98 (t, 3H), 2.47 (s, 3H), 3.43 (q, 2H), 3.85 (t, 2H), 4.93 (t, 2H), 7.86 (d, 1H), 8.48 (d, 1H), 10.08 (s, 1H). MS ES- m/z 360 [M-H]⁻ |
| 169 | 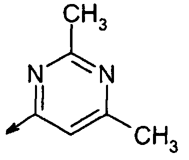 | ¹H NMR (CDCl₃, 400MHz) δ: 1.30 (m, 3H), 2.60 (s, 6H), 3.70 (t, 2H), 4.00 (t, 2H), 4.90 (m, 2H), 6.80 (m, 1H), 10.30 (s, 1H), 10.35 (s, 1H). MS APCI- m/z 374 [M-H]⁻ |

### Preparation 170

### 5-Chloro-1-(2-ethoxyethyl)-7-(pyrimidin-4-ylamino)-1H-pyrazolo[4,3-d]pyrimidine-3-carboxylic acid

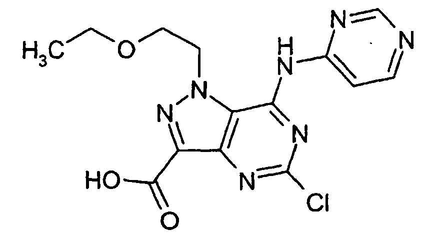

The aldehyde of preparation 166 (220mg, 0.63mmol) was dissolved in *tert*-butanol (40mL) and the solution treated with a 2M solution of 2-methylbut-2-ene in tetrahydrofuran (44mL). The solution was stirred at room temperature and then treated dropwise with a solution of sodium chlorite (683mg, 7.59mmol) and sodium dihydrogen orthophosphate (699mg, 5.82mmol) in water (8mL) over 5 minutes. The reaction mixture was stirred at room temperature for 30 minutes. Water (40mL) and dichloromethane (40mL) were added to the reaction mixture and the phases separated. The aqueous layer was extracted with dichloromethane (2x40mL) and the aqueous was then acidified to pH 3 and extracted once more with dichloromethane (2x40mL). The organics were combined, dried over magnesium sulphate and concentrated *in vacuo.* The crude product was purified by column chromatography on silica gel eluting with first dichloromethane:methanol 97:3 and then dichloromethane:methanol:acetic acid 85:15:1 to yield the title product, 194mg. ¹HNMR (CD₃OD, 400MHz) δ: 1.20 (t, 3H), 3.68 (m, 2H), 4.01 (t, 2H), 4.92 (t, 2H), 8.42 (m, 1H), 8.68 (m, 1H), 8.87 (m, 1H). MS APCI+ m/z 364 [MH]⁺

The following compounds, of the general formula shown below, were prepared by a method similar to that described for preparation 170 using the appropriate aldehyde of preparations 165, 167, 168, 169.

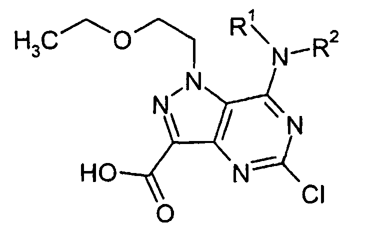

| No. | NR¹R² | Data |
|---|---|---|
| 171 | 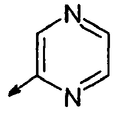 | ¹H NMR (CD₃OD, 400MHz) δ: 1.20 (m, 3H), 3.65 (m, 2H), 3.99 (m, 2H), 4.96 (m, 2H), 8.36 (m, 1H), 8.42 (m, 1H), 9.60 (m, 1H). MS APCI+ m/z 364 [MH]⁺ |
| 172 | 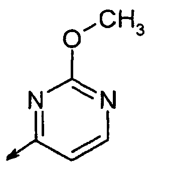 | ¹H NMR (DMSO-D₆, 400MHz) δ: 1.10 (m, 3H), 3.58 (q, 2H), 3.90 (m, 3H), 3.95 (s, 3H), 4.90 (t, 2H), 7.80 (d, 1H), 8.58 (d, 1H), 10.52 (m, 1H). MS APCI- m/z 392 [M-H]⁻ |
| 173 | 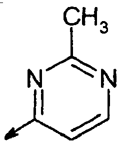 | ¹H NMR (DMSO-D₆, 400MHz) δ: 1.01 (t, 3H), 2.46 (s, 3H), 3.46 (q, 2H), 3.80 (t, 2H), 4.84 (t, 2H), 7.87 (d, 1H), 8.50 (d, 1H). MS ES- m/z 376 [M-H]⁻ |
| 174 | 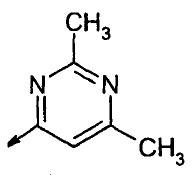 | ¹H NMR (DMSO-D₆, 400MHz) δ: 1.01 (t, 3H), 2.40 (s, 3H), 3.35 (s, 3H), 3.46 (q, 2H), 3.80 (t, 2H), 4.75 (t, 2H), 7.00 (d, 1H),. MS ES- m/z 390 [M-H]⁻ |

### Preparation 175

### Ethyl 5-chloro-1-(2-ethoxyethyl)-7-(4-methylpyridin-2-ylamino)-1H-pyrazolo[4,3-d]pyrimidine-3-carboxylate

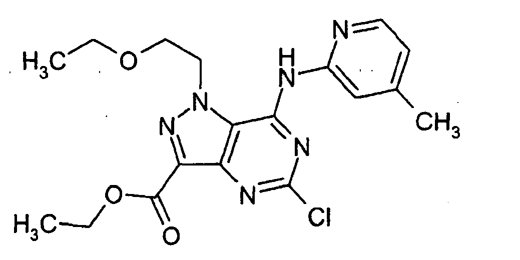

The carboxylic acid of preparation 137 (565mg, 1.5mmol) was suspended in 1-methyl-2-pyrrolidinone (5mL) and the solution treated with N-ethyldiisopropylamine (313µL, 1.8mmol) and N,N'-carbonyldiimidazole (364mg, 2.25mmol) and stirred for 30 minutes at room temperature. The solution was treated with sodium ethoxide (408mg, 6.0mmol) and the reaction mixture stirred for a further 30 minutes at room temperature. The reaction mixture was quenched with citric acid solution (5mL) and concentrated *in vacuo.* The residue was partitioned between dichloromethane (100mL) and water (50mL) and the organic phase separated, dried over magnesium sulphate and concentrated *in vacuo.* The residue was triturated with ethyl acetate (10mL) and dried *in vacuo* to yield the title product.
¹H NMR (DMSO-D₆, 400MHz) δ: 0.96 (t, 3H), 1.32 (t, 3H), 2.40 (s, 3H), 3.44 (q, 2H), 3.86 (t, 2H), 4.36 (q, 2H), 4.93 (t, 2H), 7.06 (m, 1H), 7.87 (s, 1H), 8.23 (d, 1H),
MS APCI+ m/z 405 [MH]⁺

### Preparation 176

### 2-(Dimethylamino)ethyl 5-chloro-1-(2-ethoxyethyl)-7-(4-methylpyridin-2-ylamino)-1H-pyrazolo[4,3-d]pyrimidine-3-carboxylate

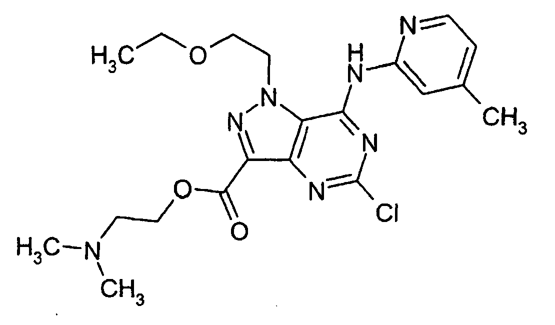

The carboxylic acid of preparation 137 (282mg, 0.75mmol) was suspended in 1-methyl-2-pyrrolidinone (2.5mL) and the solution treated with N-ethyldiisopropylamine (157µL, 0.9mmol) and N,N'-carbonyldiimidazole (182mg, 1.13mmol) and stirred at room temperature for 30 minutes. The solution was treated with 2-(dimethylamino)ethanol (309µL, 3.0mmol) and 4-(N,N-dimethylamino)pyridine (12mg, 0.1mmol) and the reaction mixture heated to 50°C for 18 hours. The reaction mixture was concentrated *in vacuo* and the residue purified by column chromatography on silica gel eluting with dichloromethane:methanol:0.88 ammonia 100:0:0 to 90:10:1 to yield the title product, 170mg.
¹H NMR (CD₃OD, 400MHz) δ: 1.08 (t, 3H), 2.40 (s, 6H), 2.45 (s, 3H), 2.84 (t, 2H), 3.60 (m, 2H), 3.98 (t, 2H), 4.57 (t, 2H), 5.01 (m, 2H), 6.98 (d, 1H), 8.12 (m, 1H).
MS APCI+ m/z 448 [MH]⁺

### Preparation 177

### 5-Chloro-1-(2-ethoxyethyl)-7-(4-methylpyridin-2-ylamino)-1H-pyrazolo[4,3-d]pyrimidine-3-carboxamide

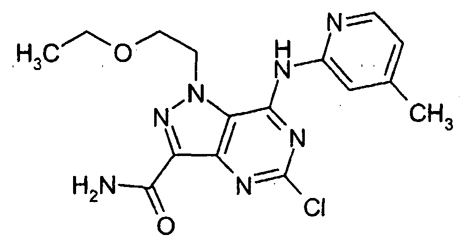

The carboxylic acid of preparation 137 (376mg, 1.0mmol) was added to a solution of *N*-[(dimethylamino)-1*H*-1,2,3-triazolo-[4,5-*b*]pyridin-1-yl-methylene]-*N-*methylmethanaminium hexafluorophosphate *N*-oxide (HATU, 380mg, 1.0mmol) and N-ethyldiisopropylamine (1 mL, 5.6mmol) in N,N-dimethylformamide (15mL) The mixture was then treated with a saturated solution of ammonia in tetrahydrofuran (600µL) and the reaction mixture stirred at room temperature for 48 hours. The reaction mixture was concentrated *in vacuo* and the residue partitioned between ethyl acetate (50mL) and water (50mL). The aqueous was extracted with ethyl acetate (2x50mL) and dichloromethane (50mL). The organics were combined, dried over magnesium sulphate and concentrated *in vacuo.* The residue was purified by column chromatography on silica gel eluting with dichloromethane:methanol 100:0 to 95:5 to yield the title product.
¹H NMR (CDCl₃, 400MHz) δ: 1.17 (t, 3H), 2.47 (s, 3H), 3.68 (m, 2H), 4.01 (t, 2H), 4.92 (m, 2H), 6.94 (m, 1H), 8.08 (m, 1H), 8.22 (m, 1H). MS APCI+ m/z 376 [MH]⁺

### Preparation 178

### 5-Chloro-1-(2-ethoxyethyl)-7-(4-fluoro-3-methylphenylamino)-1H-pyrazolo[4,3-d]pyrimidine-3-carboxamide

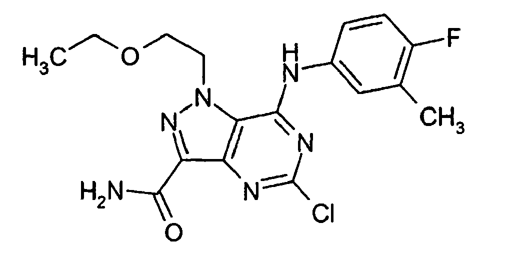

The title compound was prepared by a method similar to that described for preparation 177 using the carboxylic acid of preparation 152.
¹H NMR (DMSO-D₆, 400MHz) δ: 0.96 (t, 3H), 2.26 (s, 3H), 3.45 (q, 2H), 3.82 (m, 2H), 4.93 (m, 2H), 7.23 (t, 1H), 7.50 (m, 2H), 7.64 (s, 1H), 7.81 (s, 1H), 9.37 (s, 1H)
MS APCI+ m/z 393 [MH]⁺

### Preparation 179

### 5-Chloro-1-(2-ethoxyethyl)-7-(4-methylpyridin-2-ylamino)-1H-pyrazolo[4,3-d]pyrimidine-3-carbonitrile

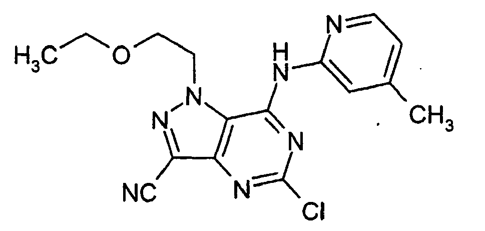

The amide of preparation 177 (140mg, 0.37mmol) was dissolved in a solution of trifluoroacetic anhydride (53µL, 0.37mmol) and pyridine (59mg, 0.75mmol) in tetrahydrofuran (5mL) and the reaction mixture stirred at room temperature for 18 hours. The reaction mixture was concentrated *in vacuo* and the residue purified by column chromatography on silica gel eluting with dichloromethane to yield the title product.
¹H NMR (CDCl₃, 400MHz) δ: 1.09 (t, 3H), 2.40 (s, 3H), 3.66 (m, 2H), 3.91 (m, 2H), 5.00 (m, 2H), 6.85 (m, 1H), 8.05 (m, 1H), 8.08 (m, 1H). MS APCI+ m/z 358 [MH]⁺

### Preparation 180

### 5-Chloro-1-(2-ethoxyethyl)-7-(4-fluoro-3-methylphenylamino)-1H-pyrazolo[4,3-d]pyrimidine-3-carbonitrile

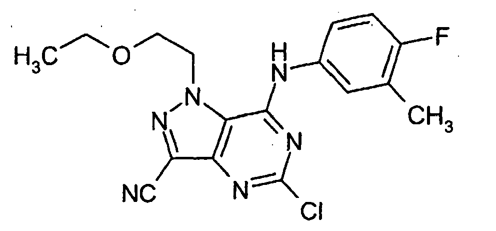

The title compound was prepared by a method similar to that described for preparation 179 using the amide of preparation 178.
¹H NMR (CDCl₃, 400MHz) δ: 1.19 (t, 3H), 2.32 (s, 3H), 3.68 (q, 2H), 4.04 (m, 2H), 4.80 (m, 2H), 7.05 (t, 1H), 7.56 (m, 2H), 9.37 (s, 1H). MS ES+ m/z 397 [MNa]⁺

### Preparation 181

### 5-Chloro-1-(2-ethoxyethyl)-N-hydroxy-7-(4-methylpyridin-2-ylamino)-1H-pyrazolo[4,3-d]pyrimidine-3-carboxamidine

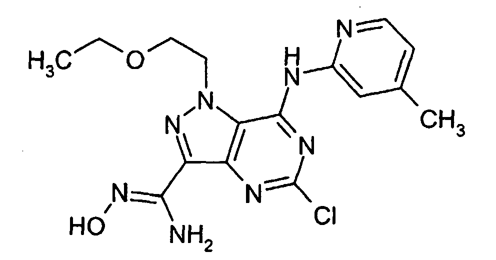

The nitrile of preparation 179 (100mg, 0.28mmol) was dissolved in a solution of hydroxylamine (23mg, 0.34mmol) in ethanol (2mL) and the solution treated with a 5M aqueous solution of sodium hydroxide (68µL, 0.34mmol). The reaction mixture was stirred at 50°C for 18 hours and then concentrated *in vacuo* to yield the title product.
¹H NMR (DMSO-D₆, 400MHz) δ: 1.02 (m, 3H), 2.37 (s, 3H), 3.50 (m, 2H), 3.85 (m, 2H), 4.84 (m, 2H), 6.96 (m, 1H), 8.16 (m, 1H), 8.20 (m, 1H). MS ES+ m/z 358 [MH]⁺

### Preparation 182

### 5-Chloro-1-(2-ethoxyethyl)-7-(4-fluoro-3-methylphenylamino)-N-hydroxy-1H-pyrazolo[4,3-d]pyrimidine-3-carboxamidine

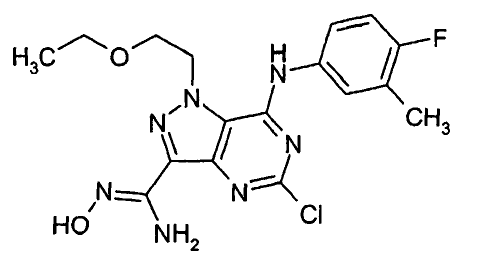

The title compound was prepared by a method similar to that described for preparation 181 using the nitrile of preparation 180.
¹H NMR (DMSO-D₆, 400MHz) δ: 0.95 (t, 3H), 2.26 (s, 3H), 3.44 (d, 2H), 3.79 (m, 2H), 4.88 (m, 2H), 7.21 (t, 1H), 7.50 (m, 2H), 9.30 (m, 1H), 9.95 (s, 1H)
MS ES- m/z 406 [M-H]⁻

### Preparation 183

### 3-[5-Chloro-1-(2-ethoxyethyl)-7-(4-methyripyridin-2-ylamino)-1H-pyrazolo[4,3-d]pyrimidin-3-yl]-2H-1,2,4-oxadiazol-5-one

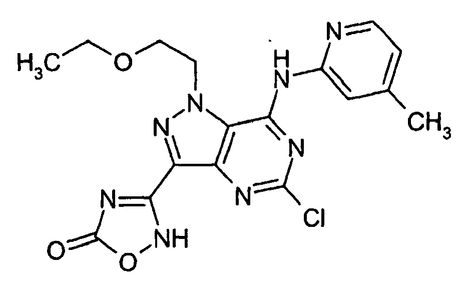

The product of preparation 181 (109mg, 0.28mmol) was dissolved in a solution of N,N'-carbonyldiimidazole (49mg, 0.30mmol) in N,N-dimethylformamide (2mL) and the reaction mixture stirred at 80°C for 2 hours. The reaction mixture was concentrated *in vacuo* and the residue triturated with acetone (3mL), filtered and recrystalised from acetonitrile to yield the title product.
¹H NMR (DMSO-D₆, 400MHz) δ: 1.00 (t, 3H), 2.39 (s, 3H), 3.47 (m, 2H), 3.87 (t, 2H), 4.95 (t, 2H), 6.98 (d, 1H), 7.87 (s, 1H), 8.17 (m, 1H). MS APCI+ m/z 417 [MH]⁺

### Preparation 184

### 3-[5-Chloro-1-(2-ethoxyethyl)-7-(4-fluoro-3-methylphenylamino)-1H-pyrazolo[4,3-d]pyrimidin-3-yl]-2H-1,2,4-oxadiazol-5-one

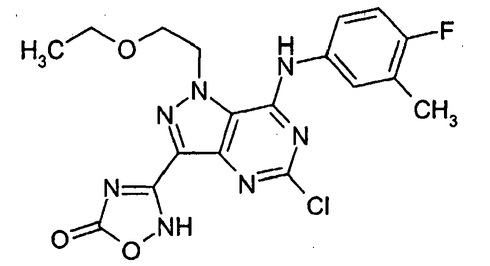

The title compound was prepared by a method similar to that described for preparation 183 using the product of preparation 182.
¹H NMR (DMSO-D₆, 400MHz) δ: 0.96 (t, 3H), 2.27 (s, 3H), 3.45 (m, 2H), 3.83 (m, 2H), 4.97 (m, 2H), 7.24 (t, 1H), 7.50 (m, 2H), 9.40 (s, 1H). MS APCI+ m/z 434 [MH]⁺

### Preparation 185

### N-[5-Chloro-1-(2-ethoxyethyl)-3-(2H-tetrazol-5-yl)-1H-pyrazolo[4,3-d]pyrimidin-7-yl]-(4-methylpyridin-2-yl)amine

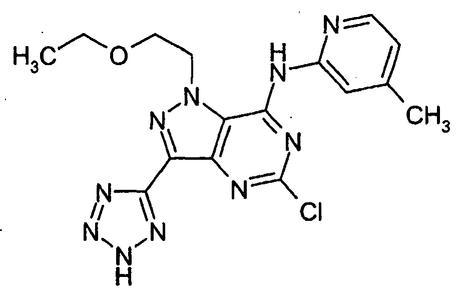

The nitrile of preparation 179 (100mg, 0.28mmol) was added to a solution of azidotributyltin (104mg, 0.32mmol) in dioxane (3mL) and the reaction mixture heated to reflux for 18 hours. The reaction mixture was treated with further azidotributyltin (104mg, 0.32mmol) and the reaction mixture heated to reflux for a further 18 hours. The reaction mixture was diluted with a 2M solution of hydrochloric acid in ether (20mL) and the mixture stirred at room temperature for 30 minutes. The reaction mixture was concentrated *in vacuo* and the residue adsorbed onto silica and purified by column chromatography on silica gel eluting with dichloromethane:methanol:acetic acid 100:0:0 to 90:10:1 to yield the title product. ¹H NMR (CD₃OD, 400MHz) δ: 1.11 (t, 3H), 2.57 (s, 3H), 3.64 (q, 2H), 4.05 (t, 2H), 5.09 (t, 2H), 7.25 (d, 1H), 7.90 (s, 1H), 8.35 (d, 1H). MS APCI+ m/z 401 [MH]⁺

### Preparation 186

### N-[5-Chloro-1-(2-ethoxyethyl)-7-(4-methylpyridin-2-ylamino)-1H-Dyrazolo[4,3-d]pyrimidine-3-carbonyl]methanesulfonamide

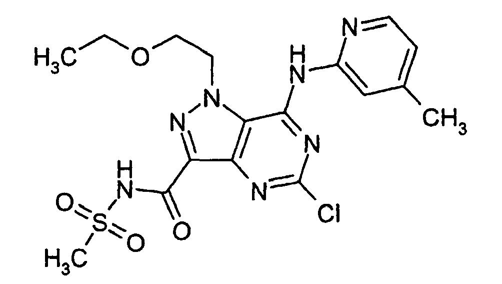

The carboxylic acid of preparation 137 (1.0g, 2.70mmol), methanesulphonamide (330mg, 3.5mmol), 1-(3-dimethylaminopropyl)-3-ethylcarbodiimide hydrochloride (660mg, 3.5mmol) and 4-dimethylaminopyridine (390mg, 3.5mmol) were dissolved in N,N-dimethylformamide (10mL) and the reaction mixture stirred at room temperature for 60 hours. Additional methanesulphonamide (165mg, 1.7mmol), 1-(3-dimethylaminopropyl)-3-ethylcarbodiimide hydrochloride (330 1.7mmol) and 4-dimethylaminopyridine (195 1.7mmol) were added and the reaction mixture stirred for a further 20 hours. Further methanesulphonamide (165 1.7mmol), 1-(3-dimethylaminopropyl)-3-ethylcarbodiimide hydrochloride (330 1.7mmol) and 4-dimethylaminopyridine (195 1.7mmol) were added and the reaction mixture stirred for a final 18 hours. The reaction mixture was concentrated *in vacuo* and the residue partitioned between dichloromethane (25mL) and water (25mL). The organic phase was separated, washed with water (2x25mL), dried over magnesium sulphate and concentrated *in vacuo.* The residue was purified by column chromatography on silica gel eluting with dichloromethane:methanol:acetic acid 100:0:0 to 96:3.5:0.5. The crude product was triturated in warm ethyl acetate (10mL) to yield the title product, 290mg.
¹H NMR (DMSO-D₆, 400MHz) δ: 0.95 (t, 3H), 2.40 (s, 3H), 3.40 (s, 3H), 3.45 (d, 2H), 3.85 (m, 2H), 4.95 (m, 2H), 7.15 (d, 1H), 7.85 (s, 1H), 8.25 (d, 1H)
MS ES- m/z 452 [M-H]⁻

### Preparation 187

### 3-(Methoxycarbonyl)-1-[(2S)-2-methoxypropyl]-4-nitro-1H-pyrazole-5-carboxylic acid

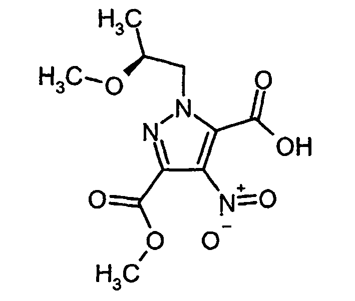

Diisopropyl azodicarboxylate (14.9mL, 76mmol) was added dropwise to a solution of dimethyl 4-nitropyrazole-3,5-dicarboxylate (15.73g, 69mmol), (*S*)-(+)-2-methoxypropanol (6.81 g, 76mmol) and triphenylphosphine (19.9g, 76mmol) in tetrahydrofuran (220mL) with stirring under nitrogen, keeping the reaction temperature between 0°C and 10°C by cooling in an ice bath. Once addition was complete the reaction was allowed to stir at room temperature for 18 hours. The reaction mixture was concentrated under reduced pressure and the residual oil redissolved in methanol (200mL). Potassium hydroxide (3.88g, 69mmol) was added and the reaction was allowed to stir at room temperature for 18 hours. The mixture was concentrated under reduced pressure and the residue suspended in water (50mL), and washed with dichloromethane (2x100mL). The aqueous solution was acidified to pH 1 using concentrated hydrochloric acid, and then extracted with dichloromethane (3x100mL). The combined organic extracts from extraction of the acidic solution were evaporated to dryness, then taken up in saturated aqueous sodium bicarbonate solution (100ml). The aqueous solution was washed sequentially with dichloromethane (100mL), and ethyl acetate (2x100ml), then acidified to pH1 with concentrated hydrochloric acid and extracted with ethyl acetate (3x100ml). The combined organic extracts from extraction of the acidic solution were dried over magnesium sulphate and concentrated under reduced pressure to afford the title compound as a yellow oil.
¹H NMR (DMSO-D₆, 400MHz) δ: 1.05 (d, 3H), 3.10 (s, 3H), 3.70 (m, 1H), 3.85 (d, 3H), 4.45-4.70 (m, 2H).
MS APCI+ m/z 288 [MH]⁺

### Preparation 188

### 2-(Cyclobutyloxy)ethanol

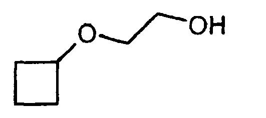

Butyl lithium (2.5M in hexanes, 61 mL, 0.152mol) was added dropwise to an ice-cold solution of cyclobutanol (10g, 0.139mol) in tetrahydrofuran (250mL) so as to maintain the reaction temperature below 10°C. The mixture was then stirred for a further 2 hours at 5-10°C, and a solution of 1,3,2-dioxathiolane 2,2-dioxide (18.90g, 0.152mol) in tetrahydrofuran (50mL) was added dropwise so as to maintain the reaction temperature below 15°C. Once addition was complete the reaction was stirred for a further 3 hours at room temperature, water (3mL) followed by concentrated sulphuric acid (7.5mL) then added and the reaction stirred for an additional 18 hours. The reaction was carefully neutralised by the addition of solid sodium carbonate and sodium bicarbonate, and the mixture concentrated under reduced pressure at room temperature. The residue was diluted with water, saturated with sodium chloride added until saturation was achieved and the solution then extracted with ethyl acetate (4x100mL). The combined organic extracts were dried over magnesium sulphate and evaporated under reduced pressure at room temperature. The residual orange oil was purified by Kugelrohr distillation to afford the title compound, 7.7g. bp 70-80°C at 10mmHg.
¹H NMR (CDCl₃ 400 MHz) δ: 1.38-1.57 (m, 1H), 1.63 (m, 1H), 1.80-1.98 (m, 2H), 2.06-2.15 (m, 2H), 3.40 (t, 2H), 3.65 (t, 2H), 3.95 (m, 1H).

### Preparation 189

### 1-[2-(Cyclobutyloxy)ethyl]-3-(methoxycarbonyl)-4-nitro-1H-pyrazole-5-carboxylic acid

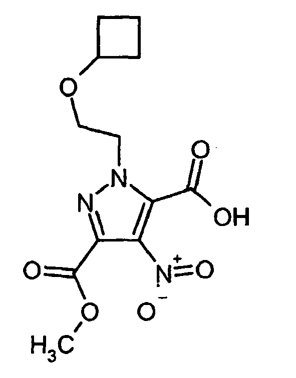

The title compound was obtained as a white solid from the alcohol from preparation 188 and dimethyl 4-nitropyrazole-3,5-dicarboxylate following a similar procedure to that described in preparation 187.
1H NMR (CDCl₃, 400MHz) δ: 1.38-1.50 (m, 1H), 1.62 (m, 1H), 1.70-1.81 (m, 2H), 2.10 (m, 2H), 3.76 (m, 2H), 3.90 (m, 4H), 4.78 (t, 2H), 9.68 (br s, 1H).
MS ES+ m/z 331 [MNH₄]⁺

### Preparation 190

### 2-(2,2-Difluoroethoxy)ethanol

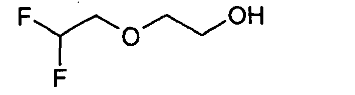

Tetra-butyl ammonium bromide (1.96g, 6.08mmol) was added portionwise to a solution of 2,2-difluoroethanol (25g, 304.9mmol) in triethylamine (45mL, 322.9mmol) and the mixture stirred for 5 minutes. Ethylene carbonate (29.53g, 335.3mmol) was added and the reaction mixture was heated at 100°C for 18 hours. The cooled mixture was then distilled under reduced pressure, and the distillate containing the desired product was redistilled at atmospheric pressure to provide the title compound as a yellow liquid, 4.95g (b.p.127-128°C).
¹H NMR (CDCl₃, 400MHz) δ: 2.04 (br s, 1H), 3.65 (m, 2H), 3.72 (m, 4H), 5.70-6.02 (m, 1H).

### Preparation 191

### 1-[2-(2,2-Difluoroethoxy)ethyl]-3-(methoxycarbonyl)-4-nitro-1H-pyrazole-5-carboxylic acid

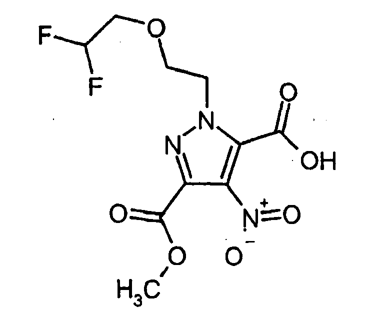

The title compound was obtained as a white solid, from the alcohol from preparation 190 and dimethyl 4-nitropyrazole-3,5-dicarboxylate, using a similar procedure to that described in preparation 187.
¹H NMR (CDCl₃, 400MHz) δ: 3.61 (m, 2H), 3.92 (m, 5H), 4.80 (t, 2H), 5.60-5.88 (m, 1H).
MS ES+ m/z 324 [MH]⁺

### Preparation 192

### 3-(Methoxycarbonyl)-4-nitro-1-[2-(2,2,2-trifluoroethoxy)ethyl]-1H-pyrazole-5-carboxylic acid

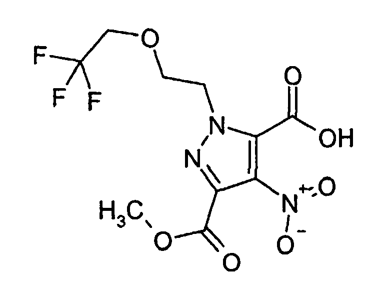

A solution of diisopropyl azodicarboxylate (71.9mL, 366mmol) in tetrahydrofuran (80mL) was added dropwise to a solution of dimethyl 4-nitropyrazole-3,5-dicarboxylate (60g, 260mmol), 2,2,2-trifluoroethoxyethanol (Journal of Fluorine Chemistry (1992), 59(3), 387-96), (45.2g, 314mmol) and triphenylphosphine (96.15g, 366mmol) in tetrahydrofuran (650mL) with stirring under nitrogen, maintaining the reaction temperature between 0°C and 10°C by cooling in an ice bath. After the addition was complete, the mixture was allowed to warm to room temperature and stirred for 2 days. The solvent was removed under reduced pressure and the residue was dissolved in methanol (800mL) and cooled to 0°C. A solution of potassium hydroxide (16.16g, 288mmol) in methanol (200mL) was added at 0°C and the reaction was allowed to warm to room temperature and stirred for 16 hours. The solvent was removed under reduced pressure and the residue was partitioned between water (600mL) and ethyl acetate (600mL). The aqueous layer was washed with ethyl acetate (2 x 200mL) and the aqueous phase then acidified with hydrochloric acid to pH1. The aqueous solution was extracted with ethyl acetate (3 x 400mL), the combined extracts were dried over sodium sulphate and concentrated under reduced pressure to afford a colourless solid (52.86g, 59%). The product was a mixture of 3-methoxycarbonyl-4-nitro-1-(2,2,2-trifluoroethoxy)ethylpyrazole-5-carboxylic acid (major) and 5-methoxycarbonyl-4-nitro-1-(2,2,2-trifluoroethoxy)ethylpyrazole-3-carboxylic acid (minor) and was used directly for the next step.
¹H NMR (CDCl₃, 400MHz) δ: 3.77 (q, 2H), 3.93 (s, 3H), 4.00 (t, 2H), 4.84 (t, 2H).

### Preparation 193

### 2-(3,3,3-Trifluoropropoxy)ethanol

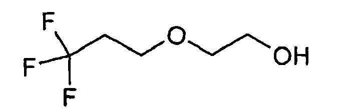

n-Butyl lithium (39mL, 2.5M in hexanes, 97.5mmol) was added dropwise to an ice-cooled solution of 3,3,3-trifluoropropan-1-ol (10g, 87.7mmol) in tetrahydrofuran (130mL), so as to maintain the temperature below 5°C, and once addition was complete the reaction was stirred for a further hour at 0°C.

A solution of 1,3,2-dioxathiolane 2,2-dioxide (11.97g, 96.5mmol) in tetrahydrofuran (35mL) was then added dropwise so as to maintain the internal temperature below 5°C, and once addition was complete the reaction was stirred at room temperature for 18 hours. Water (2mL) followed by concentrated sulphuric acid (5mL) were added and the reaction stirred for a further 6 hours at room temperature. The mixture was neutralised by the addition of sodium carbonate, then diluted with water (20mL) and the resulting solid filtered off and washed with ethyl acetate. The filtrate was concentrated under reduced pressure and the residue suspended in brine and extracted with ethyl acetate (3x). The combined organic extracts were dried over magnesium sulphate and evaporated under reduced pressure. The residual gum was distilled under high vacuum to afford the title compound as a colourless liquid, 6.75g (b.p.57-80°C).
¹H NMR (CDCl_{3,} 400 MHz) δ: 2.38 (m, 2H), 2.57 (m, 2H), 3.69 (m, 4H).

### Preparation 194

### 3-(Methoxycarbonyl)-4-nitro-1-[2-(3,3,3-trifluoropropoxy)ethyl]-1H-pyrazole-5-carboxylic acid

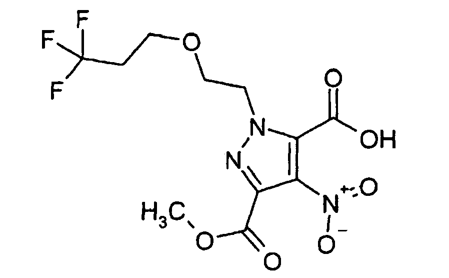

The title compound was obtained as a white solid, from the alcohol from preparation 193 and dimethyl 4-nitropyrazole-3,5-dicarboxylate, following the procedure described in preparation 187.
¹H NMR (CDCl_{3,} 400 MHz) δ: 2.39 (m, 2H), 3.54 (t, 2H), 3.78 (m, 2H), 3.80 (s, 3H), 4.69 (t, 2H).
MS ES+ m/z 356 [MH]⁺

### Preparation 195

### 2-(3-Fluoropropoxy)ethanol

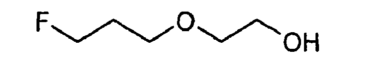

The title compound was obtained in 71 % yield, from 3-fluoropropan-1-ol and 1,3,2-dioxathiolane 2,2-dioxide, following a similar procedure to that described in preparation 193.
¹H NMR (CDCl_{3,} 400 MHz) δ: 1.96 (m, 2H), 2.10 (bs, 1H), 3.58 (t, 2H), 3.62 (t, 2H), 3.75 (t, 2H), 4.50 (dd, 1H), 4.62 (dd, 1H).

### Preparation 196

### 1-[2-(3-Fluoropropoxxy)ethyl]-3-(methoxycarbonyl)-4-nitro-1H-pyrazole-5-carboxylic acid

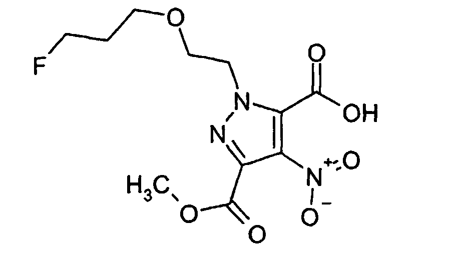

The title compound was obtained in 92% yield from dimethyl 4-nitropyrazole-3,5-dicarboxylate and the alcohol from preparation 195 following the procedure described in preparation 187.
¹H NMR (CDCl_{3.} 400 MHz) δ: 1.81-1.95 (m, 2H), 3.56 (t, 2H), 3.83 (t, 2H), 3.97 (s, 3H), 4.38 (m, 1H), 4.48 (m, 1H), 4.82 (m, 2H).
MS ES+ m/z 320 [MH]⁺

### Preparation 197

### Methyl 5-(aminocarbonyl)-1-[(2S)-2-methoxyprooyl]-4-nitro-1H-pyrazole-3-carboxylate

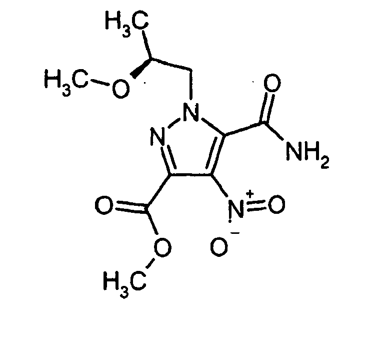

Oxalyl chloride (6.83mL, 78.3mmol) was added to a solution of the acid from preparation 187 (15g, 52.2mmol) in dichloromethane (250mL) at 0°C. N,N-Dimethylformamide (0.15mL) was added and the mixture was allowed to stir for 18 hours at room temperature. Tic analysis (dichloromethane:methanol:0.88 ammonia, 95:5:1) showed starting material remaining, so additional oxalyl chloride (0.91 mL, 10mmol) was added dropwise and the reaction stirred for a further 18 hours at room temperature. The solution was evaporated under reduced pressure and the residue was dissolved in tetrahydrofuran (250mL). The solution was cooled to 0°C, 0.88 ammonia (20mL) added dropwise, and once addition was complete, the reaction was stirred at room temperature for 1 hour. The reaction was concentrated under reduced pressure and the residue partitioned between dichloromethane (200mL) and water (50mL) and the layers separated. The aqueous solution was extracted with further dichloromethane (200mL), the organic solutions combined, dried over magnesium sulphate and evaporated under reduced pressure to give the title compound as a white solid.
¹H NMR (DMSO-D₆, 400MHz) δ: 1.25 (d, 3H), 3.30 (s, 3H), 3.85 (m, 1H), 4.00 (s, 3H), 4.40-4.50 (m, 2H), 6.20 (s, 1H), 7.50 (s, 1H).
MS APCI+ m/z 287 [MH]⁺

### Preparation 198

### Methyl 5-(aminocarbonyl)-1-[2-(cyclobulyloxy)ethyl]-4-nitro-1H-pyrazole-3-carboxylate

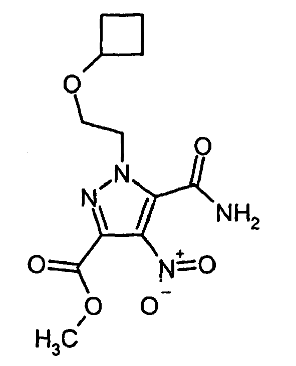

A solution of oxalyl chloride (6.71 mL, 76.7mmol) in dichloromethane (30mL) was added slowly to a solution of the acid from preparation 189 (20g, 63.9mmol) and N,N-dimethylformamide (0.28mL) in dichloromethane (140mL) with stirring and the mixture stirred at room temperature for 2 hours. The mixture was concentrated under reduced pressure and the residue azeotroped with dichloromethane (4x200mL) to give an orange oil that was dried *in vacuo.* The residue was dissolved in tetrahydrofuran (170mL), the solution cooled to -78°C and concentrated aqueous ammonia (23.2 mL, 0.42mol) was added dropwise. Once addition was complete, the reaction was stirred for a further 2 hours at -78°C. The reaction was quenched by the addition of excess 6N hydrochloric acid (17mL) at -78°C. The mixture was allowed to warm to room temperature and the tetrahydrofuran was removed under reduced pressure. The resulting aqueous suspension was filtered, and the resulting solid washed with saturated sodium bicarbonate solution (2x50mL). The solid was then washed with water until the filtrate was neutral, then dried *in vacuo.* The solid was stirred for 1 hour in a solution of ether:methanol (10:1 by volume, at 5mL/g solid), then filtered and dried. The solid was then stirred in a solution of ether:methanol (5:1 by volume, 5mL/g solid), filtered and dried *in vacuo* to afford the title compound, 10.34g.
1H NMR (CDCl₃, 400 MHz) δ: 1.41-1.82 (m, 4H), 2.17 (m, 2H), 3.74 (t, 2H), 3.86 (m, 1H), 3.97 (s, 3H), 4.60 (t, 2H), 6.06 (br s, 1H), 7.54 (br s, 1H).
MS ES+ m/z 330 [MNH₄]⁺

### Preparation 199

### Methyl 5-(aminocarbonyl)-4-nitro-1-[2-(2,2,2-trifluoroethoxy)ethyl]-1H-pyrazole-3-carboxylate

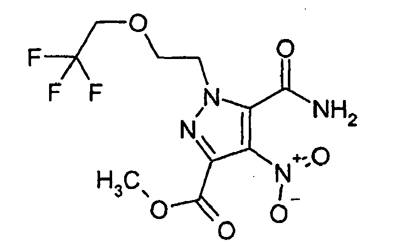

The carboxylic acid from preparation 192 (70.0g, 204mmol) was dissolved in a mixture of dichloromethane (1000mL) and N,N-dimethylformamide (1mL) under nitrogen at 20°C. Oxalyl chloride (25mL, 366mmol) was added dropwise with stirring. The mixture was stirred for 16 hours then concentrated under reduced pressure. Three portions of dichloromethane (200mL) were added and evaporated sequentially to remove excess oxalyl chloride. The residue was dissolved in tetrahydrofuran (1000mL) and cooled to -78°C. Concentrated aqueous 0.88 ammonia (70mL) was added dropwise maintaining the mixture at -78°C. After the addition was complete the mixture was stirred for 1 hour, and then an excess of hydrochloric acid was added at -78°C (to give pH1). The mixture was allowed to warm to room temperature and the solvent was removed under reduced pressure. The resulting cream-coloured solid was collected by filtration and washed with water (3 x 100mL) to give a colourless solid (47.01 g). Trituration of the solid with a mixture of diethyl ether and methanol (20:1, 20mL/g) gave the title compound as a colourless solid (40.0g, 61 %).
¹H NMR (CDCl₃, 400 MHz) 3.78 (q, 2H), 3.95 (s, 3H), 3.98 (t, 2H), 4.76 (t, 2H), 5.91 (br s, 1H), 7.03 (br s, 1H).

### Preparation 200

### Methyl 5-(aminocarbonyl)-1-[2-(2,2-difluoroethoxy)ethyl]-4-nitro-1H-pyrazole-3-carboxylate

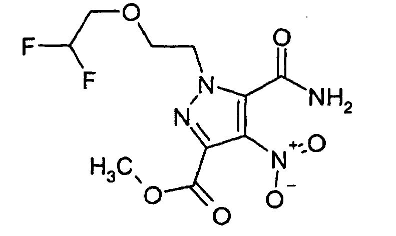

The title compound was obtained as a white solid from the compound from preparation 191, following the procedure described in preparation 199.
¹H NMR (DMSO-d₆, 400 MHz) 3.63 (m, 2H), 3.85 (m, 5H), 4.39 (t, 2H), 5.84-6.19 (m, 1H), 8.38 (s, 1H), 8.45 (s, 1H).
MS ES+ m/z 323 [MH]⁺

### Preparation 201

### Methyl 5-(aminocarbonyl)-4-nitro-1-[2-(3,3,3-trifluoropropoxy)ethyl]-1H-pyrazole-3-carboxylate

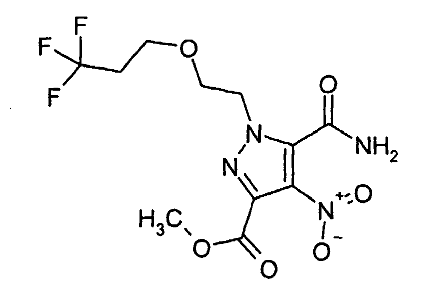

The title compound was obtained as a white solid from the acid from preparation 194, following a similar procedure to that described in preparation 199.
¹H NMR (DMSO-d₆, 400 MHz) 2.43 (m, 2H), 2.55 (m, 2H), 3.76 (t, 2H), 3.94 (s, 3H), 4.28 (m, 2H), 8.38 (m, 2H).
MS ES- m/z 353 [M-H]⁻

### Preparation 202

### Methyl 5-(aminocarbonyl)-1-[2-(3-fluoropropoxy)ethyl]-4-nitro-1H-pyrazole-3-carboxylate

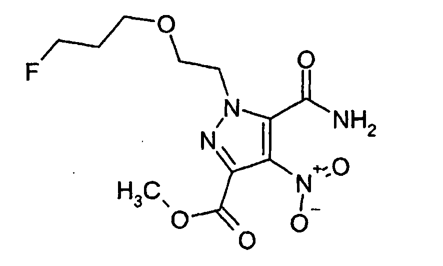

The title compound was obtained as a white solid from the acid from preparation 196, following a similar procedure to that described in preparation 199.
¹H NMR (CDCl_{3,} 400 MHz) δ: 1.83-1.99 (m, 2H), 3.58 (t, 2H), 3.84 (t, 2H), 3.98 (s, 3H), 4.40 (m, 1H), 4.54 (m, 1H), 4.70 (t, 2H).
MS APCI+ 319 [MH]⁺

### Preparation 203

### Methyl 4-amino-5-(aminocarbonyl)-1-[(2S)-2-methoxypropyl]-1H-pyrazole-3-carboxylate

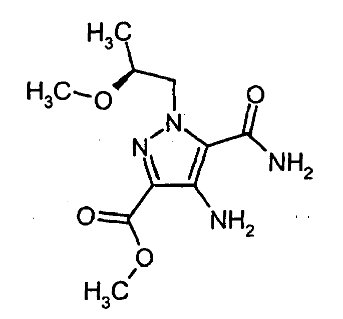

A solution of the compound from preparation 197 (7.1g, 25mmol) and palladium hydroxide (500mg) in methanol (200mL) was warmed to gentle reflux, and then ammonium formate (5.95g, 94mmol) added portionwise (care exotherm). Once the addition was complete the reaction was stirred under reflux for 18 hours under nitrogen. The cooled mixture was filtered through wet Arbocel®, and the filtrate evaporated under reduced pressure to give the title compound as a yellow oil, 5.4g. ¹H NMR (CDCl₃, 400 MHz) δ : 1.25 (d, 3H), 3.30 (s, 3H), 3.90 (m, 4H), 4.21-4.50 (m, 2H).
MS APCI+ m/z 279 [MNa]⁺

### Preparation 204

### Methyl 4-amino-5-(aminocarbonyl)-1-[2-(cyclobuyloxy)ethyl]-1H-pyrazole-3-carboxylate

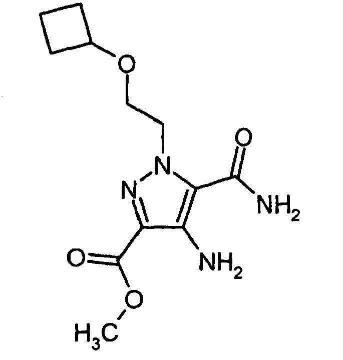

A solution of the compound from preparation 198 (10.34g, 33mmol) in methanol (400mL) was hydrogenated over 10% palladium on charcoal (Degussa 101 type, 2.1g) at 50psi H₂ and 50°C for 5 hours. The solution was filtered through Arbocel® filter aid. The filtrate was concentrated under reduced pressure, to afford the title compound as a colourless liquid, 9.32g.
¹H NMR (CDCl₃, 400MHz) δ : 1.39-1.52 (m, 1H), 1.60-1.80 (m, 3H), 2.12 (m, 2H), 3.80 (t, 2H), 3.90 (m, 4H), 4.32-4.70 (m, 2H).
MS ES+ m/z 305 [MNa]⁺

### Preparation 205

### Methyl 4-amino-5-(aminocarbony)-1-[2-(2,2-difluoroethoxy)ethyl]-1H-pyrazole-3-carboxylate

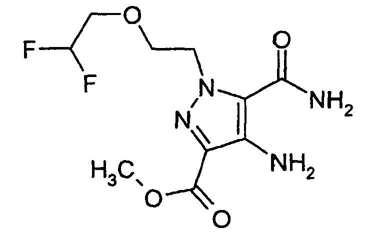

A mixture of the compound from preparation 200 (4.83g, 15mmol) and 10% palladium on charcoal (1.2g) in methanol (250mL) was hydrogenated at 3 Bar of hydrogen and room temperature for 24 hours. The mixture was warmed to 50°C, filtered through Arbocel®, washing through with warm methanol (500mL). The filtrate was concentrated under reduced pressure, and the residue azeotroped with acetonitrile to afford the title compound as a white solid, 3.8g.
¹H NMR (CDCl₃, 400MHz) δ: 3.68 (m, 2H), 3.91 (s, 3H), 4.03 (t, 2H), 4.61 (t, 2H), 5.61-5.96 (m, 1H), 6.20-6.39 (br s, 2H).
MS ES+ m/z 293 [MH]⁺

### Preparation 206

### Methyl 4-amino-5-(aminocarbonyl)-1-[2-(2,2,2-trifluoroethoxy)ethyl]-1H-pyrazole-3-carboxylate

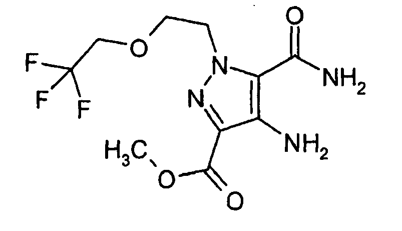

A solution of the compound from preparation 199 (40.0g, 118mmol) in methanol (640mL) was hydrogenated over 10% palladium on charcoal (10.0g) at 3 bar and 50°C for 3 hours. The hot solution was filtered through Arbocel® filter aid and the filter cake was washed with dichloromethane. The filtrate was concentrated under reduced pressure. The residue was kept under vacuum overnight at room temperature to provide the title product as an off-white solid, (34.2g, 94%).
¹H NMR (CDCl₃, 400MHz) δ: 3.80 (q, 2H), 3.91 (s, 3H), 4.07 (t, 2H), 4.63 (t, 2H), 6.29 (br s, 2H).

### Preparation 207

### Methyl 4-amino-5-(aminocarbonyl)1-[2-(3,3,3-trifluoropropoxy)ethyl]-1H-pyrazole-3-carboxylate

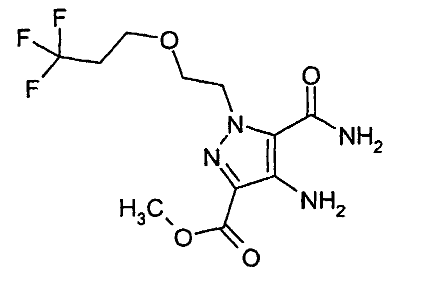

The title compound was obtained as an off-white solid, from the compound from preparation 201 following a similar procedure to that described in preparation 205. ¹H NMR (DMSO-d₆, 400MHz) δ: 2.41 (m, 2H), 3.52 (t, 2H), 3.68 (t, 2H), 3.74 (s, 3H), 4.49 (t, 2H), 5.09 (s, 2H), 7.40 (s, 2H).
MS APCI+ m/z 325 [MH]⁺

### Preparation 208

### Methyl 4-amino-5-(aminocarbonyl)-1-[2-(3-fluoropropoxy)ethyl]-1H-pyrazole-3-carboxylate

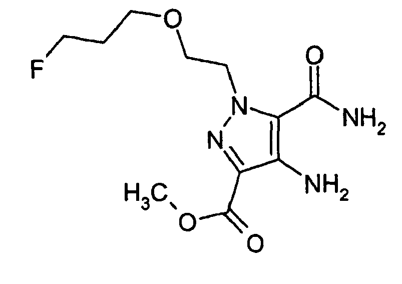

The title compound was prepared in quantitative yield from the compound from preparation 202, following the procedure described in preparation 206.
¹H NMR (CDCl₃, 400 MHz) δ: 1.83-1.99 (m, 2H), 3.61 (t, 2H), 3.95 (m, 5H), 4.38 (m, 1H), 4.50 (m, 1H), 4.58 (m, 2H).

### Preparation 209

### Methyl 1-[(2S)-2-methoxyoropyl]-5,7-dioxo-4,5,6,7-tetrahydro-1H-pyrazolo[4,3-d]pyrimidine-3-carboxylate

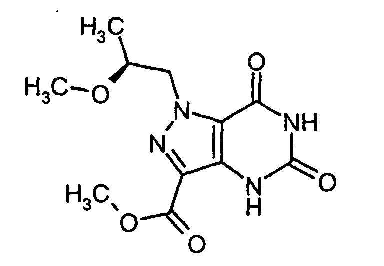

A mixture of the amine from preparation 203 (2.7g, 9.7mmol) and 1,1'-carbonyldiimidazole (1.89g, 11.7mmol) in N,N-dimethylformamide (80mL) was stirred at room temperature for 1 hour. The mixture was concentrated under reduced pressure and the residue dissolved in acetone. The mixture was sonicated for 30 minutes, and the resulting precipitate filtered off and dried. The filtrate was sonicated again, the precipitate was filtered, dried and combined to afford the title compound, 740mg.
¹H NMR (DMSO-d₆, 400MHz) δ: 1.05 (m, 3H), 3.15 (s, 3H), 3.75-3.85 (m, 1H), 3.88 (s, 3H), 4.40, 4.60 (2xm, 2H).
MS APCI+ 305 [MNa]⁺

### Preparation 210

### Methyl 1-[2-(cyclobutyloxy)ethyl]-5,7-dioxo-4,5,6,7-tetrahydro-1H-pyrazolo[4,3-d]pyrimidine-3-carboxylate

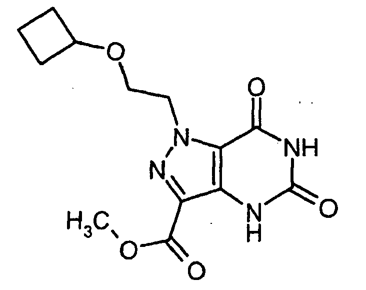

A solution of the amide from preparation 204 (9.32g, 33mmol) in acetonitrile (70mL) was added dropwise to a refluxing solution of 1,1'-carbonyldiimidazole (13.38g, 82.5mmol) in acetonitrile (230mL). The reaction was then stirred for a further 18 hours under reflux, and then cooled to 0°C. The resulting yellow precipitate was filtered off, washed with ice-cold acetonitrile and dried *in vacuo* to afford the title compound, 7.28g.
¹H NMR (CDCl₃, 400MHz) δ: 1.26-1.40 (m, 1H), 1.54 (m, 1H), 1.63 (m, 2H), 2.01 (m, 1H), 3.63 (t, 2H), 3.81 (m, 4H), 4.59 (t, 2H), 11.78 (br s, 1H), 11.38 (br s, 1H).
MS ES⁻ m/z 307 [M-H]⁻

### Preparations 211

### Methyl 1-[2-(2,2-difluoroethoxy)ethyl]-5,7-dioxo-4,5,6,7-tetrahydro-1H-pyrazolo[4,3-d]pyrimidine-3-carboxylate

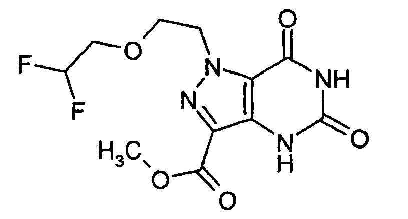

A solution of 1,1'-carbonyldiimidazole (3.16g, 19.5mmol) in acetonitrile (60mL) was added portionwise over 3 hours to a solution of the compound from preparation 205 (3.8g, 13.0mmol) in acetonitrile (150mL) stirring under reflux. The reaction was then stirred under reflux for a further 3 hours and allowd to cool. The reaction mixture was concentrated under reduced pressure and the residue triturated with water, the resulting solid filtered off, washed with water and dried *in vacuo* to afford the title compound as a pale grey solid, 3.17g.
¹H NMR (DMSO-d₆, 400MHz) δ: 3.61 (m, 2H), 3.79 (s, 3H), 3.90 (t, 2H), 3.64 (t, 2H), 5.99 (m, 1H), 10.78 (bs, 1H), 11.35 (bs, 1H).
MS ES+ m/z 318 [MH]⁺

### Preparation 212

### Methyl 5,7-dioxo-1-[2-(2,2,2-trifluoroethoxy)ethyl]-4,5,6,7-tetrahydro-1H-pyrazolo[4,3-d]pyrimidine-3-carboxylate

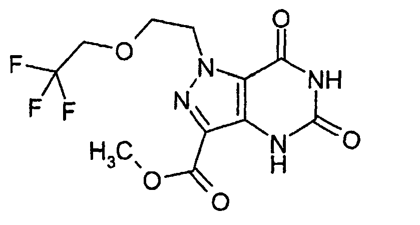

A solution of the amine from preparation 206 (21.7g, 70.0mmol) in acetonitrile (150mL) was added dropwise over 2 hours to a stirred solution of 1,1'-carbonyldiimidazole (17.02g, 105mmol) in refluxing acetonitrile (850mL) under nitrogen. The mixture was heated under reflux for 2 hours, cooled and the solvent was removed under reduced pressure. The residue was treated with water (150mL). The resulting pale grey solid was filtered off, washed with water (3 x 100mL), and dried *in vacuo* at 80°C to provide the title compound, 21.26g.
¹H NMR (CDCl₃, 400MHz) δ: 3.79 (q, 2H), 3.98 (s, 3H), 4.07 (t, 2H), 4.77 (t, 2H), 7.87 (br s, 1H), 8.41 (br s, 1H).
MS ES- m/z 335 [M-H]⁻

### Preparation 213

### Methyl 5,7-dioxo-1-[2-(3,3,3-trifluoropropoxy)ethyl]-4,5,6,7-tetrahydro-1H-pyrazolo[4,3,d]pyrimidine-3-carboxylate

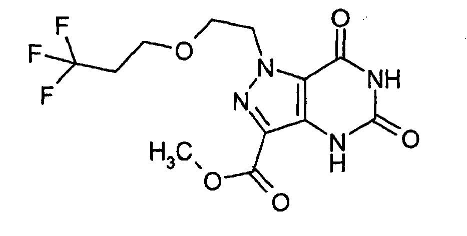

The title compound was obtained as a pale yellow solid from the compound from preparation 207 and 1,1'-carbonyldiimidazole, following a similar procedure to that described in preparation 212.
¹H NMR (CDCl₃, 400MHz) δ: 2.26 (m, 2H), 3.61 (t, 2H), 3.88 (t, 2H), 3.98 (s, 3H), 4.75 (t, 2H), 8.05 (s, 1H), 8.49 (s, 1H).
MS m/z 351 [MH]⁺

### Preparation 214

### Methyl 5,7-dioxo-1-[2-(3-fluoropropoxy)ethyl]-4,5,6,7-tetrahydro-1H-pyrazolo[4,3-d]pyrimidine-3-carboxylate

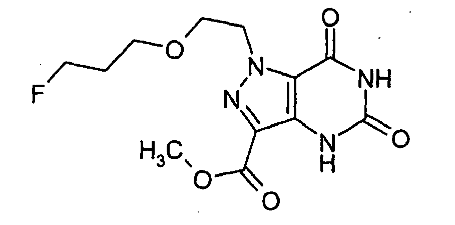

A solution of the amine from preparation 208 (2.3g, 8.0mmol) in acetonitrile (35mL) was added dropwise to a stirred solution of 1,1'-carbonyldiimidazole (2.0g, 12.3mmol) in refluxing acetonitrile (35mL) under nitrogen. The mixture was then heated under reflux for 2 hours, and cooled to room temperature. The resulting solid was filtered off, washed with acetonitrile and the filtrate evaporated under reduced pressure. The residue was triturated with water, the solid filtered off and the two isolated solids combined and dried *in vacuo* to afford the title compound, 2.3g.
¹H NMR (DMSO-D₆, 400 MHz) δ: 1.70-1.92 (m, 2H), 3.42 (t, 2H), 3.79 (t, 2H), 3.83 (s, 3H), 4.27 (dd, 1H), 4.40 (dd, 1H), 4.65 (m, 2H).
MS APCI+ m/z 315 [MH]⁺

### Preparation 215

### Methyl 5.7-dichloro-1-[(2S)-2-methoxypropyl]-1H-pyrazolo[4,3-d]pyrimidine-3-carboxylate

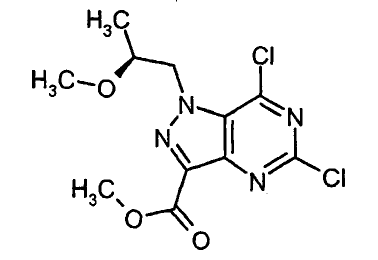

Phosphorous oxychloride (3.46mL, 37.2mmol) was added to a solution of the compound from preparation 209 (700mg, 2.48mmol) and tetraethylammonium chloride hydrate (616mg, 3.72mmol) in acetonitrile (8mL) and the reaction mixture heated under reflux for 24 hours. The cooled mixture was concentrated under reduced pressure and the residue azeotroped with toluene (3x) to provide the title compound as a white solid.
¹H NMR (CDCl₃, 400MHz) δ: 1.30 (d, 3H), 3.15 (s, 3H), 3.90 (m, 1H), 4.10 (s, 3H), 4.68 (dd, 1H), 4.98 (dd, 1H).

### Preparation 216

### Methyl 5,7-dichloro-1-[2-(cyclobutyloxy)ethyl]-1H-pyrazolo[4,3-d]pyrimidine-3-carboxylate

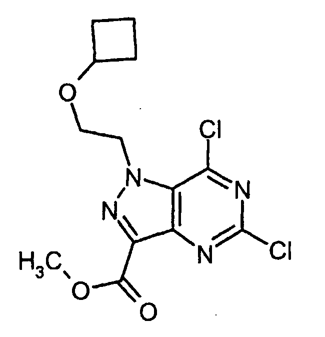

N,N-Diisopropylethylamine (3.4mL, 19.5mmol) was added dropwise to a solution of the compound from preparation 210 (2g, 6.5mmol), phosphorous oxychloride (9.04mL, 97.3mmol) and tetraethylammonium chloride (2.15g, 13.0mmol) in acetonitrile (25mL) and the reaction heated under reflux for 18 hours. Tic analysis showed starting material remaining, so additional phosphorous oxychloride (10mL, 107mmol) was added and the reaction heated under reflux for a further 24 hours. The cooled mixture was concentrated under reduced pressure and the residue azeotroped with toluene (2x100mL). The product was dissolved in dichloromethane (500mL), washed with water (3x200mL), dried over magnesium sulphate and evaporated under reduced pressure. The crude product was purified by column chromatography using an Isolute® silica gel cartridge and an elution gradient of ethyl acetate:pentane (20:80 to 100:0) to provide the title compound as a white solid, 1.0g.
¹H NMR (CDCl₃, 400MHz) δ: 1.40 (m, 1H), 1.55-1.75 (m, 3H), 2.10 (m, 2H), 3.80 (m, 3H), 4.10 (s, 3H), 5.00 (t, 2H).

### Preparation 217

### Methyl 5,7-dichloro-1-[2-(2,2-difluoroethoxy)ethyl]-1H-pyrazolo[4,3-d]pyrimidine-3-carboxylate

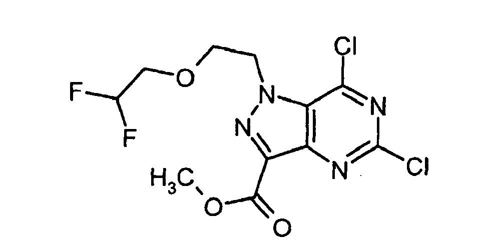

Phosphorous oxychloride (14mL, 148mmol) was added portionwise to a solution of the compound from preparation 211 (3.13g, 9.84mmol) and tetraethylammonium chloride (4.08g, 2.46mmol) in propionitrile (50mL) and the reaction then stirred under reflux for 18 hours. The cooled mixture was concentrated under reduced pressure and the residue azeotroped with toluene (2x). The residual solid was triturated with pentane:ether (40mL:10mL), and the resulting solid filtered off. This was pre-adsorbed onto silica gel and purified by column chromatography on silica gel using ethyl acetate:pentane (34:66) to afford the title compound as a white solid, 2.69g.
¹H NMR (CDCl₃, 400MHz) δ: 3.55 (m, 2H), 4.03 (t, 2H), 4.06 (s, 3H), 5.00 (t, 2H), 5.66 (m, 1H).
Microanalysis found: C, 37.14; H, 2.85; N, 15.68. C₁₁H₁₀Cl₂F₂N₄O₃ requires C, 37.20; H, 2.84; N, 15.78%.

### Preparation 218

### Methyl 5,7-dichloro-1-[2-(2,2,2-trifluoroethoxy)ethyl]-1H-pyrazolo[4,3-d]pyrimidine-3-carboxylate

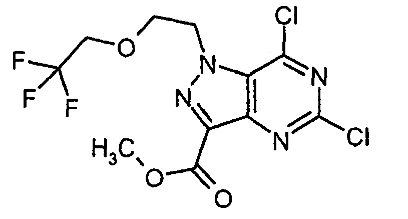

A mixture of the compound from preparation 212 (10g, 29.8mmol), phosphorous oxychloride (42mL, 447mmol) and tetraethylammonium chloride hydrate (14.8g, 89.4mmol) in propionitrile (125mL) was stirred under reflux for 8 hours. The cooled mixture was concentrated under reduced pressure and the residue azeotroped with toluene. The product was partitioned between dichloromethane (600mL) and water (500mL) and the layers separated. The aqueous solution was further extracted with dichloromethane (2x500mL) and the combined organic solutions washed with water (500mL) and brine (200mL), then dried over magnesium sulphate and concentrated under reduced pressure. The crude product was purified by column chromatography on silica gel using an elution gradient of ethyl acetate:pentane (33:67 to 50:50) to afford the title compound as a white solid, 5.4g.
¹H NMR (CDCl₃, 400MHz) δ: 3.75 (q, 2H), 4.10 (s, 3H), 4.15 (t, 2H), 5.05 (t, 2H).
MS APCI+ m/z 373 [M]⁺

### Preparation 219

### Methyl 5,7-dichloro-1-[2-(3,3,3-trifluoropropoxy)ethyl]-1H-pyrazolo[4,3-d]pyrimidine-3-carboxylate

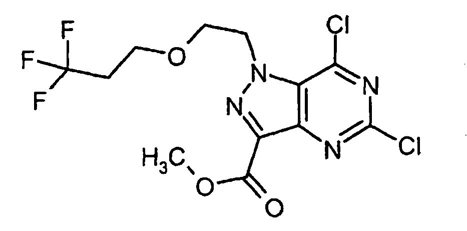

A mixture of the compound from preparation 213 (3.28g, 9.37mmol), phosphorous oxychloride (13.1mL, 140mmol) and tetraethylammonium chloride hydrate (3.88g, 23.4mmol) in propionitrile (50mL) was stirred under reflux for 18 hours. The cooled mixture was concentrated under reduced pressure and the residue azeotroped with toluene. The product was partitioned between dichloromethane (50mL) and water (50mL) and the layers separated. The aqueous solution was further extracted with dichloromethane (2x50mL) and the combined organic solutions dried over magnesium sulphate and concentrated under reduced pressure. The residue was triturated with pentane:ether, the resulting solid filtered off, washed with pentane and dried *in vacuo* to give the title compound as a solid, 3.2g
¹H NMR (CDCl₃, 400MHz) δ: 2.20 (m, 2H), 3.57 (t, 2H), 3.90 (t, 2H), 4.06 (s, 3H), 4.99 (t, 2H).
MS+ m/z 387 [MH]⁺

### Preparation 220

### Methyl 5,7-dichloro-1-[2-(3-fluoropropoxy)ethyl]-1H-pyrazolo[4,3-d]pyrimidine-3-carboxylate

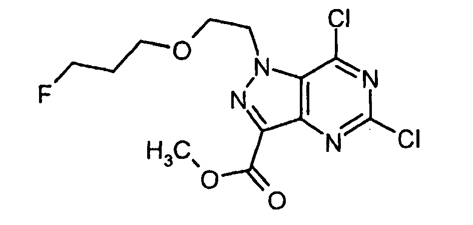

The title compound was obtained as a cream coloured solid in 86% yield, from the compound from preparation 214, following a similar procedure to that described in preparation 219.
¹H NMR (CDCl₃, 400 MHz) δ: 1.76-86 (m, 2H), 3.48 (t, 2H), 3.95 (t, 2H), 4.09 (s, 3H), 4.29 (dd, 1H), 4.42 (dd, 1H), 5.01 (t, 2H).
MS APCI+ m/z 351 [MH]⁺

### Preparation 221

### Methyl 5-chloro-1-[(2S)-2-methoxypropyl]-7-[(4-methylpyridin-2-yl)amino]-1H-pyrazolo[4,3-d]pyrimidine-3-carboxylate

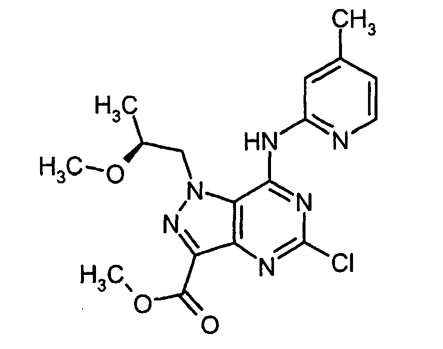

A solution of 2-amino-4-methylpyridine (850mg, 7.83mmol) in dimethylsulphoxide (7mL) was warmed to 30°C, and the dichloro compound from preparation 215 (500mg, 1.56mmol) added. The reaction was stirred for a further 2 hours at 30°C and then cooled to room temperature. The reaction mixture was poured into water (100mL) and extracted with dichloromethane (2x200mL). The combined organic solutions were washed with water (200mL), 1 M citric acid solution (100mL) then dried over magnesium sulphate and concentrated under reduced pressure. The product was triturated with ether, the solid filtered and dried to afford the title compound as yellow crystals, 200mg.
¹H NMR (DMSO-D_{6,} 400MHz) δ: 1.18 (d, 3H), 2.40 (s, 3H), 3.25 (m, 1H), 3.30 (s, 3H), 3.90 (s, 3H), 4.85 (d, 2H), 7.00 (br s, 1H), 8.20 (br s, 1H).
MS APCI⁻ m/z 389 [MH]⁻

### Preparation 222

### Methyl 7-{[4-({[tert-butyl(dimethyl)silyl]oxy}methyl)pyridin-2-yl]amino}-5-chloro-1-(2-ethoxyethyl)-1H-pyrazolo[4,3-d]pyrimidine-3-carboxylate

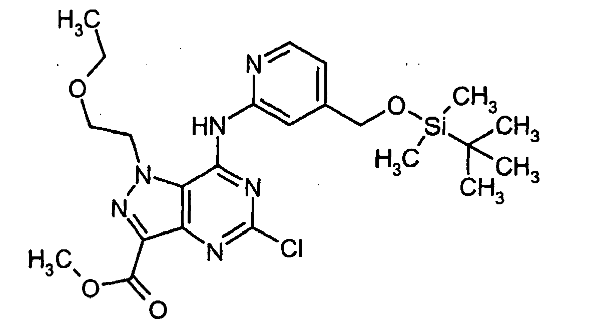

A mixture of the dichloro compound from preparation 58 (400mg, 1.25mmol) and 4-(*tert*-butyl-dimethyl-silanyloxymethyl)-pyridin-2-ylamine (WO 2001 017995, prep 8-5) (746mg, 3.13mmol) in dichloromethane (10mL) was stirred at room temperature for 18 hours. The mixture was partitioned between water (30mL) and dichloromethane (30mL), the layers separated and the organic phase dried over magnesium sulphate and evaporated under reduced pressure. The resulting yellow oil was purified by column chromatography on an Isolute® silica gel cartridge using an elution gradient of ethyl acetate:pentane (0:100 to 70:30) to afford the title compound, 229mg.
¹H NMR (MeOD-D₆, 400MHz) δ: 0.06 (s, 6H), 0.87 (s, 9H), 0.99 (t, 3H), 3.48 (q, 2H), 3.87 (m, 2H), 3.88 (s, 3H), 4.53 (s, 2H), 4.88 (m, 2H), 6.96 (m, 1H), 8.12 (m, 1H) MS ES+ m/z 521 [MH]⁺

### Preparation 223

### Methyl 5-chloro-1-(2-propoxyethyl)-7-(pyridin-2-ylamino)-1H-pyrazolo[4,3-d]pyrimidine-3-carboxylate

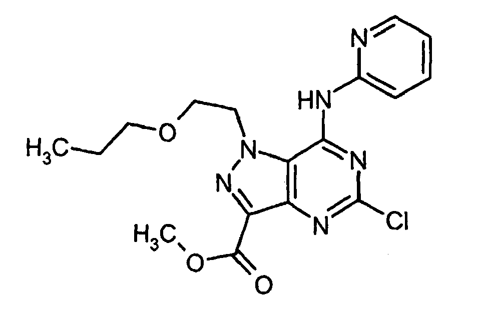

A mixture of the dichloro compound from preparation 57 (1.33g, 4mmol) and 2-aminopyridine (1.88g, 20mmol) in dichloromethane (16mL) was stirred at 35°C for 18 hours. The reaction was diluted with dichloromethane (200mL), the mixture washed with 1 M citric acid solution (2x 50mL), dried over magnesium sulphate and evaporated under reduced pressure to afford the title compound as a yellow solid, 1.48g.
¹H NMR (DMSO-D₆ + 1 drop TFA-d, 400MHz) δ: 0.80 (t, 3H), 1.38 (m, 2H), 3.37 (t, 2H), 3.85 (t, 2H), 3.88 (s, 3H), 4.94 (t, 2H), 7.20 (m, 1H), 8.01 (m, 1H), 8.10 (d, 1H), 8.38 (d, 1H).
MS APCI+ m/z 391 [MH]⁺

### Preparation 224

### Methyl 5-chloro-1-[2-(cyclobutyloxy)ethyl]-7-[(4-methylpyridin-2-yl)amino]-1H-prazolo[4,3-d]pyrimidine-3-carboxylate

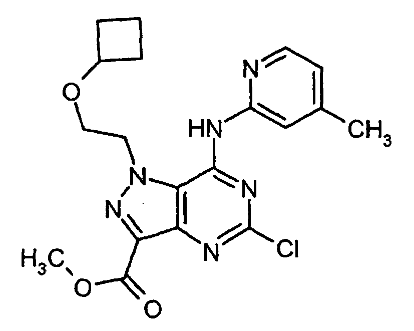

A mixture of the dichloro compound from preparation 216 (1.0g, 2.90mmol) and 2-amino-4-picoline (1.57g, 14.53mmol) in dichloromethane (12mL) were stirred at room temperature for 18 hours. The mixture was partitioned between dichloromethane (250mL) and 1 M citric acid solution (100mL) and the layers separated. The organic layer was washed again with 1 M citric acid solution (100mL), water (100mL), and brine (20mL) then dried over magnesium sulphate and evaporated under reduced pressure. The product was suspended in ether (50mL), the mixture sonicated, then filtered and the solid dried *in vacuo* to afford the title compound as a yellow solid, 618mg.
¹H NMR (DMSO-D₆+TFA-d, 400MHz) δ: 1.35 (m, 1H), 1.50 (m, 1H), 1.70 (m, 2H), 2.00 (m, 2H), 2.42 (s, 3H), 3.75 (t, 2H), 3.90 (m, 4H), 4.95 (t, 2H), 7.10 (d, 1H), 7.82 (s, 1H), 8.30 (d, 1H).
MS APCI+ m/z 417 [MH]⁺

### Preparation 225

### Methyl 5-chloro-1-[2-(2,2-difluoroethoxy)ethyl]-7-[(4-methylpyridin-2-yl)amino]-1H-pyrazolo[4,3-d]pyrimidine-3-carboxylate

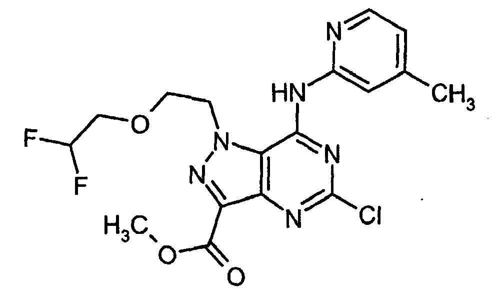

The title compound was prepared as a yellow solid n 56% yield from the chloro compound from preparation 217 and 2-amino-4-picoline (1.62g, 15mmol) following the procedure described in preparation 224.
¹H NMR (DMSO-D₆ + 1 drop TFA-d, 400MHz) δ: 2.40 (s, 3H), 3.68 (m, 2H), 3.88 (s, 3H), 4.00( t, 2H), 5.05 (t, 2H), 6.00 (m, 1H), 7.04 (d, 1H), 7.76 (s, 1H), 8.24 (d, 1H).

### Preparation 226

### Methyl 5-chloro-7-[(4-methylpyridin-2-yl)amino]-1-[2-(2,2,2-trifluoroethoxy)ethyl]-1H-pyrazolo[4,3-d]pyrimidine-3-carboxylate

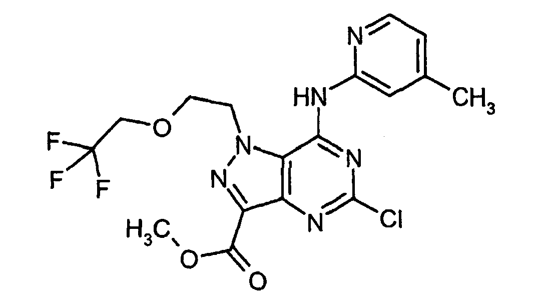

A mixture of the chloro compound from preparation 218 (5.6g, 14.9mmol) and 2-amino-4-picoline (4.85g, 44.8mmol) in acetonitrile (60mL) was stirred under reflux for 5 hours. The reaction mixture was cooled and diluted with 10% aqueous citric acid solution (33.6mL) and the mixture stirred for 10 minutes. The mixture was then cooled in ice for 30 minutes, the resulting precipitate filtered off, washed with ice-cold acetonitrile:water solution (50:50 by volume, 37mL) and ice-cold water (19mL). The solid was then dried *in vacuo* to afford the title compound, 5.05g.
¹H NMR (DMSO-D₆, 400MHz) δ: 2.38 (s, 3H), 3.81 (s, 3H), 4.00 (m, 4H), 5.02 (br s, 2H), 6.85 (br s, 1H), 7.64 (br s, 1H), 8.04 (br s, 1H).
MS ES+ m/z 445 [MH]⁺

### Preparation 227

### Methyl 5-chloro-7-[(3-methylphenyl)amino]-1-[2-(2,2,2-trifluoroethoxy)ethyl]-1H-pyrazolo[4,3-d]pyrimidine-3-carboxylate

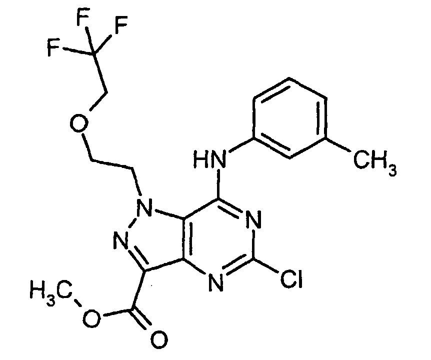

A mixture of the chloro compound from preparation 218 (746mg, 2mmol) and 3-methylaniline (650µL, 6mmol) in dimethylsulphoxide (8mL) was stirred at room temperature for 3 hours. The mixture was partitioned between dichloromethane (200mL) and water (50mL), and the layers separated. The organic phase was washed with 1 M hydrochloric acid (20mL) and water (2x50mL), then dried over magnesium sulphate and evaporated under reduced pressure to afford the title compound as a white solid, 880mg.
¹H NMR (CDCl₃, 400MHz) δ: 2.38 (s, 3H), 3.98 (q, 2H), 4.05 (s, 3H), 4.30 (t, 2H), 4.90 (t, 2H), 7.00 (d, 1H), 7.31 (m, 1H), 7.35 (s, 1H), 7.55 (d, 1H), 8.45 (s, 1H).
MS APCI+ m/z 444 [MH]⁺

### Preparation 228

### Methyl 5-chloro-7-[(4-fluoro-3-methylphenyl)amino]-1-[2-(2,2,2-trifluoroethoxy)ethyl]-1H-pyrazolo[4,3-d]pyrimidine-3-carboxylate

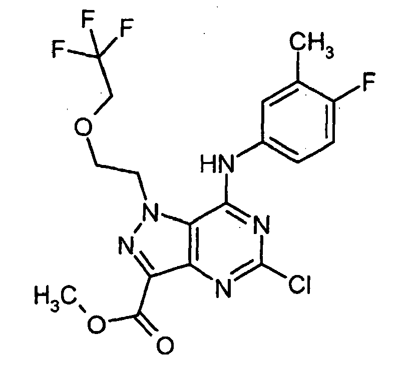

The title compound was obtained from the chloro compound from preparation 218 and 4-fluoro-3-methylamine, following the procedure described in preparation 227. ¹H NMR (CDCl₃, 400MHz) δ: 2.30 (s, 3H), 3.98 (q, 2H), 4.05 (s, 3H), 4.27 (t, 2H), 4.90 (s, 2H), 7.06 (m, 1H), 7.38 (m, 1H), 7.47 (m, 1H), 8.36 (s, 1H).
MS APCI+ m/z 462 [MH]⁺

### Preparation 229

### Methyl 5-chloro-7-[(4-methylpyridin-2-yl)amino]-1-[2-(3,3,3-trifluoropropoxy)ethyl]-1H-pyrazolo[4,3-d]pyrimidine-3-carboxylate

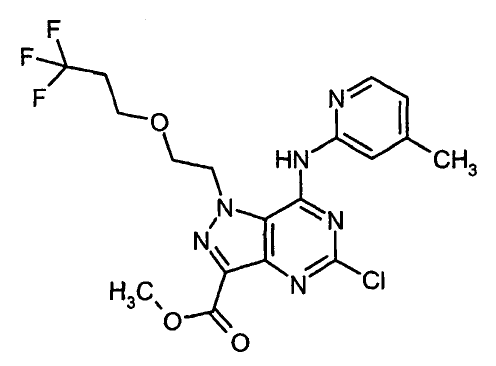

The title compound was obtained as a solid in 74% yield from the compound from preparation 219 and 2-amino-4-picoline, following the procedure described in preparation 223.
¹H NMR (DMSO-D₆+ 1 drop TFA-d, 400MHz) δ: 2.41 (s, 3H), 2.44 (t, 2H), 3.63 (t, 2H), 3.88 (s, 3H), 3.91 (t, 2H), 5.01 (t, 2H), 7.04 (d, 1H), 7.79 (s, 1H), 8.21 (d, 1H).
MS APCI+ m/z 459 [M]⁺

### Preparation 230

### Methyl 5-chloro-1-[2-(3-fluoropropoxy)ethyl]-7-[(4-methylpyridin-2-yl)amino]-1H-pyrazolo[4,3-d]pyrimidine-3-carboxylate

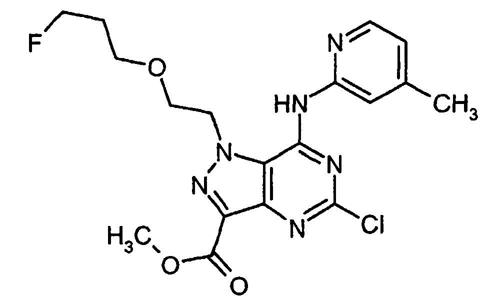

The title compound was obtained as a solid in 75% yield from the compound from preparation 220 and 2-amino-4-picoline, following the procedure described in preparation 223.
¹H NMR (DMSO-D₆+ 1 drop TFA-d, 400MHz) δ: 1.68-1.82 (m, 2H), 2.40 (s, 3H), 3.49 (t, 2H), 3.85-3.89 (m, 5H), 4.21-4.36 (m, 2H), 4.99 (t, 2H), 7.01 (d, 1H), 7.81 (s, 1H), 8.19 (d, 1H).
MS APCI+ m/z 423 [M]⁺

### Preparation 231

### 5-Chloro-1-(2-ethoxyethyl)-7-{[4-(hydroxymethyl)pyridin-2-yl]amino}-1H-pyrazolo[4,3-d]pyrimidine-3-carboxylic acid

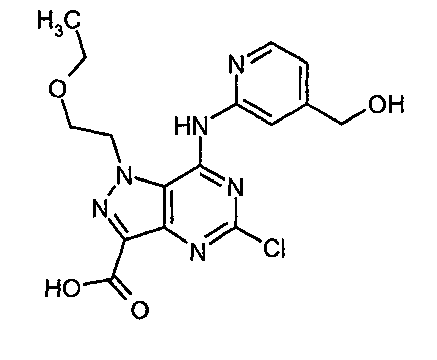

A solution of the compound from preparation 222 (229mg, 0.44mmol) in 1N sodium hydroxide solution (2.2mL) and dioxan (10mL) was stirred at room temperature for 72 hours. The mixture was acidified to pH 4 using 1 N hydrochloric acid and extracted with a solution of 10% methanol in dichloromethane. The combined organic extracts were dried over magnesium sulphate and evaporated under reduced pressure to afford the title compound as a yellow solid, 140mg.
¹H NMR (DMSO-D₆, 400MHz) δ: 1.07 (t, 3H), 3.55 (q, 2H), 3.93 (t, 2H), 4.65 (t, 2H), 4.99 (s, 2H), 5.60 (m, 1H), 7.10 (m, 1H), 8.29 (m, 1H).

### Preparation 232

### 5-Chloro-1-[2-(cyclobutyloxy)ethyl]-7-[(4-methylpyridin-2-yl)amino]-1H-pyrazolo[4,3-d]pyrimidine-3-carboxylic acid

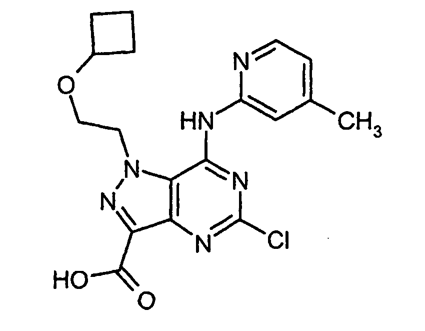

A solution of the ester from preparation 224 (600mg, 1.44mmol) in dioxane (5mL) and 1 N sodium hydroxide solution (5mL) was stirred at room temperature for 18 hours. The solution was concentrated under reduced pressure and diluted with 1 M citric acid solution (25mL). The resulting precipitate was filtered off, washed with ether and dried *in vacuo* at 50°C to afford the title compound as a yellow solid, 566mg.
¹H NMR (DMSO-D₆+TFA-d, 400MHz) δ: 1.35 (m, 1H), 1.55 (m, 1H), 1.75 (t, 2H), 2.05 (m, 2H), 2.40 (s, 3H), 3.79 (t, 2H), 3.95 (m, 1H), 4.90 (t, 2H), 6.98 (d, 1H), 7.85 (s, 1H), 8.20 (d, 1H).
MS APCI+ m/z 403 [MH]⁺

### Preparation 233

### 5-Chloro-7-[(4-methylpyridin-2-yl)amino]-1-[2-(2,2,2-trifluoroethoxy)ethyl]-1H-pyrazolo[4.3-d]pyrimidine-3-carboxylic acid

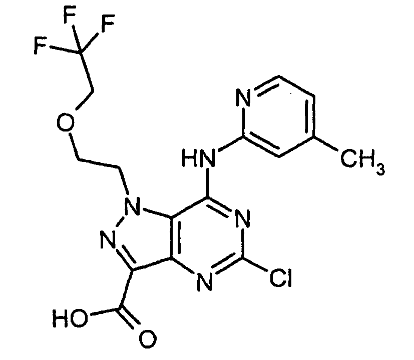

A mixture of the ester from preparation 226 (1.2g, 2.70mmol) and 1 M sodium hydroxide solution (4.1 mL, 4.1mmol) in dioxane (17.4mL) was stirred at room temperature for 18 hours. The reaction mixture was concentrated under reduced pressure and the residue dissolved in water (50mL). The solution was washed with dichloromethane (10mL), and then acidified using 1M citric acid. The resulting solid was filtered off and dried *in vacuo* to afford the title compound, 925mg.
¹H NMR (DMSO-D₆, 400MHz) δ: 2.50 (s, 3H), 3.32 (q, 2H), 4.07 (t, 2H), 5.06 (t, 2H), 6.93 (d, 1H), 7.73 (s, 1H), 8.13 (d, 1H).

### Preparation 234

### 3-({[tert-Butyl(dimethyl)silyl]oxy}methyl)-5-chloro-1-(2-ethoxyethyl)-N-(6-methylpyrimidin-4-yl)-1H-pyrazolo[4,3-d]pyrimidin-7-amine

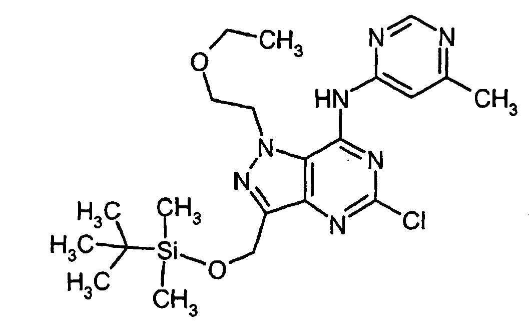

A solution of 4-amino-6-methylpyrimidine (1.13g, 10.4mmol) and sodium bis(trimethylsilyl)amide (3.80g, 20.74mmol) in tetrahydrofuran (40mL) was stirred at room temperature for 15 minutes. A solution of the dichloro compound from preparation 154 (3.5g, 8.64mmol) in tetrahydrofuran (50mL) was added and the reaction stirred at room temperature for 1.5 hours. The reaction mixture was concentrated under reduced pressure and the residue partitioned between dichloromethane and saturated ammonium chloride solution and the layers separated. The organic phase was dried over magnesium sulphate and evaporated under reduced pressure to give a red solid. The product was purified by column chromatography using an Isolute® silica gel cartridge and an elution gradient of methanol:dichloromethane (0:100 to 3:97) to provide the title compound as an orange solid, 3.7g.
¹H NMR (DMSO-D₆, 400MHz) δ: 0.02 (s, 6H), 0.79 (s, 9H), 1.06 (t, 3H), 2.44 (s, 3H), 3.53 (q, 2H), 3.82 (t, 2H), 4.71 (t, 2H), 4.89 (s, 2H), 8.19 (s, 1H), 8.59 (s, 1H).
MS ES+ m/z 478 [MH]⁺

### Preparation 235

### {5-Chloro-1-(2-ethoxyethyl)-7-[(6-methylpyrimidin-4-yl)amino]-1H-pyrazolo[4,3-d]pyrimidin-3-yl}methanol

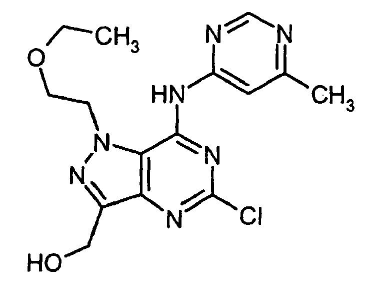

A mixture of the compound from preparation 234 (3.7g, 7.75mmol) and tetrabutylammonium fluoride (23.2mL, 1 M in tetrahydrofuran, 23.2mmol) in tetrahydrofuran (61 mL) was stirred at room temperature for 18 hours. The mixture was concentrated under reduced pressure and the residue partitioned between ethyl acetate (100mL) and water (100mL) and the layers separated. The aqueous solution was extracted with further ethyl acetate (2x50mL) and the combined organic solutions were concentrated under reduced pressure.

The residue was purified by column chromatography using an Isolute® silica gel cartridge and an elution gradient of methanol:dichloromethane (0:100 to 2:98) to provide the title compound, 2.6g.
¹H NMR (CD₃OD, 400MHz) δ: 1.19 (t, 3H), 2.57 (s, 3H), 3.66 (q, 2H), 3.96 (t, 2H), 4.84 (t, 2H), 4.90 (s, 2H), 8.33 (s, 1H), 8.72 (s, 1H).
MS ES+ m/z 364 [MH]⁺

### Preparation 236

### 5-Chloro-1-(2-ethoxyethyl)-7-[(6-methylpyrimidin-4-yl)amino]-1H-pyrazolo[4,3-d]pyrimidine-3-carbaldehyde

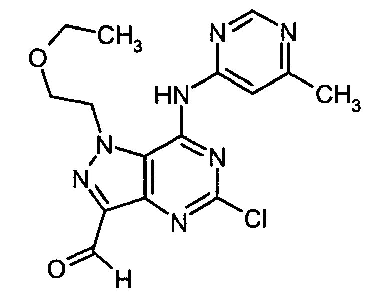

Dess-Martin periodinane (4.56g, 10.73mmol) was added portionwise to an ice-cooled solution of the alcohol from preparation 235 (2.6g, 7.15mmol) in dichloromethane (150mL) and the reaction then stirred at room temperature for a further 2 hours. A solution of sodium thiosulphate (7.5g, 30mmol) in water (75mL) was added dropwise, followed by saturated sodium bicarbonate solution (75mL) and then ether (75mL). The mixture was stirred for 15 minutes, and the layers separated. The aqueous solution was extracted with further dichloromethane (2x40mL) and the combined organic solutions dried over magnesium sulphate and evaporated under reduced pressure. The residual brown solid was purified by column chromatography using an Isolute® silica gel cartridge and an elution gradient of ethyl acetate:pentane (0:100 to 100:0) to provide the title compound as a solid, 1.66g.
¹H NMR (CDCl₃, 400MHz) δ: 1.25 (t, 3H), 2.63 (s, 3H), 3.72 (q, 2H), 4.06 (t, 2H), 4.91 (t, 2H), 8.29 (s, 1H), 8.81 (s, 1H), 10.34 (s, 1H), 10.42 (s, 1H).
MS ES+ m/z 362 [MH]⁺

### Preparation 237

### 5-Chloro-1-(2-ethoxyethyl)-7-[(6-methylpyrimidin-4-yl)amino]-1H-pyrazolo[4,3-d]pyrimidine-3-carboxylic acid

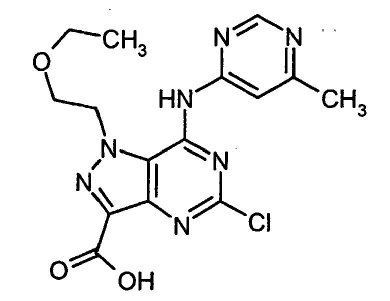

2-Methyl-2-butene (160mL, 0.32mol) was added to a solution of the aldehyde from preparation 236 (1.66g, 4.59mmol) in t-butanol (300mL). A solution of sodium chlorite (4.96g, 55.1mmol) and sodium dihydrogen phosphate (5.07g, 42.2mmol) in water (60mL) was added dropwise over 5 minutes, and the reaction then stirred at room temperature for 1 hour. The reaction mixture was diluted with dichloromethane (300mL) and water (150mL) and the layers separated. The aqueous layer was allowed to evaporate and the resulting precipitate was filtered off and dried *in vacuo* to give the title compound, 1.02g.
¹H NMR (400 MHz, DMSO-D₆) δ: 1.07 (t, 3H), 2.48 (s, 3H), 3.51 (m, 2H), 3.88 (t, 2H), 4.90 (t, 2H), 8.02 (br s, 1H), 8.78 (s, 1H).
MS APCI+ m/z 378 [MH]⁺

### Preparation 238

### 5-Chloro-1-(2-ethoxyethyl)-7-[(3-methylphenyl)amino]-1H-indazole-3-carboxylic acid

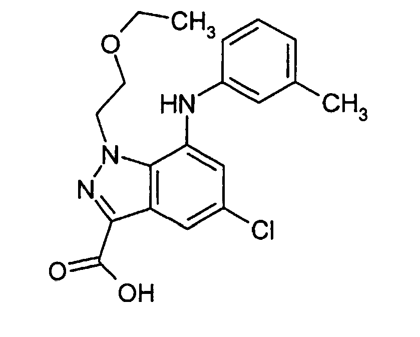

A mixture of the ester from preparation 81 (800mg, 2.06mmol) and 1 N sodium hydroxide solution (5mL, 5mmol) in dioxan (3mL) was stirred at room temperature for 18 hours. The reaction was concentrated under reduced pressure and the residue diluted with 1 M citric acid solution and the mixture sonicated. The resulting precipitate was filtered off, washed with water and ether and dried *in vacuo* to give the title compound as a white solid, 600mg.
¹H NMR (DMSO-D₆, 400MHz) δ: 1.00 (t, 3H), 2.35 (s, 3H), 3.50 (q, 2H), 3.82 (t, 2H), 4.95 (t, 2H), 7.01 (d, 1H), 7.35 (t, 1H), 7.41 (s, 1H), 7.50 (d, 1H), 9.39 (s, 1H).
MS APCI+ m/z 376 [MH]⁺

### Preparation 239

### 5-Chloro-1-(2-ethoxyethyl)-7-[(1-methyl-6-oxo-1,6-dihydropyridin-3-yl)amino]-1H-indazole-3-carboxylic acid

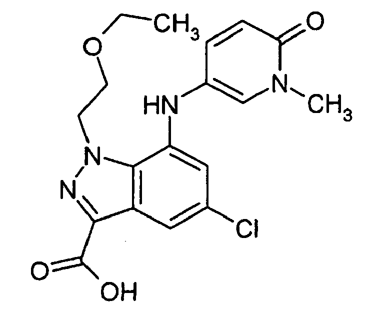

The compound from preparation 58 (10.85g, 34mmol) was added portionwise to a solution of 3-amino-1-methyl-1,6-dihydropyridin-6-one (EP 677519) (4.6g, 37mmol) and N-ethyldiisopropylamine (5.92mL, 34mmol) in dimethylsulphoxide (40mL), and the reaction then stirred at room temperature for 4 hours. The mixture was diluted with water (600mL), and the resulting solid filtered off, washed with water and dried *in vacuo,* 10.8g.

A portion of this solid (6.75g, 16.59mmol) was dissolved in dioxan (65mL) and the solution treated with 1 N sodium hydroxide (33mL, 1 M, 33mmol), and the reaction stirred at room temperature for 18 hours. The reaction was concentrated under reduced pressure, the residue dissolved in water (120mL), washed with dichloromethane (15mL), then acidified to pH 3 using solid citric acid. The resulting precipitate was filtered off, washed with water (3x20mL) and dried *in vacuo* to afford the title compound as a yellow solid, 6.19g.
¹H NMR (DMSO-D₆, 400MHz) δ: 0.95 (t, 3H), 3.40 (q, 2H), 3.47 (s, 3H), 3.79 (t, 2H), 4.92 (t, 2H), 6.49 (d, 1H), 7.58 (dd, 1H), 7.90 (s, 1H),
MS APCI+ m/z 376 [MS]⁺

### Preparation 240

### Benzyl cyclobutylcarbamate

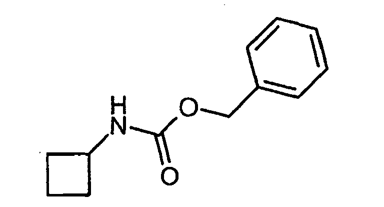

Benzyl chloroformate (5.2mL, 36.4mmol) was added dropwise to an ice-cold solution of cyclobutylamine (2g, 28.1 mmol) in dichloromethane (20mL), with stirring. Triethylamine (4.7mL, 33.7mmol) was added dropwise to the ice-cold solution, and once the addition was complete the reaction was allowed to warm to room temperature and stirred for 18 hours. The reaction was washed with saturated sodium bicarbonate solution (x2), dried over sodium sulphate and concentrated under reduced pressure. The residue was purified by column chromatography on silica gel using dichloromethane as eluant to afford the title compound, 3.72g.
¹H NMR (CDCl₃, 300MHz) δ: 1.68 (m, 2H), 1.82 (m, 2H), 2.35 (m, 2H), 4.19 (m, 1H), 4.92 (m, 1H); 5.14 (s, 2H), 7.25-7.39 (m, 5H).
MS TSP+ m/z 223.2 [MH]⁺

### Preparation 241

### N-Cyclobutyl-N-methylamine hydrochloride

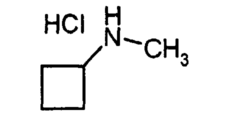

A solution of the compound from preparation 240 (500mg, 2.43mmol) was added dropwise to an ice-cold solution of lithium aluminium hydride (12.18mL, 1 M in tetrahydrofuran, 12.18mmol) in tetrahydrofuran (12mL), and the reaction mixture stirred at room temperature for 24 hours. The mixture was cooled to 0°C, water (0.46mL), followed by 15% sodium hydroxide solution (0.46mL) and finally further water (1.4mL) were added dropwise. The resulting precipitate was filtered off and washed with ether. The filtrate was washed with water and acidified to pH 2 using 1 M hydrochloric acid in ether. The solution was allowed to evaporate at room temperature, the residual oil dissolved in methanolic ether, dried over sodium sulphate and evaporated under reduced pressure to provide the title compound. ¹H NMR (CDCl₃. 400MHz) δ: 1.78-2.04 (m, 2H), 2.34 (m, 2H), 2.44 (m, 2H), 2.54 (s, 3H), 3.58 (m, 1H), 9.60 (br s, 2H).

### Preparations 242 to 244

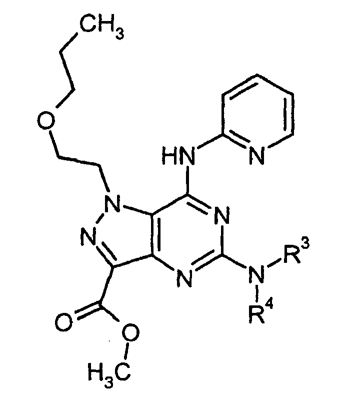

The appropriate amine (HNR³R⁴) (2mmol) and cesium fluoride (100mg, 0.67mmol) were added to a solution of the chloride from preparation 233 (260mg, 0.67mmol) in dimethylsulphoxide (2mL) in a Reactivial®. The reaction mixture was then sealed and heated at 120°C for 18 hours. The cooled solution was partitioned between dichloromethane (50mL) and water (50mL) and the layers separated. The organic phase was washed with water (50mL), dried over magnesium sulphate and evaporated under reduced pressure. The crude products were purified by column chromatography on silica gel using dichloromethane:methanol (98:2) as eluant to afford the title compounds.

| Prep No | -NR³R⁴ | Data |
|---|---|---|
| 242 | 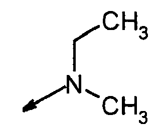 | ¹H NMR (DMSO-d₆ +TFAd, 400MHz) δ: 0.65 (t, 3H), 1.15 (t, 3H), 1.38 (m, 2H), 3.20 (s, 3H), 3.33 (t, 2H), 3.64 (q, 2H), 3.84 (t, 2H), 3.90 (s, 3H), 4.98 (t, 2H), 7.28 (m, 1H), 8.18 (m, 2H), 8.36 (d, 1H). MS APCI+ m/z 414 [MH]⁺ |
| 243 | 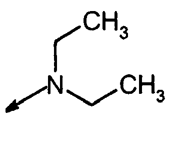 | ¹H NMR (DMSO-d₆ +TFAd, 400MHz) δ: 0.65 (t, 3H), 1.17 (t, 6H), 1.35 (m, 2H), 3.28 (t, 2H), 3.64 (m, 4H), 3.84 (t, 2H), 3.87 (s, 3H), 4.96 (t, 2H), 7.27 (m, 1H), 8.17 (m, 2H), 8.35 (m, 1H). MS APCI+ m/z 428 [MH]⁺ |
| 244 | 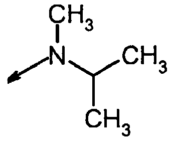 | ¹H NMR (DMSO-d₆ +TFAd, 400MHz) δ: 0.65 (t, 3H), 1.18 (d, 6H), 1.36 (m, 2H), 3.05 (s, 3H), 3.30 (t, 2H), 3.82 (t, 2H), 3.90 (s, 3H), 4.72 (m, 1H), 4.95 (t, 2H), 7.29 (m, 1H), 8.18 (m, 2H), 8.36 (d, 1H). MS APCI+ m/z 428 [MH]⁺ |

### Preparations 245 to 249

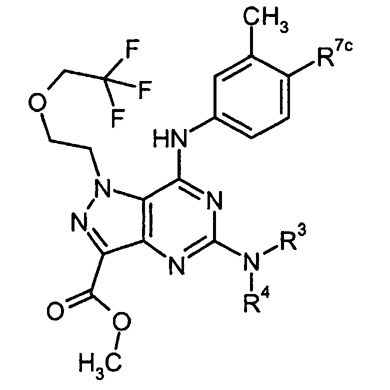

The appropriate amine (HNR³R⁴) (2mmol) was added to a solution of the chloride from preparations 227 or 228 (296mg, 0.67mmol) and cesium fluoride (101 mg, 0.67mmol) in dimethylsulphoxide (2.5mL) in a Reactivial®. The reaction mixture was then sealed and heated to 120°C for 12 hours. The cooled solution was partitioned between dichloromethane (200mL) and water (50mL) and the layers separated. The organic phase was washed with water (2x50mL), dried over magnesium sulphate and evaporated under reduced pressure, to afford the title compound.

| Prep No | -NR³R⁴ | R^{7c} | Yield/Data |
|---|---|---|---|
| 245 | 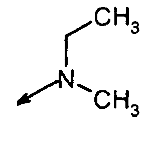 | H | 96% yellow gum. ¹H NMR (CD₃OD, 400MHz) δ: 1.17 (t, 3H), 2.37 (s, 3H), 3.16 (s, 3H), 3.66 (q, 2H), 3.94 (s, 3H), 4.05 (q, 2H), 4.18 (t, 2H), 4.87 (t, 2H), 6.93 (d, 1H), 7.24 (dd, 1H), 7.43 (d, 1H), 7.55 (s, 1H). MS APCI+ m/z 467 [MH]⁺ |
| 246 | 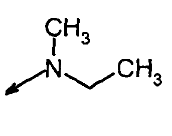 | F | Quantitative, Yellow gum ¹H NMR (CD₃OD, 400MHz) δ: 1.12 (t, 3H), 2.27 (s, 3H), 3.14 (s, 3H), 3.66 (q, 2H), 3.96 (s, 3H), 4.04 (q, 2H), 4.18 (t, 2H), 4.87 (t, 2H), 7.04 (t, 1H), 7.42 (m, 1H), 7.58 (m, 1H). MS APCI+ 485 [MH]⁺ |
| 247 | 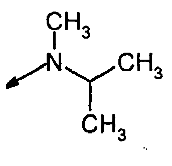 | H | 96% yellow gum ¹H NMR (CD₃OD, 400MHz) δ: 1.20 (d, 6H), 2.34 (s, 3H), 3.05 (s, 3H), 3.95 (s, 3H), 4.09 (q, 2H), 4.18 (t, 2H), 4.87 (t, 2H), 5.10 (m, 1H), 6.95 (d, 1H), 7.24 (dd, 1H), 7.40 (d, 1H), 7.55 (s, 1H). MS APCI+ m/z 481 [MH]⁺ |
| 248 | 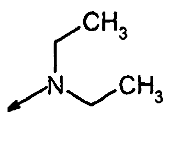 | F | 96% as a yellow gum ¹H NMR (CD₃OD, 400MHz) δ: 1.08 (t, 6H), 2.28 (s, 3H), 3.66 (q, 4H), 3.96 (s, 3H), 4.04 (q, 2H), 4.15 (t, 2H), 4.86 (t, 2H), 7.01 (m, 1H), 7.38 (m, 1H), 7.58 (s, 1H). MS APCI+ m/z 499 [MS]⁺ |
| 249 | 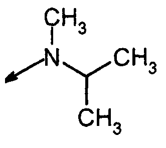 | F | 93% as a yellow gum ¹H NMR (CD₃OD, 400MHz) δ: 1.18 (d, 6H), 2.26 (s, 3H), 3.02 (s, 3H), 3.95 (s, 3H), 4.02 (q, 2H), 4.16 (t, 2H), 4.87 (t, 2H), 5.04 (m, 1H), 7.02 (t, 1H), 7.40 (m, 1H), 7.55 (m, 1H). MS APCI+ m/z 499 [MH]⁺ |

### Preparations 250 to 258

The compounds of the general formulae shown in the table below were prepared using the method described for preparations 245 to 249, from the compound from preparations 225, 229 and 230 and the appropriate HNR³R⁴ amines.

| | | |
|---|---|---|
| 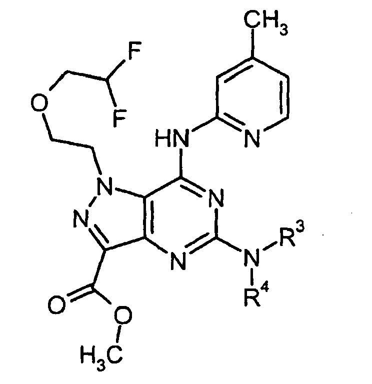 | | |

| Prep No | -NR³R⁴ | Data |
|---|---|---|
| 250 | 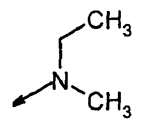 | ¹H NMR (CD₃OD, 400MHz) δ: 1.27 (t, 3H), 2.38 (s, 3H), 3.20 (s, 3H), 3.74 (m, 4H), 3.95 (s, 3H), 4.10 (t, 2H), 4.80 (m, 2H), 6.00 (m, 1H), 6.98 (m, 1H), 8.18 (m, 1H), 8.25 (m, 1H). MS APCI+ m/z 450 [MH]⁺ |
| 251 | 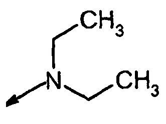 | ¹H NMR (CD₃OD, 400MHz) δ: 1.25 (t, 6H), 2.40 (s, 3H), 3.75 (m, 6H), 3.95 (s, 3H), 4.10 (t, 2H), 4.82 (m, 2H), 6.00 (m, 1H), 6.95 (m, 1H), 8.18 (m, 1H), 8.30 (m, 1H). MS APCI+ m/z 464 [MH]⁺ |
| 252 | 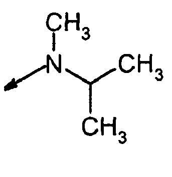 | ¹H NMR (CD₃OD, 400MHz) δ: 1.24 (d, 6H), 2.40 (s, 3H), 3.06 (s, 3H), 3.60 (m, 2H), 3.95 (s, 3H), 4.08 (t, 2H), 4.80 (m, 2H), 5.10 (m, 1H), 6.00 (m, 1H), 6.95 (m, 1H), 8.18 (m, 1H), 8.20 (m, 1H). MS APCI+ m/z 464 [MH]⁺ |
| 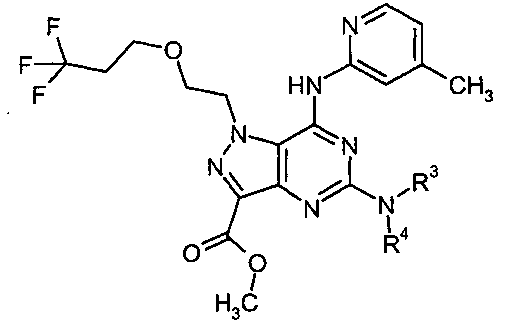 | | |
| 253 | 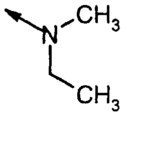 | ¹H NMR (DMSO-d₆ +TFAd, 400MHz) δ: 1.12 (t, 3H), 2.46 (s, 3H), 2.37-2.50 (m, 2H), 3.20 (s, 3H), 3.60 (t, 2H), 3.65 (m, 2H), 3.89-3.91 (m, 5H), 5.00 (t, 2H), 7.19 (d, 1H), 8.10 (s, 1H), 8.25 (d, 1H). MS APCI+ m/z 482 [MH]⁺ |
| 254 | 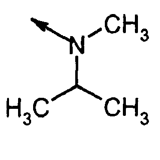 | ¹H NMR (DMSO-d₆ +TFAd, 400MHz) δ: 1.20 (d, 6H), 2.45 (s, 3H), 2.37-2.50 (m, 2H), 3.04 (s, 3H), 3.60 (t, 2H), 3.89-3.91 (m, 5H), 4.70-4.78 (m, 1H), 4.99 (t, 2H), 7.18 (d, 1H), 8.08 (s, 1H), 8.25 (d, 1H). MS APCI+ m/z 496 [MH]⁺ |
| 255 | 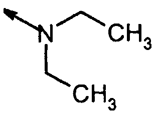 | ¹H NMR (DMSO-d₆ +TFAd, 400MHz) δ: 1.21 (t, 6H), 2.35-2.50 (m, 5H), 3.60 (t, 2H), 3.65 (q, 4H), 3.90 (s, 3H), 4.99 (t, 2H), 7.19 (d, 1H), 8.10 (s, 1H), 8.25 (d, 1H). MS APCI+ m/z 496 [MH]⁺ |
| 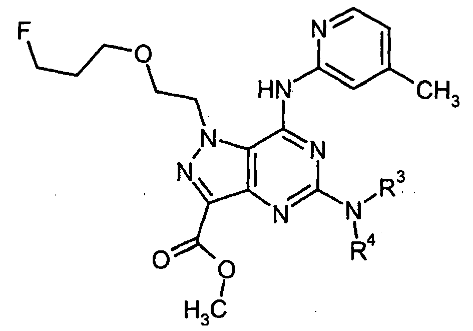 | | |
| 256 | 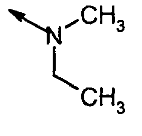 | ¹H NMR (DMSO-d₆ +TFAd, 400MHz) δ: 1.12 (t, 3H), 1.67-1.80 (m, 2H), 2.40 (s, 3H), 3.20 (s, 3H), 3.45 (t, 2H), 3.65 (q, 2H), 3.87 (t, 2H), 3.90 (s, 3H), 4.23-4.38 (m, 2H), 4.98 (t, 2H), 7.18 (d, 1H), 8.10 (s, 1H), 8.25 (d, 1H). MS APCI+ m/z 446 [MH]⁺ |
| 257 | 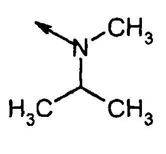 | ¹H NMR (DMSO-d₆ +TFAd, 400MHz) δ: 1.20 (d, 6H), 1.69-1.78 (m, 2H), 2.45 (s, 3H), 3.00 (s, 3H), 3.45 (t, 2H), 3.86 (t, 2H), 3.90 (s, 3H), 4.23-4.38 (m, 2H), 4.98 (t, 2H), 4.69-4.76 (m, 1H), 7.18 (d, 1H), 8.08 (s, 1H), 8.24 (d, 1H). MS APCI+ m/z 460 [MH]⁺ |
| 258 | 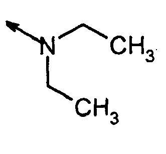 | ¹H NMR (DMSO-d₆ +TFAd, 400MHz) δ: 1.20 (t, 6H), 1.68-1.80 (m, 2H), 2.45 (s, 3H), 3.45 (t, 2H), 3.65 (q, 4H), 3.87 (t, 2H), 3.90 (s, 3H), 4.24-4.39 (m, 2H), 4.97 (t, 2H), 7.18 (d, 1H), 8.04 (s, 1H), 8.25 (d, 1H). MS APCI+ m/z 460 [MH]⁺ |

### Preparation 259

### Methyl 1-(2-ethoxyethyl)-7-[(4-methylpyridin-2-yl)amino]-5-pyrrolidin-1-yl-1H-pyrazolo[4,3-d]pyrimidine-3-carboxylate

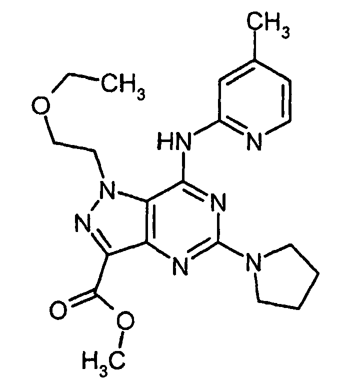

The title compound was obtained as a yellow oil, from the chloride from preparation 72 and pyrrolidine, following a similar procedure to that described in preparation 245-249, except 5 eq N-ethyldiisopropylamine was added to the reaction, and the product was purified by column chromatography using an Isolute® silica gel cartridge and dichloromethane:methanol:0.88 ammonia (100:0:0 to 95:5:0.5) as eluant.
¹H NMR (CD₃OD, 400MHz) δ: 1.05 (t, 3H), 2.02 (m, 4H), 2.40 (s, 3H), 3.60 (q, 2H), 3.65 (m, 4H), 3.90 (m, 5H), 4.80 (t, 2H), 6.95 (d, 1H), 8.18 (d, 1H), 8.50 (s, 1H).
MS APCI+ m/z 426 [MH]⁺

### Preparation 260

### Methyl 5-[isopropyl(methyl)amino]-1-[(2S)-2-methoxypropyl]-7-[(4-methylpyridin-2-yl)amino]-1H-pyrazolo[4,3-d]pyrimidine-3-carboxylate

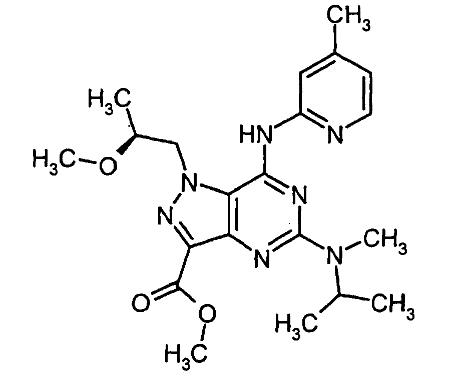

A mixture of the chloride from preparation 221 (110mg, 0.28mmol), N-ethyldiisopropylamine (0.25mL, 1.40mmol), N-methylisopropylamine (0.15mL, 1.40mmol) and tetraethylammonium fluoride (37mg, 0.28mmol) in 1-methyl-2-pyrrolidinone (1 mL) was heated in a Reactivial® at 120°C for 18 hours. The cooled mixture was concentrated under reduced pressure and the residue partitioned between dichloromethane (50mL) and water (50mL) and the layers separated. The aqueous phase was extracted with additional dichloromethane (50mL), and the combined organic solutions washed with water (100mL) dried over magnesium sulphate and evaporated under reduced pressure. The crude product was purified by column chromatography using an Isolute® silica gel cartridge using dichloromethane as eluant to provide the title compound as a yellow oil, 43mg.
¹H NMR (CD₃OD, 400MHz) δ: 1.21 (m, 6H), 1.28 (d, 3H), 2.39 (s, 3H), 3.03 (s, 3H), 3.40 (s, 3H), 3.98 (m, 4H), 4.50-4.70 (m, 2H), 5.17 (m, 1H), 6.92 (d, 1H), 8.18 (m, 2H).
MS APCI+ m/z 428 [MH]⁺

### Preparation 261

### 5-Chloro-7-[(4-methylpyridin-2-yl)amino]-N-(methylsulfonyl)-1-[2-(2,2,2-trifluoroethoxy)ethyl]-1H-pyrazolo[4,3-d]pyrimidine-3-carboxamide

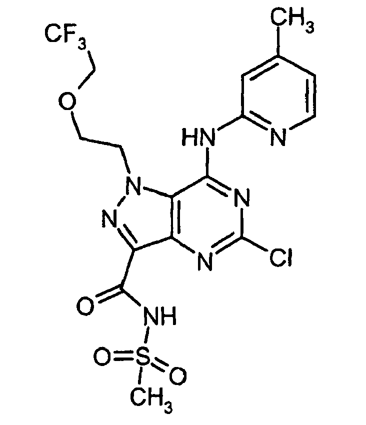

A mixture of the acid from preparation 233 (300mg, 0.70mmol), methanesulphonamide (87mg, 0.91mmol), 1-(3-dimethylaminopropyl)-3-ethylcarbodiimide hydrochloride (175mg, 0.91 mmol) and 4-dimethylamino pyridine (102mg, 0.91mmol) in N,N-dimethylformamide (3mL) was stirred at room temperature for 18 hours. Tlc analysis showed starting material remaining, so additional methanesulphonamide (43mg, 0.45mmol), 1-(3-dimethylaminopropyl)-3-ethylcarbodiimide hydrochloride (87mg, 0.45mmol) and 4-dimethylamino pyridine (51 mg, 0.45mmol) were added and the mixture stirred for a further 4 hours. The mixture was partitioned between dichloromethane (50mL) and water (50mL) and the layers separated. The organic solution was washed with 1 N hydrochloric acid (5mL) and water (3x50mL) then dried over sodium sulphate and evaporated under reduced pressure, to give the title compound, 100mg.
¹H NMR (CD₃OD, 400MHz) δ: 2.42 (s, 3H), 3.39 (s, 3H), 4.02 (q, 2H), 4.17 (t, 2H), 5.07 (t, 2H), 6.99 (d, 1H), 7.81 (s, 1H), 8.18 (d, 1H).
MS ES- m/z 506 [M-H]⁻

### Example 1

### Methyl 1-(2-ethoxyethyl)-5-(N-isopropyl-N-methylamino)-7-(4-methylpyridin-2-ylamino)-1H-pyrazolo[4,3-d]pyrimidine-3-carboxylate

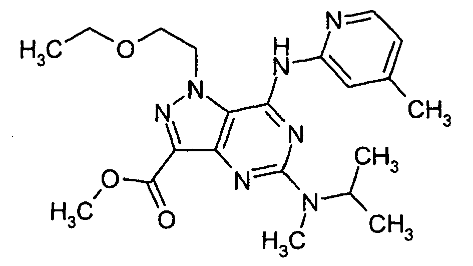

The chloro compound of preparation 72 (130mg, 0.33mmol) was dissolved in dimethyl sulphoxide (1 mL) and the solution treated with tetraethylammonium fluoride (50mg, 0.33mmol) and N-methylisopropylamine (104µL, 1.0mmol). The reaction mixture was stirred in a ReactiVial™ at 120°C for 18 hours before being allowed to cool and concentrated *in vacuo.* The residue was partitioned between ethyl acetate (50mL) and water (50mL) and the organic phase dried over magnesium sulphate and concentrated *in vacuo.* The residue was purified by column chromatography on silica gel eluting with dichloromethane:methanol 100:0 to 97:3 to yield the title product.
¹H NMR (CDCl₃, 400MHz) δ: 1.18 (t, 3H), 1.24 (s, 6H), 2.40 (m, 3H), 3.11 (s, 3H), 3.60 (q, 2H), 3.96 (t, 2H), 4.02 (s, 3H), 4.80 (t, 2H), 5.10 (m, 1H), 6.91 (d, 1H), 8.18 (m, 1H), 8.37 (d, 1H). MS APCI+ m/z 428 [MH]⁺

### Example 2

### Methyl 1-(2-ethoxyethyl)5-(N-ethyl-N-methylamino)-7-(4-methylpyridin-2-ylamino)-1H-pyrazolo[4,3-d]pyrimidine-3-carboxylate

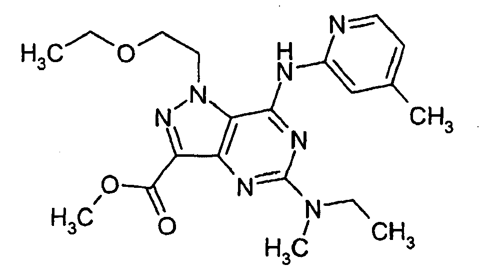

The chloro compound of preparation 72 (130mg, 0.33mmol) was dissolved in dimethyl sulphoxide (1mL) and the solution treated with tetraethylammonium fluoride (50mg, 0.33mmol) and N-methylethylamine (86µL, 1.0mmol). The reaction mixture was heated to 110°C in a ReactiVial™ for 18 hours and then allowed to cool to room temperature. The reaction mixture was partitioned between dichloromethane (50mL) and water (50mL) and the organic phase washed with water (2x30mL), dried over magnesium sulphate and concentrated *in vacuo.* The residue was purified by column chromatography on silica gel eluting with dichloromethane:methanol 100:0 to 90:10 to yield the title product.
¹H NMR (CD₃OD, 400MHz) δ: 1.10 (t, 3H), 1.25 (m, 3H), 2.40 (s, 3H), 3.25 (s, 3H), 3.60 (q, 2H), 3.78 (q, 2H), 3.86 (m, 5H), 4.80 (t, 2H), 6.93 (d, 1H), 8.15 (d, 1H), 8.32 (s, 1H). MS APCI+ m/z 414 [MH]⁺

The following compounds, of the general formula shown below, were prepared by a method similar to that described for example 2 using the appropriate HNR³R⁴ amine.

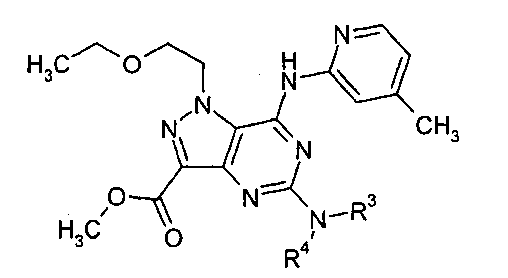

| No. | NR³R⁴ | Data |
|---|---|---|
| 3 | 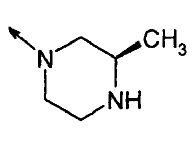 | ¹H NMR (CD₃OD, 400MHz) δ: 1.13 (t, 3H), 1.18 (d, 3H), 2.40 (s, 3H), 2.64 (m, 1H), 2.84 (m, 2H), 3.03 (m, 2H), 3.42 (q, 2H), 3.94 (m, 5H), 4.64 (m, 2H), 4.80 (t, 2H), 6.94 (d, 1H), 8.18 (m, 2H). MS APCI+ m/z 455 [MH]⁺ |
| 4 | 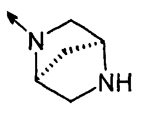 | ¹H NMR (CD₃OD, 400MHz) δ: 1.08 (t, 3H), 1.84 (d, 1H), 1.96 (d, 1H), 2.40 (s, 3H), 3.08 (m, 2H), 3.60 (m, 3H), 3.73 (m, 1H), 3.85 (m, 1H), 3.92 (m, 5H), 4.82 (m, 2H), 4.97 (m, 1H), 6.95 (d, 1H), 8.16 (d, 1H), 8.30 (m, 1H). MS APCI+ m/z 453 [MH]⁺ |
| 5 | -N(CH₃)₂ | ¹H NMR (CD₃OD, 400MHz) δ: 1.10 (t, 3H), 2.38 (s, 3H), 3.24 (s, 6H), 3.40 (q, 2H), 3.83 (m, 5H), 4.77 (m, 2H), 6.93 (d, 1H), 8.15 (d, 1H), 8.34 (s, 1H). MS APCI+ m/z 400 [MH]⁺ |

- Example 4 was prepared using *tert*-butyl (1*S*,4*S*)-2,5-diaza-bicyclo[2.2.1]heptane-2-carboxylate (Aldrich Chem.) as the HNR³R⁴ amine. Prior to being purified by column chromatography, the crude product was dissolved in dichloromethane (5mL) and the solution treated with trifluoroacetic acid (5mL) at room temperature for 4 hours. The reaction mixture was concentrated *in vacuo* and the residue partitioned between dichloromethane (50mL) and saturated sodium hydrogencarbonate solution (50mL). The organic phase was separated, dried over magnesium sulphate and concentrated *in vacuo.*

### Example 6

### Methyl 1-(2-ethoxyethyl)-5-(N-isopropyl-N-methylamino)-7-(6-methylpyridin-2-ylamino)-1H-pyrazolo[4,3-d]pvrimidine-3-carboxylate

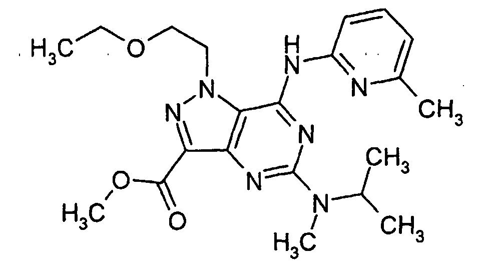

The chloro compound of preparation 93 (200mg, 0.51 mmol) was added to a solution of N-ethyldiisopropylamine (440µL, 2.55mmol), isopropylmethylamine (260µL, 2.55mmol) and caesium fluoride (77mg, 0.51 mmol) in dimethyl sulphoxide (1mL) and the reaction mixture heated to 120°C in a ReactiVial™ for 18 hours. The reaction mixture was concentrated *in vacuo* and the residue taken up in 1 M citric acid solution (5mL) and extracted with dichloromethane (3x25mL). The organics were combined, dried over magnesium sulphate and concentrated *in vacuo* to yield the title product.
¹H NMR (CDCl₃, 400MHz) δ: 1.16 (t, 3H), 1.24 (d, 6H), 2.37 (s, 3H), 3.11 (s, 3H), 3.62 (q, 2H), 3.98 (t, 2H), 4.01 (s, 3H), 4.77 (m, 2H), 5.15 (m, 1H), 6.82 (d, 1H), 8.18 (d, 1H), 8.26 (s, 1H), 9.76 (s, 1H).

### Example 7

### Ethyl 1-(2-ethoxyethyl)-5-(N-ethyl-N-methylamino)-7-(4-methylpyridin-2-ylamino)-1H-pyrazolo[4,3-d]pyrimidine-3-carboxylate

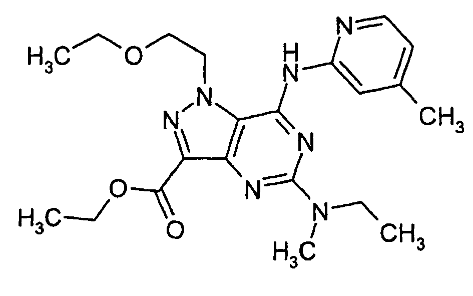

The ethyl ester of preparation 175 (100mg, 0.25mmol) was dissolved in dimethyl sulphoxide (1 mL) and the solution treated with N-methyl-ethylamine (78µL, 0.75mmol) and tetraethylammonium fluoride (37mg, 0.25mmol). The reaction mixture was then heated to 120°C in a ReactiVia™ for 18 hours before being allowed to cool. The reaction mixture was concentrated *in vacuo,* the residue partitioned between ethyl acetate (50mL) and water (50mL) and the organic phase dried over magnesium sulphate and concentrated *in vacuo.* The residue was purified by column chromatography on silica gel eluting with dichloromethane:acetonitrile 100:0 to 90:10. The crude product was partitioned between dichloromethane (30mL) and saturated sodium hydrogencarbonate solution (10mL). The organic phase was separated, dried over magnesium sulphate and concentrated *in vacuo.* The residue was purified by column chromatography on silica gel eluting with dichloromethane:methanol:0.88 ammonia 95:5:0.5 to yield the title product.
¹H NMR (CDCl₃, 400MHz) δ: 1.14 (t, 3H), 1.24 (t, 3H), 1.46 (t, 3H), 2.39 (s, 3H), 3.25 (s, 3H), 3.62 (q, 2H), 3.80 (q, 2H), 3.95 (t, 2H), 4.52 (q, 2H), 4.78 (t, 2H), 6.82 (d, 1H), 8.20 (d, 1H), 8.30 (s, 1H), 9.75 (m, 1H). MS APCI+ m/z 428 [MH]⁺

The following compounds, of the general formula shown below, were prepared by a method similar to that described for example 7 using the appropriate HNR³R⁴ amine.

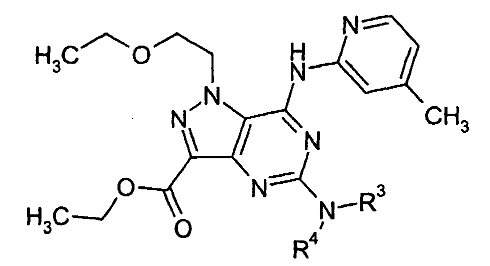

| No. | NR³R⁴ | Data |
|---|---|---|
| 8 | 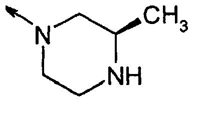 | ¹H NMR (CDCl₃, 400MHz) δ: 1.12 (t, 3H), 1.14 (d, 3H), 1.42 (t, 3H), 2.40 (s, 3H), 2.66 (m, 1H), 2.84 (m, 2H), 3.04 (m, 2H), 3.62 (q, 2H), 3.94 (t, 2H), 4.43 (q, 2H), 4.64 (m, 2H), 4.80 (t, 2H), 6.96 (d, 1H), 8.14 (m, 2H). MS APCI+ m/z 469 [MH]⁺ |
| 9 | 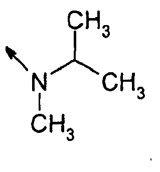 | ¹H NMR (CDCl₃, 400MHz) δ: 1.05 (t, 3H), 1.30 (m, 6H), 1.48 (t, 3H), 2.44 (s, 3H), 3.08 (s, 3H), 3.55 (q, 2H), 3.94 (t, 2H), 4.50 (q, 2H), 4.95 (m, 3H), 6.96 (d, 1H), 8.12 (s, 1H), 8.33 (d, 1H): MS APCI+ m/z 442 [MH]⁺ |

- Example 9 - This compound was isolated without purification by column chromatography

### Example 10

### 2-(Dimethylamino)ethyl 5-dimethylamino-1-(2-ethoxyethyl)-7-(4-methylpyridin-2-ylamino)-1H-pyrazolo[4,3-d]pyrimidine-3-carboxylate

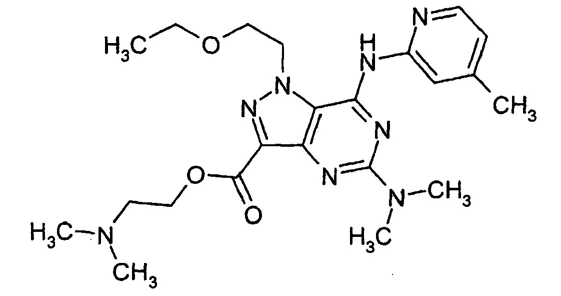

The title compound was prepared by a method similar to that described for example 7, using the ester of preparation 176 and a 2M solution of dimethylamine in methanol.
¹H NMR (CD₃OD, 400MHz) δ: 1.10 (t, 3H), 2.38 (s, 6H), 2.40 (s, 3H), 2.84 (t, 2H), 3.26 (s, 6H), 3.60 (q, 2H), 3.94 (t, 2H), 4.52 (t, 2H), 4.80 (m, 2H), 6.94 (d, 1H), 8.15 (d, 1H), 8.34 (s, 1H). MS APCI+ m/z 457 [MH]⁺

### Examples 11 to 41

The appropriate monochloro precursor (1 eq) was dissolved in dimethyl sulphoxide (1-2mLmmol⁻¹) and the solution treated with the appropriate HNR³R⁴ amine (3eq) and N-ethyldiisopropylamine (3eq). The reaction mixture was then stirred at 120°C for 18 hours, allowed to cool to room temperature and concentrated *in vacuo*. The residue was dissolved in dichloromethane and the organic phase washed with citric acid solution (20mL), dried over magnesium sulphate and concentrated *in vacuo.* The crude product was purified by column chromatography on silica gel eluting with dichloromethane:methanol 100:0 to 94:6 to yield the desired product.

Monochloro precursors from preparations 135, 136, 137, 140, 141, 142, 143, 144, 146, 147, 148, 149, 170 and 171were used.

| | | | |
|---|---|---|---|
| | 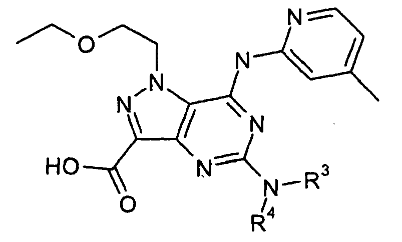 | | |

| Ex | -NR³R⁴ | Data | |
|---|---|---|---|
| 11 | -N(CH₂CH₃)₂ | ¹H NMR (CD₃OD, 400MHz) δ: 1.18 (t, 3H), 1.35 (t, 6H), 2.45 (s, 3H), 3.60 (m, 2H), 3.78 (m, 4H), 3.98 (m, 2H), 4.90 (m, 2H), 7.05 (m, 1H), 8.10 (m, 2H). MS APCI- m/z 412 [M-H]⁻ | |
| 12 | 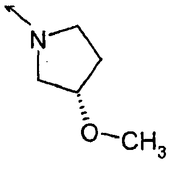 | ¹H NMR (CD₃OD, 400MHz) δ: 1.10 (t, 3H), 2.20 (m, 2H), 2.50 (s, 3H), 3.40 (s, 3H), 3.58 (m, 2H), 3.80 (m, 4H), 3.98 (t, 2H), 4.18 (s, 1H), 4.90 (s, 2H), 7.05 (m, 1H), 8.18 (m, 2H). MS APCI- m/z 440 [M-H]⁻ | |
| 13 | 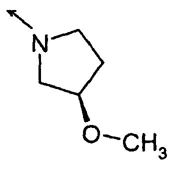 | ¹H NMR (CD₃OD, 400MHz) δ: 1.10 (t, 3H), 2.20 (m, 2H), 2.50 (s, 3H), 3.40 (s, 3H), 3.58 (m, 2H), 3.80 (m, 4H), 3.98 (t, 2H), 4.18 (s, 1H), 4.90 (s, 2H), 7.05 (m, 1H), 8.05 (m, 2H), 8.15 (d, 1H). MS APCI- m/z 440 [M-H]⁻ | |
| 14 | -N(CH₃)₂ | ¹H NMR (DMSO-D₆, 400MHz) δ: 1.00 (t, 3H), 2.40 (s, 3H), 3.18 (s, 6H), 3.50 (m, 2H), 3.85 (t, 2H), 4.90 (m, 2H), 7.10 (m, 1H), 8.10 (m, 1H), 8.25 (m, 1H). MS APCI- m/z 384 [M-H]⁻ | |
| 15 | 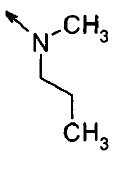 | ¹H NMR (DMSO-D₆, 400MHz) δ: 0.81 (t, 3H), 1.05 (t, 3H), 1.60 (m, 2H), 2.31 (s, 3H), 3.13 (s, 3H), 3.45-3.60 (m, 4H), 3.83 (t, 2H), 4.74 (t, 2H), 6.93 (d, 1H), 8.05 (m, 1H), 8.19 (d, 1H), 9.73 (m, 1H). MS APCI+ m/z 414 [MH]⁺ | |
| 16 | 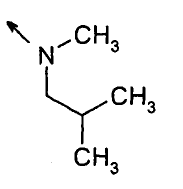 | ¹H NMR (DMSO-D₆, 400MHz) δ: 0.82 (d, 6H), 1.04 (t, 3H), 2.06 (m, 1H), 2.38 (s, 3H), 3.14 (s, 3H), 3.45 (m, 2H), 3.57 (m, 2H), 3.85 (m, 2H), 4.73 (m, 2H), 6.92 (m, 1H), 8.06 (m, 1H), 8.20 (m, 1H), 9.70 (m, 1H). MS APCI+ m/z 428 [MH]⁺ | |
| 17 | 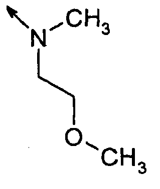 | ¹H NMR (CD₃OD, 400MHz) δ: 1.12 (t, 3H), 2.45 (s, 3H), 3.36 (s, 3H), 3.41 (s, 3H), 3.58 (m, 2H), 3.74 (m, 2H), 3.88 (m, 2H), 3.97 (m, 2H), 4.88 (m, 2H), 7.05 (m, 1H), 8.09 (m, 1H), 8.16 (m, 1H). MS APCI- m/z 428 [M-H]⁻ | |
| 18 | 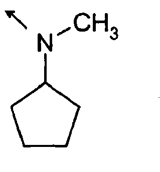 | ¹H NMR (DMSO-D₆, 400MHz) δ: 1.04 (t, 3H), 1.15-1.85 (m, 8H), 2.32 (s, 3H), 3.00 (s, 3H), 3.52 (q, 2H), 3.84 (t, 2H), 4.75 (t, 2H), 5.13 (s, 1H), 6.92 (d, 1H), 8.11 (m, 1H), 8.18 (d, 1H), 9.72 (m, 1H). MS APCI+ m/z 440 [MH]⁺ | |
| | 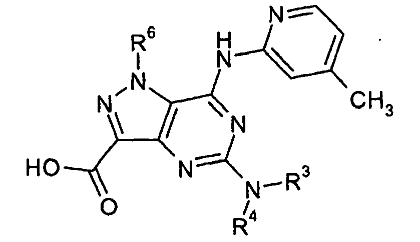 | | |

| Ex | -NR³R⁴ | R⁶ | Data |
|---|---|---|---|
| 19 | 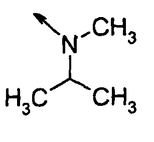 | -(CH₂)₂O(CH₂)₂CH₃ | ¹H NMR (CDCl₃, 400MHz) δ: 0.77 (t, 3H), 1.24 (s, 6H), 1.59 (m, 2H), 2.37 (s, 3H), 3.02 (s, 3H), 3.56 (m, 2H), 3.95 (m, 2H), 4.80 (m, 2H), 5.02 (m, 1H), 6.85 (m, 1H), 8.20 (m, 1H), 8.25 (m, 1H). MS ES+ m/z 450 [MNa]⁺ |
| 20 | 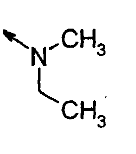 | -(CH₂)₂O(CH₂)₂CH₃ | ¹H NMR (CDCl₃, 400MHz) δ: 0.74 (t, 3H), 1.26 (t, 3H), 1.49 (m, 2H), 2.40 (s, 3H), 3.22 (s, 3H), 3.54 (m, 2H), 3.75 (m, 2H), 3.97 (m, 2H), 4.82 (m, 2H), 6.88 (d, 1H), 8.21 (d, 1H), 8.29 (m, 1H). MS APCI+ m/z 426 [MH]⁺ |
| 21 | 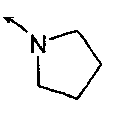 | -(CH₂)₂O(CH₂)₂CH₃ | ¹H NMR (CD₃OD, 400MHz) δ: 0.74 (t, 3H), 1.49 (m, 2H), 2.12 (m, 4H), 2.48 (s, 3H), 3.48 (m, 2H), 3.75 (m, 4H), 3.95 (m, 2H), 4.85 (m, 2H), 7.08 (d, 1H), 8.17 (m, 1H). MS APCI+ m/z 428 [MH]⁺ |
| 22 | 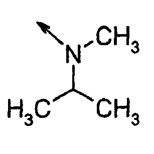 | -(CH₂)₂OCH₃ | ¹H NMR (DMSO-D₆, 400MHz) δ: 1.24 (d, 6H), 2.37 (s, 3H), 3.00 (s, 3H), 3.80 (m, 2H), 4.77 (m, 2H), 5.00 (m, 1H), 6.90 (m, 1H), 8.04 (m, 1H), 8.20 (m, 1H), 9.80 (m, 1H). MS APCI- m/z 398 [M-H]⁻ |
| 23 | -N(CH₃)₂ | -(CH₂)₂OCH₃ | ¹H NMR (DMSO-D₆, 400MHz) δ: 2.34 (s, 3H), 3.12 (s, 6H), 3.31 (s, 3H), 3.80 (m, 2H), 4.78 (m, 2H), 6.90 (d, 1H), 8.02 (m, 1H), 8.20 (d, 1H). MS APCI+ m/z 372 [M-H]⁺ |
| | 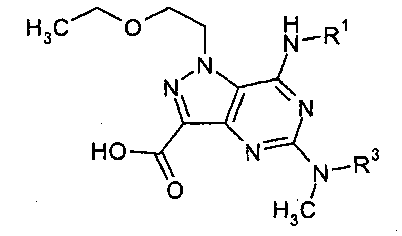 | | |

| Ex | R¹ | R³ | Data |
|---|---|---|---|
| 24 | 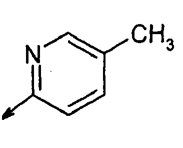 | -CH₃ | ¹H NMR (DMSO-D₆, 400MHz) δ: 1.06 (t, 3H), 2.24 (s, 3H), 3.13 (s, 6H), 3.51 (m, 2H), 3.83 (m, 2H), 4.72 (m, 2H), 7.63 (m, 1H), 8.16 (m, 2H), 9.65 (m, 1H). MS ES- m/z 384 [M-H]⁻ |
| 25 | 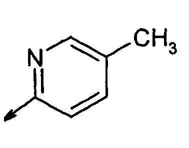 | -CH(CH₃)₂ | ¹H NMR (DMSO-D₆, 400MHz) δ: 1.10 (t, 3H), 1.18 (d, 6H), 2.26 (s, 3H), 2.98 (s, 3H), 3.58 (q, 2H), 3.90 (m, 2H), 4.77 (m, 2H), 4.99 (m, 1H), 7.62 (m, 1H), 8.10 (m, 1H), 8.19 (m, 1H). MS APCI- m/z 412 [M-H]⁻ |
| 26 | 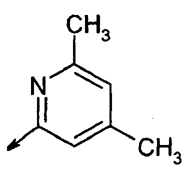 | -CH(CH₃)₂ | ¹H NMR (CD₃OD, 400MHz) δ: 1.11 (t, 3H), 1.30 (d, 6H), 2.40 (s, 3H), 2.49 (s, 3H), 3.10 (s, 3H), 3.55 (m, 2H), 3.93 (m, 2H), 4.90 (m, 2H), 6.90 (m, 1H), 7.87 (m, 1H). MS ES- m/z 426 [M-H]⁻ |
| 27 | 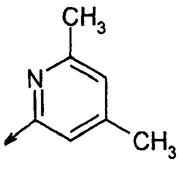 | -CH₃ | ¹H NMR (CD₃OD, 400MHz) δ: 1.08 (t, 3H), 2.41 (s, 3H), 2.53 (s, 3H), 3.30 (s, 6H), 3.55 (m, 2H), 3.94 (m, 2H), 4.91 (m, 2H), 6.91 (m, 1H), 7.82 (m, 1H), . MS ES+ m/z 400 [MH]⁺ |
| 28 | 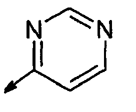 | -CH(CH₃)₂ | ¹H NMR (CD₃OD, 400MHz) δ: 1.22 (t, 3H), 1.26 (d, 6H), 3.10 (s, 3H), 3.67 (q, 2H), 3.97 (t, 2H), 4.80 (t, 2H), 5.11 (m, 1H), 8.28 (m, 1H), 8.61 (d, 1H), 9.83 (s, 1H). MS APCI+ m/z 401 [MH]⁺ |
| 29 | 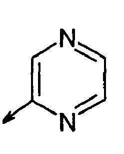 | -CH(CH₃)₂ | ¹H NMR (CD₃OD, 400MHz) δ: 1.20 (t, 3H), 1.28 (d, 6H), 3.10 (s, 3H), 3.67 (q, 2H), 3.98 (t, 2H), 4.85 (m, 2H), 5.04 (m, 1H), 5.48 (s, 2H), 8.31 (m, 1H), 8.42 (m, 1H), 9.48 (m, 1H). MS APCI+ m/z 401 [MH]⁺ |
| 30 | 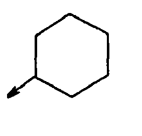 | -(CH₂)₂CH₃ | ¹H NMR (CD₃OD, 400MHz) δ: 1.00 (t, 3H), 1.16 (t, 3H), 1.32 (m, 1H), 1.47 (m, 4H), 1.74 (m, 3H), 1.88 (m, 2H), 2.14 (m, 2H), 3.28 (s, 3H), 3.58 (q, 2H), 3.69 (t, 2H), 3.89 (t, 2H), 4.16 (m, 1H), 4.70 (t, 2H). MS ES+ m/z 405 [MH]⁺ |
| 31 | 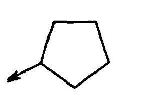 | -CH(CH₃)₂ | ¹H NMR (CDCl₃, 400MHz) δ: 0.86 (t, 3H), 1.18 (d, 6H), 1.57 (m, 2H), 1.73 (m, 2H), 1.88 (m, 2H), 2.16 (m, 2H), 2.98 (s, 3H), 3.51 (m, 2H), 3.86 (m, 2H), 4.40 (m, 1H), 4.61 (m, 2H), 7.07 (m, 1H). MS ES+ m/z 413 [MNa]⁺ |
| 32 | 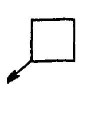 | -CH(CH₃)₂ | ¹H NMR (CD₃OD, 400MHz) δ: 1.19 (t, 3H), 1.31 (d, 6H), 1.96 (m, 2H), 2.22 (m, 2H), 2.52 (m, 2H), 3.12 (s, 3H), 3.57 (q, 2H), 3.90 (m, 2H), 4.65 (m, 1H), 4.76 (m, 2H), 5.03 (m, 1H). MS ES- m/z 375 [M-H]⁻ |
| 33 | 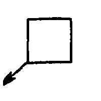 | -CH₃ | ¹H NMR (CD₃OD, 400MHz) δ: 1.19 (t, 3H), 1.92 (m, 2H), 2.22 (m, 2H), 2.52 (m, 2H), 3.32 (s, 6H), 3.57 (q, 2H), 3.90 (m, 2H), 4.69 (m, 1H), 4.76 (m, 2H). MS ES- m/z 347 [M-H]⁻ |
| 34 | 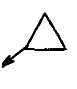 | -CH(CH₃)₂ | ¹H NMR (CD₃OD, 400MHz) δ: 0.80 (m, 2H), 0.98 (m, 2H), 1.17 (t, 3H), 1.31 (d, 6H), 3.07 (m, 1H), 3.15 (s, 3H), 3.52 (q, 2H), 3.86 (m, 2H), 4.70 (m, 2H), 5.10 (m, 1H). MS ES- m/z 361 [M-H]⁻ |
| 35 | 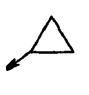 | -CH₃ | ¹H NMR (CD₃OD, 400MHz) δ: 0.80 (m, 2H), 0.98 (q, 2H), 1.17 (t, 3H), 3.09 (m, 1H), 3.35 (s, 6H), 3.52 (q, 2H), 3.86 (m, 2H), 4.71 (m, 2H). MS ES- m/z 333 [M-H]⁻ |
| 36 | 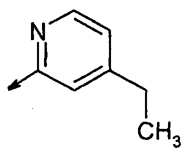 | -CH(CH₃)₂ | ¹H NMR (CD₃OD, 400MHz) δ: 1.13 (t, 3H), 1.29 (m, 6H), 2.75 (q, 2H), 3.13 (s, 3H), 3.60 (q, 2H), 3.96 (t, 2H), 4.88 (m, 2H), 5.10 (m, 1H), 7.08 (d, 1H), 8.16 (s, 1H), 8.21 (d, 1H). MS ES- m/z 426 [M-H]⁻ |
| 37 | 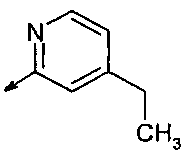 | -CH₃ | ¹H NMR (CD₃OD, 400MHz) δδ: 1.11 (t, 3H), 1.31 (t, 3H), 2.75 (q, 2H), 3.30 (s, 6H), 3.58 (q, 2H), 3.95 (t, 2H), 4.88 (m, 2H), 7.08 (d, 1H), 8.19 (s, 1H), 8.20 (d, 1H). MS ES- m/z 398 [M-H]⁻ |
| 38 | 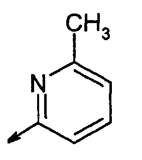 | -CH₂CH₃ | ¹H NMR (CD₃OD, 400MHz) δ: 1.17 (t, 3H), 1.28 (t, 3H), 2.54 (s, 3H), 3.27 (s, 3H), 3.62 (q, 2H), 3.76 (q, 2H), 3.97 (t, 2H), 4.88 (t, 2H), 7.03 (d, 1H), 7.80 (t, 1H), 8.02 (d, 1H). MS ES+ m/z 400 [MH]⁺ |
| 39 | 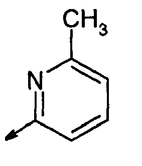 | -CH₃ | ¹H NMR (CD₃OD, 400MHz) δ: 1.16 (t, 3H), 2.54 (s, 3H), 3.29 (s, 6H), 3.60 (q, 2H), 3.95 (t, 2H), 4.89 (t, 2H), 7.02 (d, 1H), 7.80 (t, 1H), 8.02 (d, 1H). MS ES+ m/z 386 [MH]⁺ |
| 40 | 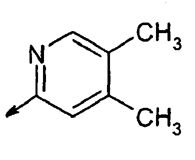 | -CH₂CH₃ | ¹H NMR (CD₃OD, 400MHz) δ: 1.10 (t, 3H), 1.31 (t, 3H), 2.29 (s, 3H), 2.39 (s, 3H), 3.27 (s, 3H), 3.60 (q, 2H), 3.77 (q, 2H), 3.96 (t, 2H), 4.88 (t, 2H), 8.04 (s, 1H), 8.10 (s, 1H). MS ES+ m/z 414 [MH]⁺ |
| 41 | 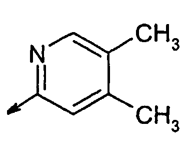 | -CH₃ | ¹H NMR (CD₃OD, 400MHz) δ: 1.10 (t, 3H), 2.29 (s, 3H), 2.39 (s, 3H), 3.30 (s, 3H), 3.31 (s, 3H), 3.58 (q, 2H), 3.94 (q, 2H), 4.85 (t, 2H), 8.03 (s, 1H), 8.06 (s, 1H). MS ES+ m/z 400 [MH]⁺ |

- Examples 14 and 24-31 were performed without N-ethyldiisopropylamine
- Examples 38-41 were performed using caesium fluoride instead of N-ethyldiisopropylamine
- Example 12 used the amine of preparation 4 as the HNR³R⁴ amine
- Example 13 used the amine of preparation 3 as the HNR³R⁴ amine

### Examples 42 to 48

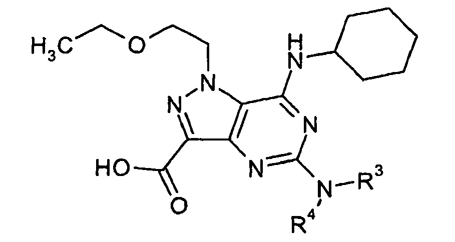

The monochloro compound of preparation 144 (99mg, 0.27mmol) was dissolved in dimethyl sulphoxide (3mL) and the solution treated with the appropriate HNR³R⁴ amine (1.08mmol). The reaction mixture was heated to 120°C for 18 hours before being allowed to cool to room temperature. The reaction mixture was diluted with dichloromethane and washed with water, brine (x2) and citric acid. The dichloromethane phase was dried over magnesium sulphate and concentrated *in vacuo.* The residue was purified by column chromatography on silica gel eluting with dichloromethane:methanol 100:0 to 85:15. The crude product was triturated with ether to give the desired product.

| Ex | -NR³R⁴ | Data |
|---|---|---|
| 42 | -N(CH₃)₂ | ¹H NMR (CD₃OD, 400MHz) δ: 1.16 (t, 3H), 1.30 (m, 1H), 1.48 (m, 4H), 1.72 (m, 1H), 1.86 (m, 2H), 2.15 (m, 2H), 3.30 (s, 6H), 3.58 (q, 2H), 3.89 (t, 2H), 4.20 (m, 1H), 4.70 (t, 2H). MS ES+ m/z 375 [MH]⁺ |
| 43 | 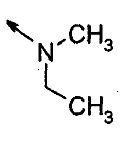 | ¹H NMR (CD₃OD, 400MHz) δ: 1.16 (t, 3H), 1.29 (t, 4H), 1.48 (m, 4H), 1.73 (m, 1H), 1.86 (m, 2H), 2.14 (m, 2H), 3.27 (s, 3H), 3.58 (q, 2H), 3.77 (q, 2H), 3.90 (t, 2H), 4.19 (m, 1H), 4.71 (t, 2H). MS ES+ m/z 389 [MH]⁺ |
| 44 | 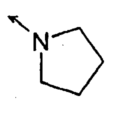 | ¹H NMR (CD₃OD, 400MHz) δ: 1.16 (t, 3H), 1.31 (m, 1H), 1.48 (m, 4H), 1.73 (m, 1H), 1.86 (m, 2H), 2.13 (m, 6H), 3.58 (q, 2H), 3.71 (m, 4H), 3.90 (t, 2H), 4.21 (m, 1H), 4.71 (t, 2H). MS ES+ m/z 401 [MH]⁺ |
| 45 | -NHCH₂CH₃ | ¹H NMR (CD₃OD, 400MHz) δ: 1.17 (t, 3H), 1.30 (t, 4H), 1.48 (m, 4H), 1.73 (m, 1H), 1.86 (m, 2H), 2.14 (m, 2H), 3.57 (m, 4H), 3.90 (t, 2H), 4.23 (m, 1H), 4.70 (t, 2H). MS ES+ m/z 375 [MH]⁺ |
| 46 | 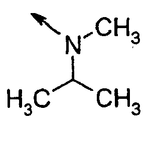 | ¹H NMR (CD₃OD, 400MHz) δ: 1.16 (t, 3H), 1.30 (d, 7H), 1.48 (m, 4H), 1.72 (m, 1H), 1.87 (m, 2H), 2.14 (m, 2H), 3.12 (s, 3H), 3.58 (q, 2H), 3.90 (t, 2H), 4.16 (m, 1H), 4.70 (t, 2H), 4.95 (m, 1H). MS ES+ m/z 403 [MH]⁺ |
| 47 | -N(CH₂CH₃)₂ | ¹H NMR (CD₃OD, 400MHz) δ: 1.16 (t, 3H), 1.32 (t, 7H), 1.48 (m, 4H), 1.72 (m, 1H), 1.86 (m, 2H), 2.13 (m, 2H), 3.58 (q, 2H), 3.72 (q, 4H), 3.90 (t, 2H), 4.17 (m, 1H), 4.71 (t, 2H). MS ES+ m/z 403 [MH]⁺ |
| 48 | -NHCH₃ | ¹H NMR (CD₃OD, 400MHz) δ: 1.15 (t, 3H), 1.32 (m, 1H), 1.47 (m, 4H), 1.72 (m, 1H), 1.85 (m, 2H), 2.16 (m, 2H), 3.05 (s, 3H), 3.56 (q, 2H), 3.89 (m, 2H), 4.22 (m, 1H), 4.72 (m, 2H). MS ES+ m/z 367 [MH]⁺ |

### Examples 49 to 74

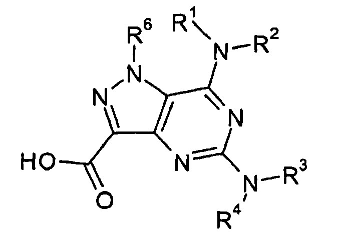

The appropriate monochloro precursor (0.266mmol) and tetraethylammonium fluoride (39.6mg, 0.266mmol) were dissolved in dimethyl sulphoxide (1.0mL) and the solution treated with N-ethyldiisopropylamine (230µL, 1.33mmol) and a solution of the appropriate HNR³R⁴ amine (1.33mmol) in dimethyl sulphoxide (500µL). The reaction mixture was placed in a sealed vessel and shaken at 350rpm at 120°C for 18 hours. The reaction mixture was diluted with dichloromethane and washed with 1M citric acid solution and water. The dichloromethane phase was then dried over magnesium sulphate and concentrated *in vacuo.* The residue was purified by column chromatography on silica gel eluting with dichloromethane:methanol 100:0 to 90:10 to yield the desired product.

Monochloro precursors from preparations 137, 138, 139, 145, 150, 151, 172, 173 and 174 were used.

| | | | |
|---|---|---|---|
| | 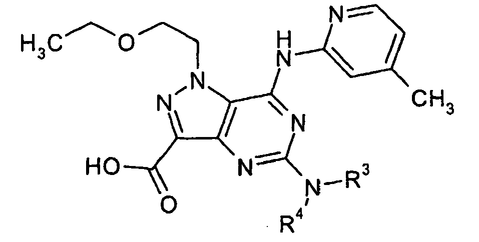 | | |

| Ex | -NR³R⁴ | Data | |
|---|---|---|---|
| 49 | 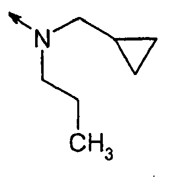 | ¹H NMR (CD₃OD, 400MHz) δ: 0.40 (q, 2H), 0.60 (q, 2H), 1.00 (t, 3H), 1.14 (t, 3H), 1.23 (m, 1H), 1.78 (q, 2H), 2.47 (q, 2H), 3.60 (m, 4H), 3.69 (m, 2H), 3.96 (t, 2H), 4.88 (m, 2H), 7.03 (d, 1H), 8.16 (m, 2H). MS ES+ m/z 454 [MH]⁺ | |
| 50 | 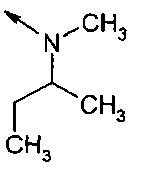 | ¹H NMR (CD₃OD, 400MHz) δ: 0.93 (t, 3H), 1.14 (t, 3H), 1.29 (d, 3H), 1.69 (m, 2H), 2.46 (s, 3H), 3.11 (s, 3H), 3.33 (m, 1H), 3.61 (m, 2H), 3.95 (t, 2H), 4.88 (m, 2H), 7.04 (d, 1H), 8.15 (s, 1H), 8.17 (d, 1H). MS ES+ m/z 428 [MH]⁺ | |
| 51 | 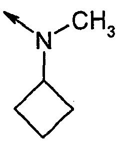 | ¹H NMR (CD₃OD, 400MHz) δ: 1.12 (t, 3H), 1.81 (m, 2H), 2.35 (m, 4H), 2.48 (s, 3H), 3.24 (s, 3H), 3.33 (m, 1H), 3.59 (m, 2H), 3.95 (t, 2H), 4.88 (m, 2H), 7.04 (d, 1H), 8.16 (m, 2H). MS ES+ m/z 426 [MH]⁺ | |
| 52 | 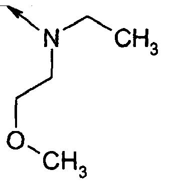 | ¹H NMR (CD₃OD, 400MHz) δ: 1.13 (t, 3H), 1.34 (t, 3H), 2.47 (s, 3H), 3.44 (s, 3H), 3.60 (m, 2H), 3.74-3.84 (m, 6H), 3.96 (t, 2H), 4.89 (m, 2H), 7.05 (d, 1H), 8.12 (s, 1H), 8.16 (d, 1H). MS ES+ m/z 444 [MH]⁺ | |
| 53 | 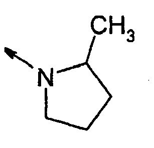 | ¹H NMR (CD₃OD, 400MHz) δ: 1.12 (t, 3H), 1.39 (d, 3H), 1.87 (m, 1H), 2.23 (m, 3H), 2.48 (s, 3H), 3.60 (m, 3H), 3.80 (m, 1H), 3.96 (t, 2H), 4.46 (m, 1H), 4.89 (m, 2H), 7.07 (d, 1H), 8.18 (m, 2H). MS ES+ m/z 426 [MH]⁺ | |
| 54 | 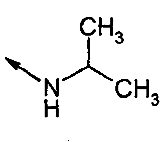 | ¹H NMR (CD₃OD, 400MHz) δ: 1.11 (t, 3H), 1.35 (d, 6H), 2.54 (s, 3H), 3.55 (m, 2H), 3.98 (t, 2H), 4.18 (m, 1H), 5.03 (t, 2H), 7.16 (d, 1H), 8.10 (s, 1H), 8.12 (d, 1H). MS ES+ m/z 400 [MH]⁺ | |
| 55 | 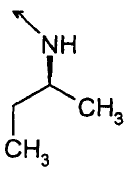 | ¹H NMR (CD₃OD, 400MHz) δ: 1.00 (t, 3H), 1.10 (t, 3H), 1.30 (t, 3H), 1.67 (m, 2H), 2.53 (s, 3H), 3.54 (m, 2H), 3.98 (m, 3H), 5.02 (t, 2H), 7.17 (d, 1H), 8.10 (s, 1H), 8.13 (d, 1H). MS ES+ m/z 414 [MH]⁺ | |
| 56 | 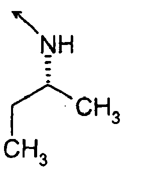 | ¹H NMR (CD₃OD, 400MHz) δ: 1.00 (t, 3H), 1.10 (t, 3H), 1.30 (t, 3H), 1.67 (m, 2H), 2.53 (s, 3H), 3.54 (m, 2H), 3.98 (m, 3H), 5.02 (t, 2H), 7.17 (d, 1H), 8.10 (s, 1H), 8.13 (d, 1H). MS ES+ m/z 414 [MH]⁺ | |
| | 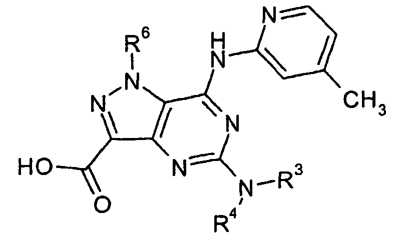 | | |

| Ex | -NR³R⁴ | R⁶ | Data |
|---|---|---|---|
| 57 | -N(CH₃)₂ | 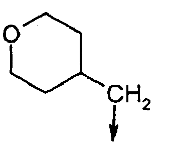 | ¹H NMR (CD₃OD, 400MHz) δ: 1.47 (m, 4H), 2.30 (m, 1H), 2.50 (s, 3H), 3.28 (s, 6H), 3.36 (m, 2H), 3.90 (m, 2H), 4.70 (d, 2H), 7.08 (d, 1H), 7.80 (s, 1H), 8.10 (d, 1H). MS APCI+ m/z 412 [MH]⁺ |
| 58 | 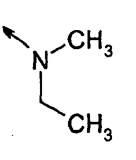 | 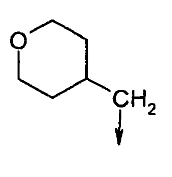 | ¹H NMR (CD₃OD, 400MHz) δ: 1.28 (t, 3H), 1.44 (m, 4H), 2.30 (m, 1H), 2.48 (s, 3H), 3.24 (s, 3H), 3.34 (m, 2H), 3.73 (q, 2H), 3.90 (m, 2H), 4.70 (d, 2H), 7.07 (d, 1H), 7.84 (s, 1H), 8.08 (d, 1H). MS APCI+ m/z 426 [MH]⁺ |
| 59 | -N(CH₃)₂ | 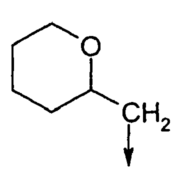 | ¹H NMR (DMSO-D₆, 400MHz) δ: 1.20 (m, 1H), 1.50 (m, 3H), 1.74 (m, 1H), 1.82 (m, 1H), 2.35 (s, 3H), 3.14 (s, 6H), 3.46 (m, 1H), 3.84 (m, 1H), 4.06 (m, 1H), 4.58 (m, 1H), 4.70 (m, 1H), 6.92 (d, 1H), 8.08 (m, 1H), 8.19 (m, 1H). MS APCI+ m/z 412 [MH]⁺ |
| 60 | 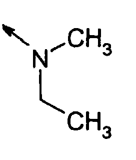 | 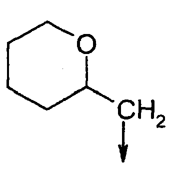 | ¹H NMR (DMSO-D₆, 400MHz) δ: 1.17 (t, 3H), 1.20 (m, 1H), 1.47 (m, 3H), 1.78 (m, 2H), 2.32 (s, 3H), 3.12 (s, 3H), 3.42 (m, 1H), 3.68 (m, 2H), 3.84 (m, 1H), 4.07 (m, 1H), 4.58 (m, 1H), 4.68 (m, 1H), 6.95 (d, 1H), 8.05 (m, 1H), 8.20 (m, 1H). MS APCI+ m/z 426 [MH]⁺ |
| 61 | -N(CH₃)₂ | -(CH₂)₂OCH(CH₃)₂ | ¹H NMR (CD₃OD, 400MHz) δ: 1.06 (m, 6H), 2.42 (s, 3H), 3.27 (s, 6H), 3.67 (m, 1H), 3.94 (t, 2H), 4.82 (m, 2H), 7.07 (d, 1H), 8.17 (m, 2H). MS APCI+ m/z 400 [MH]⁺ |
| 62 | 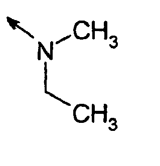 | -(CH₂)₂OCH(CH₃)₂ | ¹H NMR (CD₃OD, 400MHz) δ: 1.08 (d, 6H), 1.27 (t, 3H), 2.43 (s, 3H), 3.28 (s, 3H), 3.65 (m, 1H), 3.80 (q, 2H), 3.95 (t, 2H), 4.85 (m, 2H), 7.06 (d, 1H), 8.20 (m, 2H). MS APCI+ m/z 414 [MH]⁺ |
| 63 | -NHCH₂CH₃ | -(CH₂)₂OCH(CH₃)₂ | ¹H NMR (DMSO-D₆, 400MHz) δ: 0.97 (d, 6H), 1.16 (t, 3H), 2.44 (s, 3H), 3.37 (m, 2H), 3.50 (m, 1H), 3.81 (t, 2H), 4.90 (t, 2H), 7.05 (d, 1H), 8.08 (s, 1H), 8.22 (d, 1H). MS APCI+ m/z 400 [MH]⁺ |
| 64 | -N(CH₃)₂ | 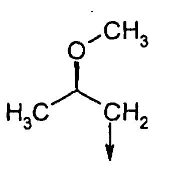 | ¹H NMR (CD₃OD, 400MHz) δ: 1.28 (d, 3H), 2.45 (s, 3H), 3.30 (s, 6H), 3.42 (s, 3H), 3.98 (m, 1H), 4.74 (m, 2H), 7.03 (d, 1H), 8.08 (m, 1H), 8.18 (m, 1H). MS APCI+ m/z 386 [MH]⁺ |
| 65 | 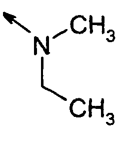 | 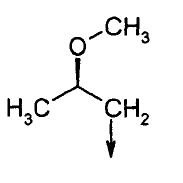 | ¹H NMR (CD₃OD, 400MHz) δ: 1.32 (m, 6H), 2.45 (s, 3H), 3.36 (s, 3H), 3.42 (s, 3H), 3.78 (q, 2H), 4.00 (m, 1H), 4.74 (m, 2H), 7.04 (d, 1H), 8.10 (m, 1H), 8.20 (m, 1H). MS APCI+ m/z 400 [MH]⁺ |
| 66 | -N(CH₂CH₃)₂ | 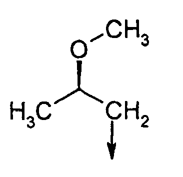 | ¹H NMR (CD₃OD, 400MHz) δ: 1.30 (m, 9H), 2.45 (s, 3H), 3.44 (s, 3H), 3.64 (q, 2H), 4.00 (m, 1H), 4.72 (m, 2H), 7.03 (m, 1H), 8.11 (m, 1H), 8.19 (m, 1H). MS APCI+ m/z 414 [MH]⁺ |
| | 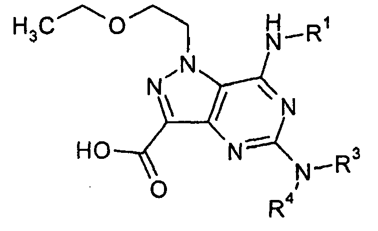 | | |

| Ex | R¹ | -NR³R⁴ | Data |
|---|---|---|---|
| 67 | 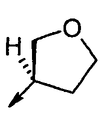 | 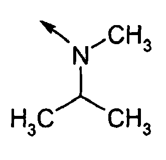 | ¹H NMR (DMSO-D₆, 400MHz) δ: 1.03 (t, 3H), 1.23 (d, 6H), 2.09 (m, 1H), 2.33 (m, 1H), 3.07 (s, 3H), 3.44 (q, 2H), 3.77 (m, 2H), 3.95 (m, 2H), 4.75 (m, 2H), 4.84 (m, 2H). MS ES+ m/z 393 [MH]⁺ |
| 68 | 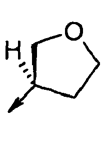 | -N(CH₂CH₃)₂ | ¹H NMR (DMSO-D₆, 400MHz) δ: 1.04 (t, 3H), 1.22 (t, 6H), 2.09 (m, 1H), 2.32 (m, 1H), 3.44 (q, 2H), 3.77 (m, 8H), 3.94 (m, 2H), 4.75 (m, 1H), 4.84 (m, 2H). MS ES+ m/z 393 [MH]⁺ |
| 69 | 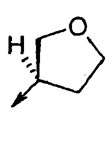 | 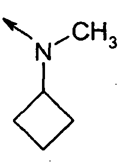 | ¹H NMR (CD₃OD, 400MHz) δ: 1.16 (t, 3H), 1.84 (m, 2H), 2.09 (m, 1H), 2.35 (m, 4H), 2.48 (m, 1H), 3.26 (s, 3H), 3.58 (q, 2H), 3.89 (m, 4H), 4.05 (m, 2H), 4.73 (t, 2H), 4.85 (m, 2H). MS ES+ m/z 405 [MH]⁺ |
| 70 | 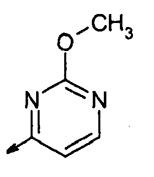 | 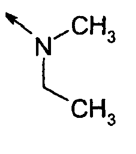 | ¹H NMR (CDCl₃, 400MHz) δ: 1.25 (m, 6H), 3.22 (s, 3H), 3.70 (m, 4H), 3.99 (t, 2H), 4.01 (s, 3H), 4.80 (t, 2H), 7.90 (d, 1H), 8.42 (d, 1H), 10.18 (s, 1H). MS APCI- m/z 415 [M-H]⁻ |
| 71 | 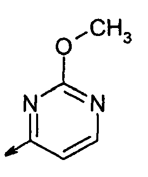 | -N(CH₃)₂ | ¹H NMR (CDCl₃, 400MHz) δ: 1.25 (t, 3H), 3.30 (s, 6H), 3.70 (q, 2H), 3.99 (t, 2H), 4.02 (s, 3H), 4.80 (t, 2H), 8.42 (d, 1H), 8.45 (d, 1H), 10.18 (s, 1H). MS APCI- m/z 401 [M-H]⁻ |
| 72 | 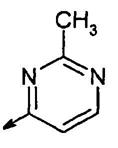 | 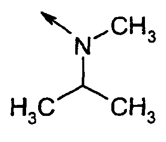 | ¹H NMR (CD₃OD, 400MHz) δ: 1.24 (t, 3H), 1.28 (d, 6H), 2.60 (s, 3H), 3.10 (s, 3H), 3.69 (q, 2H), 3.99 (t, 2H), 4.82 (t, 2H), 5.09 (m, 1H), 8.09 (d, 1H), 8.51 (m, 1H). MS ES- m/z 413 [M-H]⁻ |
| 73 | 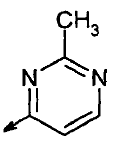 | 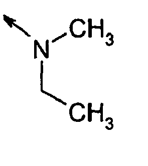 | ¹H NMR (CD₃OD, 400MHz) δ: 1.23 (t, 6H), 2.60 (s, 3H), 3.23 (s, 3H), 3.71 (q, 2H), 3.78 (q, 2H), 3.98 (t, 2H), 4.81 (t, 2H), 8.11 (d, 1H), 8.51 (m, 1H). MS ES- m/z 399 [M-H]⁻ |
| 74 | 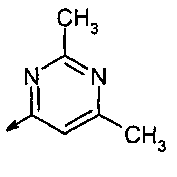 | 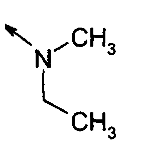 | ¹H NMR (CD₃OD, 400MHz) δ: 1.25 (m, 6H), 2.45 (s, 6H), 3.25 (s, 3H) 3.61 (q, 2H), 3.78 (q, 2H), 3.98 (t, 2H), 4.90 (t, 2H), 6.99 (d, 1H). MS ES- m/z 413 [M-H]⁻ |

- Example 51 was prepared using cyclobutyl-methyl-amine (J. Med. Chem., 1994, 37, 3482-3491) as the HNR³R⁴ amine.
- Examples 67, 68 and 69 were purified by column chromatography on silica gel eluting with dichloromethane:methanol:acetic acid 90:10:1
- Examples 70 and 71 were prepared without the use of N-ethyldiisopropylamine.

### Example 75

### 3-[1-(2-Ethoxyethyl)-5-(N-isopropyl-N-methylamino)-7-(4-methylpyridin-2-ylamino)-1H-pyrazolo[4,3-d]pyrimidin-3-yl]-2H-1,2,4-oxadiazol-5-one

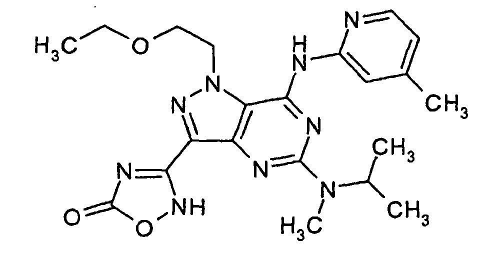

The oxadiazolone of preparation 183 (50mg, 0.12mmol) was added to a solution of methylisopropylamine (44mg, 0.60mmol) and N-ethyldiisopropylamine (83µL, 0.60mmol) in dimethyl sulphoxide (1mL) and the reaction mixture stirred at 120°C for 18 hours. The reaction mixture was diluted with ethyl acetate (20mL) and washed with water (15mL). The aqueous phase was then extracted with ethyl acetate (2x20mL), acidified with acetic acid solution and extracted with further ethyl acetate (2x20mL). The organics were combined, dried over magnesium sulphate and concentrated *in vacuo.* The residue was purified by column chromatography on silica gel eluting with dichloromethane:methanol:acetic acid 100:0:0 to 97.5:2.5:0.25 to yield the title product.
¹H NMR (CD₃OD, 400MHz) δ: 1.11 (t, 3H), 1.25 (d, 6H), 2.41 (s, 3H), 3.09 (s, 3H), 3.60 (m, 2H), 3.95 (t, 2H), 4.83 (t, 2H), 5.16 (m, 1H), 6.95 (d, 1H), 8.16 (m, 1H), 8.25 (m, 1H)
MS ES+ m/z 454 [MH]⁺

The following compounds, of the general formula shown below, were prepared by a method similar to that described for example 75 using the monochloro precursor from preparations 183, 184 and 185.

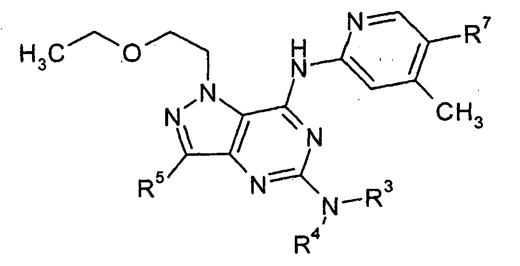

| | | | |
|---|---|---|---|
| | 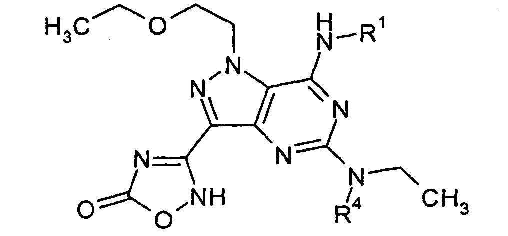 | | |

| Ex | R¹ | R⁴ | Data |
|---|---|---|---|
| 76 | 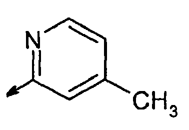 | -CH₃ | ¹H NMR (CD₃OD, 400MHz) δ: 1.12 (t, 3H), 1.25 (t, 3H), 2.41 (s, 3H), 3.24 (s, 3H), 3.61 (m, 2H), 3.77 (m, 2H), 3.94 (t, 2H), 4.84 (t, 2H), 6.95 (d, 1H), 8.16 (s, 1H), 8.31 (m, 1H). MS ES+ m/z 440 [MH]⁺ |
| 77 | 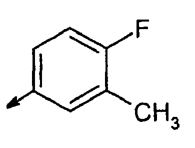 | -CH₃ | ¹H NMR (DMSO-D₆, 400MHz) δ: 0.99 (t, 3H), 1.10 (t, 3H), 2.26 (s, 3H), 3.07 (s, 3H), 3.50 (q, 2H), 3.58 (q, 2H), 3.84 (m, 2H), 4.84 (m, 2H), 7.14 (t, 1H), 7.47 (m, 1H), 7.68 (m, 1H), 8.87 (s, 1H),. MS ES+ m/z 457 [MH]⁺ |
| 78 | 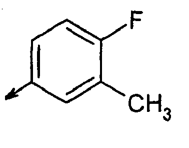 | -CH₂CH₃ | ¹H NMR (DMSO-D₆, 400MHz) δ: 1.00 (t, 3H), 1.10 (t, 6H), 2.25 (s, 3H), 3.50 (q, 2H), 3.58 (q, 4H), 3.84 (m, 2H), 4.84 (m, 2H), 7.15 (t, 1H), 7.40 (m, 1H), 7.69 (m, 1H), 8.85 (s, 1H). MS APCI- m/z 469 [M-H]⁻ |
| | 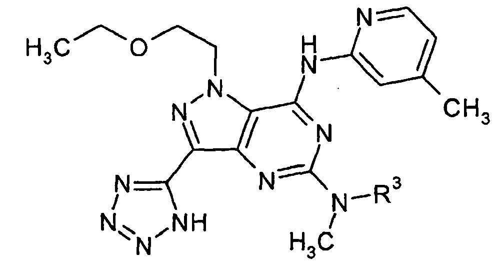 | | |
| | R³ | | |
| 79 | -CH(CH₃)₂ | ¹H NMR (CD₃OD, 400MHz) δ: 1.13 (t, 3H), 1.25 (d, 6H), 2.42 (s, 3H), 3.12 (s, 3H), 3.61 (m, 2H), 3.95 (m, 2H), 4.84 (m, 2H), 5.20 (m, 1H), 6.95 (d, 1H), 8.15 (m, 1H), 8.30 (m, 1H). MS ES- m/z 436 [M-H]⁻ | |
| 80 | -CH₃ | ¹H NMR (CD₃OD, 400MHz) δ: 1.13 (t, 3H), 2.44 (s, 3H), 3.30 (s, 6H), 3.61 (m, 2H), 3.72 (t, 2H), 4.90 (t, 2H), 7.00 (d, 1H), 8.17 (m, 1H), 8.25 (m, 1H). MS APCI+ m/z 410 [MH]⁺ | |

### Example 81

### 1-(2-Ethoxyethyl)-7-(5-fluoropyridin-2-ylamino)-5-(N-isopropyl-N-methylamino)-1H-pyrazolo[4,3-d]pyrimidine-3-carboxylic acid

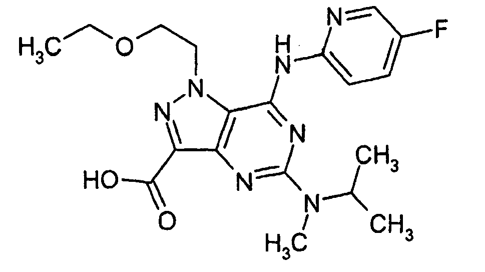

The ester of preparation 108 (30mg, 0.07mmol) and a 1M aqueous solution of sodium hydroxide (105µL, 0.105mmol) were dissolved in dioxane (1 mL) and the reaction mixture stirred at room temperature for 18 hours. The reaction mixture was concentrated *in vacuo* and the residue treated with 1M citric acid solution (5mL) and extracted with dichloromethane (3x50mL). The organics were combined, dried over sodium sulphate and concentrated *in vacuo.* The crude product was triturated with ether and then filtered to yield the title product as a white solid, 27mg.
¹H NMR (CD₃OD, 400MHz) δ: 1.22 (t, 3H), 1.30 (d, 6H), 3.12 (s, 3H), 3.70 (q, 2H), 3.98 (t, 2H), 4.84 (t, 2H), 5.01 (m, 1H), 7.71 (m, 1H), 8.29 (m, 1H), 8.31 (d, 1H)
MS ES- m/z 416 [M-H]⁻

The following compounds, of the general formula shown below, were prepared by a method similar to that described for example 81 using the appropriate ester of preparations 103, 106, 108, 109, 110, 111, 112, 113, 114, 115, 116, 117, 118, 119, 121, 122, 123, 124, 125, 126, 128, 129, 130, 131, 132, 133, 134 and example 6.

| | | | |
|---|---|---|---|
| | 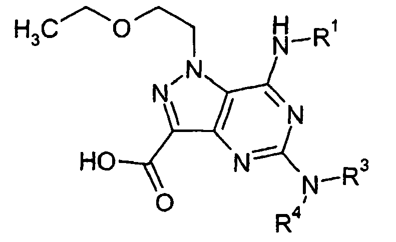 | | |

| Ex | R¹ | -NR³R⁴ | Data |
|---|---|---|---|
| 82 | 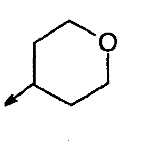 | -N(CH₃)₂ | ¹H NMR (CD₃OD, 400MHz) δ: 1.16 (t, 3H), 1.78 (m, 2H), 2.14 (m, 2H), 3.32 (s, 6H), 3.59 (m, 4H), 3.90 (t, 2H), 4.04 (m, 2H), 4.40 (m, 1H), 4.74 (t, 2H). MS ES- m/z 377 [M-H]⁻ |
| 83 | 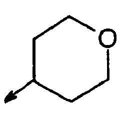 | 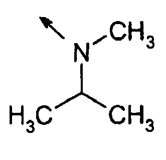 | ¹H NMR (CD₃OD, 400MHz) δ: 1.19 (t, 3H), 1.32 (d, 6H), 1.85 (m, 2H), 2.10 (m, 2H), 3.16 (s, 3H), 3.60 (m, 4H), 3.93 (t, 2H), 4.05 (t, 2H), 4.45 (m, 1H), 4.89 (t, 2H), 5.01 (m, 1H). MS ES-m/z 405 [M-H]⁻ |
| 84 | 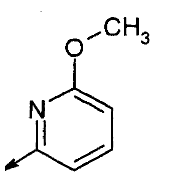 | 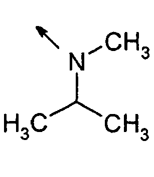 | ¹H NMR (CD₃OD, 400MHz) δ: 1.15 (t, 3H), 2.40 (d, 6H), 3.05 (s, 3H), 3.65 (q, 2H), 3.95 (s, 3H), 3.98 (q, 2H), 4.78 (t, 2H), 5.01 (m, 1H), 6.49 (d, 1H), 7.66 (t, 1H), 7.82 (d, 1H). MS ES- m/z 428 [M-H]⁻ |
| 85 | 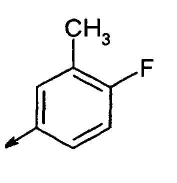 | 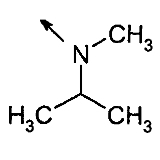 | ¹H NMR (CD₃OD, 400MHz) δ: 1.13 (t, 3H), 1.26 (d, 6H), 2.33 (s, 3H), 3.11 (s, 3H), 3.61 (q, 2H), 3.98 (t, 2H), 4.88 (t, 2H), 4.90 (m, 1H), 7.10 (t, 1H), 7.42 (m, 1H), 7.60 (m, 1H). MS ES+ m/z 431 [MH]⁺ |
| 86 | 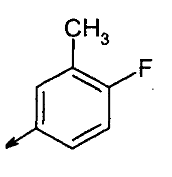 | -N(CH₂CH₃)₂ | ¹H NMR (DMSO-D₆, 400MHz) δ: 1.00 (t, 3H), 1.15 (t, 6H), 2.25 (s, 3H), 3.45 (m, 4H), 3.50 (m, 2H), 3.82 (t, 2H), 4.81 (t, 2H), 7.08 (t, 1H), 7.41 (m, 1H), 7.70 (m, 1H). MS APCI- m/z 429 [M-H]⁻ |
| 87 | 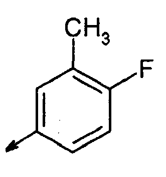 | 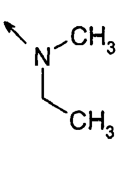 | ¹H NMR (CD₃OD, 400MHz) δ: 1.10 (t, 3H), 1.25 (t, 3H), 2.30 (s, 3H), 3.20 (s, 3H), 3.60 (q, 2H), 3.70 (q, 2H), 3.98 (t, 2H), 4.82 (m, 2H), 7.15 (t, 1H), 7.50 (t, 1H), 7.63 (m, 1H). MS APCI+ m/z 439 [MNa]⁺ |
| 88 | 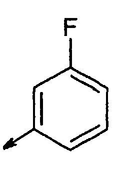 | 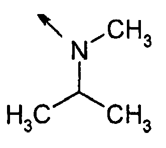 | ¹H NMR (CD₃OD, 400MHz) δ: 1.15 (t, 3H), 1.31 (d, 6H), 3.17 (s, 3H), 3.64 (q, 2H), 3.99 (t, 2H), 4.89 (m, 1H), 4.96 (t, 2H), 7.08 (m, 1H), 7.43 (m, 1H), 7.51 (m, 1H), 7.62 (m, 1H). MS ES- m/z 415 [M-H]⁻ |
| 89 | 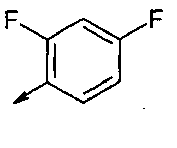 | 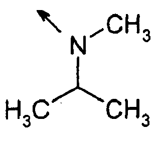 | ¹H NMR (CDCl₃, 400MHz) δ: 1.10 (m, 9H), 2.98 (s, 3H), 3.62 (q, 2H), 3.95 (m, 2H), 4.78 (m, 2H), 4.90 (m, 1H), 6.95 (m, 2H), 8.15 (m, 1H), 9.01 (s, 1H). MS APCI- m/z 453 [M-H]⁻ |
| 90 | 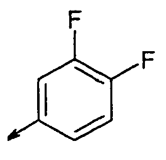 | 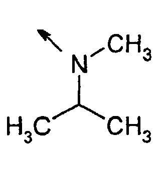 | ¹H NMR (CDCl₃, 400MHz) δ: 1.22 (m, 9H), 3.01 (s, 3H), 3.65 (q, 2H), 4.00 (m, 2H), 4.78 (m, 2H), 4.98 (m, 1H), 7.18 (m, 2H), 7.82 (m, 1H), 9.20 (m, 1H). MS APCI- m/z 433 [M-H]⁻ |
| 91 | 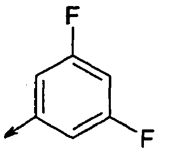 | 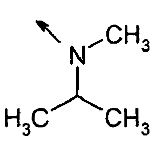 | ¹H NMR (CDCl₃, 400MHz) δ: 1.20 (m, 9H), 3.02 (s, 3H), 3.65 (q, 2H), 4.00 (t, 2H), 4.78 (t, 2H), 4.98 (m, 1H), 6.58 (m, 1H), 7.30 (m, 2H), 9.35 (m, 1H). MS APCI- m/z 433 [M-H]⁻ |
| 92 | 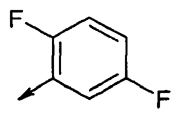 | 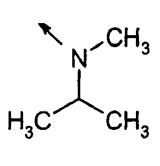 | ¹H NMR (CDCl₃, 400MHz) δ: 1.18 (t, 3H), 1.28 (m, 6H), 3.05 (s, 3H), 3.62 (q, 2H), 3.98 (t, 2H), 4.78 (t, 2H), 4.99 (m, 1H), 6.78 (m, 1H), 7.10 (m, 1H), 8.25 (m, 1H), 9.26 (m, 1H). MS APCI+ m/z 435 [MH]⁺ |
| 93 | 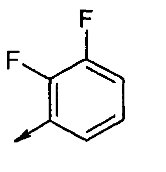 | 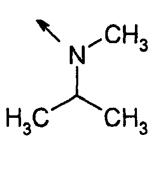 | ¹H NMR (CDCl₃, 400MHz) δ: 1.20 (m, 9H), 3.00 (s, 3H), 3.65 (q, 2H), 3.98 (t, 2H), 4.79 (t, 2H), 4.90 (m, 1H), 6.95 (m, 1H), 7.10 (m, 1H), 8.01 (m, 1H), 9.22 (m, 1H). MS APCI- m/z 433 [M-H]⁻ |
| 94 | 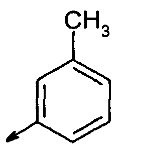 | 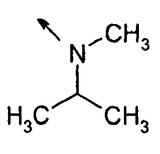 | ¹H NMR (CD₃OD, 400MHz) δ: 1.15 (t, 3H), 1.30 (d, 6H), 2.42 (s, 3H), 3.15 (s, 3H), 3.64 (q, 2H), 4.00 (t, 2H), 4.88 (m, 1H), 4.94 (m, 2H), 7.16 (d, 1H), 7.34 (t, 1H), 7.42 (d, 1H), 7.57 (s, 1H), . MS ES- m/z 411 [M-H]⁻ |
| 95 | 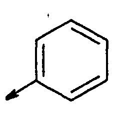 | 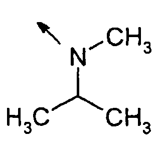 | ¹H NMR (CD₃OD, 400MHz) δ: 1.15 (t, 3H), 1.23 (d, 6H), 3.05 (s, 3H), 3.65 (q, 2H), 4.00 (t, 2H), 4.79 (t, 2H), 5.11 (m, 1H), 7.09 (t, 1H), 7.40 (t, 2H), 7.71 (d, 2H). MS ES- m/z 397 [M-H]⁻ |
| 96 | 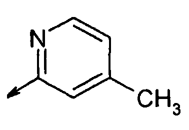 | 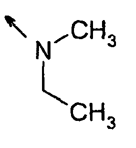 | ¹H NMR (CD₃OD, 400MHz) δ: 1.12 (t, 3H), 1.30 (t, 3H), 2.41 (s, 3H), 3.22 (s, 3H), 3.58 (q, 2H), 3.76 (q, 2H), 3.97 (t, 2H), 4.82 (t, 2H), 7.02 (d, 1H), 8.12 (s, 1H), 8.14 (d, 1H). MS APCI+ m/z 400 [MH]⁺ |
| 97 | 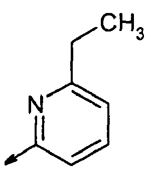 | 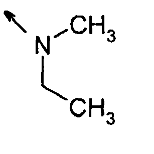 | ¹H NMR (CDCl₃, 400MHz) δ: 1.20-1.40 (m, 9H), 2.78 (q, 2H), 3.20 (s, 3H), 3.70 (m, 4H), 4.00 (t, 2H), 4.81 (t, 2H), 6.87 (d, 1H), 7.62 (t, 1H), 8.10 (d, 1H), 9.85 (s, 1H). MS APCI+ m/z 414 [MH]⁺ |
| 98 | 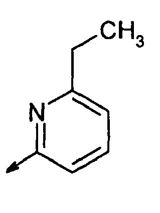 | -N(CH₃)₂ | ¹H NMR (CDCl₃, 400MHz) δ: 1.30 (m, 6H), 2.78 (q, 2H), 3.25 (s, 6H), 3.70 (q, 2H), 4.00 (m, 2H), 4.82 (m, 2H), 6.90 (d, 1H), 7.65 (t, 1H), 8.10 (d, 1H), 9.90 (m, 1H). MS APCI+ m/z 400 [MH]⁺ |
| 99 | 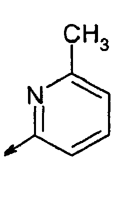 | 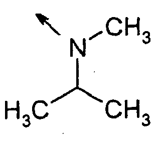 | ¹H NMR (CD₃OD, 400MHz) δ: 1.17 (t, 3H), 1.30 (d, 6H), 2.53 (s, 3H), 3.12 (s, 3H), 3.65 (q, 2H), 3.97 (t, 2H), 4.89 (t, 2H), 4.96 (m, 1H), 7.06 (d, 1H), 7.84 (t, 1H), 7.99 (d, 1H). MS ES- m/z 412 [M-H]⁻ |
| 100 | 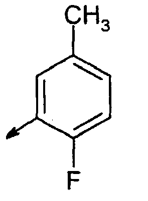 | -N(CH₃)₂ | ¹H NMR (CD₃OD, 400MHz) δ: 1.10 (t, 3H), 2.37 (s, 3H), 3.23 (s, 6H), 3.61 (q, 2H), 3.96 (t, 2H), 4.85 (t, 2H), 7.08 (m, 1H), 7.14 (m, 1H), 7.86 (d, 1H). MS ES+ m/z 403 [MH]⁺ |
| 101 | 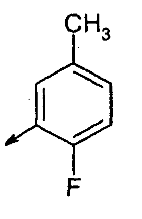 | 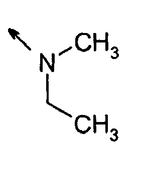 | ¹H NMR (CD₃OD, 400MHz) δ: 1.11 (t, 3H), 1.20 (t, 3H), 2.38 (s, 3H), 3.23 (s, 3H), 3.59 (q, 2H), 3.61 (q, 2H), 3.95 (t, 2H), 4.85 (t, 2H), 7.09 (m, 1H), 7.14 (m, 1H), 7.84 (d, 1H). MS ES+ m/z 417 [MH]⁺ |
| 102 | 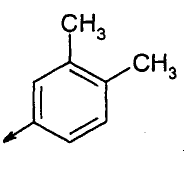 | -N(CH₃)₂ | ¹H NMR (CD₃OD, 400MHz) δ: 1.12 (t, 3H), 2.30 (s, 3H), 2.32 (s, 3H), 3.29 (s, 6H), 3.63 (q, 2H), 3.98 (t, 2H), 4.84 (t, 2H), 7.21 (d, 1H), 7.45 (d, 1H), 7.53 (s, 1H). MS ES+ m/z 399 [MH]⁺ |
| 103 | 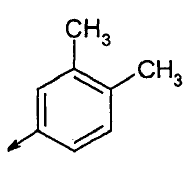 | 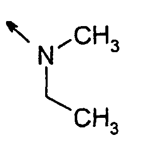 | ¹H NMR (CD₃OD, 400MHz) δ: 1.02 (t, 3H), 1.16 (t, 3H), 2.19 (s, 3H), 2.21 (s, 3H), 3.16 (s, 3H), 3.52 (q, 2H), 3.63 (q, 2H), 3.87 (t, 2H), 4.73 (t, 2H), 7.09 (d, 1H), 7.30 (d, 1H), 7.43 (m, 1H). MS ES+ m/z 413 [MH]⁺ |
| 104 | 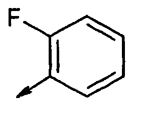 | 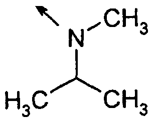 | ¹H NMR (CD₃OD, 400MHz) δ: 1.11 (t, 3H), 1.21 (d, 6H), 3.06 (s, 3H), 3.61 (q, 2H), 3.96 (t, 2H), 4.83 (m, 1H), 4.85 (t, 2H), 7.28 (m, 3H), 7.90 (t, 1H). MS ES- m/z 415 [M-H]⁻ |
| 105 | 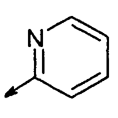 | 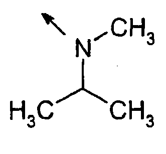 | ¹NMR (CD₃OD, 400MHz) δ: 1.17 (t, 3H, 1.31 (d, 6H), 3.13 (s, 3H), 3.66 (q, 2H), 3.98 (t, 2H), 4.88 (t, 2H), 4.99 (m, 1H), 7.19 (t, 1H), 7.90 (t, 1H), 8.22 (d, 1H), 8.35 (d, 1H). MS ES- m/z 398 [M-H]⁻ |
| | 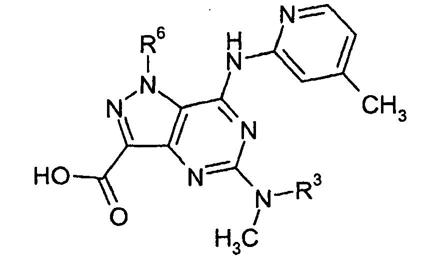 | | |

| | R³ | R⁶ | Data |
|---|---|---|---|
| 106 | -CH(CH₃)₂ | -(CH₂)₂O(CH₂)₂OCH₃ | ¹H NMR (CD₃OD, 400MHz) δ: 1.30 (d, 6H), 2.42 (s, 3H), 3.02 (s, 3H), 3.10 (s, 3H), 3.30 (s, 1H), 3.45 (t, 2H), 3.62 (t, 2H), 4.00 (t, 2H), 4.98 (m, 2H), 7.05 (d, 1H), 8.10 (d, 1H), 8.20 (m, 1H). MS APCI+ m/z 458 [MH]⁺ |
| 107 | -CH(CH₃)₂ | -(CH₂)₂CH(CH₃)OCH₃ | ¹H NMR (CD₃OD, 400MHz) δ: 1.18 (d, 3H), 1.35 (d, 6H), 2.10 (m, 2H), 2.50 (s, 3H), 3.10 (s, 3H), 3.32 (s, 3H), 3.38 (m, 1H), 4.80-4.90 (m, 3H), 7.10 (d, 1H), 7.90 (s, 1H), 8.10 (d, 1H). MS APCI+ m/z 428 [MH]⁺ |
| 108 | -CH(CH₃)₂ | 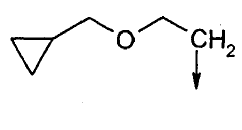 | ¹H NMR (CD₃OD, 400MHz) δ: 0.12 (m, 2H), 0.37 (m, 2H), 1.06 (m, 1H), 1.30 (d, 6H), 2.44 (s, 3H), 3.09 (s, 3H), 3.44 (d, 2H), 4.04 (t, 2H), 4.91 (m, 2H), 5.13 (m, 1H), 7.02 (d, 1H), 8.23 (m, 2H). MS ES- m/z 438 [M-H]⁻ |
| 109 | -CH₂CH₃ | 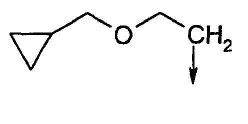 | ¹H NMR (CD₃OD, 400MHz) δ: 0.75 (m, 2H), 0.81 (m, 2H), 0.99 (m, 1H), 1.31 (t, 3H), 2.44 (s, 3H), 3.27 (s, 3H), 3.38 (d, 2H), 3.79 (q, 2H), 4.01 (m, 2H), 4.89 (m, 2H), 7.03 (d, 1H), 8.19 (m, 2H). MS ES+ m/z 426 [MH]⁺ |

### Example 110

### 1-(2-Ethoxyethyl)-5-(N-isopropyl-N-methylamino)-7-(4-methylpyridin-2-ylamino)-1H-pyrazolo[4,3-d]pyrimidine-3-carboxylic acid

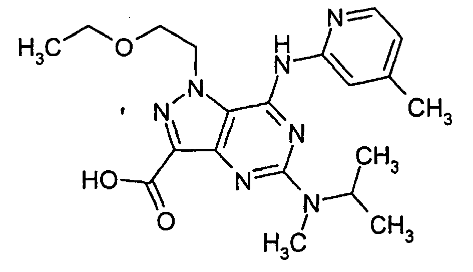

The ester of preparation 121 (219mg, 0.51 mmol) was dissolved in a solution of 1M aqueous sodium hydroxide solution (3mL) in dioxane (1.5mL) and the reaction mixture stirred at room temperature for 18 hours. The reaction mixture was diluted with 1M citric acid solution (50mL) and the mixture washed with dichloromethane (3x100mL). The combined dichloromethane extracts were dried over magnesium sulphate and concentrated *in vacuo.* The residue was purified by column chromatography on silica gel eluting with dichloromethane:methanol 100:0 to 92:8 to yield the title product as a yellow oil, 80mg (38%).
¹H NMR (CD₃OD, 400MHz) δ: 1.12 (t, 3H), 1.30 (d, 6H), 2.45 (s, 3H), 3.12 (s, 3H), 3.60 (m, 2H), 3.96 (t, 2H), 4.88 (m, 2H), 4.98 (m, 1H), 7.04 (d, 1H), 8.14 (s, 1H), 8.18 (d, 1H). MS APCI- m/z 412 [M-H]⁻

### Example 111

### 1-(2-Isopropoxyethyl)-5-(N-isopropyl-N-methylamino)-7-(4-methylpyridin-2-ylamino)-1H-pyrazolo[4,3-d]pyrimidine-3-carboxylic acid

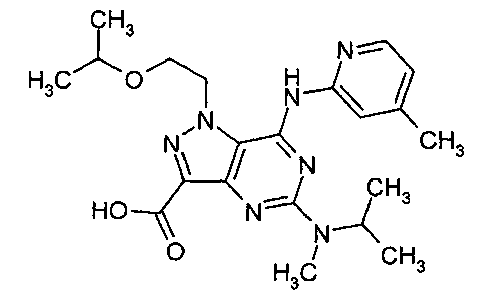

The ester of preparation 106 (140mg, 0.32mmol) was dissolved in methanol (2mL) and the solution treated with 1M aqueous sodium hydroxide solution (640µL). The reaction mixture was stirred at room temperature for 18 hours before being concentrated *in vacuo.* The residue was dissolved in water (20mL), washed with ethyl acetate (10mL), acidified with citric acid and extracted with dichloromethane (2x20mL). The organics were combined, dried over magnesium sulphate and concentrated *in vacuo.* The residue was purified by column chromatography on silica gel eluting with dichloromethane:methanol 100:0 to 95:5. The product was triturated with ether to yield the title product as a white solid, 45mg.
¹H NMR (CD₃OD, 400MHz) δ: 1.10 (d, 6H), 1.32, (d, 6H), 2.42 (s, 3H), 3.12 (s, 3H), 3.66 (m, 1H), 3.94 (t, 2H), 4.83 (t, 2H), 5.05 (m, 1H), 7.04 (d, 1H), 8.16 (s, 1H), 8.20 (d, 1H). MS ES+ m/z 428 [MH]⁺

### Example 112

### (2'R)-5-(N-Isopropyl-N-methylamino)-1-(2'-methoxypropyl)-7-(4-methylpyridin-2-ylamino)-1H-pyrazolo[4,3-d]pyrimidine-3-carboxylic acid

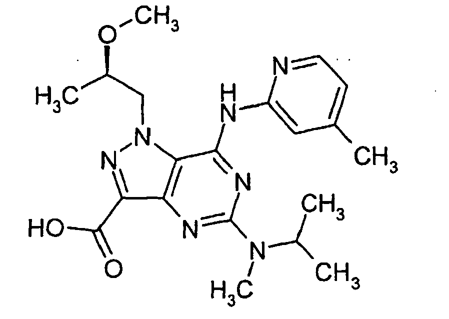

The title compound was prepared by a method similar to that described for example 111 using the ester of preparation 105.
¹H NMR (CD₃OD, 400MHz) δ: 1.32 (m, 9H), 2.44 (s, 3H), 3.10 (s, 3H), 3.42 (s, 3H), 3.97 (m, 1H), 4.73 (m, 2H), 4.99 (m, 1H), 7.08 (m, 1H), 8.08 (s, 1H), 8.20 (d, 1H). MS APCI+ m/z 414 [MH]⁺

### Example 113

### N-[1-(2-Ethoxyethyl)-5-(N-isopropyl-N-methylamino)-7-(4-methylpyridin-2-ylamino)-1H-pyrazolo[4,3-d]pyrimidine-3-carbonyl]methanesulfonamide

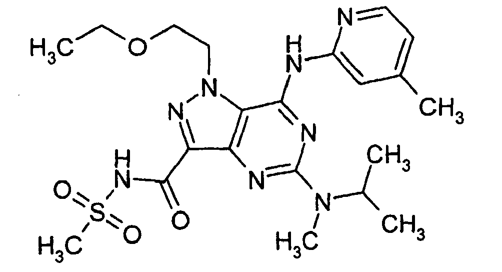

The chloro compound of preparation 186 (90mg, 0.20mmol), N-methyl-isopropylamine (73mg, 1.0mmol), N-ethyldiisopropylamine (170µL, 1.0mmol) and caesium fluoride (30mg, 0.20mmol) were dissolved in dimethyl sulphoxide (1mL) and the reaction mixture stirred at 110°C for 5 hours. The reaction mixture was allowed to cool and was then diluted with ethyl acetate (10mL) and water (10mL). The organic phase was separated and washed with water (2x10mL), dried over magnesium sulphate and concentrated *in vacuo.* The residue was purified twice by column chromatography on silica gel eluting with dichloromethane:methanol 99:1 to 97:3. The crude product was dissolved in ethyl acetate (2mL) and treated with pentane. The precipitate formed was filtered off and dried *in vacuo* to yield the title product, 42mg.
¹H NMR (DMSO-D₆ + CF₃CO₂D, 400MHz) δ: 0.98 (t, 3H), 1.18 (d, 6H), 2.44 (s, 3H), 3.02 (s, 3H), 3.40 (s, 3H), 3.44 (d, 2H), 3.86 (t, 2H), 4.75 (m, 1H), 4.94 (t, 2H), 7.18 (d, 1H), 8.06 (s, 1H), 8.25 (d, 1H). MS ES- m/z 489 [M-H]⁻

### Example 114

### N-[5-(Dimethylamino)-1-(2-ethoxyethyl)-7-(4-methylpyridin-2-ylamino)-1H-pyrazolo[4,3-d]pyrimidine-3-carbonyl]methanesulfonamide

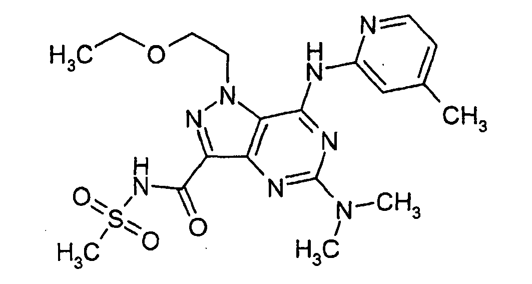

The title product was prepared by a method similar to that described for example 113 using a 33% solution of dimethylamine in ethanol as the source of the HNR³R⁴ amine. 55mg of the desired product was produced.
¹H NMR (DMSO-D₆ + CF₃CO₂D, 400MHz) δ: 0.97 (t, 3H), 2.45 (s, 3H), 3.20 (s, 6H), 3.40 (s, 3H), 3.44 (d, 2H), 3.88 (t, 2H), 4.95 (t, 2H), 7.18 (d, 1H), 8.07 (s, 1H), 8.25 (d, 1H), 13.40 (s, 1H). MS ES+ m/z 485 [MNa]⁺

### Example 115

### N-[1-(2-Ethoxyethyl)-5-(N-ethyl-N-methylamino)-7-(4-methylpyridin-2-ylamino)-1H-pyrazolo[4,3-d]pyrimidine-3-carbonyl]methanesulfonamide

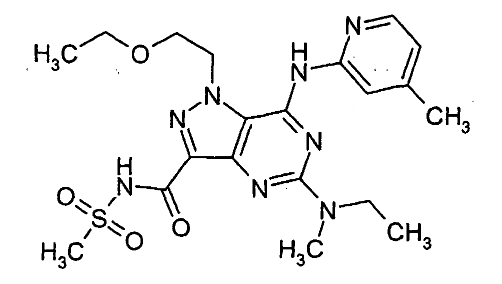

The chloro compound of preparation 186 (110mg, 0.24mmol), N-methyl-ethylamine (79mg, 1.2mmol), N-ethyldiisopropylamine (210µL, 1.20mmol) and caesium fluoride (37mg, 0.24mmol) were dissolved in dimethyl sulphoxide (1mL) and the reaction mixture heated to 110°C for 5 hours in a ReactiVial™. The reaction mixture was partitioned between ethyl acetate (10mL) and water (10mL) and the organic phase separated and washed with water (2x10mL). The organic phase was then dried over magnesium sulphate and concentrated *in vacuo.* The residue was purified by column chromatography on silica gel eluting with dichloromethane:methanol 99:1 to 97:3 to yield the title product as a pale yellow solid, 66mg.
Alternatively, example 115 may be prepared using the carboxylic acid of Example 96. The carboxylic acid of example 96 (1.0g, 2.50mmol), methanesulphonamide (356mg, 3.75mmol), 1-(3-dimethylaminopropyl)-3-ethylcarbodiimide hydrochloride (1.0g, 5.2mmol) and 4-dimethylaminopyridine (305mg, 2.5mmol) were dissolved in dichloromethane (5mL) and the reaction mixture stirred at room temperature for 16 hours. The reaction mixture was diluted with 10% aqueous citric acid (3mL) and the organic phase was separated, washed with water (3mL), dried over magnesium sulphate and concentrated *in vacuo.*
¹H NMR (DMSO-D₆ + CF₃CO₂D, 400MHz) δ: 0.99 (t, 3H), 1.17 (t, 3H), 2.44 (s, 3H), 3.18 (s, 3H), 3.41 (s, 3H), 3.44 (d, 2H), 3.66 (d, 2H), 3.88 (t, 2H), 4.93 (t, 2H), 7.16 (d, 1H), 8.09 (s, 1H), 8.26 (d, 1H). MS ES- m/z 475 [M-H]⁻

### Example 116

### Methyl 1-(2-ethoxyethyl)-5-[ethyl(methyl)amino]-7-[(4-fluoro-3-methylohenyl)amino]-1H-pyrazolo[4,3-d]pyrimidine-3-carboxylate

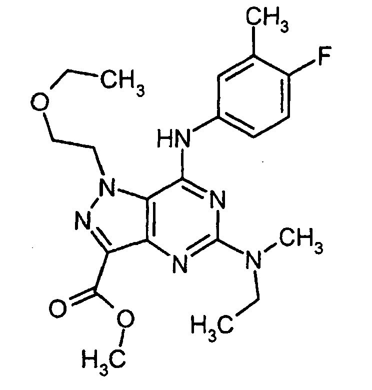

A mixture of the chloride from preparation 92 (200mg, 0.49mmol), N-ethylmethylamine (0.084mL, 0.98mmol) and N-ethyldiisopropylamine (0.17mL, 0.98mmol) in dimethylsulphoxide (2mL) was heated in a Reactivial® at 120°C for 18 hours. The cooled mixture was concentrated under reduced pressure and the residue partitioned between dichloromethane (100mL) and water (100mL) and the layers separated. The aqueous solution was extracted with further dichloromethane (50mL) and the combined organic solutions were washed with water (100mL), brine (50mL), dried over magnesium sulphate and evaporated under reduced pressure. The crude product was purified by column chromatography on an Isolute® silica gel cartridge using dichloromethane:methanol (100:0 to 95:5) as an elution gradient to provide the title compound as a white crystalline solid, 70mg.
¹H NMR (CD₃OD, 400MHz) δ: 1.15 (t, 3H), 1.20 (t, 3H), 2.30 (s, 3H), 3.19 (s, 3H), 3.60 (q, 2H), 3.70 (q, 2H), 3.96 (s, 3H), 3.98 (m, 2H), 4.80 (t, 2H), 7.01 (m, 1H), 7.42 (m, 1H), 7.67 (m, 1H).
MS APCI+ m/z431 [MH]⁺

### Example 117

### Methyl 5-(diethylamino)-1-(2-ethoxyethyl)-7-[(4-fluoro-3-methylphenyl)amino]-1H-pyrazolo[4,3-d]pyrimidine-3-carboxylate

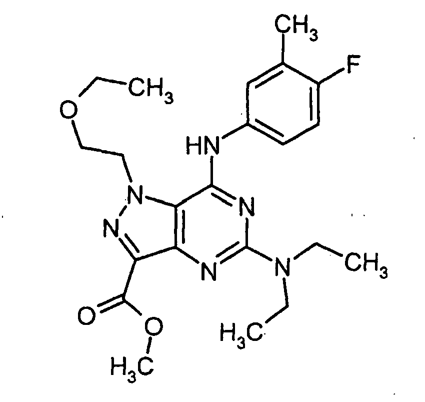

The title compound was obtained as a white crystalline solid from the compound from preparation 92, after re-crystallisation from methanol, following a similar procedure to that described in example 116.
¹H NMR (CDCl₃, 400MHz) δ: 1.20 (m, 9H), 2.30 (s, 3H), 3.65 (m, 6H), 4.00 (m, 5H), 4.75 (t, 2H), 6.95 (m, 1H), 7.35 (m, 1H), 7.60 (m, 1H).
MS APCI+ m/z445 [MH]⁺

### Example 118

### 1-(2-Ethoxyethyl)-5-[ethyl(methyl)amino]-7-[(3-methylphenyl)amino]-1H-pyrazolo[4,3-d]pyrimidine-3-carboxylic acid

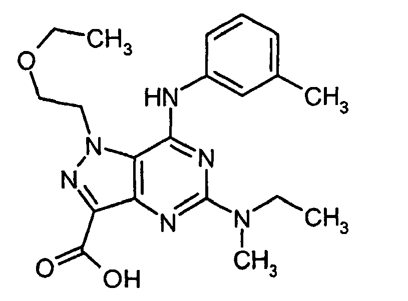

A mixture of the chloride from preparation 238 (200mg, 0.53mmol), cesium fluoride (81 mg, 0.53mmol) and *N*-ethylmethylamine (0.25mL, 2.65mmol) in dimethylsulphoxide (1.5mL) was heated in a Reactivial® at 110°C for 18 hours. The cooled mixture was partitioned between dichloromethane (50mL) and 1N citric acid solution (100mL) and the layers separated. The aqueous solution was extracted with further dichloromethane (50mL) and the combined organic solutions washed with water (2x100mL) and then brine (50mL). The solution was dried over magnesium sulphate and concentrated under reduced pressure to provide the title compound as a white solid, 150mg.
¹H NMR (400 MHz, CD₃OD) δ: 1.10 (t, 3H), 1.25 (t, 3H), 2.40 (s, 3H), 3.25 (s, 3H), 3.65 (q, 2H), 3.75 (q, 2H), 4.00 (t, 2H), 4.85 (t, 2H), 7.10 (d, 1H), 7.35 (m, 1H), 7.50 (d, 1H), 7.61 (s, 1H).
MS APCI+ m/z 399 [MH]⁺

### Examples 119 to 124

The following compounds of the general formula shown below: 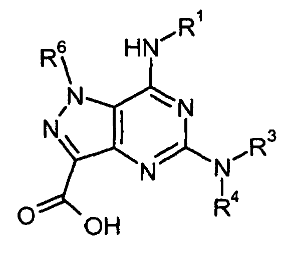 were prepared from the corresponding chloride compounds from preparations 149, 231, 232 and 237, following a similar procedure to that described in example 118.

| | | | |
|---|---|---|---|
| 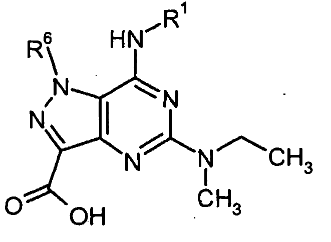 | | | |

| Ex | HNR¹, | R⁶ | Yield/Data |
|---|---|---|---|
| 119 | 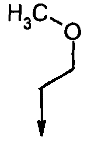 | 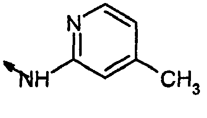 | Yellow crystals (93%) ¹H NMR (DMSO-D₆+drop TFA-d, 400MHz) δ: 1.14 (t, 3H), 2.45 (s, 3H), 3.20 (s, 6H), 3.63 (q, 2H), 3.81 (t, 2H), 4.98 (t, 2H), 7.16 (d, 1H), 8.11 (s, 1H), 8.24 (d, 1H). MS m/z 386 [MH]⁺ |
| 120 | 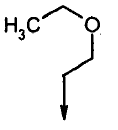 | 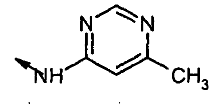 | Yellow solid (85%) ¹H NMR (DMSO-D₆+drop TFA-d, 400MHz) δ: 1.02 (t, 3H), 1.19 (t, 3H), 2.57 (s, 3H), 3.19 (s, 3H), 3.48 (q, 2H), 3.68 (q, 2H), 3.83 (t, 2H), 4.82 (t, 2H), 8.08 (s, 1H), 9.02 (s, 1H). MS m/z 401 [MH]⁺ |
| 121 | 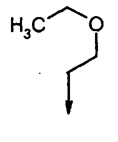 | 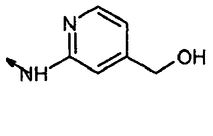 | Yellow oil, (88%) ¹H NMR (DMSO-D₆, 400MHz) δ: 0.95 (t, 3H), 1.15 (t, 3H), 3.17 (s, 3H), 3.39 (q, 2H), 3.66 (q, 2H), 3.83 (t, 2H), 4.63 (s, 2H), 4.95 (t, 2H), 7.14 (d, 1H), 8.19 (d, 1H), 8.34 (s, 1H). MS m/z 416 [MH]⁺ |
| 122^{A} | 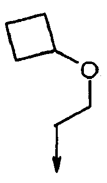 | 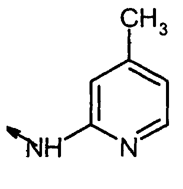 | Yellow powder (50%) ¹H NMR (CD₃OD, 400MHz) δ: 1.30 (t, 3H), 1.50 (m, 1H), 1.60 (m, 1H), 1.90 (m, 2H), 2.12 (m, 2H), 2.45 (s, 3H), 3.30 (s, 3H), 3.80 (q, 2H), 3.90 (t, 2H), 4.00 (m, 1H), 4.90 (m, 2H), 7.05 (d, 1H), 8.20 (m, 2H). |
| | | | |
| 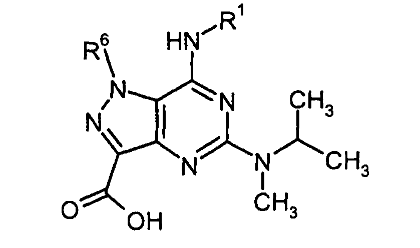 | | | |
| 123 | 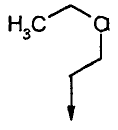 | 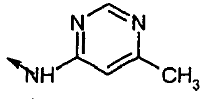 | Yellow solid (69%) ¹H NMR (DMSO-D₆+drop TFA-d, 400MHz) δ: 1.02 (t, 3H), 1.21 (d, 6H), 2.57 (s, 3H), 3.02 (s, 3H), 3.48 (q, 2H), 3.82 (t, 2H), 4.82 (t, 2H), 4.95 (m, 1H), 8.04 (s, 1H), 9.03 (s, 1H). MS m/z 415 [MH]⁺ |
| 124^{A} | 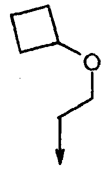 | 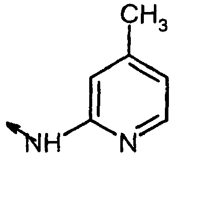 | Pale yellow powder (45%) ¹H NMR (CD₃OD, 400MHz) δ: 1.35 (d, 6H), 1.45 (m, 1H), 1.60 (m, 1H), 1.90 (m, 2H), 2.10 (m, 2H), 2.45 (s, 3H), 3.15 (s, 3H), 3.30 (m, 1H), 3.90 (t, 2H), 4.05 (m, 1H), 5.02 (m, 2H), 7.05 (d, 1H), 8.20 (m, 2H). MS APCI+ m/z 440 [MH]⁺ |

A-The product was recrystallised from dichloromethane, then sonicated in ether and dried *in vacuo.*

### Example 125

### 5-(Diethylamino)-1-(2-ethoxyethyl)-7-[(1-methyl-6-oxo-1,6-dihydropyridin-3-yl)amino]-1H-pyrazolo[4,3-d]pyrimidine-3-carboxylic acid

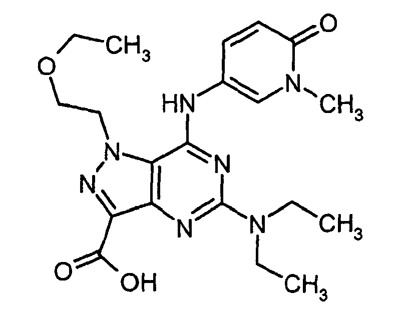

A mixture of the chloro compound from preparation 239 (170mg, 0.43mmol), diethylamine (0.18mL, 1.73mmol) and cesium fluoride (66mg, 0.43mmol) in dimethylsulphoxide (1mL) was stirred at 110°C for 2 hours. Additional diethylamine (0.18mL, 1.73mmol) in dimethylsulphoxide (0.5mL) was added, the mixture transferred to a Reactivial® and stirred at 110°C for a further 2 hours. The cooled mixture was diluted with dichloromethane (40mL) and washed with 1M citric acid solution (3x20mL). The combined aqueous solutions were washed with dichloromethane (20mL), then basified to pH 6 using solid sodium bicarbonate. This solution was extracted with dichloromethane (3x30mL) and the combined organic solutions washed with water (20mL) and brine (20mL) then dried (using a phase separation cartridge) and concentrated under reduced pressure. The resulting oil was suspended in water (30mL) and the mixture sonicated for 30 minutes. The resulting solid was filtered off and dried *in vacuo* to provide the title compound as a solid.
¹H NMR (CD₃OD, 400MHz) δ: 1.12 (t, 3H), 1.25 (t, 6H), 3.59 (q, 2H), 3.62 (s, 3H), 3.65 (m, 4H), 3.92 (t, 2H), 4.90 (m, 2H), 6.65 (d, 1H), 7.75 (m, 1H), 8.10 (s, 1H). MS ES- m/z 428 [M-H]⁻

### Example 126

### 5-[Isopropyl(methyl)amino]-7-[(4-methylpyridin-2-yl)amino]-1-[2-(2,2,2-trifluoroethoxy)ethyl]-1H-pyrazolo[4,3-d]pyrimidine-3-carboxylic acid

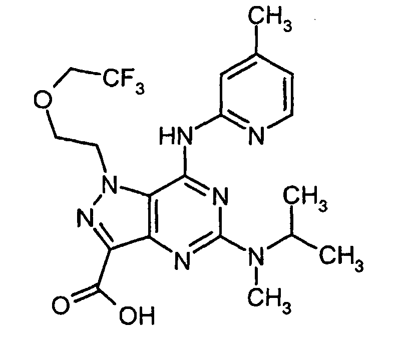

A mixture of the chloride from preparation 233 (75mg, 0.17mmol), N-ethyldiisopropylamine (0.15mL, 0.85mmol), cesium fluoride (26mg, 0.17mmol) and N-methylisopropylamine (0.09mL, 0.85mmol) in 1-methyl-2-pyrrolidinone (1 mL) was stirred at 110°C in a Reactivial® for 4 hours.
The cooled reaction mixture was purified directly using a Phenomenex Luna C18 reverse phase silica gel column and acetonitrile:95% water/5% methanol/0.1% trifluoroacetic acid (5:95 to 95:5) as elution gradient. The product was dissolved in dichloromethane and the solution washed with sodium bicarbonate solution, dried over magnesium sulphate and evaporated under reduced pressure to provide the title compound, 24mg.
¹H NMR (CD₃OD, 400MHz) δ: 1.31 (d, 6H), 2.48 (s, 3H), 3.11 (s, 3H), 3.98 (q, 2H), 4.15 (t, 2H), 4.95 (m, 1H), 4.99 (t, 2H), 7.08 (d, 1H), 8.00 (s, 1H), 8.12 (d, 1H). MS ES- m/z 466 [M-H]⁻

### Example 127

### 5-[Ethyl(methyl)amino]-7-[(4-methylpyridin-2-yl)amino]-1-[2-(2,2,2-trifluoroethoxy)ethyl]-1H-pyrazolo[4,3-d]pyrimidine-3-carboxylic acid

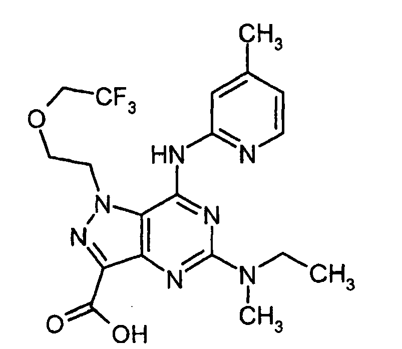

The title compound was prepared from the compound from preparation 233 and N-ethylmethylamine, following a similar procedure to that described in example 126, except only 2 equivalents of N-ethylmethylamine and N-ethyldiisopropylamine were used.
¹H NMR (CD₃OD, 400MHz) δ: 1.30 (t, 3H), 2.49 (s, 3H), 3.26 (s, 3H), 3.73 (q, 2H), 3.98 (q, 2H), 4.16 (t, 2H), 4.98 (t, 2H), 7.05 (d, 1H), 8.00 (s, 1H), 8.10 (d, 1H).
MS ES- m/z 452 [M-H]⁻

### Example 128

### 5-(Diethylamino)-7-[(4-methylpyridin-2-yl)amino]-1-[2-(2,2,2-trifluoroethoxy)ethyl]-1H-pyrazolo[4,3-d]pyrimidine-3-carboxylic acid

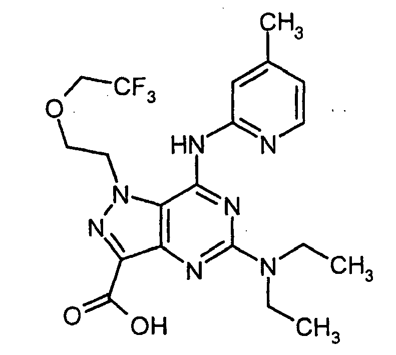

A mixture of the chloride from preparation 233 (100mg, 0.23mmol), cesium fluoride (35mg, 0.23mmol) and diethylamine (0.07mL, 0.69mmol) in dimethylsulphoxide (1 mL) was stirred at 120°C in a Reactivial® for 18 hours.

Tlc analysis showed starting material remaining, so additional diethylamine (0.07mL, 0.69mmol) was added and the reaction heated for a further 3 hours at 135°C. The cooled mixture was suspended in 1M citric acid solution (200mL) and extracted with dichloromethane (3x50mL). The combined organic extracts were washed with water (50mL), brine (25mL) and dried over sodium sulphate then concentrated under reduced pressure. The crude product was purified by column chromatography using a silica gel Isolute® cartridge and an elution gradient of 10% acetic acid in methanol:dichloromethane: (1:99 to 7:93). The product was triturated with ether and dried *in vacuo* to afford the title compound as a yellow powder, 44mg.
¹H NMR (CD₃OD, 400MHz) δ: 1.35 (t, 6H), 2.50 (s, 3H), 3.70 (q, 4H), 4.00 (q, 2H), 4.18 (t, 2H), 5.00 (t, 2H), 7.10 (d, 1H), 8.05 (s, 1H), 8.13 (d, 1H).
MS ES+ m/z 468 [MH]⁺

### Example 129

### 7-[(4-Methylpyridin-2-yl)amino]-5-(2-methylpyrrolidin-1-yl)-1-[2-(2,2,2-trifluoroethoxy)ethyl]-1H-pyrazolo[4,3-d]pyrimidine-3-carboxylic acid

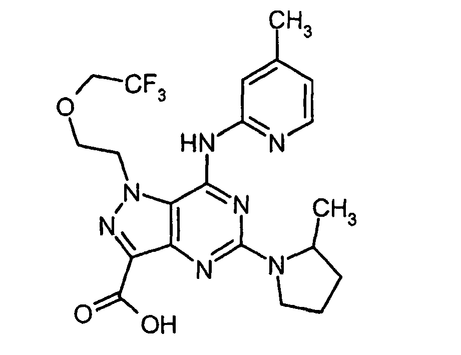

The title compound was obtained as a solid in 45% yield from 2-methylpyrrolidine and the chloride from preparation 233, following the procedure described in example 128.
¹H NMR (CD₃OD, 400MHz) δ: 1.38 (d, 3H), 1.85 (m, 1H), 2.10 (m, 1H), 2.25 (m, 2H), 2.50 (s, 3H), 3.60 (m, 1H), 3.79 (m, 1H), 3.98 (q, 2H), 4.15 (t, 2H), 4.45 (m, 1H), 5.00 (t, 2H), 7.10 (d, 1H), 7.98 (s, 1H), 8.15 (d, 1H).
MS ES+ m/z480 [MH]⁺

### Example 130

### 5-[Cyclobutyl(methyl)amino]-7-[(4-methylpyridin-2-yl)amino]-1-[2-(2,2,2-trifluoroethoxy)ethyl]-1H-pyrazolo[4,3-d]pyrimidine-3-carboxylic acid

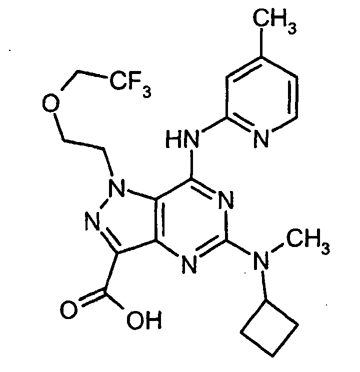

The title compound was obtained in 42% yield as a yellow solid, from the amine from preparation 241 and the chloro compound from preparation 233, following a similar procedure to that described in example 128, except 5eq N-ethyldiisopropylamine was also added.
¹H NMR (CD₃OD, 400MHz) δ: 1.80 (m, 2H), 2.35 (m, 4H), 2.50 (s, 3H), 3.21 (s, 3H), 3.99 (q, 2H), 4.18 (t, 2H), 4.80 (m, 1H), 4.99 (t, 2H), 7.10 (d, 1H), 8.05 (s, 1H), 8.18 (d, 1H).
MS APCI+ m/z480 [MH]⁺

### Examples 131 to 133

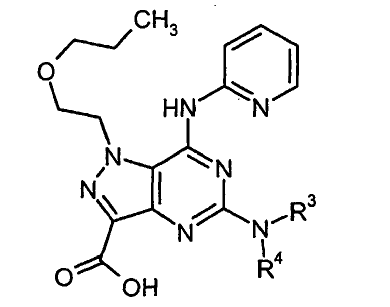

A solution of the appropriate esters from preparations 240-242 (0.5mmol) in sodium hydroxide (1 N, 4mL, 4mmol) and dioxan (2 mL) was stirred at room temperature for 18 hours. The solution was concentrated under reduced pressure and the residue partitioned between dichloromethane (20mL) and 1 M citric acid solution (10mL). The layers were separated and the organic phase dried over magnesium sulphate and evaporated under reduced pressure to give the title compounds.

| Ex No | -NR³R⁴ | Data |
|---|---|---|
| 131 | 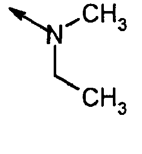 | ¹H NMR (DMSO-D₆ + 1dp TFAD), 400MHz) δ: 0.65 (t, 3H), 1.18 (t, 3H), 1.38 (m, 2H), 3.18 (s, 3H), 3.34 (t, 2H), 3.62 (q, 2H), 3.82 (t, 2H), 4.98 (t, 2H), 7.28 (m, 1H), 8.20 (m, 2H), 8.38 (d, 1H). MS APCI+ m/z400 [MH]⁺ |
| 132 | 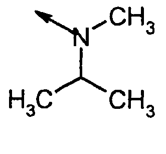 | ¹H NMR (DMSO-D₆+ 1 dp TFAD, 400MHz) δ: 0.65 (t, 3H), 1.18 (d, 6H), 1.38 (m, 2H), 3.04 (s, 3H), 3.22 (t, 2H), 3.84 (t, 2H), 4.70 (m, 1H), 4.98 (t, 2H), 7.30 (m, 1H), 8.20 (m, 2H), 8.40 (d, 1H). MS APCI+ m/z414 [MH]⁺ |
| 133 | 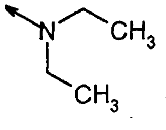 | ¹H NMR (DMSO-D₆+ 1dp TFAD, 400MHz) δ: 0.66 (t, 3H), 1.20 (t, 6H), 1.39 (m, 2H), 3.30 (t, 2H), 3.62 (q, 4H), 3.84 (t, 2H), 4.98 (t, 2H), 7.28 (m, 1H), 8.20 (m, 2H), 8.38 (d, 1H). MS APCI+ m/z414 [MH]⁺ |

### Example 134

### 1-(2-Ethoxyethyl)-7-[(4-methylpyridin-2-yl)amino]-5-pyrrolidin-1-yl-1H-pyrazolo[4,3-d]pyrimidine-3-carboxylic acid

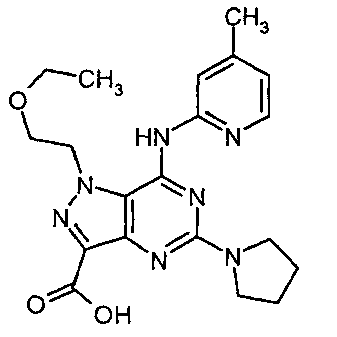

A solution of the ester from preparation 259 (50mg, 0.12mmol) in 1 N sodium hydroxide solution (1 mL) and dioxan (0.5mL) was stirred at room temperature for 18 hours. The mixture was diluted with 1 M citric acid solution (50mL) and extracted with dichloromethane (3x200mL). The combined organic extracts were dried over magnesium sulphate and evaporated under reduced pressure. The crude product was purified by column chromatography using an Isolute® silica gel cartridge and an elution gradient of dichloromethane:methanol (100:0 to 90:10) to provide the title compound as a yellow solid, 23mg.
¹H NMR (CD₃OD,400MHz) δ: 1.10 (t, 3H), 2.10 (m, 4H), 2.45 (s, 3H), 3.59 (m, 2H), 3.70 (m, 4H), 3.90 (t, 2H), 4.90 (m, 2H), 7.05 (d, 1H), 8.20 (m, 2H).
MS APCI+ m/z 412 [MH]⁺

### Example 135

### 5-[Isopropyl(methyl)amino]-1-[(2S)-2-methoxypropyl]-7-[(4-methylpyridin-2-yl)amino]-1H-pyrazolo[4,3-d]pyrimidine-3-carboxylic acid

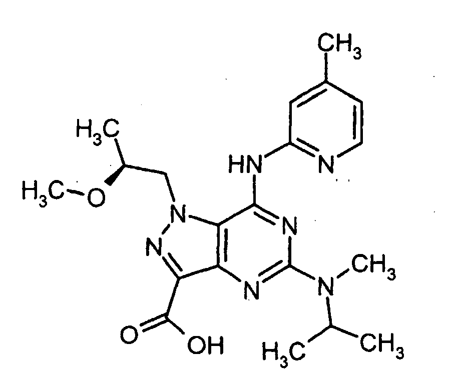

A solution of the ester from preparation 260 (43mg, 0.1 mmol) in dioxan (2mL) and sodium hydroxide (1N, 4mL) was stirred at room temperature for 18 hours. The mixture was concentrated under reduced pressure and the residue diluted with citric acid solution (1M, 50mL). This solution was extracted with dichloromethane (3x50mL), the combined organic solutions washed with sodium bicarbonate solution (3x15mL), dried over magnesium sulphate and evaporated under reduced pressure. The crude product was purified by column chromatography using an Isolute® silica gel cartridge and an elution gradient of dichloromethane:methanol (100:0 to 94:6) to give a yellow oil. This was triturated with ether and the resulting solid filtered off and dried to give the title compound as a white solid, 26mg.
¹H NMR (CD₃OD,400MHz) δ: 1.30 (m, 9H), 2.42 (s, 3H), 3.10 (s, 3H), 3.43 (s, 3H), 4.00 (m, 1H), 4.75 (m, 2H), 4.99 (m, 1H), 7.02 (d, 1H), 8.08 (s, 1H), 8.10 (d, 1H).
MS APCI+ m/z 414 [MH]⁺

### Examples 136 to 140

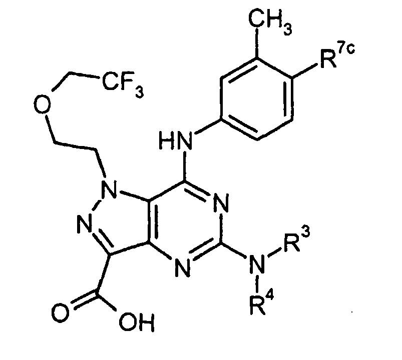

A solution of the appropriate esters from preparations 245-249 (1eq) in sodium hydroxide (1N, 1.5-3eq) and dioxan (6.5-7.5 mLmmol⁻¹) was stirred at room temperature for 18 hours. The solution was concentrated under reduced pressure and the residue partitioned between dichloromethane and 1 M citric acid solution and the layers separated. The aqueous phase was extracted with additional dichloromethane, the combined organic solutions dried over magnesium sulphate and evaporated under reduced pressure. The products were triturated with ethyl acetate, and the solids filtered and dried to afford the title compounds as white crystalline solids.

| Ex No | -NR³R⁴ | R^{7c} | Data |
|---|---|---|---|
| 136 | 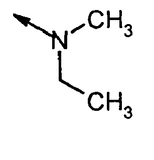 | H | ¹H NMR (CD₃OD, 400MHz) δ: 1.20 (t, 3H), 2.38 (s, 3H), 3.24 (s, 3H), 3.57 (q, 2H), 4.02 (q, 2H), 4.15 (t, 2H), 4.94 (t, 2H), 7.10 (d, 1H), 7.32 (m, 1H), 7.40 (d, 1H), 7.50 (s, 1H). MS APCI+ m/z453 [MH]⁺ |
| 137 | 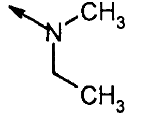 | F | ¹H NMR (CD₃OD, 400MHz) δ: 1.08 (t, 3H), 2.30 (s, 3H), 3.22 (s, 3H), 3.64 (q, 2H), 4.00 (q, 2H), 4.10 (t, 2H), 4.94 (t, 2H), 7.10 (d, 1H), 7.40 (m, 1H), 7.50 (s, 1H). MS APCI+ m/z471 [MH]⁺ |
| 138 | 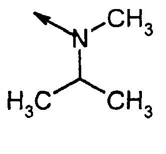 | H | ¹H NMR (CD₃OD, 400MHz) δ: 1.22 (d, 6H), 2.40 (s, 3H), 3.08 (s, 3H), 4.04 (q, 2H), 4.13 (t, 2H), 4.85 (m, 1H), 4.94 (t, 2H), 7.12 (d, 1H), 7.32 (m, 1H), 7.39 (d, 1H), 7.50 (s, 1H). MS APCI+ m/z467 [MS]⁺ |
| 139 | 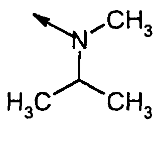 | F | ¹H NMR (CD₃OD, 400MHz) δ: 1.22 (d, 6H), 2.30 (s, 3H), 3.08 (s, 3H), 4.00 (q, 2H), 4.12 (t, 2H), 4.80 (m, 1H), 4.94 (t, 2H), 7.10 (m, 1H), 7.37 (m, 1H), 7.49 (m, 1H). MS APCI+ m/z 485 [MH]⁺ |
| 140 | 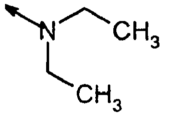 | F | ¹H NMR (CD₃OD, 400MHz) δ: 1.24 (t, 6H), 2.31 (s, 3H), 3.62 (q, 4H), 4.02 (q, 2H), 4.15 (t, 2H), 4.86 (t, 2H), 7.10 (m, 1H), 7.37 (m, 1H), 7.52 (m, 1H). MS APCI+ m/z 485 [MH]⁺ |

### Examples 141 to 146

Sodium hydroxide solution (1M, 3eq) was added to a solution of the esters from preparations 253-258 (1 eq) in dioxane (8.5-10.5mLmmol⁻¹), and the reaction mixture stirred at room temperature for 18 hours. The solvent was removed under reduced pressure and the residue partitioned between citric acid (15mL) and dichloromethane (15mL). The phases were separated and the organic layer evaporated under reduced pressure to provide the title compounds.

| | | | |
|---|---|---|---|
| 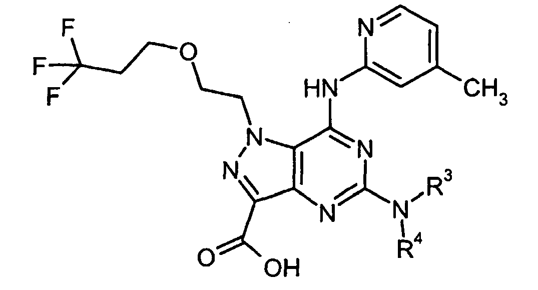 | | | |

| Ex. No | | Yield(%) | Data |
|---|---|---|---|
| 141 | 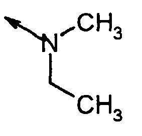 | 96 | MS APCI+ m/z 468 [MH]⁺ |
| 142 | 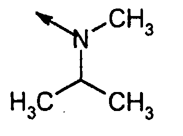 | 89 | MS APCI+ m/z 482 [MH]⁺ |
| 143 | 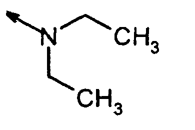 | 99 | MS APCI+ m/z 482 [MH]⁺ |
| 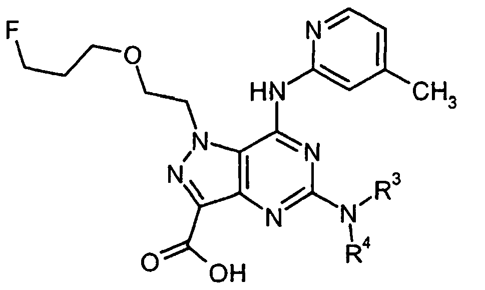 | | | |
| 144 | 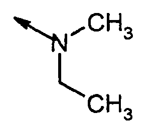 | 99 | MS APCI+ m/z 432 [MH]⁺ |
| 145 | 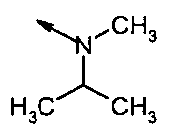 | 90 | MS APCI+ m/z 446 [MH]⁺ |
| 146 | 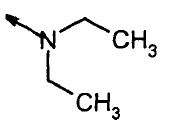 | 95 | MS APCI+ m/z 446 [MH]⁺ |

### Example 147

### 2-(Dimethylamino)ethyl 1-(2-ethoxyethyl)-5-[isopropyl(methyl)amino]-7-[(4-methylpyridin-2-yl)amino]-1H-pyrazolo[4,3-d]pyrimidine-3-carboxylate

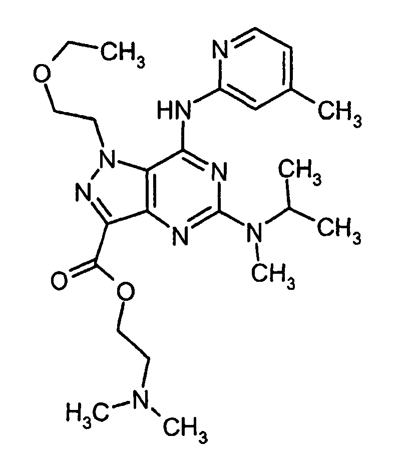

A mixture of the chloride from preparation 176 (160mg, 0.36mmol), cesium fluoride (54mg, 0.36mmol) and N-methylisopropylamine (186µl, 1.79mmol) in dimethylsulphoxide (3mL) was heated at 110°C in a Reactivial® for 18 hours. The cooled mixture was partitioned between dichloromethane (20mL) and water (20mL) and the layers separated. The aqueous solution was extracted further with dichloromethane (20mL) and the combined organic solutions washed with water (3x20mL), dried over magnesium sulphate and evaporated under reduced pressure. The residual orange oil was purified by column chromatography using an Isolute® silica gel cartridge and an elution gradient of methanol:dichloromethane (0:100 to 10:90), and then on reverse phase silica gel using acetonitrile:water :trifluoroacetic acid (95:5:0.1) as eluant to provide the title compound, 15mg.
¹H NMR (CD₃OD, 400MHz) δ: 1.09 (t, 3H), 1.33 (d, 6H), 2.54 (s, 3H), 3.05 (s, 6H), 3.16 (s, 3H), 3.51 (q, 2H), 3.65 (t, 2H), 3.98 (t, 2H), 4.78 (t, 2H), 4.89 (m, 1H), 5.04 (t, 2H), 7.18 (d, 1H), 8.08 (s, 1H), 8.14 (d, 1H).
MS m/z 485 [MH]⁺

### Examples 148 to 164

4-Dimethylaminopyridine (1.3 eq) was added to a solution of the appropriate acid from examples 11,14,15,17, 38, 96, 118-124, 136, 137 and 139 (1eq), 1-(3-dimethylaminopropyl)-3-ethylcarbodiimide hydrochloride (1.3eq) and the appropriate sulphonamide (1.2-1.3eq) in dichloromethane (13-30mLmmol⁻¹) and the reaction stirred at room temperature for 18 hours. The mixture was diluted with dichloromethane, washed with 1 M citric acid solution, dried over magnesium sulphate and evaporated under reduced pressure to afford the title compounds.

| | |
|---|---|
| | 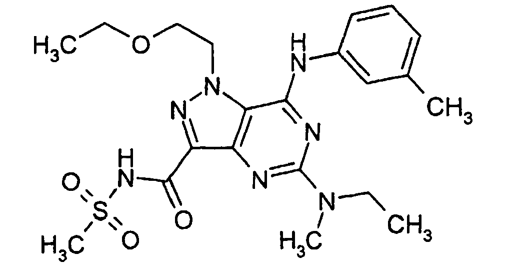 |
| 148^{A} | ¹H NMR (CDCl₃, 400MHz) δ: 1.15 (t, 3H), 1.25 (t, 3H), 2.38 (s, 3H), 3.20 (s, 3H), 3.38 (s, 3H), 3.62 (q, 2H), 3.70 (q, 2H), 3.98 (t, 2H), 4.82 (t, 2H), 7.00 (d, 1H), 7.25 (m, 1H), 7.50 (d, 1H), 7.70 (s, 1H). MS APCI+ m/z 476 [MH]⁺ |

| | |
|---|---|
| | 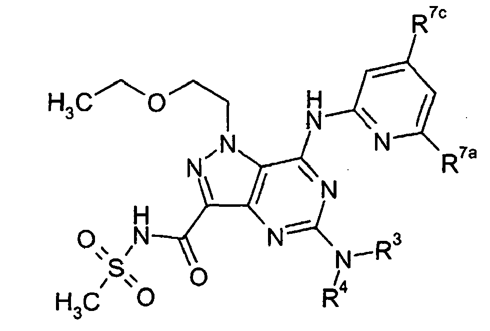 |
| 149 | R³ = -CH₂CH₃; R⁴ = -CH₃; R^{7A} = CH₃; R^{7C} = H ¹H NMR (CDCl₃, 400MHz) δ: 1.19 (t, 3H), 1.26 (t, 3H), 2.50 (s, 3H), 3.23 (s, 3H), 3.41 (s, 3H), 3.65 (q, 2H), 3.75 (q, 2H), 3.97 (t, 2H), 4.87 (m, 2H), 6.98 (d, 1H), 7.71 (m, 1H), 8.18 (br m, 1H). MS m/z 477 [MH]⁺ |
| 150^{A} | R³ = -(CH₂)₂CH₃; R⁴ = -CH₃; R^{7A} = H; R^{7C} = CH₃ ¹H NMR (DMSO-D₆+ drop TFAd, 400MHz) δ: 0.88 (t, 3H), 1.01 (t, 3H), 1.63 (m, 2H), 2.41 (s, 3H), 3.18 (s, 3H), 3.42 (s, 3H), 3.47 (q, 2H), 3.55 (t, 2H), 3.87 (t, 2H), 4.89 (m, 2H), 7.09 (m, 1H), 8.05 (s, 1H), 8.24 (d, 1H). MS ESI+ m/z 491 [MH]⁺ |
| 151^{A} | R³ = -CH₂CH₃; R⁴ = -CH₂CH₃; R^{7A} = H; R^{7C} = CH₃ ¹H NMR (DMSO-D₆+ drop TFAd, 400MHz) δ: 1.02 (t, 3H), 1.20 (t, 6H), 2.39 (s, 3H), 3.41 (s, 3H), 3.48 (q, 2H), 3.61 (q, 4H), 3.87 (t, 2H), 4.87 (m, 2H), 7.08 (m, 1H), 8.10 (s, 1H), 8.24 (d, 1H). MS ESI+ m/z 491 [MH]⁺ |
| 152^{A} | R³ = -(CH₂)₂OCH₃; R⁴ = -CH₃; R^{7A} = H; R^{7C} = CH₃ ¹H NMR (DMSO-D₆+ drop TFAd, 400MHz) δ: 0.99 (t, 3H), 2.44 (s, 3H), 3.21 (s, 3H), 3.30 (br s, 3H), 3.40 (s, 3H), 3.44 (q, 2H), 3.62 (m, 2H), 3.77 (m, 2H), 3.88 (t, 2H), 4.93 (t, 2H), 7.14 (d, 1H), 8.06 (s, 1H), 8.24 (d, 1H). MS ESI+ m/z 507 [MH]⁺ |
| 153 | R³ = -CH₂CH₃; R⁴ = -CH₃; R^{7A} = H; R^{7C} = CH₂OH ¹H NMR (CD₃OD+ drop TFAd, 400MHz) δ: 1.10 (t, 3H), 1.30 (t, 3H), 3.28 (s, 3H), 3.40 (s, 3H), 3.56 (q, 2H), 3.79 (q, 2H), 3.99 (q, 2H), 4.78 (s, 2H), 5.06 (m, 2H), 7.24 (d, 1H), 8.19 (s, 1H), 8.40 (s, 1H). MS ESI- m/z 491 [M-H]⁻ |
| | 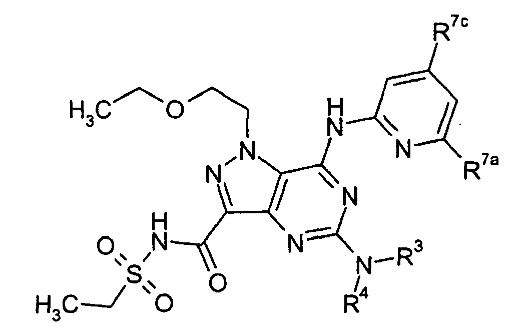 |
| 154 ^{A, B} | R³ = -CH₃; R⁴ = -CH₃; R^{7A} = H; R^{7C} = CH₃ |
| | ¹H NMR, (CD₃OD, 400MHz) δ: 1.13 (t, 3H), 1.41 (t, 3H), 2.43 (s, 3H), 3.29 (s, 6H), 3.57 (q, 2H), 3.62 (q, 2H), 3.96 (t, 2H), 4.87 (m, 2H), 6.98 (d, 1H), 8.18 (d, 1H), 8.36 (s, 1H). MS m/z 477 [MH]⁺ |
| 155 ^{A, B} | R³ = -CH₂CH₃; R⁴ = -CH₃; R^{7A} = H; R^{7C} = CH₃ |
| | ¹H NMR (CD₃OD, 400MHz) δ: 1.14 (t, 3H), 1.29 (t, 3H), 1.41 (t, 3H), 2.43 (s, 3H), 3.25 (s, 3H), 3.57 (q, 2H), 3.63 (q, 2H), 3.78 (q, 2H), 3.97 (t, 2H), 4.87 (m, 2H), 6.99 (d, 1H), 8.18 (d, 1H), 8.35 (s, 1H). MS m/z 491 [MH]⁺ |
| 156 ^{A, C} | R³ = -CH₂CH₃; R⁴ = -CH₂CH₃; R^{7A} = H; R^{7C} = CH₃ |
| | ¹H NMR (CD₃OD, 400MHz) δ: 1.14 (t, 3H), 1.32 (t, 6H), 1.40 (t, 3H), 2.43 (s, 3H), 3.56 (q, 2H), 3.63 (q, 2H), 3.73 (q, 4H), 3.97 (t, 2H), 4.85 (m, 2H), 6.98 (d, 1H), 8.19 (d, 1H), 8.34 (s, 1H). MS m/z 505 [MH]⁺ |
| | 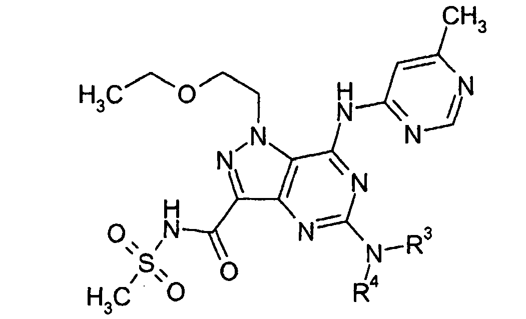 |
| 157 | R³ = -CH₂CH₃; R⁴ = -CH₃ |
| | ¹H NMR (CD₃OD, 400MHz) δ: 1.21 (t, 3H), 1.30 (t, 3H), 2.54 (s, 3H), 3.27 (s, 3H), 3.43 (s, 3H), 3.67 (q, 2H), 3.79 (q, 2H), 3.98 (t, 2H), 4.84 (t, 2H), 8.30 (s, 1H), 8.71 (s, 1H). MS m/z 478 [MH]⁺ |
| 158 | R³ = -CH₂CH₃; R⁴ = -CH₂CH₃ |
| | ¹H NMR (CD₃OD, 400MHz) δ: 1.21 (t, 3H), 1.33 (t, 6H), 2.54 (s, 3H), 3.43 (s, 3H), 3.67 (q, 2H), 3.75 (q, 4H), 3.98 (t, 2H), 4.86 (t, 2H), 8.31 (s, 1H), 8.72 (s, 1H). MS m/z 492 [MH]⁺ |
| | 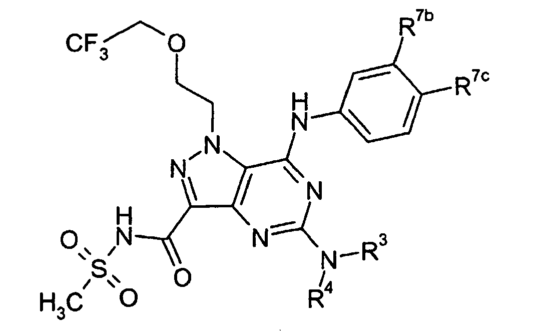 |
| 159^{D} | R³ = -CH₂CH₃; R⁴ = -CH₃; R^{7B} = -CH₃; R^{7C} = F |
| | ¹H NMR (CDCl₃, 400MHz) δ: 1.18 (m, 3H), 2.30 (s, 3H), 3.17 (s, 3H), 3.43 (s, 3H), 3.66 (q, 2H), 3.93 (q, 2H), 4.26 (q, 2H), 4.80 (q, 2H), 7.00 (dd, 1H), 7.36 (br s, 1H), 8.05 (s, 1H). MS APCI+ m/z 548 [MH]⁺ |
| 160^{D} | R³ = -CH₂CH₃; R⁴ = -CH₂CH₃; R^{7B} = -CH₃; R^{7C} = F ¹H NMR (CDCl₃, 400MHz) δ: 1.20 (m, 6H), 2.28 (s, 3H), 3.42 (s, 3H), 3.60 (q, 4H), 3.94 (q, 2H), 4.25 (t, 2H), 4.81 (t, 2H), 7.00 (dd, 1H), 7.30 (m, 1H), 7.50 (m, 1H), 8.06 (br s, 1H). MS APCI+ m/z 562 [MH]⁺ |
| 161^{D} | R³ = -CH₂CH₃; R⁴ = -CH₃; R^{7B} = -CH₃; R^{7C} = H ¹H NMR (CDCl₃, 400MHz) δ: 1.20 (t, 3H), 2.38 (s, 3H), 3.17 (s, 3H), 3.44 (s, 3H), 3.67 (q, 2H), 3.95 (q, 2H), 4.26 (t, 2H), 4.82 (t, 2H), 6.95 (d, 1H), 7.26 (m, 1H), 7.40 (m, 1H), 7.50 (s, 1H), 8.18 (s, 1H). MS APCI+ m/z 530 [MH]⁺ |
| | 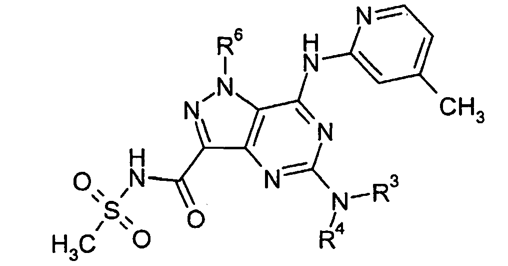 |
| 162 | R⁶ = -(CH₂)₂OCH₃; R³ = -CH₂CH_{3;} R⁴ = -CH₃ |
| | ¹H NMR (CD₃OD, 400MHz) δ: 1.31 (t, 3H), 2.54 (s, 3H), 3.30 (s, 3H), 3.37 (s, 3H), 3.41 (s, 3H), 3.77 (q, 2H), 3.94 (t, 2H), 5.07 (t, 2H), 7.19 (d, 1H), 8.06 (s, 1H), 8.14 (d, 1H). MS m/z 463 [MH]⁺ |
| 163^{A,E} | 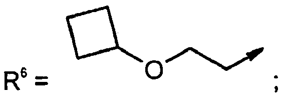 R³ = -CH₂CH_{3;} R⁴ = -CH₃ |
| | ¹H NMR (CD₃OD, 400MHz) δ: 1.30 (t, 3H), 1.45 (m, 1H), 1.60 (m, 1H), 1.90 (m, 2H), 2.10 (m, 2H), 2.41 (s, 3H), 3.25 (s, 3H), 3.42 (s, 3H), 3.75 (q, 2H), 3.90 (t, 2H), 4.00 (m, 1H), 4.85 (m, 2H), 7.00 (d, 1H), 8.20 (m, 1H), 8.35 (m, 1H). MS APCI+ m/z 503 [MH]⁺ |
| 164 ^{A,E} | 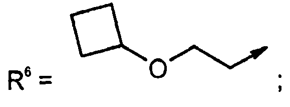 R³ = -CH(CH₃)_{2;} R⁴ = -CH₃ |
| | ¹H NMR (CD₃OD, 400MHz) δ: 1.30 (d, 6H), 1.50 (m, 1H), 1.62 (m, 1H), 1.90 (m, 2H), 2.10 (m, 2H), 2.40 (s, 3H), 3.10 (s, 3H), 3.40 (s, 3H), 3.85 (t, 2H), 4.00 (m, 1H), 4.85 (m, 2H), 5.05 (m, 1H), 6.99 (d, 1H), 8.20 (m, 1H), 8.30 (m, 1H). MS APCI+ m/z 517 [MH]⁺ |

A-crude compounds were purified by column chromatography on an Isolute® silica gel cartridge using dichloromethane:methanol as eluant.

B-an additional 0.5eq of sulphonamide and 4-dimethylaminopyridine were added after 18 hours, and the reaction stirred for a further 6 hours.

C-1.5 eq of 1-(3-dimethylaminopropyl)-3-ethylcarbodiimide hydrochloride, 4-dimethylaminopyridine and ethylsulphonamide were used.

D-the compound was isolated after trituration with methanol. E-4-dimethylaminopyridine (0.5eq), 1-(3-dimethylaminopropyl)-3-ethylcarbodiimide hydrochloride (2eq) and the appropriate sulphonamide (2eq) were used.

### Examples 165 to 171

4-Dimethylaminopyridine (1.3 eq) was added to a solution of the appropriate acid from examples 20 and 131-133 (1eq), 1-(3-dimethylaminopropyl)-3-ethylcarbodiimide hydrochloride (1.3eq) and the appropriate sulphonamide (1.2-1.3eq) in dichloromethane (13-30mLmmol⁻¹) and the reaction stirred at room temperature for 18 hours. The mixture was washed with 1 M citric acid solution (5mL), the organic phase separated and purified directly by column chromatography on silica gel using an elution gradient of dichloromethane:methanol (100:0 to 95:5) to afford the title compounds as yellow solids.

| | |
|---|---|
| | 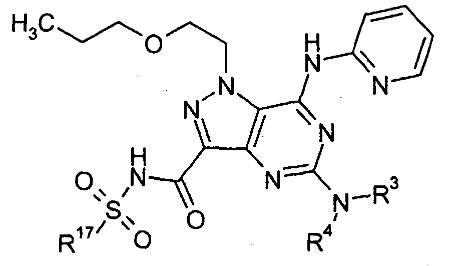 |
| 165 | R³ = -CH₃; R⁴ = -CH₂CH₃; R¹⁷ = -CH₃; |
| | ¹H NMR (CDCl₃, 400MHz) δ: 0.75 (t, 3H), 1.24 (t, 3H), 1.60 (m, 2H), 3.24 (s, 3H), 3.42 (s, 3H), 3.56 (t, 2H), 3.75 (q, 2H), 3.98 (t, 2H), 4.84 (m, 2H), 7.04 (m, 1H), 7.75 (m, 1H), 8.35 (m, 2H). MS APCI+ m/z 477 [MH]⁺ |
| 166 | R³ = -CH₃; R⁴ = -CH₂CH₃; R¹⁷ = -CH₂CH₃; |
| | ¹H NMR (CDCl₃, 400MHz) δ: 0.75 (t, 3H), 1.24 (t, 3H), 1.42 (t, 3H), 1.60 (m, 2H), 3.22 (s, 3H), 3.56 (t, 2H), 3.60 (q, 2H), 3.75 (q, 2H), 3.97 (t, 2H), 4.82 (m, 2H), 7.04 (m, 1H), 7.78 (m, 1H), 8.40 (m, 2H). MS APCI+ m/z 491 [MH]⁺ |
| 167 | R³ = -CH₃; R⁴ = -CH(CH₃)₂; R¹⁷ = -CH₃; |
| | ¹H NMR (CDCl₃, 400MHz) δ: 0.75 (t, 3H), 1.24 (d, 6H), 1.58 (m, 2H), 3.06 (s, 3H), 3.44 (s, 3H), 3.50 (m, 2H), 3.98 (t, 2H), 4.84 (m, 2H), 4.95 (m, 1H), 7.04 (m, 1H), 7.80 (m, 1H), 8.35 (m, 2H). MS APCI+ m/z 491 [MH]⁺ |
| 168 | R³ = -CH₃; R⁴ = -CH(CH₃)₂; R¹⁷ = -CH₂CH₃; |
| | ¹H NMR (CDCl₃, 400MHz) δ: 0.73 (t, 3H), 1.24 (d, 6H), 1.42 (t, 3H), 1.56 (m, 2H), 3.04 (s, 3H), 3.55 (m, 2H), 3.60 (q, 2H), 3.98 (t, 2H), 4.82 (m, 2H), 4.95 (m, 1H), 7.04 (m, 1H), 7.80 (m, 1H), 8.35 (d, 1H), 8.40 (m, 1H). MS APCI+ m/z 505 [MH]⁺ |
| 169 | R³ = -CH₂CH₃; R⁴ = -CH₂CH₃; R¹⁷ = -CH₃; |
| | ¹H NMR (CDCl₃, 400MHz) δ: 0.75 (t, 3H), 1.26 (t, 6H), 1.60 (m, 2H), 3.42 (s, 3H), 3.55 (t, 2H), 3.66 (m, 4H), 3.97 (t, 2H), 4.87 (m, 2H), 7.06 (m, 1H), 7.78 (m, 1H), 8.36 (m, 1H), 8.44 (m, 1H). MS APCI+ m/z 491 [MH]⁺ |
| 170 | R³ = -CH₂CH₃; R⁴ = -CH₂CH₃; R¹⁷ = -CH₂CH₃; |
| | ¹H NMR (CDCl₃, 400MHz) δ: 0.75 (t, 3H), 1.24 (t, 6H), 1.42 (t, 3H), 1.60 (m, 2H), 3.52 (t, 2H), 3.60 (q, 2H), 3.65 (m, 4H), 3.98 (t, 2H), 4.84 (m, 2H), 7.06 (m, 1H), 7.75 (m, 1H), 8.34 (m, 1H), 8.42 (m, 1H). MS APCI+ m/z 505 [MH]⁺ |
| | 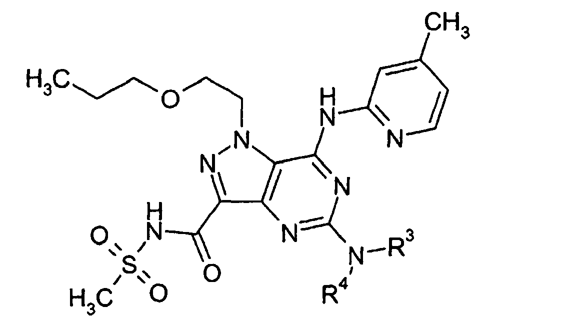 |
| 171 | R³ = -CH₃; R⁴ = -CH₂CH₃; |
| | ¹H NMR (DMSO-d₆ +TFAd, 400MHz) δ: 0.64 (t, 3H), 1.16 (t, 3H), 1.35 (m, 2H), 2.45 (s, 3H), 3.15 (s, 3H), 3.32 (t, 2H), 3.40 (s, 3H), 3.64 (q, 2H), 3.86 (t, 2H), 4.94 (m, 2H), 7.18 (d, 1H), 8.04 (s, 1H), 8.25 (d, 1H). MS APCI+ m/z 491 [MH]⁺ |

### Examples 172 to 177

4-Dimethylaminopyridine (1.3 eq) was added to a solution of the appropriate acid from examples 140-145 (1eq), 1-(3-dimethylaminopropyl)-3-ethylcarbodiimide hydrochloride (1.3eq) and methanesulphonamide (1.3eq) in dichloromethane (13.5-16mLmmol⁻¹) and the reaction stirred at room temperature for 18 hours. Tlc analysis showed starting material remaining, so additional 1-(3-dimethylaminopropyl)-3-ethylcarbodiimide hydrochloride (0.5eq) and methanesulphonamide (0.5eq) were added and the reaction stirred for a further 18 hours. The mixture was diluted with dichloromethane and 1 M citric acid solution, stirring continued for a further 40 minutes, then the phases separated. The organic phase was purified directly by column chromatography on silica gel (using a Parallel Flashmaster system) using an elution gradient of methanol:dichloromethane (0:100 to 5:95) to provide the title compounds as yellow solids.

| | | |
|---|---|---|
| 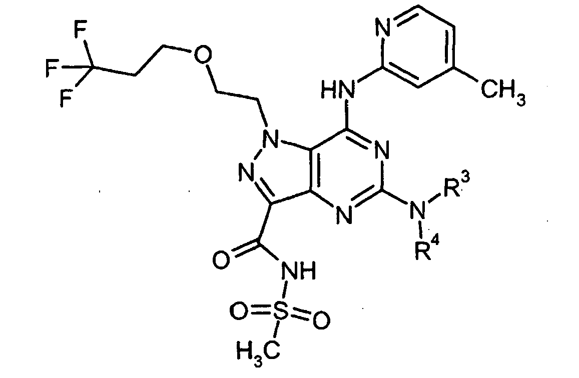 | | |

| Ex. No | -NR³R⁴ | Data |
|---|---|---|
| 172 | 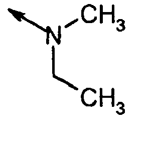 | ¹H NMR (DMSO-d₆ +TFAd, 400MHz) δ: 1.17 (t, 3H), 2.35-2.46 (m, 5H), 3.18 (s, 3H), 3.39 (s, 3H), 3.60-3.67 (m, 4H), 3.94 (t, 2H), 4.98 (t, 2H), 7.18 (d, 1H), 8.08 (s, 1H), 8.26 (d, 1H). MS APCI+ m/z 545 [MH]⁺ |
| 173 | 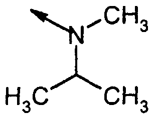 | ¹H NMR (DMSO-d₆ +TFAd, 400MHz) δ: 1.19 (d, 6H), 2.40-2.50 (m, 5H), 3.02 (s, 3H), 3.40 (s, 3H), 3.63 (t, 2H), 3.94 (t, 2H), 4.97 (t, 2H), 7.15 (d, 1H), 8.05 (s, 1H), 8.24 (d, 1H). MS APCI+ m/z 559 [MH]⁺ |
| 174 | 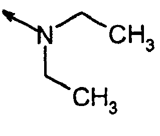 | ¹H NMR (DMSO-d₆ +TFAd, 400MHz) δ: 1.19 (t, 6H), 2.42 (s, 3H), 2.44-2.53 (m, 2H), 3.40 (s, 3H), 3.64 (m, 6H), 3.93 (t, 2H), 4.94 (t, 2H), 7.15 (d, 1H), 8.05 (s, 1H), 8.26 (d, 1H). MS APCI+ m/z 559 [MH]⁺ |
| 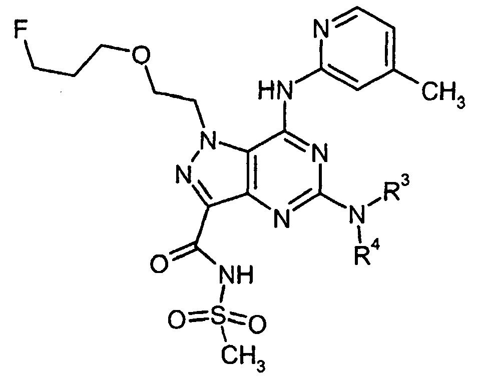 | | |
| 175 | 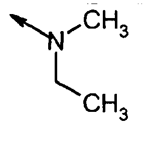 | ¹H NMR (DMSO-d₆ +TFAd, 400MHz) δ: 1.17 (t, 3H), 1.73-1.86 (m, 2H), 2.41 (s, 3H), 3.17 (s, 3H), 3.41 (s, 3H), 3.51 (t, 2H), 3.65 (q, 2H), 3.90 (t, 2H), 4.24-4.39 (m, 2H), 4.92 (m, 2H), 7.11 (d, 1H), 8.07 (s, 1H), 8.24 (d, 1H). MS APCI+ m/z 509 [MH]⁺ |
| 176 | 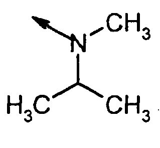 | ¹H NMR (DMSO-d₆ +TFAd, 400MHz) δ: 1.20 (d, 6H), 1.72-1.85 (m, 2H), 2.42 (s, 3H), 3.01 (s, 3H), 3.41 (s, 3H), 3.50 (t, 2H), 3.90 (t, 2H), 4.24-4.39 (m, 2H), 4.77 (m, 1H), 4.94 (t, 2H), 7.13 (d, 1H), 8.05 (s, 1H), 8.24 (d, 1H). MS APCI+ m/z 523 [MH]⁺ |
| 177 | 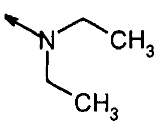 | ¹H NMR (DMSO-d₆ +TFAd, 400MHz) δ: 1.20 (t, 6H), 1.73-1.85 (m, 2H), 2.42 (s, 3H), 3.40 (s, 3H), 3.50 (t, 2H), 3.62 (q, 4H), 3.89 (t, 2H), 4.24-4.39 (m, 2H), 4.92 (t, 2H), 7.14 (d, 1H), 8.08 (s, 1H), 8.26 (d, 1H). MS APCI+ m/z 523 [MH]⁺ |

### Example 178

### 5-[Ethyl(methyl)amino]-7-[(4-methylpyridin-2-yl)amino]-N-(methylsulfonyl)-1-[2-(2,2,2-trifluoroethoxy)ethyl]-1H-pyrazolo[4,3-d]pyrimidine-3-carboxamide

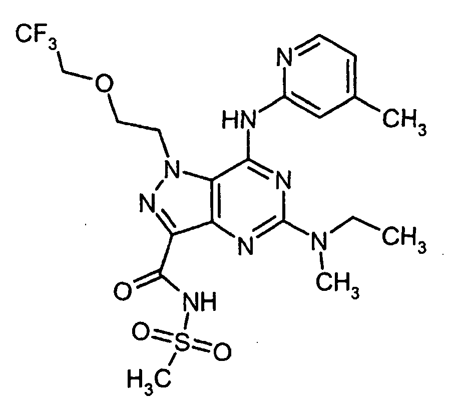

A mixture of the chloride from preparation 261 (50mg, 0.1mmol), N-ethyldiisopropylamine (0.05mL, 0.3mmol), N-ethylmethylamine (0.026mL, 0.3mmol) and cesium fluoride (15mg, 0.1mmol) in 1-methyl-2-pyrrolidinone (1 mL) was heated in a Reactivial® at 110°C for 90 minutes.

The cooled reaction mixture was purified directly using a Phenomenex Luna C18 column reverse phase column and acetonitrile:95% water/5% methanol/0.1% trifluoroacetic acid (5:95 to 95:5) as elution gradient. The product was dissolved in dichloromethane and the solution washed with sodium bicarbonate solution, dried over magnesium sulphate and evaporated under reduced pressure to provide the title compound, 24mg.

Alternatively, example 178 may be prepared by the method of examples 172-177. 4-Dimethylaminopyridine (22mg, 0.20mmol) was added to a solution of the acid from example 127 (70mg, 0.15mmol), 1-(3-dimethylaminopropyl)-3-ethylcarbodiimide hydrochloride (38mg, 0.20mmol) and methanesulphonamide (19mg, 0.20mmol) in dichloromethane (2ml) and the reaction stirred at room temperature for 18 hours. The mixture was diluted with dichloromethane (20mls) and 1 M citric acid solution (10ml), then the phases separated. The organic phase was purified directly by column chromatography on silica gel (using a Parallel Flashmaster system) using an elution gradient of methanol:dichloromethane (0:100 to 2:98) to provide the title compound as a yellow solid.
¹H NMR (DMSO-D₆ +1 drop TFAd, 400MHz) δ: 1.19 (t, 3H), 2.49 (s, 3H), 3.20 (s, 3H), 3.41 (s, 3H), 3.66 (q, 2H), 4.06 (q, 2H), 4.14 (t, 2H), 5.03 (t, 2H), 7.20 (d, 1H), 8.12 (s, 1H), 8.27 (d, 1H).
MS ES- m/z 529 [M-H]⁻

### Example 179

### 5-[Isopropyl(methyl)amino]-7-[(4-methylpyridin-2-yl)amino]-N-(methylsulfonyl)-1-[2-(2,2,2-trifluoroethoxy)ethyl]-1H-pyrazolo[4,3-d]pyrimidine-3-carboxamide

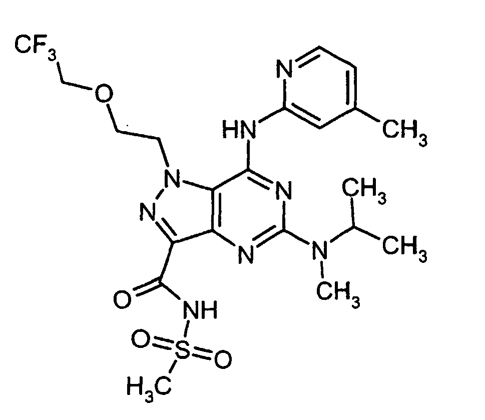

The title compound was obtained from the chloride from preparation 261 and N-methylisopropylamine, following the procedure descibed in example 178.
¹H NMR (DMSO-D₆ + 1 drop TFAd, 400MHz) δ: 1.20 (d, 6H), 2.49 (s, 3H), 3.03 (s, 3H), 3.41 (s, 3H), 4.08 (q, 2H), 4.15 (t, 2H), 4.78 (m, 1H), 5.03 (t, 2H), 7.20 (d, 1H), 8.10 (s, 1H), 8.26 (d, 1H).
MS ES- m/z 543 [M-H]⁻

### Examples 180 to 182

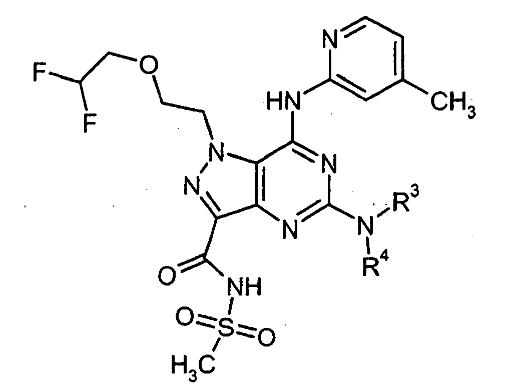

Sodium hydroxide solution (1M, 1 mL, 1mmol) was added to a solution of the appropriate ester from preparation 250-252 (0.33mmol) in dioxan (3mL) and the solution stirred at room temperature for 18 hours. The reaction was evaporated under reduced pressure and the mixture partitioned between dichloromethane (50mL) and 1 M citric acid solution. The layers were separated and the organic solution dried over magnesium sulphate and evaporated under reduced pressure. The product was dissolved in dichloromethane (5mL) and 1-(3-dimethylaminopropyl)-3-ethylcarbodiimide hydrochloride (82mg, 0.43mmol), methanesulphonamide (41 mg, 0.43mmol) and 4-dimethylaminopyridine (48mg, 0.43mmol) added and the reaction stirred at room temperature for 72 hours. The mixture was diluted with dichloromethane (10mL), 1 M citric acid solution and the mixture stirred for 30 minutes. The phases were separated and the organic solution purified directly by column chromatography on silica gel using dichloromethane:methanol (98:2) as eluant to afford the title compounds.

| Ex No | -NR³R⁴ | Data |
|---|---|---|
| 180^{A} | 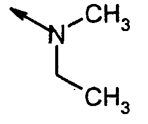 | ¹H NMR (DMSO-d₆ +TFA-d, 400MHz) δ: 1.20 (t, 3H), 2.45 (s, 3H), 3.18 (s, 3H), 3.40 (s, 3H), 3.60-3.75 (m, 4H), 4.05 (t, 2H), 5.00 (t, 2H), 6.00 (m, 1H), 7.20 (d, 1H), 8.08 (s, 1H), 8.25 (d, 1H). MS APCI+ m/z 513 [MH]⁺ |
| 181 | 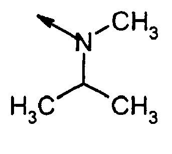 | ¹H NMR (DMSO-d₆ +TFA-d, 400MHz) δ: 1.18 (d, 6H), 2.44 (s, 3H), 3.02 (s, 3H), 3.40 (s, 3H), 3.68 (m, 2H), 4.05 (t, 2H), 4.76 (m, 1H), 5.00 (t, 2H), 6.04 (m, 1H), 7.18 (m, 1H), 8.06 (s, 1H), 8.25 (d, 1H). MS APCI+ m/z527 [MH]⁺ |
| 182 | 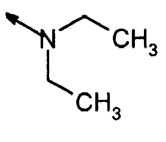 | ¹H NMR (DMSO-d₆ +TFA-d, 400MHz) δ: 1.18 (t, 6H), 2.42 (s, 3H), 3.40 (s, 3H), 3.40-3.55 (m, 6H), 4.04 (t, 2H), 5.00 (t, 2H), 5.98 (m, 1H), 7.22 (d, 1H), 8.08 (s, 1H), 8.30 (d, 1H). MS APCI+ m/z527 [MH]⁺ |

| | | |
|---|---|---|
| A = the product was crystallised from dichloromethane:isopropylalcohol | | |

### Example 183

### 5-(Diethylamino)-7-[(4-methylpyridin-2-yl)amino]-N-(methylsulfonyl)-1-(2-propoxyethyl)-1H-pyrazolo[4,3-d]pyrimidine-3-carboxamide

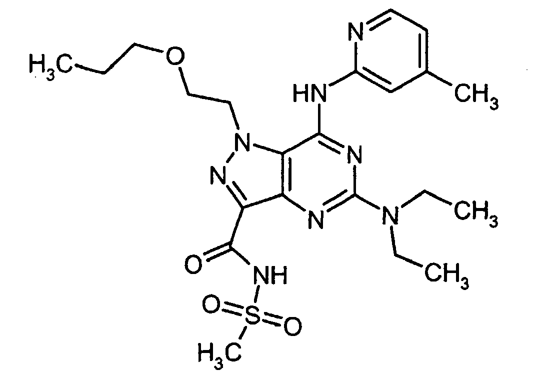

A mixture of the chloride from preparation 67 (135mg, 0.33mmol), cesium fluoride (50mg, 0.33mmol) and diethylamine (103µL, 1 mmol) in dimethylsulphoxide (1 mL) was heated in a Reactivial® at 120°C for 18 hours. The cooled mixture was partitioned between dichloromethane (20mL) and water (20mL) and the layers separated. The organic phase was washed with water (2x10mL), dried over magnesium sulphate and evaporated under reduced pressure. Sodium hydroxide solution (1M, 0.5mL, 0.5mmol) and dioxan (1 mL) were added to the residue and the solution stirred at room temperature for 18 hours. The reaction was evaporated under reduced pressure and the mixture partitioned between dichloromethane (20mL) and 1 M citric acid solution (2mL) and water (20mL). The layers were separated and the organic solution dried over magnesium sulphate and evaporated under reduced pressure. The product was dissolved in dichloromethane (5mL) and 1-(3-dimethylaminopropyl)-3-ethylcarbodiimide hydrochloride (82mg, 0.43mmol), methanesulphonamide (41 mg, 0.43mmol) and 4-dimethylaminopyridine (48mg, 0.43mmol) added and the reaction stirred at room temperature for 18 hours. The mixture was diluted with dichloromethane (30mL), washed with 1 M citric acid solution (5mL) then dried over magnesium sulphate and evaporated under reduced pressure. The crude product was purified by column chromatography on silica gel using dichloromethane:methanol (100:0 to 97:3) as eluant to afford the title compound, 75mg.
¹H NMR (DMSO-d₆ +TFAd, 400MHz) δ: 0.62 (t, 3H), 1.20 (t, 6H), 1.40 (m, 2H), 2.41 (s, 3H), 3.38 (t, 2H), 3.41 (s, 3H), 3.60 (m, 4H), 3.85 (t, 2H), 4.90 (m, 2H), 7.14 (d, 1H), 8.05 (s, 1H), 8.24 (d, 1H).
MS APCI+ m/z 505 [MH]⁺

### Example 184

### 5-[Isopropyl(methyl)amino]-7-[(4-methlpyridin-2-yl)amino]-N-(methylsulfonyl)-1-(2-propoxyethyl)-1H-pyrazolo[4,3-d]pyrimidine-3-carboxamide

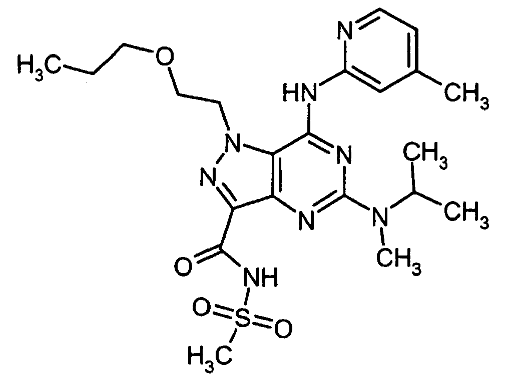

The title compound was obtained in 51 % yield as a yellow solid, from the chloride of preparation 67, following a similar procedure to that described in example 183.
¹H NMR (DMSO-D₆ +1 drop TFAd, 400MHz) δ: 0.62 (t, 3H), 1.18 (d, 6H), 1.38 (m, 2H), 2.42 (s, 3H), 3.00 (s, 3H), 3.30 (t, 2H), 3.39 (s, 3H), 3.84 (t, 2H), 4.75 (m, 1H), 4.95 (t, 2H), 7.18 (d, 1H), 8.04 (s, 1H), 8.24 (d, 1H).
MS APCI+- m/z 505 [MH]+

### Example 185

### 5-[Ethyl(methyl)amino]-1-(2-hydroxyethyl)-7-[(4-methylpyridin-2-yl)amino]-N-(methylsulfonyl)-1H-pyrazolo[4,3-d]pyrimidine-3-carboxamide

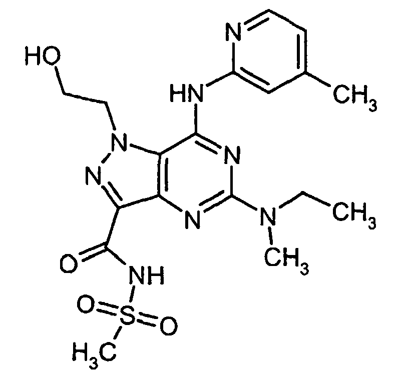

Boron tribromide (1 M in dichloromethane, 1.95mL, 1.95mmol) was added dropwise to a solution of the compound from example 119 (100mg, 0.22mmol) in dichloromethane (5mL) cooled to -25°C, so as to maintain the temperature below - 20°C. The reaction was stirred at -25°C for 3 hours, then quenched by the dropwise addition of saturated sodium bicarbonate solution until pH 7 was achieved. The mixture was allowed to warm to room temperature, then partitioned between water (10mL) and dichloromethane (20mL). The organic phase was washed with water, dried over magnesium sulphate and evaporated under reduced pressure to provide the title compound as a yellow solid, 31 mg.
¹H NMR (CD₃OD + drop TFAd, 400MHz) δ: 1.31 (t, 3H), 2.54 (s, 3H), 3.30 (s, 3H), 3.41 (s, 3H), 3.76 (q, 2H), 4.08 (t, 2H), 4.98 (t, 2H), 7.19 (d, 1H), 8.04 (s, 1H), 8.15 (d, 1H).
MS m/z 449 [MH]⁺

### Assay

The compounds of the invention are inhibitors of cyclic guanylate monophosphate (cGMP)-specific phosphodiesterase type 5 (PDE-5 inhibitors). Preferred compounds suitable for use in accordance with the present invention are potent and selective PDE5 inhibitors. *In vitro* PDE inhibitory activities against cyclic guanosine 3',5'-monophosphate (cGMP) and cyclic adenosine 3',5'-monophosphate (cAMP) phosphodiesterases can be determined by measurement of their IC₅₀ values (the concentration of compound required for 50% inhibition of enzyme activity).

The required PDE enzymes can be isolated from a variety of sources, including human corpus cavernosum, human and rabbit platelets, human cardiac ventricle, human skeletal muscle and bovine retina, essentially by a modification of the method of Thompson, WJ et al.; Biochemistry 18(23), 5228-5237, 1979, as described by Ballard SA et al.; J. Urology 159(6), 2164-2171, 1998. In particular, cGMP-specific PDE5 and cGMP-inhibited cAMP PDE3 can be obtained from human corpus cavernosum tissue, human platelets or rabbit platelets; cGMP-stimulated PDE2 was obtained from human corpus cavernosum; calcium/calmodulin (Ca/CAM)-dependent PDE1 from human cardiac ventricle; cAMP-specific PDE4 from human skeletal muscle; and photoreceptor PDE6 from bovine retina. Phosphodiesterases 7-11 can be generated from full length human recombinant clones transfected into SF9 cells.

Assays can be performed either using a modification of the "batch" method of Thompson WJ and Appleman MM; Biochemistry 10(2),311-316, 1971, essentially as described by Ballard SA et al.; J. Urology 159(6), 2164-2171, 1998, or using a scintillation proximity assay for the direct detection of [³H]-labelled AMP/GMP using a modification of the protocol described by Amersham plc under product code TRKQ7090/7100. In summary, for the scintillation proximity assay the effect of PDE inhibitors was investigated by assaying a fixed amount of enzyme in the presence of varying inhibitor concentrations and low substrate, (cGMP or cAMP in a 3:1 ratio unlabelled to [³H]-labeled at a concentration of -1/3 *Kₘ* or less) such that IC₅₀ ≅ *Kᵢ*. The final assay volume was made up to 100µl with assay buffer [20mM Tris-HCl pH 7.4, 5mM MgCl₂, 1mg/ml bovine serum albumin]. Reactions were initiated with enzyme, incubated for 30-60min at 30°C to give <30% substrate turnover and terminated with 50µl yttrium silicate SPA beads (containing 3mM of the respective unlabelled cyclic nucleotide for PDEs 9 and 11). Plates were re-sealed and shaken for 20min, after which the beads were allowed to settle for 30min in the dark and then counted on a TopCount plate reader (Packard, Meriden, CT) Radioactivity units were converted to % activity of an uninhibited control (100%), plotted against inhibitor concentration and inhibitor IC₅₀ values obtained using the 'Fit Curve' Microsoft Excel extension.

All compounds of the invention have an activity against PDE-5 of less than 10,000nM. IC₅₀ values for representative compounds are listed in the table below.

| **Example** | **IC₅₀ (nM)** | | **Example** | **IC₅₀ (nM)** |
|---|---|---|---|---|
| 1 | **0.075** | | 20 | **35.7** |
| 2 | **0.201** | | 21 | **5.30** |
| 3 | **5.01** | | 22 | **7.12** |
| 4 | **2.62** | | 23 | **8.97** |
| 5 | **0.943** | | 24 | **53.9** |
| 6 | **2.89** | | 25 | **7.19** |
| 7 | **0.082** | | 26 | **1.64** |
| 8 | **3.82** | | 27 | **36.8** |
| 9 | **0.075** | | 28 | **11.6** |
| 10 | **1.86** | | 29 | **106** |
| 11 | **1.09** | | 30 | **35.8** |
| 12 | **14.7** | | 31 | **43.4** |
| 13 | **1.46** | | 32 | **100** |
| 14 | **8.75** | | 33 | **100** |
| 15 | **1.84** | | 34 | **100** |
| 16 | **0.510** | | 35 | **100** |
| 17 | **2.93** | | 36 | **0.708** |
| 18 | **11.3** | | 37 | **23.9** |
| 19 | **2.43** | | 38 | **16.7** |
| 39 | **57.2** | | 74 | **100** |
| 40 | **0.167** | | 75 | **0.371** |
| 41 | **3.20** | | 76 | **0.465** |
| 42 | **86.6** | | 77 | **0.162** |
| 43 | **17.4** | | 78 | **0.240** |
| 44 | **94.7** | | 79 | **0.332** |
| 45 | **47.1** | | 80 | **2.88** |
| 46 | **17.9** | | 81 | **16.7** |
| 47 | **8.00** | | 82 | **55.1** |
| 48 | **100** | | 83 | **27.8** |
| 49 | **4.92** | | 84 | **2.37** |
| 50 | **1.66** | | 85 | **0.0550** |
| 51 | **2.57** | | 86 | **0.0630** |
| 52 | **3.40** | | 87 | **0.0870** |
| 53 | **0.797** | | 88 | **21.3** |
| 54 | **2.03** | | 90 | **2.83** |
| 55 | **2.60** | | 91 | **5.06** |
| 56 | **9.11** | | 92 | **41.0** |
| 57 | **100** | | 93 | **23.2** |
| 58 | **100** | | 94 | **0.303** |
| 59 | **43.6** | | 95 | **9.11** |
| 60 | **8.02** | | 96 | **3.65** |
| 61 | **13.1** | | 97 | **9.74** |
| 62 | **1.85** | | 99 | **2.45** |
| 63 | **4.58** | | 100 | **3.48** |
| 64 | **100** | | 101 | **8.45** |
| 65 | **25.0** | | 102 | **38.2** |
| 66 | **13.5** | | 103 | **21.4** |
| 67 | **100** | | 104 | **9.69** |
| 68 | **100** | | 105 | **0.741** |
| 69 | **100** | | 106 | **2.60** |
| 70 | **0.451** | | 107 | **0.307** |
| 71 | **2.46** | | 108 | **18.3** |
| 72 | **59.2** | | 109 | **9.70** |
| 73 | **52.3** | | 110 | **0.564** |
| 111 | **0.502** | | 156 | **0.162** |
| 112 | **14.7** | | 157 | **0.771** |
| 113 | **0.270** | | 158 | **0.608** |
| 114 | **4.83** | | 159 | **0.0560** |
| 115 | **0.545** | | 160 | **0.0250** |
| 116 | **0.0320** | | 161 | **0.101** |
| 117 | **0.0250** | | 162 | **3.10** |
| 118 | **1.72** | | 163 | **0.591** |
| 119 | **11.5** | | 164 | **0.324** |
| 120 | **1.12** | | 171 | **1.21** |
| 122 | **2.54** | | 175 | **0.57** |
| 123 | **0.628** | | 176 | **0.17** |
| 124 | **0.776** | | 177 | **0.17** |
| 125 | **1.53** | | 178 | **2.21** |
| 126 | **3.05** | | 179 | **0.914** |
| 127 | **6.34** | | 180 | **2.88** |
| 128 | **3.94** | | 181 | **1.29** |
| 129 | **7.78** | | 182 | **1.4** |
| 130 | **19.5** | | 183 | **0.383** |
| 134 | **5.02** | | 184 | **0.5** |
| 135 | **7.70** | | 185 | **11.5** |
| 136 | **0.414** | | | |
| 137 | **0.0680** | | | |
| 138 | **0.0650** | | | |
| 139 | **0.0710** | | | |
| 140 | **0.0990** | | | |
| 147 | **0.141** | | | |
| 148 | **0.198** | | | |
| 149 | **14.4** | | | |
| 150 | **1.55** | | | |
| 151 | **0.513** | | | |
| 152 | **4.68** | | | |
| 153 | **0.398** | | | |
| 154 | **1.16** | | | |
| 155 | **0.341** | | | |

## Claims

1. A compound of formula (I) 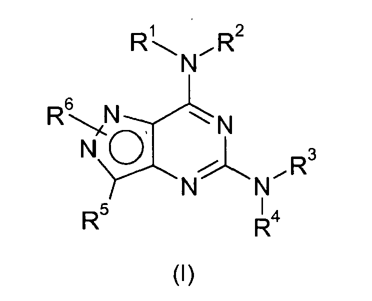 wherein
R¹ is a cyclic group selected from R^{A}, R^{B}, R^{C} and R^{D}, each of which is optionally substituted with one or more R⁷ groups;
R² is hydrogen or C₁-C₂ alkyl;
R³ and R⁴ are each independently C₁-C₈ alkyl, C₂-C₈ alkenyl, C₂-C₈ alkynyl-or C₃-C₁₀ cycloalkyl, each of which is optionally substituted with one or more R⁸ groups, or R^{E}, which is optionally substituted with one or more R⁹ groups, or hydrogen;
or -NR³R⁴ forms R^{F}, which is optionally substituted with one or more R¹⁰ groups;
R⁵ is selected from -Y-CO₂R¹⁵ and -Y-R¹⁶;
R⁶, which may be attached at N¹ or N², is C₁-C₆ alkyl, C₁-C₆ haloalkyl, C₂-C₆ alkenyl or C₂-C₆ alkynyl, each of which is optionally substituted by C₁-C₆ alkoxy, C₁-C₆ haloalkoxy or a cyclic group selected from R^{J}, R^{K}, R^{L} and R^{M}, or R⁶ is R^{N}, C₃-C₇ cycloalkyl or C₃-C₇ halocycloalkyl, each of which is optionally substituted by C₁-C₆ alkoxy or C₁-C₆ haloalkoxy, or R⁶ is hydrogen;
R⁷ is halo, C₁-C₆ alkyl, C₁-C₆ haloalkyl, C₂-C₆ alkenyl, C₂-C₆ alkynyl, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹², CONR¹²R¹³ or CN;
R⁸ is halo, phenyl, C₁-C₆ alkoxyphenyl, OR¹², OC(O)R¹², NO₂, NR¹²R¹³, NR¹²C(O)R¹³, NR¹²CO²R¹⁴, C(O)R¹², CO₂R¹², CONR¹²R¹³, CN, C₃-C₆ cycloalkyl, R^{G} or R^{H}, the last two of which are optionally substituted with one or more R⁹ groups;
R⁹ is C₁-C₆ alkyl, C₁-C₆ haloalkyl or CO₂R¹²;
R¹⁰ is halo, C₃-C₁₀ cycloalkyl, C₃-C₁₀ halocycloalkyl, phenyl, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, C₁-C₆ alkyl or C₁-C₆ haloalkyl, the last two of which are optionally substituted by R¹¹;
R¹¹ is phenyl, NR¹²R¹³ or NR¹²CO₂R¹⁴;
R¹² and R¹³ are each independently hydrogen, C₁-C₆ alkyl or C₁-C₆ haloalkyl;
R¹⁴ is C₁-C₆ alkyl or C₁-C₆ haloalkyl;
R¹⁵ is hydrogen or C₁-C₆ alkyl optionally substituted with one or more groups selected from halo, OH, C₁-C₆ alkyloxy, NH₂, NH(C₁-C₆alkyl) and N(C₁-C₆ alkyl)₂;
R¹⁶ is a carboxylic acid isostere selected from tetrazol-5-yl, 5-trifluoromethyl-1,2,4-triazol-3-yl, 5-(methylsulfonyl)-1,2,4-triazol-3-yl, 2,5-dihydro-5-oxo-1,2,4-oxadiazol-3-yl, -SO₂NHR¹⁷ and -CONHR¹⁸;
R¹⁷ is selected from C₁-C₆ alkyl, phenyl, -CO-(C₁-C₆ alkyl) and - CO-phenyl;
R¹⁸ is selected from -SO₂-(C₁-C₆ alkyl) and -SO₂-phenyl;
R^{A} and R^{J} are each independently a C₃-C₁₀ cycloalkyl or C₃-C₁₀ cycloalkenyl group, each of which may be either monocyclic or, when there are an appropriate number of ring atoms, polycyclic and which may be fused to either
(a) a monocyclic aromatic ring selected from a benzene ring and a 5- or 6-membered heteroaromatic ring containing up to three heteroatoms selected from nitrogen, oxygen and sulphur, or
(b) a 5-, 6- or 7-membered heteroalicyclic ring containing up to three heteroatoms selected from nitrogen, oxygen and sulphur;
R^{B} and R^{K} are each independently a phenyl or naphthyl group, each of which may be fused to
(a) a C₅-C₇ cycloalkyl or C₅-C₇ cycloalkenyl ring,
(b) a 5-, 6- or 7-membered heteroalicyclic ring containing up to three heteroatoms selected from nitrogen, oxygen and sulphur, or
(c) a 5- or 6-membered heteroaromatic ring containing up to three heteroatoms selected from nitrogen, oxygen and sulphur;
R^{C}, R^{L} and R^{N} are each independently a monocyclic or, when there are an appropriate number of ring atoms, polycyclic saturated or partly unsaturated ring system containing between 3 and 10 ring atoms, of which at least one is a heteroatom selected from nitrogen, oxygen and sulphur, which ring may be fused to a C₅-C₇ cycloalkyl or C₅-C₇ cycloalkenyl group or a monocyclic aromatic ring selected from a benzene ring and a 5- or 6-membered heteroaromatic ring containing up to three heteroatoms selected from nitrogen, oxygen and sulphur;
R^{D} and R^{M} are each independently a 5- or 6-membered heteroaromatic ring containing up to three heteroatoms independently selected from nitrogen, oxygen and sulphur, which ring may further be fused to
(a) a second 5- or 6-membered heteroaromatic ring containing up to three heteroatoms selected from nitrogen, oxygen and sulphur;
(b) C₅-C₇ cycloalkyl or C₅-C₇ cycloalkenyl ring;
(c) a 5-, 6- or 7-membered heteroalicyclic ring containing up to three heteroatoms selected from nitrogen, oxygen and sulphur; or
(d) a benzene ring;
R^{E}, R^{F} and R^{G} are each independently a monocyclic or, when there are an appropriate number of ring atoms, polycyclic saturated ring system containing between 3 and 10 ring atoms, of which at least one is a heteroatom selected from nitrogen, oxygen and sulphur;
R^{H} is a 5- or 6-membered heteroaromatic ring containing up to three heteroatoms independently selected from nitrogen, oxygen and sulphur; and
Y is a covalent bond, -CH₂-O-CH₂-, C₁-C₆ alkylenyl or C₃-C₇ cycloalkylenyl;
a tautomer thereof or a pharmaceutically acceptable salt, or solvate of said compound or tautomer.

2. A compound according to claim 1, wherein R¹ is R^{A}, which is optionally substituted with one or more R⁷ groups; and
R^{A} is a C₃-C₁₀ cycloalkyl group, which may be either monocyclic or, when there are an appropriate number of ring atoms, polycyclic, which may be fused to either
(a) a monocyclic aromatic ring selected from a benzene ring and a 5- or 6-membered heteroaromatic ring containing up to three heteroatoms selected from nitrogen, oxygen and sulphur, or
(b) a 5-, 6- or 7-membered heteroalicyclic ring containing up to three heteroatoms selected from nitrogen, oxygen and sulphur.

3. A compound according to claim 2 wherein R^{A} is a monocyclic C₃-C₈ cycloalkyl group.

4. A compound according to claim 3 wherein R^{A} is a monocyclic C₅-C₇ cycloalkyl group.

5. A compound according to claim 4 wherein R^{A} is cyclopentyl or cyclohexyl.

6. A compound according to claim 1 wherein R¹ is R^{B}, which is optionally substituted with one or more R⁷ groups.

7. A compound according to claim 6 wherein R^{B} is phenyl.

8. A compound according to claim 1 wherein R¹ is R^{C}, which is optionally substituted with one or more R⁷ groups.

9. A compound according to claim 8 wherein R^{C} is a monocyclic saturated or partly unsaturated ring system containing between 3 and 8 ring atoms, of which at least one is a heteroatom selected from nitrogen, oxygen and sulphur.

10. A compound according to claim 9 wherein R^{C} is a monocyclic saturated or partly unsaturated ring system containing between 5 and 7 ring atoms, of which at least one is a heteroatom selected from nitrogen, oxygen and sulphur.

11. A compound according to claim 10 wherein R^{C} is a monocyclic saturated ring system containing between 5 and 7 ring atoms, of which at least one is a heteroatom selected from nitrogen, oxygen and sulphur.

12. A compound according to claim 1 wherein R¹ is R^{D}, which is optionally substituted with one or more R⁷ groups.

13. A compound according to claim 12 wherein R^{D} is a 5- or 6-membered heteroaromatic ring containing up to three heteroatoms independently selected from nitrogen, oxygen and sulphur.

14. A compound according to claim 13 wherein R^{D} is a 5-membered heteroaromatic ring containing a heteroatom selected from nitrogen, oxygen and sulphur and optionally up to two further nitrogen atoms in the ring, or a 6-membered heteroaromatic ring including 1, 2 or 3 nitrogen atoms.

15. A compound according to claim 14 wherein R^{D} is furanyl, thienyl, pyrrolyl, pyrazolyl, imidazolyl, isoxazolyl, oxazolyl, isothiazolyl, thiazolyl, oxadiazolyl, pyridyl, pyridazinyl, pyrimidyl or pyrazinyl.

16. A compound according to claim 15 wherein R^{D} is pyrazolyl, imidazolyl, isoxazolyl, oxazolyl, oxadiazolyl, pyridyl, pyridazinyl, pyrimidyl or pyrazinyl.

17. A compound according to any one of claims 1 to 16 wherein R⁷ is halo, C₁-C₆ alkyl, C₁-C₆ haloalkyl, OR¹² or CONR¹²R¹³.

18. A compound according to claim 17 wherein R⁷ is halo, C₁-C₃ alkyl, C₁-C₃ alkoxy, hydroxy or CONH(C₁-C₃ alkyl).

19. A compound according to claim 18 wherein R⁷ is fluoro, methyl, ethyl, hydroxy, methoxy, propoxy or CONHMe.

20. A compound according to any one of claims 1 to 19 wherein R² is hydrogen or methyl.

21. A compound according to claim 20 wherein R² is hydrogen.

22. A compound according to any one of claims 1 to 21 wherein R³ is hydrogen, C₁-C₆ alkyl, which is optionally substituted with one or more R⁸ groups, or R^{E}, which is optionally substituted with one or more R⁹ groups; and wherein R^{E} is a monocyclic or, when there are an appropriate number of ring atoms, polycyclic saturated ring system containing between 3 and 7 ring atoms, of which at least one is a heteroatom selected from nitrogen, oxygen and sulphur.

23. A compound according to claim 22 wherein R³ is hydrogen, C₁-C₄ alkyl, which is optionally substituted with one or more R⁸ groups, or R^{E}, which is optionally substituted with one or more R⁹ groups; and wherein R^{E} is a monocyclic saturated ring system containing between 3 and 7 ring atoms, of which at least one is a heteroatom selected from nitrogen, oxygen and sulphur.

24. A compound according to claim 23 wherein R³ is R^{E}, which is optionally substituted with one or more R⁹ groups and wherein R^{E} is a monocyclic saturated ring system containing between 3 and 7 ring atoms containing one nitrogen atom.

25. A compound according to claim 24 wherein R^{E} is azetidinyl, pyrrolidinyl or piperidinyl.

26. A compound according to claim 23 wherein R³ is C₁-C₄ alkyl, which is optionally substituted with one or more R⁸ groups and wherein R⁸ is halo, phenyl, C₁-C₆ alkoxyphenyl, OR¹², NR¹²R¹³, NR¹²CO₂R¹⁴, CO₂R¹², CONR¹²R¹³, R^{G} or R^{H}, the last two of which are optionally substituted with one or more R⁹ groups.

27. A compound according to claim 26 wherein R⁸ is hydroxy, methoxy, methoxyphenyl, NH₂, NHMe, NMe₂, NHCO₂^{t}Bu, NMeCO₂^{t}Bu, CO₂H, CONHMe, R^{G} or R^{H}, the last two of which are optionally substituted with one or more R⁹ groups.

28. A compound according to claim 27 wherein R⁸ is R^{G}, which is optionally substituted with one or more R⁹ groups and wherein R^{G} is a monocyclic saturated ring system containing between 3 and 7 ring atoms, of which at least one is a heteroatom selected from nitrogen, oxygen and sulphur.

29. A compound according to claim 28 wherein R^{G} is a monocyclic saturated ring system containing between 3 and 7 ring atoms containing one nitrogen atom and optionally one oxygen atom.

30. A compound according to claim 29 wherein R^{G} is pyrrolidinyl, piperidinyl or morpholinyl.

31. A compound according to claim 27 wherein R⁸ is R^{H}, which is optionally substituted with one or more R⁹ groups and wherein R^{H} is a 5- or 6-membered heteroaromatic ring containing up to two nitrogen atoms.

32. A compound according to claim 31 wherein R^{H} is pyrazolyl.

33. A compound according to any one of claims 22 to 32 wherein R⁹ is methyl or CO₂^{t}Bu.

34. A compound according to claim 23 wherein R³ is hydrogen or C₁-C₄ alkyl, which is optionally substituted with one or more R⁸ groups, or R³ is azetidinyl, pyrrolidinyl or piperidinyl, each of which is optionally substituted with one or more R⁹ groups, wherein
R⁸ is hydroxy, methoxy, methoxyphenyl, NH₂, NHMe, NMe₂, NHCO₂^{t}Bu, NMeCO₂^{t}Bu, CO₂H, CONHMe, pyrrolidinyl, piperidinyl, morpholinyl or pyrazolyl, the last four of which are optionally substituted with one or more R⁹ groups and wherein
R⁹ is methyl or CO₂^{t}Bu.

35. A compound according to any one of claims 1 to 34 wherein R⁴ is hydrogen, C₁-C₆ alkyl, C₁-C₆ haloalkyl, C₂-C₆ alkenyl or C₂-C₆ alkynyl.

36. A compound according to claim 35 wherein R⁴ is hydrogen, C₁-C₆ alkyl or C₁-C₆ haloalkyl.

37. A compound according to claim 36 wherein R⁴ is hydrogen, methyl or ethyl.

38. A compound according to any one of claims 1 to 21 wherein - NR³R4 forms R^{F}, which is optionally substituted with one or more R¹⁰ groups and wherein R^{F} is a monocyclic or, when there are an appropriate number of ring atoms, polycyclic saturated ring system containing between 3 and 10 ring atoms containing at least one nitrogen atom and optionally one other atom selected from oxygen and sulphur.

39. A compound according to claim 38 wherein R^{F} is a monocyclic or, when there are an appropriate number of ring atoms, polycyclic saturated ring system containing between 3 and 10 ring atoms containing one or two nitrogen atoms and optionally one other atom selected from oxygen and sulphur.

40. A compound according to claim 39 wherein R^{F} is selected from azetidinyl, pyrrolidinyl, piperidinyl, piperazinyl, morpholinyl, 3-azabicyclo[3.1.0]hex-3-yl, homopiperazinyl, 2,5-diazabicyclo[4.3.0]non-2-yl, 3,8-diazabicyclo[3.2.1]oct-3-yl, 3,8-diazabicyclo[3.2.1]oct-8-yl, 2,5-diazabicyclo[2.2.1]hept-2-yl, 1,4-diazabicyclo[4.3.0]non-4-yl and 1,4-diazabicyclo[3.2.2]non-4-yl.

41. A compound according to any one of claims 38 to 40 wherein R¹⁰ is halo, OR¹², NR¹²R¹³, NR¹²CO₂R¹⁴, CO₂R¹³, oxo, C₁-C₆ alkyl or C₁-C₆ haloalkyl, the last two of which are optionally substituted by R¹¹.

42. A compound according to claim 41 wherein R¹⁰ is halo, methyl, ethyl, isopropyl, hydroxy, methoxy, NH₂, NHMe, NMe₂, NHCO₂^{t}Bu, CO₂H, CO₂^{t}Bu, oxo, benzyl, -CH₂NH₂, -CH₂NHMe, CH₂NMe₂ or -CH₂NMeCO₂^{t}Bu.

43. A compound according to any one of claims 1 to 42 wherein R⁵ is -Y-CO₂R¹⁵.

44. A compound according to claim 43 wherein R¹⁵ is hydrogen or C₁-C₃ alkyl and Y is a covalent bond.

45. A compound according to any one of claims 1 to 42 wherein R⁵ is -Y-R¹⁶.

46. A compound according to claim 45 wherein R¹⁶ is -CONHR¹⁸, tetrazol-5-yl or 2,5-dihydro-5-oxo-1,2,4-oxadiazol-3-yl and Y is a covalent bond or a methylene group.

47. A compound according to any one of claims 1 to 46 wherein R⁶ is positioned on N¹.

48. A compound according to any one of claims 1 to 47 wherein R⁶ is C₁-C₆ alkyl or C₁-C₆ haloalkyl, each of which is optionally substituted by C₁-C₆ alkoxy, C₁-C₆ haloalkoxy or a cyclic group selected from R^{J}, R^{L} and R^{M}, or R⁶ is R^{N} or hydrogen;
R^{J} is a C₃-C₇ monocyclic cycloalkyl group;
R^{L} and R^{N} are each independently a monocyclic, saturated or partly unsaturated ring system containing between 4 and 7 ring atoms, of which at least one is a heteroatom selected from nitrogen, oxygen and sulphur; and
R^{M} is a 5- or 6-membered heteroaromatic ring containing up to three heteroatoms independently selected from nitrogen, oxygen and sulphur.

49. A compound according to claim 48 wherein R⁶ is C₁-C₄ alkyl or C₁-C₄ haloalkyl, each of which is optionally substituted by C₁-C₄ alkoxy, C₁-C₄ haloalkoxy or a cyclic group selected from R^{J}, R^{L} and R^{M}, or R⁶ is R^{N} or hydrogen;
R^{J} is cyclopropyl or cyclobutyl;
R^{L} and R^{N} are each independently a monocyclic saturated ring system containing either 5 or 6 ring atoms, of which at least one is a heteroatom selected from nitrogen, oxygen and sulphur; and
R^{M} is a 5- or 6-membered heteroaromatic ring containing a heteroatom selected from nitrogen, oxygen and sulphur.

50. A compound according to claim 49 wherein R⁶ is C₁-C₄ alkyl or C₁-C₄ haloalkyl, each of which is optionally substituted by C₁-C₄ alkoxy or a cyclic group selected from R^{J}, R^{L} and R^{M}, or R⁶ is R^{N} or hydrogen;
R^{J} is cyclopropyl or cyclobutyl;
R^{L} and R^{N} are each independently a monocyclic saturated ring system containing either 5 or 6 ring atoms containing one heteroatom selected from nitrogen, oxygen and sulphur; and
R^{M} is a 5- or 6-membered heteroaromatic ring containing one nitrogen atom.

51. A compound according to claim 50 wherein R⁶ is C₁-C₄ alkyl or C₁-C₄ haloalkyl, each of which is optionally substituted by C₁-C₄ alkoxy, cyclopropyl, cyclobutyl, tetrahydrofuranyl, tetrahydropyranyl or pyridinyl, or R⁶ is hydrogen or tetrahydropyranyl.

52. A compound according to claim 51 wherein R⁶ is hydrogen, methyl, ethyl, isopropyl, isobutyl, methoxyethyl, methoxypropyl, ethoxyethyl, ethoxypropyl, propoxyethyl, 2,2,2-trifluoroethyl, tetrahydrofuranylmethyl, tetrahydropyranylmethyl, tetrahydropyranyl or pyridinylmethyl.

53. A compound according to claim 1 wherein
R³ is hydrogen, C₁-C₄ alkyl, which is optionally substituted with one or more R⁸ groups, or R^{E} which is optionally substituted with one or more R⁹ groups;
R⁴ is hydrogen, C₁-C₆ alkyl or C₁-C₆ haloalkyl;
or -NR³R⁴ forms R^{F}, which is optionally substituted with one or more R¹⁰ groups;
R⁶ is C₁-C₄ alkyl or C₁-C₄ haloalkyl, each of which is optionally substituted by C₁-C₄ alkoxy, C₁-C₄ haloalkoxy or a cyclic group selected from R^{J}, R^{L} and R^{M}, or R⁶ is R^{N} or hydrogen;
R^{A} is a monocyclic C₃-C₈ cycloalkyl group;
R^{B} is phenyl;
R^{C} is a monocyclic saturated or partly unsaturated ring system containing between 3 and 8 ring atoms, of which at least one is a heteroatom selected from nitrogen, oxygen and sulphur;
R^{D} is a 5- or 6-membered heteroaromatic ring containing up to three heteroatoms independently selected from nitrogen, oxygen and sulphur;
R^{E} is a monocyclic saturated ring system containing between 3 and 7 ring atoms, of which at least one is a heteroatom selected from nitrogen, oxygen and sulphur;
R^{F} is a monocyclic or, when there are an appropriate number of ring atoms, polycyclic saturated ring system containing between 3 and 10 ring atoms, of which at least one is a heteroatom selected from nitrogen, oxygen and sulphur;
R^{J} is cyclopropyl or cyclobutyl;
R^{L} and R^{N} are each independently a monocyclic saturated ring system containing either 5 or 6 ring atoms, of which at least one is a heteroatom selected from nitrogen, oxygen and sulphur;
R^{M} is a 5- or 6-membered heteroaromatic ring containing a heteroatom selected from nitrogen, oxygen and sulphur; and
Y is a covalent bond or C₁-C₆ alkylenyl.

54. A compound according to claim 53 wherein R¹ is a cyclic group selected from R^{A}, R^{B}, R^{C} and R^{D}, each of which is optionally substituted with one or more R⁷ groups;
R⁷ is halo, C₁-C₆ alkyl, C₁-C₆ haloalkyl, OR¹² or CONR¹²R¹³;
R⁸ is halo, phenyl, C₁-C₆ alkoxyphenyl, OR¹², NR¹²R¹³, NR¹²CO₂R¹⁴, CO₂R¹², CONR¹²R¹³, R^{G} or R^{H}, the last two of which are optionally substituted with one or more R⁹ groups;
R^{A} is a monocyclic C₅-C₇ cycloalkyl group;
R^{B} is phenyl;
R^{C} is a monocyclic saturated ring system containing between 5 and 7 ring atoms, of which at least one is a heteroatom selected from nitrogen, oxygen and sulphur;
R^{D} is a 5-membered heteroaromatic ring containing a heteroatom selected from nitrogen, oxygen and sulphur and optionally up to two further nitrogen atoms in the ring, or a 6-membered heteroaromatic ring including 1, 2 or 3 nitrogen atoms;
R^{E} is a monocyclic saturated ring system containing between 3 and 7 ring atoms containing one nitrogen atom;
R^{F} is a monocyclic or, when there are an appropriate number of ring atoms, polycyclic saturated ring system containing between 3 and 10 ring atoms containing at least one nitrogen atom and optionally one other atom selected from oxygen and sulphur;
R^{G} is a monocyclic saturated ring system containing between 3 and 7 ring atoms, of which at least one is a heteroatom selected from nitrogen, oxygen and sulphur;
R^{H} is a 5- or 6-membered heteroaromatic ring containing up to two nitrogen atoms; and
Y is a covalent bond or -CH₂-.

55. A compound according to claim 1 selected from:
methyl 5-((1S,4S)-2,5-diazabicyclo[2.2.1]hept-2-yl)-1-(2-ethoxyethyl)-7-(4-methylpyridin-2-ylamino)-1*H*-pyrazolo[4,3-*d*]pyrimidine-3-carboxylate,
methyl 1-(2-ethoxyethyl)-5-(*N*-isopropyl-*N*-methylamino)-7-(6-methylpyridin-2-ylamino)-1*H*-pyrazolo[4,3-*d*]pyrimidine-3-carboxylate,
ethyl 1-(2-ethoxyethyl)-5-(*N*-ethyl-*N*-methylamino)-7-(4-methylpyridin-2-ylamino)-1*H*-pyrazolo[4,3-*d*]pyrimidine-3-carboxylate,
2-(dimethylamino)ethyl 5-dimethylamino-1-(2-ethoxyethyl)-7-(4-methylpyridin-2-ylamino)-1*H*-pyrazolo[4,3-*d*]pyrimidine-3-carboxylate,
1-(2-ethoxyethyl)-5-(*N*-methyl-*N*-propylamino)-7-(4-methylpyridin-2-ylamino)-1*H*-pyrazolo[4,3-*d*]pyrimidine-3-carboxylic acid,
5-(*N*-isopropyl-*N*-methylamino)-7-(4-methylpyridin-2-ylamino)-1-(2-propoxy-ethyl)-1*H*-pyrazolo[4,3-*d*]pyrimidine-3-carboxylic acid,
7-(4,6-dimethylpyridin-2-ylamino)-1-(2-ethoxyethyl)-5-(*N*-isopropyl-*N-*methyl-amino)-1*H*-pyrazolo[4,3-*d*]pyrimidine-3-carboxylic acid,
5-(*N*-cyclobutyl-*N*-methylamino)-1-(2-ethoxyethyl)-7-(4-methylpyridin-2-ylamino)-1*H*-pyrazolo[4,3-*d*]pyrimidine-3-carboxylic acid,
1-(2-ethoxyethyl)-5-isopropylamino-7-(4-methylpyridin-2-ylamino)-1*H-*pyrazolo[4,3-*d*]pyrimidine-3-carboxylic acid,
1-(2-ethoxyethyl)-5-(*N*-ethyl-*N*-methylamino)-7-(2-methoxypyrimidin-4-ylamino)-1*H*-pyrazolo[4,3-*d*]pyrimidine-3-carboxylic acid,
3-[1-(2-ethoxyethyl)-5-(*N*-isopropyl-*N*-methylamino)-7-(4-methylpyridin-2-ylamino)-1*H*-pyrazolo[4,3-*d*]pyrimidin-3-yl]-2*H*-1,2,4-oxadiazol-5-one,
3-[1-(2-ethoxyethyl)-5-(*N*-ethyl-*N*-methylamino)-7-(4-methylpyridin-2-ylamino)-1*H*-pyrazolo[4,3-*d*]pyrimidin-3-yl]-2*H*-1,2,4-oxadiazol-5-one,
1-(2-ethoxyethyl)-7-(4-fluoro-3-methylphenylamino)-5-(*N*-isopropyl-*N-*methylamino)-1*H*-pyrazolo[4,3-*d*]pyrimidine-3-carboxylic acid,
1-(2-ethoxyethyl)-5-(*N*-ethyl-*N*-methylamino)-7-(4-fluoro-3-methylphenylamino)-1*H*-pyrazolo[4,3-*d*]pyrimidine-3-carboxylic acid,
7-(3,4-dimethylphenylamino)-1-(2-ethoxyethyl)-5-(*N*-ethyl-*N-*methylamino)-1*H*-pyrazolo[4,3-*d*]pyrimidine-3-carboxylic acid,
1-(2-(cyclopropylmethoxy)ethyl)-5-(*N*-isopropyl-*N*-methylamino)-7-(4-methyl-pyridin-2-ylamino)-1*H*-pyrazolo[4,3-*d*]pyrimidine-3-carboxylic acid,
1-(2-(cyclopropylmethoxy)ethyl)-5-(*N*-ethyl-*N*-methylamino)-7-(4-methyl-pyridin-2-ylamino)-1*H*-pyrazolo[4,3-*d*]pyrimidine-3-carboxylic acid,
1-(2-ethoxyethyl)-5-(*N*-isopropyl-*N*-methylamino)-7-(4-methylpyridin-2-ylamino)-1*H*-pyrazolo[4,3-*d*]pyrimidine-3-carboxylic acid,
1-(2-isopropoxyethyl)-5-(*N*-isopropyl-*N*-methylamino)-7-(4-methylpyridin-2-ylamino)-1*H*-pyrazolo[4,3-*d*]pyrimidine-3-carboxylic acid,
*N*-[1-(2-ethoxyethyl)-5-(*N*-isopropyl-*N*-methylamino)-7-(4-methylpyridin-2-ylamino)-1*H*-pyrazolo[4,3-*d*]pyrimidine-3-carbonyl]methanesulfonamide,
*N*-[1-(2-ethoxyethyl)-5-(*N*-ethyl-*N*-methylamino)-7-(4-methylpyridin-2-yl-amino)-1*H*-pyrazolo[4,3-*d*]pyrimidine-3-carbonyl]methanesulfonamide,
*N*-[5-(Ethyl-methyl-amino)-1-[2-(3-fluoro-propoxy)-ethyl]-7-(4-methyl-pyridin-2-ylamino)-1*H*-pyrazolo[4,3-*d*]pyrimidine-3-carbonyl]-methanesulfonamide
*N*-[1-[2-(3-Fluoro-propoxy)-ethyl]-5-(isopropyl-methyl-amino)-7-(4-methyl-pyridin-2-ylamino)-1*H*-pyrazolo[4,3-*d*]pyrimidine-3-carbonyl]-methanesulfonamide
*N*-[5-Diethylamino-1-[2-(3-fluoro-propoxy)-ethyl]-7-(4-methyl-pyridin-2-ylamino)-1*H*-pyrazolo[4,3-*d*]pyrimidine-3-carbonyl]-methanesulfonamide
*N*-[5-Diethylamino-1-[2-(2,2-difluoro-ethoxy)-ethyl]-7-(4-methyl-pyridin-2-ylamino)-1*H*-pyrazolo[4,3-*d*]pyrimidine-3-carbonyl]-methanesulfonamide
*N*-[1-[2-(2,2-Difluoro-ethoxy)-ethyl]-5-(ethyl-methyl-amino)-7-(4-methyl-pyridin-2-ylamino)-1*H*-pyrazolo[4,3-*d*]pyrimidine-3-carbonyl]-methanesulfonamide, and
*N*-[1-[2-(2,2-Difluoro-ethoxy)-ethyl]-5-(isopropyl-methyl-amino)-7-(4-methyl-pyridin-2-ylamino)-1*H*-pyrazolo[4,3-*d*]pyrimidine-3-carbonyl]-methanesulfonamide,
and tautomers thereof or pharmaceutically acceptable salts or solvates of said compounds or tautomers.

56. A compound according to claim 1 that is 1-(2-ethoxyethyl)-5-(methyl(propyl)amino)-7-(4-methylpyridin-2-ylamino)-1H-pyrazolo[4,3-d]pyrimidine-3-carboxylic acid, a tautomer thereof or a pharmaceutically acceptable salt or solvate of said compound or tautomer.

57. A compound according to claim 1 that is 3-(1-(2-ethoxyethyl)-5-(ethyl(methyl)amino)-7-(4-methylpyridin-2-ylamino)-1H-pyrazolo[4,3-d]pyrimidin-3-yl)-1,2,4-oxadiazol-5(2H)-one, a tautomer thereof or a pharmaceutically acceptable salt or solvate of said compound or tautomer.

58. A compound according to claim 1 that is 1-(2-ethoxyethyl)-7-(4-fluoro-3-methylphenylamino)-5-(isopropyl(methyl)amino)-1H-pyrazolo[4,3-d]pyrimidine-3-carboxylic acid, a tautomer thereof or a pharmaceutically acceptable salt or solvate of said compound or tautomer.

59. A compound according to claim 1 that is 1-(2-ethoxyethyl)-5-(N-isopropyl-N-methylamino)-7-(4-methylpyridin-2-ylamino)-1H-pyrazolo[4,3-d]pyrimidine-3-carboxylic acid, a tautomer thereof or a pharmaceutically acceptable salt or solvate of said compound or tautomer.

60. A compound according to claim 1 that is N-[1-(2-ethoxyethyl)-5-(N-isopropyl-N-methylamino)-7-(4-methylpyridin-2-ylamino)-1H-pyrazolo[4,3-d]pyrimidine-3-carbonyl]methanesulfonamide, a tautomer thereof or a pharmaceutically acceptable salt or solvate of said compound or tautomer.

61. A compound according to claim 1 that is N-[1-(2-ethoxyethyl)-5-(N-ethyl-N-methylamino)-7-(4-methylpyridin-2-ylamino)-1H-pyrazolo[4,3-d]pyrimidine-3-carbonyl]methanesulfonamide, a tautomer thereof or a pharmaceutically acceptable salt or solvate of said compound or tautomer.

62. A pharmaceutical composition comprising a compound according to any one of claims 1 to 61 and a pharmaceutically acceptable diluent or carrier.

63. A compound according to any one of claims 1 to 61 for use as a medicament.

64. A compound according to any one of claims 1 to 61, for use in accordance with claim 57 as a medicament for the treatment of a disease or condition where inhibition of PDE5 is known, or can be shown, to produce a beneficial effect.

65. A compound according to any one of claims 1 to 61, for use in accordance with claim 57 or 58 as a medicament for the treatment of a disease or condition selected from hypertension, congestive heart failure, angina, stroke, coronary artery disease, congestive heart failure, conditions of reduced blood vessel patency, peripheral vascular disease, atherosclerosis, nitrate-induced tolerance, nitrate tolerance, diabetes, impaired glucose tolerance, metabolic syndrome, obesity, sexual dysfunction, premature labour, pre-eclampsia, dysmenorrhea, polycystic ovary syndrome, benign prostatic hyperplasia, bladder outlet obstruction, incontinence, chronic obstructive pulmonary disease, acute respiratory failure, bronchitis, chronic asthma, allergic asthma, allergic rhinitis, gut motility disorders, Kawasaki's syndrome, multiple sclerosis, Alzheimer's disease, psoriasis, skin necrosis, scarring, fibrosis, pain, cancer, metastasis, baldness, nutcracker oesophagus, anal fissure, and haemorrhoids.

66. A compound according to any one of claims 1 to 61, for use in accordance with claim 59 as a medicament for the treatment of pulmonary hypertension.

67. A compound according to any one of claims 1 to 61, for use in accordance with claim 59 as a medicament for the treatment of neuropathic pain.

68. The use of a compound according to any one of claims 1 to 61 for the manufacture of a medicament for the treatment of a disease or condition selected from hypertension, congestive heart failure, angina, stroke, coronary artery disease, congestive heart failure, conditions of reduced blood vessel patency, peripheral vascular disease, atherosclerosis, nitrate-induced tolerance, nitrate tolerance, diabetes, impaired glucose tolerance, metabolic syndrome, obesity, sexual dysfunction, premature labour, pre-eclampsia, dysmenorrhea, polycystic ovary syndrome, benign prostatic hyperplasia, bladder outlet obstruction, incontinence, chronic obstructive pulmonary disease, acute respiratory failure, bronchitis, chronic asthma, allergic asthma, allergic rhinitis, gut motility disorders, Kawasaki's syndrome, multiple sclerosis, Alzheimer's disease, psoriasis, skin necrosis, scarring, fibrosis, pain, cancer, metastasis, baldness, nutcracker oesophagus, anal fissure, and haemorrhoids.

69. A pharmaceutical composition comprising a compound according to any one of claims 1 to 61 and a second pharmaceutically active agent selected from the group consisting of aspirin, angiotensin II receptor antagonists, calcium channel blockers, beta-blockers, CI1027, CCR5 receptor antagonists, imidazolines, sGCa's (soluble guanylate cyclase activators) antihypertensive agents, diuretics, alpha adrenergic antagonists, ACE (angiotensin converting enzyme) inhibitors, aldosterone receptor antagonists, neutral endopeptidase inhibitors, antidiabetic agents, sulfonylureas, glitazones, cholesterol lowering agents, and alpha-2-delta ligands.

70. A pharmaceutical composition comprising a compound according to any one of claims 1 to 55 and pregabalin.

71. A compound of formula (III) 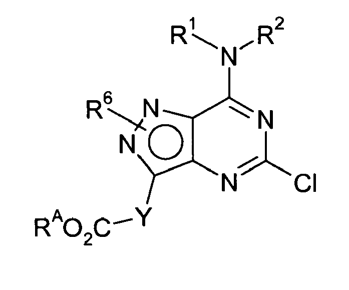 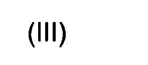 wherein R¹, R², R⁶ and Y are as defined in claim 1 and R^{A} is C₁-C₆ alkyl or benzyl,
with the proviso that the compound is not a compound of formula: 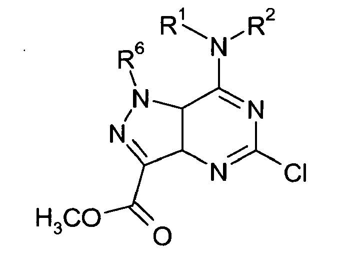 wherein R¹, R² and R⁶ are as defined in claim 1.

72. A compound according to claim 71 of formula (III^{D})

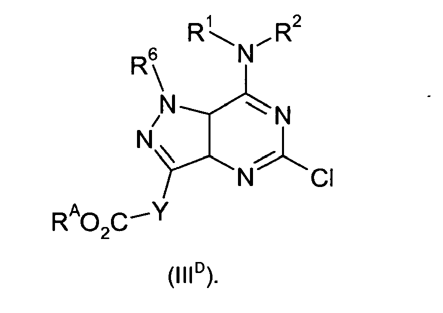

73. A compound of formula (V) 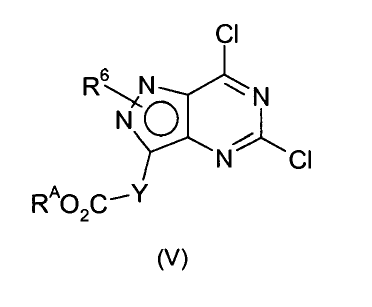 wherein R⁶ and Y are as defined in claim 1 and R^{A} is C₁-C₆ alkyl or benzyl,
with the proviso that the compound of formula (V) is not a compound of formula: 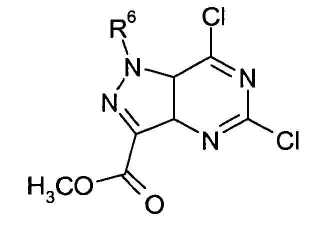 wherein R⁶ is as defined in claim 1.

74. A compound according to claim 73 of formula (V^{B})

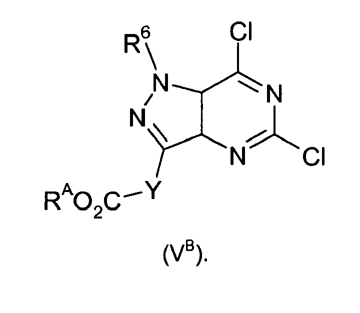

## Patentansprüche

1. Verbindung der Formel (I) 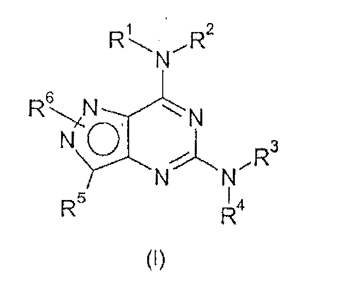 worin
R¹ eine cyclische Gruppe ist, ausgewählt aus R^{A}, R^{B}, R^{C} und R^{D}, von denen jedes gegebenenfalls mit einer oder mehreren R⁷-Gruppen substituiert ist;
R² Wasserstoff oder C₁-C₂-Alkyl ist;
R³ und R⁴ jeweils unabhängig C₁-C₈-Alkyl, C₂-C₈-Alkenyl, C₂-C₈-Alkinyl oder C₃-C₁₀-Cycloalkyl, von denen jedes gegebenenfalls mit einer oder mehreren R⁸-Gruppen substituiert ist, oder R^{E}, das gegebenenfalls mit einer oder mehreren R⁹-Gruppen substituiert ist, oder Wasserstoff sind;
oder -NR³R⁴ bildet R^{F}, das gegebenenfalls mit einer oder mehreren R¹⁰-Gruppen substituiert ist;
R⁵ ausgewählt ist aus -Y-CO₂R¹⁵ und -Y-R¹⁶;
R⁶, das an N¹ oder N² gebunden sein kann, C₁-C₆-Alkyl, C₁-C₆-Halogenalkyl, C₂-C₆-Alkenyl oder C₂-C₆-Alkinyl, von denen jedes gegebenenfalls mit C₁-C₆-Alkoxy, C₁-C₆-Halogenalkoxy oder einer cyclischen Gruppe, ausgewählt aus R^{J}, R^{K}, R^{L} und R^{M}, substituiert ist, ist oder R⁶ R^{N}, C₃-C₇-Cycloalkyl oder C₃-C₇-Halogencycloalkyl ist, von denen jedes gegebenenfalls mit C₁-C₆-Alkoxy oder C₁-C₆-Halogenalkoxy substituiert ist, oder R⁶ Wasserstoff ist;
R⁷ Halogen, C₁-C₆-Alkyl, C₁-C₆-Halogenalkyl, C₂-C₆-Alkenyl, C₂-C₆-Alkinyl, C₃-C₁₀-Cycloalkyl, C₃-C₁₀-Halogencycloalkyl, Phenyl, OR¹² OC(O)R¹², NO₂, NR¹²R¹³, NR¹²C(O)R¹³, NR¹²CO₂R¹⁴, C(O)R¹², CO₂R¹², CONR¹²R¹³ oder CN ist;
R⁸ Halogen, Phenyl, C₁-C₆-Alkoxyphenyl, OR¹², OC(O)R¹², NO₂, NR¹²R¹³, NR¹²C(O)R¹³, NR¹²CO₂R¹⁴, C(O)R¹², CO₂R¹², CONR¹²R¹³, CN, C₃-C₆-Cycloalkyl, R^{G} oder R^{H} ist, wobei die letzten zwei von diesen gegebenenfalls mit einer oder mehreren R⁹-Gruppen substituiert sind;
R⁹ C₁-C₆-Alkyl, C₁-C₆-Halogenalkyl oder CO₂R¹² ist;
R¹⁰ Halogen, C₃-C₁₀-Cycloalkyl, C₃-C₁₀-Halogencycloalkyl, Phenyl, OR¹² OC(O)R¹², NO₂, NR¹²R¹³, NR¹²C(O)R¹³, NR¹²CO₂R¹⁴, C(O)R¹², CO₂R¹³, CONR¹²R¹³, CN, Oxo, C₁-C₆-Alkyl oder C₁-C₆-Halogenalkyl ist, von denen die letzten zwei gegebenenfalls mit R¹¹ substituiert sind;
R¹¹ Phenyl, NR¹²R¹³ oder NR¹²CO₂R¹⁴ ist;
R¹² und R¹³ jeweils unabhängig Wasserstoff, C₁-C₆-Alkyl oder C₁-C₆-Halogenalkyl sind;
R¹⁴ C₁-C₆-Alkyl oder C₁-C₆-Halogenalkyl ist;
R¹⁵ Wasserstoff oder C₁-C₆-Alkyl, gegebenenfalls substituiert mit einer oder mehreren Gruppen, ausgewählt aus Halogen, OH, C₁-C₆-Alkyloxy, NH₂, NH(C₁-C₆-Alkyl) und N(C₁-C₆-Alkyl)₂, ist;
R¹⁶ ein Carbonsäure-Isoster ist, ausgewählt aus Tetrazol-5-yl, 5-Trifluormethyl-1,2,4-triazol-3-yl, 5-(Methylsulfonyl)-1,2,4-triazol-3-yl, 2,5-Dihydro-5-oxo-1,2,4-oxadiazol-3-yl, -SO₂NHR¹⁷ und -CONHR¹⁸;
R¹⁷ ausgewählt ist aus C₁-C₆-Alkyl, Phenyl, -CO-(C₁-C₆-Alkyl) und -CO-Phenyl;
R¹⁸ ausgewählt ist aus -SO₂-(C₁-C₆-Alkyl) und -SO₂-Phenyl;
R^{A} und R^{J} jeweils unabhängig eine C₃-C₁₀-Cycloalkyl- oder C₃-C₁₀-Cycloalkenylgruppe sind, von denen jede entweder monocyclisch oder, wenn es eine geeignete Anzahl von Ringatomen gibt, polycyclisch sein kann und die kondensiert sein kann an entweder
(a) einen monocyclischen aromatischen Ring, ausgewählt aus einem Benzolring und einem 5- oder 6-gliedrigen heteroaromatischen Ring, der bis zu drei Heteroatome, ausgewählt aus Stickstoff, Sauerstoff und Schwefel, enthält, oder
(b) einen 5-, 6- oder 7-gliedrigen heteroalicyclischen Ring, der bis zu drei Heteroatome, ausgewählt aus Stickstoff, Sauerstoff und Schwefel enthält;
R^{B} und R^{K} jeweils unabhängig eine Phenyl- oder Naphthylgruppe sind, von denen jede kondensiert sein kann an
(a) einen C₅-C₇-Cycloalkyl- oder C₅-C₇-Cycloalkenylring,
(b) einen 5-, 6- oder 7-gliedriegen heteroalicyclischen Ring, der bis zu drei Heteroatome, ausgewählt aus Stickstoff, Sauerstoff und Schwefel, enthält, oder
(c) einen 5- oder 6-gliedrigen heteroaromatischen Ring, der bis zu drei Heteroatome, ausgewählt aus Stickstoff, Sauerstoff und Schwefel, enthält;
R^{C}, R^{L} und R^{N} jeweils unabhängig ein monocyclisches oder, wenn es eine geeignete Anzahl von Ringatomen gibt, polycyclisches, gesättigtes oder teilweise ungesättigtes Ringsystem, das zwischen drei und zehn Ringatome enthält, von denen wenigstens eines ein Heteroatom, ausgewählt aus Stickstoff, Sauerstoff und Schwefel, ist, wobei dieser Ring an eine C₅-C₇-Cycloalkyl- oder C₅-C₇-Cycloalkenylgruppe kondensiert sein kann, oder ein monocyclischer aromatischer Ring, ausgewählt aus einem Benzolring und einem 5- oder 6-gliedrigen heteroaromatischen Ring, der bis zu drei Heteroatome, ausgewählt aus Stickstoff, Sauerstoff und Schwefel, enthält, sind;
R^{D} und R^{M} jeweils unabhängig ein 5- oder 6-gliedriger heteroaromatischer Ring, der bis zu drei Heteroatome, unabhängig ausgewählt aus Stickstoff, Sauerstoff und Schwefel, enthält, sind, wobei der Ring außerdem kondensiert sein kann an
(a) einen zweiten 5- oder 6-gliedrigen heteroaromatischen Ring, der bis zu drei Heteroatome, ausgewählt aus Stickstoff, Sauerstoff und Schwefel, enthält;
(b) einen C₅-C₇-Cycloalkyl- oder C₅-C₇-Cycloalkenylring;
(c) einen 5-, 6- oder 7-gliedrigen heteroalicyclischen Ring, der bis zu drei Heteroatome, ausgewählt aus Stickstoff, Sauerstoff und Schwefel, enthält, oder
(d) einen Benzolring;
R^{E}, R^{F} und R^{G} jeweils unabhängig ein monocyclisches oder, wenn es eine geeignete Anzahl von Ringatomen gibt, polycyclisches gesättigtes Ringsystem, das zwischen drei und zehn Ringatome enthält, von denen wenigstens eines ein Heteroatom, ausgewählt aus Stickstoff, Sauerstoff und Schwefel, ist, sind;
R^{H} ein 5- oder 6-gliedriger heteroaromatischer Ring, der bis zu drei Heteroatome, unabhängig ausgewählt aus Stickstoff, Sauerstoff und Schwefel, enthält, ist und
Y eine kovalente Bindung, -CH₂-O-CH₂-, C₁-C₆-Alkylenyl oder C₃-C₇-Cycloalkylenyl ist;
ein Tautomer davon oder ein pharmazeutisch verträgliches Salz oder Solvat der Verbindung oder des Tautomers.

2. Verbindung nach Anspruch 1, wobei R¹ R^{A} ist, welches gegebenenfalls mit einer oder mehr R⁷-Gruppen substituiert ist, und
R^{A} eine C₃-C₁₀-Cycloalkylgruppe ist, die entweder monocyclisch oder, wenn es eine geeignete Anzahl von Ringatomen gibt, polycyclisch sein kann, die kondensiert sein kann an entweder
(a) einen monocyclischen aromatischen Ring, ausgewählt aus einem Benzolring und einem 5- oder 6-gliedrigen heteroaromatischen Ring, der bis zu drei Heteroatome, ausgewählt aus Stickstoff, Sauerstoff und Schwefel, enthält, oder
(b) einen 5-, 6- oder 7-gliedrigen heteroalicyclischen Ring, der bis zu drei Heteroatome, ausgewählt aus Stickstoff, Sauerstoff und Schwefel, enthält.

3. Verbindung nach Anspruch 2, wobei R^{A} eine monocyclische C₃-C₈-Cycloalkylgruppe ist.

4. Verbindung nach Anspruch 3, wobei R^{A} eine monocyclische C₅-C₇-Cycloalkylgruppe ist.

5. Verbindung nach Anspruch 4, wobei R^{A} Cyclopentyl oder Cyclohexyl ist.

6. Verbindung nach Anspruch 1, wobei R¹ R^{B} ist, welches gegebenenfalls mit einer oder mehr R⁷-Gruppen substituiert ist.

7. Verbindung nach Anspruch 6, wobei R^{B} Phenyl ist.

8. Verbindung nach Anspruch 1, wobei R¹ R^{C}, welches gegebenenfalls mit einer oder mehr R⁷-Gruppen substituiert ist, ist.

9. Verbindung nach Anspruch 8, wobei R^{C} ein monocyclisches, gesättigtes oder teilweise ungesättigtes Ringsystem ist, das zwischen drei und acht Ringatome enthält, von denen wenigstens eines ein Heteroatom, ausgewählt aus Stickstoff, Sauerstoff und Schwefel, ist.

10. Verbindung nach Anspruch 9, wobei R^{C} ein monocyclisches, gesättigtes oder teilweise ungesättigtes Ringsystem ist, das zwischen fünf und sieben Ringatome enthält, von denen wenigstens eines ein Heteroatom, ausgewählt aus Stickstoff, Sauerstoff und Schwefel, ist.

11. Verbindung nach Anspruch 10, wobei R^{C} ein monocyclisches, gesättigtes Ringsystem ist, das zwischen fünf und sieben Ringatome enthält, von denen wenigstens eines ein Heteroatom, ausgewählt aus Stickstoff, Sauerstoff und Schwefel, ist.

12. Verbindung nach Anspruch 1, wobei R¹ R^{D}, das gegebenenfalls mit einer oder mehr R⁷-Gruppen substituiert ist, ist.

13. Verbindung nach Anspruch 12, wobei R^{D} ein 5- oder 6-gliedriger heteroaromatischer Ring ist, der bis zu drei Heteroatome, unabhängig ausgewählt aus Stickstoff, Sauerstoff und Schwefel, enthält.

14. Verbindung nach Anspruch 13, wobei R^{D} ein 5-gliedriger heteroaromatischer Ring, der ein Heteroatom, ausgewählt aus Stickstoff, Sauerstoff und Schwefel, und gegebenenfalls bis zu zwei weitere Stickstoffatome im Ring enthält, oder ein 6-gliedriger heteroaromatischer Ring, der 1, 2 oder 3 Stickstoffatome umfasst, ist.

15. Verbindung nach Anspruch 14, wobei R^{D} Furanyl, Thienyl, Pyrrolyl, Pyrazolyl, Imidazolyl, Isoxazolyl, Oxazolyl, Isothiazolyl, Thiazolyl, Oxadiazolyl, Pyridyl, Pyridazinyl, Pyrimidyl oder Pyrazinyl ist.

16. Verbindung nach Anspruch 15, wobei R^{D} Pyrazolyl, Imidazolyl, Isoxazolyl, Oxazolyl, Oxadiazolyl, Pyridyl, Pyridazinyl, Pyrimidyl oder Pyrazinyl ist.

17. Verbindung nach einem der Ansprüche 1 bis 16, wobei R⁷ Halogen, C₁-C₆-Alkyl, C₁-C₆-Halogenalkyl, OR¹² oder CONR¹²R¹³ ist.

18. Verbindung nach Anspruch 17, wobei R⁷ Halogen, C₁-C₃-Alkyl, C₁-C₃-Alkoxy, Hydroxy oder CONH(C₁-C₃-Alkyl) ist.

19. Verbindung nach Anspruch 18, wobei R⁷ Fluor, Methyl, Ethyl, Hydroxy, Methoxy, Propoxy oder CONHMe ist.

20. Verbindung nach einem der Ansprüche 1 bis 19, wobei R² Wasserstoff oder Methyl ist.

21. Verbindung nach Anspruch 20, wobei R² Wasserstoff ist.

22. Verbindung nach einem der Ansprüche 1 bis 21, wobei R³ Wasserstoff, C₁-C₆-Alkyl, das gegebenenfalls mit einer oder mehr R⁸-Gruppen substituiert ist, oder R^{E}, das gegebenenfalls mit einer oder mehr R⁹-Gruppen substituiert ist, ist und wobei R^{E} ein monocyclisches oder, wenn es eine geeignete Anzahl von Ringatomen gibt, polycyclisches gesättigtes Ringsystem ist, das zwischen drei und sieben Ringatome enthält, von denen wenigstens eines ein Heteroatom, ausgewählt aus Stickstoff, Sauerstoff und Schwefel, ist.

23. Verbindung nach Anspruch 22, wobei R³ Wasserstoff, C₁-C₄-Alkyl, das gegebenenfalls mit einer oder mehr R⁸-Gruppen substituiert ist, oder R^{E}, das gegebenenfalls mit einer oder mehr R⁹-Gruppen substituiert ist, ist und wobei R^{E} ein monocyclisches gesättigtes Ringsystem, das zwischen drei und sieben Ringatome enthält, von denen wenigstens eines ein Heteroatom, ausgewählt aus Stickstoff, Sauerstoff und Schwefel, ist, ist.

24. Verbindung nach Anspruch 23, wobei R³ R^{E}, das gegebenenfalls mit einer oder mehr R⁹-Gruppen substituiert ist, ist und wobei R^{E} ein monocyclisches gesättigtes Ringsystem, das zwischen drei und sieben Ringatome, die ein Stickstoffatom enthalten, enthält, ist.

25. Verbindung nach Anspruch 24, wobei R^{E} Azetidinyl, Pyrrolidinyl oder Piperidinyl ist.

26. Verbindung nach Anspruch 33, wobei R³ C₁-C₄-Alkyl ist, das gegebenenfalls mit einer oder mehr R⁸-Gruppen substituiert ist, und wobei R⁸ Halogen, Phenyl, C₁-C₆-Alkoxyphenyl, OR¹², NR¹²R¹³, NR¹²CO₂R¹⁴, CO₂R¹², CONR¹²R¹³, R^{G} oder R^{H} ist, von denen die letzten zwei gegebenenfalls mit einer oder mehr R⁹-Gruppen substituiert sind.

27. Verbindung nach Anspruch 26, wobei R⁸ Hydroxy, Methoxy, Methoxyphenyl, NH₂, NHMe, NMe₂, NHCO₂^{t}Bu, NMeCO₂^{t}Bu, CO₂H, CONHMe, R^{G} oder R^{H} ist, von denen die letzten zwei gegebenenfalls mit einer oder mehr R⁹-Gruppen substituiert sind.

28. Verbindung nach Anspruch 27, wobei R⁸ R^{G} ist, das gegebenenfalls mit einer oder mehr R⁹-Gruppen substituiert ist, und wobei R^{G} ein monocyclisches gesättigtes Ringsystem ist, das zwischen drei und sieben Ringatome enthält, von denen wenigstens eines ein Heteroatom, ausgewählt aus Stickstoff, Sauerstoff und Schwefel, ist.

29. Verbindung nach Anspruch 28, wobei R^{G} ein monocyclisches gesättigtes Ringsystem ist, das zwischen drei und sieben Ringatome enthält, die ein Stickstoffatom und gegebenenfalls ein Sauerstoffatom enthalten.

30. Verbindung nach Anspruch 29, wobei R^{G} Pyrrolidinyl, Piperidinyl oder Morpholinyl ist.

31. Verbindung nach Anspruch 27, wobei R⁸ R^{H} ist, das gegebenenfalls mit einer oder mehr R⁹-Gruppen substituiert ist, und wobei R^{H} ein 5- oder 6-gliedriger heteroaromatischer Ring ist, der bis zu zwei Stickstoffatome enthält.

32. Verbindung nach Anspruch 31, wobei R^{H} Pyrazolyl ist.

33. Verbindung nach einem der Ansprüche 22 bis 32, wobei R⁹ Methyl oder CO₂-^{t}BU ist.

34. Verbindung nach Anspruch 23, wobei R³ Wasserstoff oder C₁-C₄-Alkyl ist, welches gegebenenfalls mit einer oder mehr R⁸-Gruppen substituiert ist, oder R³ Azetidinyl, Pyrrolidinyl oder Piperidinyl ist, von denen jedes gegebenenfalls mit einer oder mehr R⁹-Gruppen substituiert ist, wobei
R⁸ Hydroxy, Methoxy, Methoxyphenyl, NH₂, NHMₑ, NMe₂, NHCO₂^{t}Bu, NMeCO₂^{t}Bu, CO₂H, CONHM₂, Pyrrolidinyl, Piperidinyl, Morpholinyl oder Pyrazolyl ist, von denen die letzten vier gegebenenfalls mit einer oder mehr R⁹-Gruppen substituiert sind, und wobei
R⁹ Methyl oder CO₂-^{t}Bu ist.

35. Verbindung nach einem der Ansprüche 1 bis 34, wobei R⁴ Wasserstoff, C₁-C₆-Alkyl, C₁-C₆-Halogenalkyl, C₂-C₆-Alkenyl oder C₂-C₆-Alkinyl ist.

36. Verbindung nach Anspruch 35, wobei R⁴ Wasserstoff, C₁-C₆-Alkyl oder C₁-C₆-Halogenalkyl ist.

37. Verbindung nach Anspruch 36, wobei R⁴ Wasserstoff, Methyl oder Ethyl ist.

38. Verbindung nach einem der Ansprüche 1 bis 21, wobei -NR³R⁴ R^{F} bildet, das gegebenenfalls mit einer oder mehr R¹⁰-Gruppen substituiert ist, und wobei R^{F} ein monocyclisches oder, wenn es eine geeignete Anzahl von Ringatomen gibt, polycyclisches gesättigtes Ringsystem ist, das zwischen drei und zehn Ringatome enthält, die wenigstens ein Stickstoffatom und gegebenenfalls ein anderes Atom, ausgewählt aus Sauerstoff und Schwefel, enthalten.

39. Verbindung nach Anspruch 38, wobei R^{F} ein monocyclisches oder, wenn es eine geeignete Anzahl von Ringatomen gibt, polycyclisches gesättigtes Ringsystem ist, das zwischen drei und zehn Ringatome enthält, die ein oder zwei Stickstoffatome und gegebenenfalls ein anderes Atom, ausgewählt aus Sauerstoff und Schwefel, enthalten.

40. Verbindung nach Anspruch 39, wobei R^{F} ausgewählt ist aus Azetidinyl, Pyrrolidinyl, Piperidinyl, Piperazinyl, Morpholinyl, 3-Azabicyclo[3.1.0]hex-3-yl, Homopiperazinyl, 2,5-Diazabicyclo[4.3.0]non-2-yl, 3,8-Diazabicyclo[3.2.1]oct-3-yl, 3,8-Diazabicyclo[3.2.1]oct-8-yl, 2,5-Diazabicyclo[2.2.1]hept-2-yl, 1,4-Diazabicyclo[4.3.0]non-4-yl und 1,4-Diazabicyclo[3.2.2]non-4-yl.

41. Verbindung nach einem der Ansprüche 38 bis 40, wobei R¹⁰ Halogen, OR¹², NR¹²R¹³, RN¹²CO₂R¹⁴, CO₂R¹³, Oxo, C₁-C₆-Alkyl oder C₁-C₆-Halogenalkyl ist, von denen die letzten zwei gegebenenfalls mit R¹¹ substituiert sind.

42. Verbindung nach Anspruch 41, wobei R¹⁰ Halogen, Methyl, Ethyl, Isopropyl, Hydroxy, Methoxy, NH₂, NHMe, NMe₂, NHCO₂^{t}Bu, CO₂H, CO₂^{t}Bu, Oxo, Benzyl, -CH₂NH₂, -CH₂NHMe, CH₂NMe₂ oder -CH₂NMeCO₂^{t}Bu ist.

43. Verbindung nach einem der Ansprüche 1 bis 42, wobei R⁵ -Y-CO₂R¹⁵ ist.

44. Verbindung nach Anspruch 43, wobei R¹⁵ Wasserstoff oder C₁-C₃-Alkyl ist und Y eine kovalente Bindung ist.

45. Verbindung nach einem der Ansprüche 1 bis 42, wobei R⁵ -Y-R¹⁶ ist.

46. Verbindung nach Anspruch 45, wobei R¹⁶ -CONHR¹⁸, Tetrazol-5-yl oder 2,5-Dihydro-5-oxo-1,2,4-oxadiazol-3-yl ist und Y eine kovalente Bindung oder eine Methylengruppe ist.

47. Verbindung nach einem der Ansprüche 1 bis 46, wobei R⁶ an N¹ positioniert ist.

48. Verbindung nach einem der Ansprüche 1 bis 47, wobei R⁶ C₁-C₆-Alkyl oder C₁-C₆-Halogenalkyl ist, von denen jedes gegebenenfalls mit C₁-C₆-Alkoxy, C₁-C₆-Halogenalkoxy oder einer cyclischen Gruppe, ausgewählt aus R^{J}, R^{L} und R^{M}, substituiert ist, oder R⁶ R^{N} oder Wasserstoff ist;
R^{J} eine monocyclische C₃-C₇-Cycloalkylgruppe ist;
R^{L} und R^{N} jeweils unabhängig ein monocyclisches, gesättigtes oder teilweise ungesättigtes Ringsystem sind, das zwischen vier und sieben Ringatome enthält, von denen wenigstens eines ein Heteroatom, ausgewählt aus Stickstoff, Sauerstoff und Schwefel, ist und
R^{M} ein 5- oder 6-gliederiger heteroaromatischer Ring ist, der bis zu drei Heteroatome, unabhängig ausgewählt aus Stickstoff, Sauerstoff und Schwefel, enthält.

49. Verbindung nach Anspruch 48, wobei R⁶ C₁-C₄-Alkyl oder C₁-C₄-Halogenalkyl ist, von denen jedes gegebenenfalls mit C₁-C₄-Alkoxy, C₁-C₄-Halogenalkoxy oder einer cyclischen Gruppe, ausgewählt aus R^{J}, R^{L} und R^{N}, substituiert ist, oder R⁶ R^{N} oder Wasserstoff ist;
R^{J} Cyclopropyl oder Cyclobutyl ist;
R^{L} und R^{N} jeweils unabhängig ein monocyclisches gesättigtes Ringsystem sind, das entweder fünf oder sechs Ringatome enthält, von denen wenigstens eines ein Heteroatom, ausgewählt aus Stickstoff, Sauerstoff und Schwefel, ist und
R^{M} ein 5- oder 6-gliedriger heteroaromatischer Ring ist, der ein Heteroatom, ausgewählt aus Stickstoff, Sauerstoff und Schwefel, enthält.

50. Verbindung nach Anspruch 49, wobei R⁶ C₁-C₄-Alkyl oder C₁-C₄-Halogenalkyl ist, von denen jedes gegebenenfalls mit C₁-C₄-Alkoxy oder einer cyclischen Gruppe, ausgewählt aus R^{J}, R^{L} und R^{M}, substituiert ist oder R⁶ R^{N} oder Wasserstoff ist;
R^{J} Cyclopropyl oder Cyclobutyl ist;
R^{L} und R^{N} jeweils unabhängig ein monocyclisches gesättigtes Ringsystem sind, das entweder fünf oder sechs Ringatome enthält, die ein Heteroatom, ausgewählt aus Stickstoff, Sauerstoff und Schwefel, enthalten, und
R^{M} ein 5- oder 6- gliedriger heteroaromatischer Ring, der ein Stickstoffatom enthält, ist.

51. Verbindung nach Anspruch 50, wobei R⁶ C₁-C₄-Alkyl oder C₁-C₄-Halogenalkyl ist, von denen jedes gegebenenfalls mit C₁-C₄-Alkoxy, Cyclopropyl, Cyclobutyl, Tetrahydrofuranyl, Tetrahydropyranyl oder Pyridinyl substituiert ist, oder R⁶ Wasserstoff oder Tetrahydropyranyl ist.

52. Verbindung nach Anspruch 51, wobei R⁶ Wasserstoff, Methyl, Ethyl, Isopropyl, Isobutyl, Methoxyethyl, Methoxypropyl, Ethoxyethyl, Ethoxypropyl, Propoxyethyl, 2,2,2-Trifluorethyl, Tetrahydrofuranylmethyl, Tetrahydropyranylmethyl, Tetrahydropyranyl oder Pyridinylmethyl ist.

53. Verbindung nach Anspruch 1, wobei
R³ Wasserstoff, C₁-C₄-Alkyl, das gegebenenfalls mit einer oder mehr R⁸-Gruppen substituiert ist, oder R^{E}, das gegebenenfalls mit einer oder mehr R⁹-Gruppen substituiert ist, ist;
R⁴ Wasserstoff, C₁-C₆-Alkyl oder C₁-C₆-Halogenalkyl ist; oder -NR³R⁴ R^{F} bildet, das gegebenenfalls mit einer oder mehr R¹⁰-Gruppen substituiert ist;
R⁶ C₁-C₄-Alkyl oder C₁-C₄-Halogenalkyl ist, von denen jedes gegebenenfalls mit C₁-C4-Alkoxy, C₁-C₄-Halogenalkoxy oder einer cyclischen Gruppe, ausgewählt aus R^{J}, R^{L} und R^{M} substituiert ist, oder R⁶ R^{N} oder Wasserstoff ist;
R^{A} eine monocyclische C₃-C₈-Cycloalkylgruppe ist;
R^{B} Phenyl ist;
R^{C} ein monocyclisches gesättigtes oder teilweise ungesättigtes Ringsystem ist, das zwischen drei und acht Ringatome enthält, von denen wenigstens eines ein Heteroatom, ausgewählt aus Stickstoff, Sauerstoff und Schwefel, ist;
R^{D} ein 5- oder 6-gliedriger heteroaromatischer Ring ist, der bis zu drei Heteroatome, unabhängig ausgewählt aus Stickstoff, Sauerstoff und Schwefel, enthält;
R^{E} ein monocyclisches gesättigtes Ringsystem ist, das zwischen drei und sieben Ringatome enthält, von denen wenigstens eines ein Heteroatom, ausgewählt aus Stickstoff, Sauerstoff und Schwefel, ist;
R^{F} ein monocyclisches oder, wenn es eine geeignete Anzahl von Ringatomen gibt, polycyclisches gesättigtes Ringsystem ist, das zwischen drei und zehn Ringatome enthält, von denen wenigstens eines ein Heteroatom, ausgewählt aus Stickstoff, Sauerstoff und Schwefel, ist;
R^{J} Cyclopropyl oder Cyclobutyl ist;
R^{L} und R^{N} jeweils unabhängig ein monocyclisches gesättigtes Ringsystem sind, das entweder fünf oder sechs Ringatome enthält, von denen wenigstens eines ein Heteroatom, ausgewählt aus Stickstoff, Sauerstoff und Schwefel, ist;
R^{M} ein 5- oder 6-gliedriger heteroaromatischer Ring ist, der ein Heteroatom, ausgewählt aus Stickstoff, Sauerstoff und Schwefel, enthält, und
Y eine kovalente Bindung oder ein C₁-C₆-Alkylenyl ist.

54. Verbindung nach Anspruch 53, wobei R¹ eine cyclische Gruppe, ausgewählt aus R^{A}, R^{B}, R^{C} und R^{D}, von denen jede gegebenenfalls mit einer oder mehr R⁷-Gruppen substituiert ist, ist;
R⁷ Halogen, C₁-C₆-Alkyl, C₁-C₆-Halogenalkyl, OR¹² oder CONR¹²R¹³ ist;
R⁸ Halogen, Phenyl, C₁-C₆-Alkoxyphenyl, OR¹², NR¹²R¹³, NR¹²CO₂R¹⁴, CO₂R¹², CONR¹²R¹³, R^{G} oder R^{H} ist, von denen die letzten zwei gegebenenfalls mit einer oder mehr R⁹-Gruppen substituiert sind;
R^{A} eine monocyclische C₅-C₇-Cycloalkylgruppe ist;
R^{B} Phenyl ist;
R^{C} ein monocyclisches gesättigtes Ringsystem ist, das zwischen fünf und sieben Ringatome enthält, von denen wenigstens eines ein Heteroatom, ausgewählt aus Stickstoff, Sauerstoff und Schwefel, ist;
R^{D} ein 5-gliedriger heteroaromatischer Ring, der ein Heteroatom, ausgewählt aus Stickstoff, Sauerstoff und Schwefel, und gegebenenfalls bis zu zwei weitere Stickstoffatome im Ring enthält, oder ein 6-gliedriger heteroaromatischer Ring, der eins, zwei oder drei Stickstoffatome enthält, ist;
R^{E} ein monocyclisches gesättigtes Ringsystem ist, das zwischen drei und sieben Ringatome, die ein Stickstoffatom enthalten, enthält;
R^{F} ein monocyclisches oder, wenn es eine geeignete Anzahl von Ringatomen gibt, polycyclisches gesättigtes Ringsystem ist, das zwischen drei und zehn Ringatome enthält, die wenigstens ein Stickstoffatom und gegebenenfalls ein anderes Atom, ausgewählt aus Sauerstoff und Schwefel, enthalten;
R^{G} ein monocyclisches gesättigtes Ringsystem ist, das zwischen drei und sieben Ringatome enthält, von denen wenigstens eines ein Heteroatom, ausgewählt aus Stickstoff, Sauerstoff und Schwefel, ist;
R^{H} ein 5- oder 6-gliedriger heteroaromatischer Ring, der bis zu zwei Stickstoffatome enthält, ist und
Y ein kovalente Bindung oder -CH₂- ist.

55. Verbindung nach Anspruch 1, ausgewählt aus:
Methyl-5-((1*S*,4*S*)-2,5-diazabicyclo[2.2.1]hept-2-yl)-1-(2-ethoxyethyl)-7-(4-methylpyridin-2-ylamino)-1*H*-pyrazolo[4,3-d]pyrimidin-3-carboxylat,
Methyl-1-(2-ethoxyethyl)-5-(*N*-isopropyl-*N*-methylamino)-7-(6-methylpyridin-2-ylamino)-1*H*-pyrazolo[4,3-*d*]pyrimidin-3-carboxylat,
Ethyl-1-(2-ethoxyethyl)-5-(*N*-ethyl-*N*-methylamino)-7-(4-methylpyridin-2-ylamino)-1*H*-pyrazolo[4,3-*d*]pyrimidin-3-carboxylat,
2-(Dimethylamino)ethyl-5-dimethylamino-1-(2-ethoxyethyl)-7-(4-methylpyridin-2-ylamino)-1*H*-pyrazolo[4,3-*d*]pyrimidin-3-carboxylat,
1-(2-Ethoxyethyl)-5-(*N*-methyl-*N*-propylamino)-7-(4-methylpyridin-2-ylamino)-1*H*-pyrazolo[4,3-*d*]pyrimidin-3-carbonsäure,
5-(*N*-Isopropyl-*N*-methylamino)-7-(4-methylpyridin-2-ylamino)-1-(2-propoxy-ethyl)-1*H*-pyrazolo[4,3-*d*]pyrimidin-3-carbonsäure,
7-(4,6-dimethylpyridin-2-ylamino)-1-(2-ethoxyethyl)-5-(*N-*isopropyl-*N*-methyl-amino)-1*H*-pyrazolo[4,3-*d*]pyrimidin-3-carbonsäure,
5-(*N*-Cyclobutyl-*N*-methylamino)-1-(2-ethoxyethyl)-7-(4-methylpyridin-2-ylamino)-1*H*-pyrazolo[4,3-*d*]pyrimidin-3-carbonsäure,
1-(2-Ethoxyethyl)-5-isopropylamino-7-(4-methylpyridin-2-ylamino)-1*H*-pyrazolo[4,3-*d*]pyrimidin-3-carbonsäure;
1-(2-Ethoxyethyl)-5-(*N*-ethyl-*N*-methylamino)-7-(2-methoxypyrimidin-4-ylamino)-1*H*-pyrazolo[4,3-*d*]pyrimdin-3-carbonsäure,
3-[1-(2-Ethoxyethyl)-5-(*N*-isopropyl-*N*-methylamino)-7-(4-methylpyridin-2-ylamino)-1*H*-pyrazolo[4,3-*d*]pyrimidin-3-yl]-2H-1,2,4-oxadiazol-5-on,
3-[1-(2-Ethoxyethyl)-5-(*N*-ethyl-*N*-methylamino)-7-(4-methylpyridin-2-ylamino)-1*H*-pyrazolo[4,3-*d*]pyrimidin-3-yl]-2H-1,2,4-oxadiazol-5-on,
1-(2-Ethoxyethyl)-7-(4-fluor-3-methylphenylamino)-5-(*N-*isopropyl-*N*-methylamino)-1*H*-pyrazolo[4,3-*d*]pyrimidin-3-carbonsäure,
1-(2-Ethoxyethyl)-5-(*N*-ethyl-*N*-methylamino)-7-(4-fluor-3-methyl-phenylamino)-1*H*-pyrazolo[4,3-*d*]pyrimidin-3-carbonsäure,
7-(3,4-Dimethylphenylamino)-1-(2-ethoxyethyl)-5-(*N*-ethyl-*N*-methylamino)-1*H*-pyrazolo[4,3-*d*]pyrimidin-3-carbonsäure,
1-(2-(Cyclopropylmethoxy)ethyl)-5-(N-isopropyl-N-methylamino)-7-(4-methyl-pyridin-2-ylamino)-1*H*-pyrazolo[4,3-*d*]pyrimidin-3-carbonsäure,
1-(2-(Cyclopropylmethoxy)ethyl)-5-(*N*-ethyl-*N-*methylamino)-7-(4-methyl-pyridin-2-ylamino)-1*H*-pyrazolo[4,3-*d*]pyrimidin-3-carbonsäure,
1-(2-Ethoxyethyl)-5-(*N*-isopropyl-*N*-methylamino)-7-(4-methylpyridin-2-ylamino)-1*H*-pyrazolo[4,3-*d*]pyrimidin-3-carbonsäure,
1-(2-Isopropoxyethyl)-5-(*N*-isopropyl-*N*-methylamino)-7-(4-methylpyridin-2-ylamino)-1*H*-pyrazolo[4,3-*d*]pyrimidin-3-carbonsäure,
*N*-[1-(2-Ethoxyethyl)-5-(*N*-isopropyl-*N*-methylamino)-7-(4-methylpyridin-2-ylamino)-1*H*-pyrazolo[4,3-*d*]pyrimidin-3-carbonyl]methansulfonamid,
N-[1-(2-Ethoxyethyl)-5-(*N*-ethyl-*N*-methylamino)-7-(4-methylpyridin-2-yl-amino)-1*H*-pyrazolo[4,3-*d*]pyrimidin-3-carbonyl]methansulfonamid,
*N*-[5-(Ethyl-methyl-amino)-1-[2-(3-fluor-propoxy)-ethyl]-7-(4-methyl-pyridin-2-ylamino)-1*H*-pyrazolo[4,3-*d*]pyrimidin-3-carbonyl]-methansulfonamid,
*N*-[1-[2-(3-Fluor-propoxy)-ethyl]-5-(isopropyl-methyl-amino)-7-(4-methyl-pyridin-2-ylamino)-1*H*-pyrazolo[4,3-*d*]pyrimidin-3-carbonyl]-methansulfonamid,
*N*-[5-Diethylamino-1-[2-(3-fluor-propoxy)-ethyl]-7-(4-methyl-pyridin-2-ylamino)-1*H*-pyrazolo[4,3-*d*]pyrimidin-3-carbonyl]-methansulfonamid,
*N*-[5-Diethylamino-1-[2-(2,2-difluor-ethoxy)-ethyl]-7-(4-methyl-pyridin-2-ylamino)-1*H*-pyrazolo[4,3-*d*]pyrimidin-3-carbonyl]-methansulfonamid,
*N*-[1-[2-(2,2-Difluor-ethoxy)-ethyl]-5-(ethyl-methyl-amino)-7-(4-methyl-pyridin-2-ylamino)-1*H*-pyrazolo[4,3-*d*]pyrimidin-3-carbonyl]-methansulfonamid und
*N*-[1-[2-(2,2-Difluor-ethoxy)-ethyl]-5-(isopropyl-methyl-amino)-7-(4-methyl-pyridin-2-ylamino)-1*H*-pyrazolo[4,3-*d*]pyrimidin-3-carbonyl]-methansulfonamid
und Tautomere davon oder pharmazeutisch verträgliche Salze oder Solvate der Verbindungen oder der Tautomeren.

56. Verbindung nach Anspruch 1, nämlich 1-(2-Ethoxyethyl)-5-(methyl(propyl)amino)-7-(4-methylpyridin-2-ylamino)-1H-pyrazolo[4,3-d]pyrimidin-3-carbonsäure, ein Tautomer davon oder ein pharmazeutisch verträgliches Salz oder Solvat der Verbindung oder des Tautomeren.

57. Verbindung nach Anspruch 1, nämlich 3-(1-(2-Ethoxyethyl)-5-(ethyl(methyl)amino)-7-(4-methylpyridin-2-ylamino)-1H-pyrazolo[4,3-d]pyrimidin-3-yl)-1,2,4-oxadiazol-5(2H)-on, ein Tautomer davon oder ein pharmazeutisch verträgliches Salz oder Solvat der Verbindung oder des Tautomeren.

58. Verbindung nach Anspruch 1, nämlich 1-(2-Ethoxyethyl)-7-(4-fluor-3-methylphenylamino)-5-(isopropyl(methyl)amino)-1H-pyrazolo[4,3-d]pyrimidin-3-carbonsäure, ein Tautomer davon oder ein pharmazeutisch verträgliches Salz oder Solvat der Verbindung oder des Tautomeren.

59. Verbindung nach Anspruch 1, nämlich 1-(2-Ethoxyethyl)-5-(N-isopropyl-N-methylamino)-7-(4-methylpyridin-2-ylamino)-1H-pyrazolo[4,3-d]pyrimidin-3-carbonsäure, ein Tautomer davon oder ein pharmazeutisch verträgliches Salz oder Solvat der Verbindung oder des Tautomeren.

60. Verbindung nach Anspruch 1, nämlich N-[1-(2-Ethoxyethyl)-5-(N-isopropyl-N-methylamino)-7-(4-methylpyridin-2-ylamino)-1H-pyrazolo[4,3-d]pyrimidin-3-carbonyl]methansulfonamid, ein Tautomer davon oder ein pharmazeutisch verträgliches Salz oder Solvat der Verbindung oder des Tautomeren.

61. Verbindung nach Anspruch 1, nämlich N-[1-(2-Ethoxyethyl)-5-(N-ethyl-N-methylamino)-7-(4-methylpyridin-2-ylamino)-1H-pyrazolo[4,3-d]pyrimidin-3-carbonyl]methansulfonamid, ein Tautomer davon oder ein pharmazeutisch verträgliches Salz oder Solvat der Verbindung oder des Tautomeren.

62. Pharmazeutische Zusammensetzung, umfassend eine Verbindung nach einem der Ansprüche 1 bis 61 und ein pharmazeutisch verträgliches Verdünnungsmittel oder einen pharmazeutisch verträglichen Träger.

63. Verbindung nach einem der Ansprüche 1 bis 61 zur Verwendung als Medikament.

64. Verbindung nach einem der Ansprüche 1 bis 61 zur Verwendung nach Anspruch 57 als Medikament für die Behandlung einer Erkrankung oder eines Zustandes, von der/dem bekannt ist, dass eine Inhibierung von PDE5 eine günstige Wirkung erzeugt, oder bei/dem gezeigt werden kann, dass eine Inhibierung von PDE5 eine günstige Wirkung erzeugt.

65. Verbindung nach einem der Ansprüche 1 bis 61 zur Verwendung nach Anspruch 57 oder 58 als Medikament für die Behandlung einer Erkrankung oder eines Zustandes, ausgewählt aus Bluthochdruck, kongestiver Herzinsuffizienz, Angina, Schlaganfall, Koronararterienerkrankung, kongestiver Herzinsuffizienz, Zuständen verringerter Blutgefäßdurchgängigkeit, Erkrankung der peripheren Gefäße, Atherosklerose, Nitratinduzierter Toleranz, Nitrattoleranz, Diabetes, beeinträchtigter Glucosetoleranz, metabolischem Syndrom, Adipositas, sexueller Dysfunktion, Frühgeburt, Prä-Eklampsie, Dysmenorrhö, polyzystischem Ovarialsyndrom, benigner Prostatahyperplasie, Blasenobstruktion, Inkontinenz, chronischobstruktiver Lungenerkrankung, akutem respiratorischem Versagen, Bronchitis, chronischem Asthma, allergischem Asthma, allergischer Rhinitis, Darmmotilitätsstörungen, Kawasaki-Syndrom, Multipler Sklerose, Alzheimer'scher Erkrankung, Psoriasis, Hautnekrose, Narbenbildung, Fibrose, Schmerz, Krebs, Metastasenbildung, Kahlköpfigkeit, Nussknacker-Ösophagus, Analfissur und Hämorrhoiden.

66. Verbindung nach einem der Ansprüche 1 bis 61 zur Verwendung nach Anspruch 59 als Medikament für die Behandlung von Lungenhypertonie.

67. Verbindung nach einem der Ansprüche 1 bis 61 zur Verwendung nach Anspruch 59 als Medikament für die Behandlung von neuropathischem Schmerz.

68. Verwendung einer Verbindung nach einem der Ansprüche 1 bis 61 für die Herstellung eines Medikaments für die Behandlung einer Erkrankung oder eines Zustandes, ausgewählt aus Bluthochdruck, kongestiver Herzinsuffizienz, Angina, Schlaganfall, Koronararterien-Erkrankung, kongestiver Herzinsuffizienz, Zuständen verringerter Blutgefäßdurchgängigkeit, Erkrankung der peripheren Gefäße, Atherosklerose, Nitratinduzierter Toleranz, Nitrattoleranz, Diabetes, beeinträchtigter Glucosetoleranz, metabolischem Syndrom, Adipositas, sexueller Dysfunktion, Frühgeburt, Prä-Eklampsie, Dysmenorrhö, polyzystischem Ovarialsyndrom, benigner Prostatahyperplasie, Blasenobstruktion, Inkontinenz, chronischobstruktiver Lungenerkrankung, akutem respiratorischem Versagen, Bronchitis, chronischem Asthma, allergischem Asthma, allergischer Rhinitis, Darmmotilitätsstörungen, Kawasaki-Syndrom, Multipler Sklerose, Alzheimer'scher Erkrankung, Psoriasis, Hautnekrose, Narbenbildung, Fibrose, Schmerz, Krebs, Metastasenbildung, Kahlköpfigkeit, Nussknacker-Ösophagus, Analfissur und Hämorrhoiden.

69. Pharmazeutische Zusammensetzung, umfassend eine Verbindung nach einem der Ansprüche 1 bis 61 und ein zweites pharmazeutisch aktives Mittel, ausgewählt aus der Gruppe, bestehend aus Aspirin, Angiotensin II-Rezeptor-Antagonisten, Calciumkanalblockern, Beta-Blockern, CI1027, CCR5-Rezeptor-Antagonisten, Immidazolinen, sGCa (lösliche Guanylatcyclaseaktivatoren), Antihypertensiva, Diuretika, alpha-adrenergen Antagonisten, ACE (Angiotensin-umwandelndes Enzym)-Inhibitoren, Aldosteron-Rezeptor-Antagonisten, neurale Endopeptidase-Inhibitoren, Antidiabetika, Sulfonylharnstoffverbindungen, Glitazonen, cholesterinsenkenden Mitteln und alpha-2-delta-Liganden.

70. Pharmazeutische Zusammensetzung, umfassend eine Verbindung nach einem der Ansprüche 1 bis 55 und Pregabalin.

71. Verbindung der Formel (III) 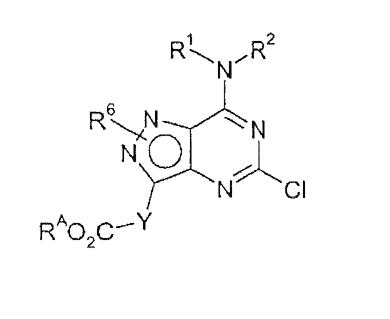 worin R¹, R², R⁶ und Y wie in Anspruch 1 definiert sind und R^{A} C₁-C₆-Alkyl oder Benzyl ist,
mit der Maßgabe, dass die Verbindung keine Verbindung der Formel: 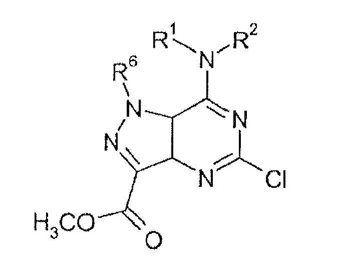 ist, worin R¹, R² und R⁶ wie in Anspruch 1 definiert sind.

72. Verbindung nach Anspruch 71 mit der Formel (III^{D})

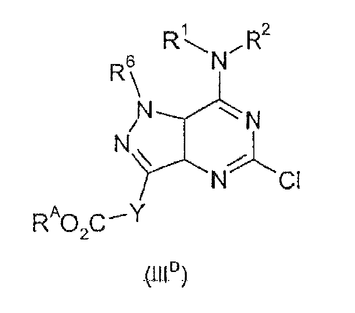

73. Verbindung der Formel (V) 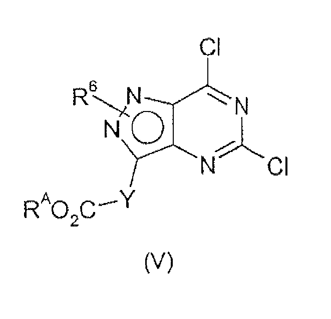 worin R⁶ und Y wie in Anspruch 1 definiert sind und R^{A} C₁-C₆-Alkyl oder Benzyl ist,
mit der Maßgabe, dass die Verbindung der Formel (V) keine Verbindung der Formel: 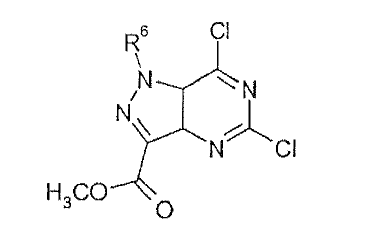 ist, worin R⁶ wie in Anspruch 1 definiert ist.

74. Verbindung nach Anspruch 73 mit der Formel (V^{B})

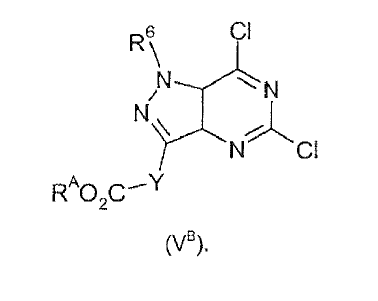

## Revendications

1. Composé de formule (I) 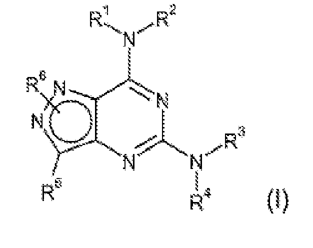 dans laquelle
R¹ représente un groupe cyclique choisi entre des groupes R^{A}, R^{B}, R^{C} et R^{D}, chacun étant facultativement substitué avec un ou plusieurs groupes R⁷ ;
R² représente un atome d'hydrogène ou un groupe alkyle en C₁ ou C₂ ;
R³ et R⁴ représentent chacun indépendamment un groupe alkyle en C₁ à C₈, alcényle en C₂ à C₈, alcynyle en C₂ à C₈ ou cycloalkyle en C₃ à C₁₀, chacun étant facultativement substitué avec un ou plusieurs groupes R⁸, ou un groupe R^{E},
qui est facultativement substitué avec un ou plusieurs groupes R⁹, ou un atome d'hydrogène ;
ou bien le groupe -NR³R⁴ forme un groupe R^{F}, qui est facultativement substitué avec un ou plusieurs groupes R¹⁰ ;
R⁵ est choisi entre des groupes -Y-CO₂R¹⁵ et -Y-R¹⁶ ;
R⁶, qui peut être fixé à N¹ ou N², représente un groupe alkyle en C₁ à C₆, halogénalkyle en C₁ à C₆, alcényle en C₂ à C₆ ou alcynyle en C₂ à C₆, chacun étant facultativement substitué avec un substituant alkoxy en C₁ à C₆, halogénalkoxy en C₁ à C₆ ou un groupe cyclique choisi entre R^{J}, R^{K}, R^{L} et R^{M}, ou bien R⁶ représente un groupe R^{N},
cycloalkyle en C₃ à C₇ ou halogénocycloalkyle en C₃ à C₇, dont chacun est facultativement substitué avec un substituant alkoxy en C₁ à C₆ ou halogénalkoxy en C₁ à C₆, ou bien R⁶ représente un atome d'hydrogène ;
R⁷ représente un groupe halogéno, alkyle en C₁ à C₆, halogénalkyle en C₁ à C₆, alcényle en C₂ à C₆, alcynyle en C₂ à C₆, cycloalkyle en C₃ à C₁₀, halogénocycloalkyle en C₃ à C₁₀, phényle, OR¹², OC(O)R¹², NO₂, NR¹²R¹³, NR¹²C(O)R¹³, NR¹²CO₂R¹⁴, C(O)R¹², CO₂R¹², CONR¹²R¹³ ou CN ;
R⁸ représente un groupe halogéno, phényle, (alkoxy en C₁ à C₆)phényle, OR¹², OC(O)R¹², NO₂, NR¹²R¹³, NR¹²C(O)R¹³, NR¹²CO₂R¹⁴, C(O)R¹², CO₂R¹², CONR¹²R¹³, CN, cycloalkyle en C₃ à C₆, R^{G} ou R^{H}, les deux derniers groupes étant facultativement substitués avec un ou plusieurs groupes R⁹ ;
R⁹ représente un groupe alkyle en C₁ à C₆, halogénalkyle en C₁ à C₆ ou CO₂R¹² ;
R¹⁰ représente un groupe halogéno, cycloalkyle en C₃ à C₁₀, halogénocycloalkyle en C₃ à C₁₀, phényle, OR¹², OC(O)R¹², NO₂, NR¹²R¹³, NR¹²C(O)R¹³, NR¹²CO₂R¹⁴, C(O)R¹², CO₂R¹³, CONR¹²R¹³, CN, oxo, alkyle en C₁ à C₆ ou halogénalkyle en C₁ à C₆, ces deux derniers groupes étant facultativement substitués avec R¹¹ ;
R¹¹ représente un groupe phényle, NR¹²R¹³ ou NR¹²CO₂R¹⁴ ;
R¹² et R¹³ représentent chacun indépendamment un atome d'hydrogène, un groupe alkyle en C₁ à C₆ ou halogénalkyle en C₁ à C₆ ;
R¹⁴ représente un groupe alkyle en C₁ à C₆ ou halogénalkyle en C₁ à C₆ ;
R¹⁵ représente un atome d'hydrogène ou un groupe alkyle en C₁ à C₆ facultativement substitué avec un ou plusieurs groupes choisis entre des groupes halogéno, OH, alkyloxy en C₁ à C₆, NH₂, NH (alkyle en C₁ à C₆) et N (alkyle en C₁ à C₆)₂ ;
R¹⁶ représente un isostère d'acide carboxylique choisi entre des groupes tétrazole-5-yle, 5-trifluorométhyl-1,2,4-triazole-3-yle, 5-(méthylsulfonyl)-1,2,4-triazole-3-yle, 2,5-dihydro-5-oxo-1,2,4-oxadiazole-3-yle, -SO₂NHR¹⁷ et -CONHR¹⁸ ;
R¹⁷ est choisi entre des groupes alkyle en C₁ à C₆, phényle, -CO-(alkyle en C₁ à C₆) et -CO-phényle ;
R¹⁸ est choisi entre des groupes -SO₂-(alkyle en C₁ à C₆) et -SO₂-phényle ;
R^{A} et R^{J} représentent chacun indépendamment un groupe cycloalkyle en C₃ à C₁₀ ou cycloalcényle en C₃ à C₁₀, chacun pouvant être monocyclique ou, lorsqu'il existe un nombre approprié d'atomes dans le noyau, polycyclique, et qui peut être condensé avec soit
(a) un noyau aromatique monocyclique choisi entre un noyau benzénique et un noyau hétéroaromatique penta- ou hexagonal contenant jusqu'à trois hétéroatomes choisis entre l'azote, l'oxygène et le soufre, soit
(b) un noyau hétéroalicyclique penta-, hexa- ou heptagonal contenant jusqu'à trois hétéroatomes choisis entre l'azote, l'oxygène et le soufre ;
R^{B} et R^{K} représentent chacun indépendamment un groupe phényle ou naphtyle, dont chacun peut être condensé avec
(a) un noyau cycloalkyle en C₅ à C₇ ou cycloalcényle en C₅ à C₇,
(b) un noyau hétéroalicyclique penta-, hexa- ou heptagonal contenant jusqu'à trois hétéroatomes choisis entre l'azote, l'oxygène et le soufre, ou
(c) un noyau hétéroaromatique penta- ou hexagonal contenant jusqu'à trois hétéroatomes choisis entre l'azote, l'oxygène et le soufre ;
R^{C}, R^{L} et R^{N} représentent chacun indépendamment un système de noyau monocyclique ou, lorsqu'il existe un nombre approprié d'atomes dans le noyau, polycyclique, saturé ou partiellement insaturé, contenant 3 à 10 atomes dans le noyau, dont au moins l'un est un hétéroatome choisi entre l'azote, l'oxygène et le soufre, noyau qui peut être condensé avec un groupe cycloalkyle en C₅ à C₇ ou cycloalcényle en C₅ à C₇ ou un noyau aromatique monocyclique choisi entre un noyau benzénique et un noyau hétéroaromatique penta- ou hexagonal contenant jusqu'à trois hétéroatomes choisis entre l'azote, l'oxygène et le soufre ;
R^{D} et R^{M} représentent chacun indépendamment un noyau hétéroaromatique penta- ou hexagonal contenant jusqu'à trois hétéroatomes choisis indépendamment entre l'azote, l'oxygène et le soufre, noyau qui peut être en outre condensé avec
(a) un second noyau hétéroaromatique penta- ou hexagonal contenant jusqu'à trois hétéroatomes choisis entre l'azote, l'oxygène et le soufre ;
(b) un noyau cycloalkyle en C₅ à C₇ ou cycloalcényle en C₅ à C₇ ;
(c) un noyau hétéroalicyclique penta-, hexa- ou heptagonal contenant jusqu'à trois hétéroatomes choisis entre l'azote, l'oxygène et le soufre, ou
(d) un noyau benzénique ;
R^{E}, R^{F} et R^{G} représentent chacun indépendamment un système de noyau monocyclique ou, lorsqu'il existe un nombre approprié d'atomes dans le noyau, polycyclique, saturé contenant 3 à 10 atomes dans le noyau, dont au moins l'un est un hétéroatome choisi entre l'azote, l'oxygène et le soufre ;
R^{H} représente un noyau hétéroaromatique penta- ou hexagonal contenant jusqu'à trois hétéroatomes choisis indépendamment entre l'azote, l'oxygène et le soufre ; et
Y représente une liaison covalente, un groupe -CH₂-O-CH₂-, alkylényle en C₁ à C₆ ou cycloalkylényle en C₃ à C₇ ;
une de ses formes tautomères ou un sel ou produit de solvatation pharmaceutiquement acceptable dudit composé ou de ladite forme tautomère.

2. Composé suivant la revendication 1, dans lequel R¹ représente un groupe R^{A}, qui est facultativement substitué avec un ou plusieurs groupes R⁷ ; et
R^{A} représente un groupe cycloalkyle en C₃ à C₁₀, qui peut être monocyclique ou, lorsqu'il existe un nombre approprié d'atomes dans le noyau, polycyclique, qui peut être condensé avec
(a) un noyau aromatique monocyclique choisi entre un noyau benzénique et un noyau hétéroaromatique penta- ou hexagonal contenant jusqu'à trois hétéroatomes choisis entre l'azote, l'oxygène et le soufre, ou
(b) un noyau hétéroalicyclique penta-, hexa- ou heptagonal contenant jusqu'à trois hétéroatomes choisis entre l'azote, l'oxygène et le soufre.

3. Composé suivant la revendication 2, dans lequel R^{A} représente un groupe cycloalkyle en C₃ à C₈ monocyclique.

4. Composé suivant la revendication 3, dans lequel R^{A} représente un groupe cycloalkyle en C₅ à C₇ monocyclique.

5. Composé suivant la revendication 4, dans lequel R^{A} représente un groupe cyclopentyle ou cyclohexyle.

6. Composé suivant la revendication 1, dans lequel R¹ représente un groupe R^{B}, qui est facultativement substitué avec un ou plusieurs groupes R⁷.

7. Composé suivant la revendication 6, dans lequel R^{B} représente un groupe phényle.

8. Composé suivant la revendication 1, dans lequel R¹ représente un groupe R^{C}, qui est facultativement substitué avec un ou plusieurs groupes R⁷.

9. Composé suivant la revendication 8, dans lequel R^{C} représente un système de noyau monocyclique, saturé ou partiellement insaturé, contenant 3 à 8 atomes dans le noyau, dont au moins l'un est un hétéroatome choisi entre l'azote, l'oxygène et le soufre.

10. composé suivant la revendication 9, dans lequel R^{C} représente un système de noyau monocyclique, saturé ou partiellement insaturé, contenant 5 à 7 atomes dans le noyau, dont au moins l'un est un hétéroatome choisi entre l'azote, l'oxygène et le soufre.

11. Composé suivant la revendication 10, dans lequel R^{C} représente un système de noyau monocyclique saturé contenant 5 à 7 atomes dans le noyau, dont au moins l'un est un hétéroatome choisi entre l'azote, l'oxygène et le soufre.

12. Composé suivant la revendication 1, dans lequel R¹ représente un groupe R^{D}, qui est facultativement substitué avec un ou plusieurs groupes R⁷.

13. Composé suivant la revendication 12, dans lequel R^{D} représente un noyau hétéroaromatique penta- ou hexagonal contenant jusqu'à trois hétéroatomes choisis indépendamment entre l'azote, l'oxygène et le soufre.

14. composé suivant la revendication 13, dans lequel R^{D} représente un noyau hétéroaromatique pentagonal contenant un hétéroatome choisi entre l'azote, l'oxygène et le soufre et contenant facultativement jusqu'à deux atomes d'azote supplémentaires dans le noyau, ou un noyau hétéroaromatique hexagonal comprenant 1, 2 ou 3 atomes d'azote.

15. Composé suivant la revendication 14, dans lequel R^{D} représente un groupe furannyle, thiényle, pyrrolyle, pyrazolyle, imidazolyle, isoxazolyle, oxazolyle, isothiazolyle, thiazolyle, oxadiazolyle, pyridyle, pyridazinyle, pyrimidyle ou pyrazinyle.

16. Composé suivant la revendication 15, dans lequel R^{D} représente un groupe pyrazolyle, imidazolyle, isoxazolyle, oxazolyle, oxadiazolyle, pyridyle, pyridazinyle, pyrimidyle ou pyrazinyle.

17. Composé suivant l'une quelconque des revendications 1 à 16, dans lequel R⁷ représente un groupe halogéno, alkyle en C₁ à C₆, halogénalkyle en C₁ à C₆, OR¹² ou CONR¹²R¹³.

18. Composé suivant la revendication 17, dans lequel R⁷ représente un groupe halogéno, alkyle en C₁ à C₃, alkoxy en C₁ à C₃, hydroxy ou CONH(alkyle en C₁ à C₃).

19. Composé suivant la revendication 18, dans lequel R⁷ représente un groupe fluoro, méthyle, éthyle, hydroxy, méthoxy, propoxy ou CONHMe.

20. Composé suivant l'une quelconque des revendications 1 à 19, dans lequel R² représente un atome d'hydrogène ou un groupe méthyle.

21. composé suivant la revendication 20, dans lequel R² représente un atome d'hydrogène.

22. Composé suivant l'une quelconque des revendications 1 à 21, dans lequel R³ représente un atome d'hydrogène, un groupe alkyle en C₁ à C₆, qui est facultativement substitué avec un ou plusieurs groupes R⁸, ou un groupe R^{E}, qui est facultativement substitué avec un ou plusieurs groupes R⁹ ; et dans lequel R^{E} représente un système de noyau saturé monocyclique ou, lorsqu'il existe un nombre approprié d'atomes dans le noyau, polycyclique, contenant 3 à 7 atomes dans le noyau, dont au moins l'un est un hétéroatome choisi entre l'azote, l'oxygène et le soufre.

23. Composé suivant la revendication 22, dans lequel R³ représente un atome d'hydrogène, un groupe alkyle en C₁ à C₄, qui est facultativement substitué avec un ou plusieurs groupes R⁸, ou un groupe R^{E}, qui est facultativement substitué avec un ou plusieurs groupes R⁹ ; et dans lequel R^{E} représente un système de noyau monocyclique saturé contenant 3 à 7 atomes dans le noyau, dont au moins l'un est un hétéroatome choisi entre l'azote, l'oxygène et le soufre.

24. Composé suivant la revendication 23, dans lequel R³ représente un groupe R^{E}, qui est facultativement substitué avec un ou plusieurs groupes R⁹, et dans lequel R^{E} représente un système de noyau monocyclique saturé contenant 3 à 7 atomes dans le noyau, contenant un atome d'azote.

25. Composé suivant la revendication 24, dans lequel R^{E} représente un groupe azétidinyle, pyrrolidinyle ou pipéridinyle.

26. composé suivant la revendication 23, dans lequel R³ représente un groupe alkyle en C₁ à C₄, qui est facultativement substitué avec un ou plusieurs groupes R⁸, et dans lequel R⁸ représente un groupe halogéno, phényle, (alkoxy en C₁ à C₆)phényle, OR¹², NR¹²R¹³, NR¹²CO₂R¹⁴, CO₂R¹², CONR¹²R¹³, R^{G} ou R^{H}, les deux derniers étant facultativement substitués avec un ou plusieurs groupes R⁹.

27. Composé suivant la revendication 26, dans lequel R⁸ représente un groupe hydroxy, méthoxy, méthoxyphényle, NH₂, NHMe, NMe₂, NHCO₂^{t}Bu, NMeCO₂^{t}Bu, CO₂H, CONHMe, R^{G} ou R^{H}, les deux derniers étant facultativement substitués avec un ou plusieurs groupes R⁹.

28. Composé suivant la revendication 27, dans lequel R⁸ représente un groupe R^{G}, qui est facultativement substitué avec un ou plusieurs groupes R⁹, et dans lequel R^{G} représente un système de noyau monocyclique saturé contenant 3 à 7 atomes dans le noyau, dont au moins l'un est un hétéroatome choisi entre l'azote, l'oxygène et le soufre.

29. Composé suivant la revendication 28, dans lequel R^{G} représente un système de noyau monocyclique saturé contenant 3 à 7 atomes dans le noyau, contenant un atome d'azote, et facultativement un atome d'oxygène.

30. Composé suivant la revendication 29, dans lequel R^{G} représente un groupe pyrrolidinyle, pipéridinyle ou morpholinyle.

31. Composé suivant la revendication 27, dans lequel R⁸ représente un groupe R^{H}, qui est facultativement substitué avec un ou plusieurs groupes R⁹, et dans lequel R^{H} représente un noyau hétéroaromatique penta- ou hexagonal contenant jusqu'à deux atomes d'azote.

32. Composé suivant la revendication 31, dans lequel R^{H} représente un groupe pyrazolyle.

33. composé suivant l'une quelconque des revendications 22 à 32, dans lequel R⁹ représente un groupe méthyle ou CO₂^{t}Bu.

34. Composé suivant la revendication 23, dans lequel R³ représente un atome d'hydrogène ou un groupe alkyle en C₁ à C₄, qui est facultativement substitué avec un ou plusieurs groupes R⁸, ou bien R³ représente un groupe azétidinyle, pyrrolidinyle ou pipéridinyle, dont chacun est facultativement substitué avec un ou plusieurs groupes R⁹, et dans lequel
R⁸ représente un groupe hydroxy, méthoxy, méthoxyphényle, NH₂, NHMe, NMe₂, NHCO₂^{t}Bu, NMeCO₂^{t}Bu, CO₂H, CONHMe, pyrrolidinyle, pipéridinyle, morpholinyle ou pyrazolyle, les quatre derniers étant facultativement substitués avec un ou plusieurs groupes R⁹, et dans lequel
R⁹ représente un groupe méthyle ou CO₂^{t}Bu.

35. Composé suivant l'une quelconque des revendications 1 à 34, dans lequel R⁴ représente un atome d'hydrogène, un groupe alkyle en C₁ à C₆, halogénalkyle en C₁ à C₆, alcényle en C₂ à C₆ ou alcynyle en C₂ à C₆.

36. Composé suivant la revendication 35, dans lequel R⁴ représente un atome d'hydrogène, un groupe alkyle en C₁ à C₆ ou halogénalkyle en C₁ à C₆.

37. Composé suivant la revendication 36, dans lequel R⁴ représente un atome d'hydrogène, un groupe méthyle ou éthyle.

38. Composé suivant l'une quelconque des revendications 1 à 21, dans lequel -NR³R⁴ forme un groupe R^{F}, qui est facultativement substitué avec un ou plusieurs groupes R¹⁰, et dans lequel R^{F} représente un système de noyau saturé monocyclique ou, lorsqu'il existe un nombre approprié d'atomes dans le noyau, polycyclique, contenant 3 à 10 atomes dans le noyau, contenant au moins un atome d'azote et facultativement un autre atome choisi entre l'oxygène et le soufre.

39. Composé suivant la revendication 38, dans lequel R^{F} représente un système de noyau saturé monocyclique ou, lorsqu'il existe un nombre approprié d'atomes dans le noyau, polycyclique, contenant 3 à 10 atomes dans le noyau, contenant un ou deux atomes d'azote et facultativement un autre atome choisi entre l'oxygène et le soufre.

40. Composé suivant la revendication 39, dans lequel R^{F} est choisi entre des groupes azétidinyle, pyrrolidinyle, pipéridinyle, pipérazinyle, morpholinyle, 3-azabicyclo[3.1.0]hex-3-yle, homopipérazinyle, 2,5-diaza-bicyclo[4.3.0]non-2-yle, 3,8-diazabicyclo[3.2.1]oct-3-yle, 3,8-diazabicyclo[3.2.1]oct-8-yle, 2,5-diazabicyclo[2.2.1]-hept-2-yle, 1,4-diazabicyclo[4.3.0]non-4-yle et 1,4-diazabicyclo[3.2.2]non-4-yle.

41. Composé suivant l'une quelconque des revendications 38 à 40, dans lequel R¹⁰ représente un groupe halogéno, OR¹², NR¹²R¹³, NR¹²CO₂R¹⁴, CO₂R¹³, oxo, alkyle en C₁ à C₆ ou halogénalkyle en C₁ à C₆, les deux derniers étant facultativement substitués avec R¹¹.

42. Composé suivant la revendication 41, dans lequel R¹⁰ représente un groupe halogéno, méthyle, éthyle, isopropyle, hydroxy, méthoxy, NH₂, NHMe, NMe₂, NHCO₂^{t}Bu, CO₂H, CO₂^{t}Bu, oxo, benzyle, -CH₂NH₂, -CH₂NHMe, CH₂NMe₂ ou -CH₂NMeCO₂^{t}Bu.

43. Composé suivant l'une quelconque des revendications 1 à 42, dans lequel R⁵ représente un groupe -Y-CO₂R¹⁵.

44. Composé suivant la revendication 43, dans lequel R¹⁵ représente un atome d'hydrogène ou un groupe alkyle en C₁ à C₃ et Y représente une liaison covalente.

45. Composé suivant l'une quelconque des revendications 1 à 42, dans lequel R⁵ représente un groupe -Y-R¹⁶.

46. Composé suivant la revendication 45, dans lequel R¹⁶ représente un groupe -CONHR¹⁸, tétrazole-5-yle ou 2,5-dihydro-5-oxo-1,2,4-oxadiazole-3-yle et Y représente une liaison covalente ou un groupe méthylène.

47. Composé suivant l'une quelconque des revendications 1 à 46, dans lequel R⁶ est en position sur N¹.

48. Composé suivant l'une quelconque des revendications 1 à 47, dans lequel R⁶ représente un groupe alkyle en C₁ à C₆ ou halogénalkyle en C₁ à C₆, chacun étant facultativement substitué avec un groupe alkoxy en C₁ à C₆, un groupe halogénalkoxy en C₁ à C₆ ou un groupe cyclique choisi entre R^{J}, R^{L} et R^{M}, ou bien R⁶ représente un groupe
R^{N} ou un atome d'hydrogène ;
R^{J} représente un groupe cycloalkyle monocyclique en C₃ à C₇ ;
R^{L} et R^{N} représentent chacun indépendamment un système de noyau monocyclique, saturé ou partiellement insaturé,
contenant 4 à 7 atomes dans le noyau, dont au moins l'un est un hétéroatome choisi entre l'azote, l'oxygène et le soufre ; et
R^{M} représente un noyau hétéroaromatique penta- ou hexagonal contenant jusqu'à trois hétéroatomes choisis indépendamment entre l'azote, l'oxygène et le soufre.

49. Composé suivant la revendication 48, dans lequel R⁶ représente un groupe alkyle en C₁ à C₄ ou halogénalkyle en C₁ à C₄, chacun étant facultativement substitué avec un groupe alkoxy en C₁ à C₄, un groupe halogénalkoxy en C₁ à C₄ ou un groupe cyclique choisi entre
R^{J}, R^{L} et R^{M}, ou bien R⁶ représente un groupe R^{N} ou un atome d'hydrogène ;
R^{J} représente un groupe cyclopropyle ou cyclobutyle ;
R^{L} et R^{N} représentent chacun indépendamment un système de noyau monocyclique saturé contenant 5 ou 6 atomes dans le noyau, dont au moins l'un est un hétéroatome choisi entre l'azote, l'oxygène et le soufre ; et
R^{M} représente un noyau hétéroaromatique penta- ou hexagonal contenant un hétéroatome choisi entre l'azote, l'oxygène et le soufre.

50. Composé suivant la revendication 49, dans lequel R⁶ représente un groupe alkyle en C₁ à C₄ ou halogénalkyle en C₁ à C₄, chacun étant facultativement substitué avec un groupe alkoxy en C₁ à C₄ ou un groupe cyclique choisi entre R^{J}, R^{L} et R^{M}, ou bien R⁶ représente un groupe R^{N} ou un atome d'hydrogène ;
R^{J} représente un groupe cyclopropyle ou cyclobutyle ;
R^{L} et R^{N} représentent chacun indépendamment un système de noyau monocyclique saturé contenant 5 ou 6 atomes dans le noyau, contenant un hétéroatome choisi entre l'azote, l'oxygène et le soufre ; et
R^{M} représente un noyau hétéroaromatique penta- ou hexagonal contenant un atome d'azote.

51. Composé suivant la revendication 50, dans lequel R⁶ représente un groupe alkyle en C₁ à C₄ ou halogénalkyle en C₁ à C₄, chacun étant facultativement substitué avec un substituant alkoxy en C₁ à C₄, cyclopropyle, cyclobutyle, tétrahydrofurannyle, tétrahydropyrannyle ou pyridinyle, ou bien R⁶ représente un atome d'hydrogène ou un groupe tétrahydropyrannyle.

52. Composé suivant la revendication 51, dans lequel R⁶ représente un atome d'hydrogène, un groupe méthyle, éthyle, isopropyle, isobutyle, méthoxyéthyle, méthoxypropyle, éthoxyéthyle, éthoxypropyle, propoxyéthyle, 2,2,2-trifluoréthyle, tétrahydrofurannylméthyle, tétra-hydropyrannylméthyle, tétrahydropyrannyle ou pyridinyl-méthyle.

53. Composé suivant la revendication 1, dans lequel
R³ représente un atome d'hydrogène, un groupe alkyle en C₁ à C₄ qui est facultativement substitué avec un ou plusieurs groupes R⁸, ou un groupe R^{E} qui est facultativement substitué avec un ou plusieurs groupes R⁹ ;
R⁴ représente un atome d'hydrogène, un groupe alkyle en C₁ à C₆ ou halogénalkyle en C₁ à C₆ ;
ou bien -NR³R⁴ forme un groupe R^{F}, qui est facultativement substitué avec un ou plusieurs groupes R¹⁰ ;
R⁶ représente un groupe alkyle en C₁ à C₄ ou halogénalkyle en C₁ à C₄, dont chacun est facultativement substitué avec un groupe alkoxy en C₁ à C₄, un groupe halogénalkoxy en C₁ à C₄ ou un groupe cyclique choisi entre R^{J}, R^{L} et R^{M}, ou bien R⁶ représente un groupe R^{N} ou un atome d'hydrogène ;
R^{A} représente un groupe cycloalkyle en C₃ à C₈ monocyclique ;
R^{B} représente un groupe phényle ;
R^{C} représente un système de noyau monocyclique, saturé ou partiellement insaturé, contenant 3 à 8 atomes dans le noyau, dont au moins l'un est un hétéroatome choisi entre l'azote, l'oxygène et le soufre ;
R^{D} représente un noyau hétéroaromatique penta- ou hexagonal contenant jusqu'à trois hétéroatomes choisis indépendamment entre l'azote, l'oxygène et le soufre ;
R^{E} représente un système de noyau saturé monocyclique contenant 3 à 7 atomes dans le noyau, dont au moins l'un est un hétéroatome choisi entre l'azote, l'oxygène et le soufre ;
R^{F} représente un système de noyau saturé monocyclique ou, lorsqu'il existe un nombre approprié d'atomes dans le noyau, polycyclique, contenant 3 à 10 atomes dans le noyau, dont au moins l'un est un hétéroatome choisi entre l'azote, l'oxygène et le soufre ;
R^{J} représente un groupe cyclopropyle ou cyclobutyle ;
R^{L} et R^{N} représentent chacun indépendamment un système de noyau saturé monocyclique contenant 5 ou 6 atomes dans le noyau, dont au moins l'un est un hétéroatome choisi entre l'azote, l'oxygène et le soufre ;
R^{M} représente un noyau hétéroaromatique penta- ou hexagonal contenant un hétéroatome choisi entre l'azote, l'oxygène et le soufre ; et
Y représente une liaison covalente ou un groupe alkylényle en C₁ à C₆.

54. Composé suivant la revendication 53, dans lequel R¹ représente un groupe cyclique choisi entre des groupes R^{A}, R^{B}, R^{C} et R^{D}, dont chacun est facultativement substitué avec un ou plusieurs groupes R⁷ ;
R⁷ représente un groupe halogéno, alkyle en C₁ à C₆, halogénalkyle en C₁ à C₆, OR¹² ou CONR¹²R¹³;
R⁸ représente un groupe halogéno, phényle, (alkoxy en C₁ à C₆)phényle, OR¹², NR¹²R¹³, NR¹²CO₂R¹⁴, CO₂R¹², CONR¹²R¹³, R^{G} ou
R^{H}, les deux derniers étant facultativement substitués avec un ou plusieurs groupes R⁹ ;
R^{A} représente un groupe cycloalkyle en C₅ à C₇ monocyclique ;
R^{B} représente un groupe phényle ;
R^{C} représente un système de noyau monocyclique saturé contenant 5 à 7 atomes dans le noyau, dont au moins l'un est un hétéroatome choisi entre l'azote, l'oxygène et le soufre ;
R^{D} représente un noyau hétéroaromatique pentagonal contenant un hétéroatome choisi entre l'azote, l'oxygène et le soufre et facultativement jusqu'à deux atomes d'azote supplémentaires dans le noyau, ou un noyau hétéroaromatique hexagonal comprenant 1, 2 ou 3 atomes d'azote ;
R^{E} représente un système de noyau saturé monocyclique contenant 3 à 7 atomes dans le noyau, contenant un atome d'azote ;
R^{F} représente un système de noyau saturé monocyclique ou, lorsqu'il existe un nombre approprié d'atomes dans le noyau, polycyclique, contenant 3 à 10 atomes dans le noyau, contenant au moins un atome d'azote et facultativement un autre atome choisi entre l'oxygène et le soufre ;
R^{G} représente un système de noyau monocyclique saturé contenant 3 à 7 atomes dans le noyau, dont au moins l'un est un hétéroatome choisi entre l'azote, l'oxygène et le soufre ;
R^{H} représente un noyau hétéroaromatique penta- ou hexagonal contenant jusqu'à deux atomes d'azote ; et
Y représente une liaison covalente ou un groupe -CH₂-.

55. Composé suivant la revendication 1, choisi entre :
le 5-((1*S*,4*S*)-2,5-diazabicyclo[2.2.1]hept-2-yl)-1-(2-éthoxyéthyl)-7-(4-méthyl-pyridine-2-ylamino)-1*H*-pyrazolo-[4,3-*d*]pyrimidine-3-carboxylate de méthyle,
le 1-(2-éthoxyéthyl)-5-(*N*-isopropyl-*N*-méthylamino)-7-(6-méthylpyridine-2-ylamino)-1*H*-pyrazolo[4,3-d]pyrimidine-3-carboxylate de méthyle,
le 1-(2-éthoxyéthyl)-5-(*N*-éthyl-*N*-méthylamino)-7-(4-méthyl-pyridine-2-ylamino)-1*H*-pyrazolo[4,3-*d*]pyrimidine-3-carboxylate d'éthyle,
le 5-diméthylamino-1-(2-éthoxyéthyl)-7-(4-méthylpyridine-2-ylamino)-1*H*-pyrazolo[4,3-*d*]pyrimidine-3-carboxylate de 2-(diméthylamino)éthyle,
l'acide 1-(2-éthoxyéthyl)-5-(*N*-méthyl-*N*-propylamino)-7-(4-méthylpyridine-2-ylamino)-1*H*-pyrazolo[4,3-*d*]pyrimidine-3-carboxylique,
l'acide 5-(*N*-isopropyl-*N*-méthylamino)-7-(4-méthylpyridine-2-ylamino)-1-(2-propoxyéthyl)-1*H*-pyrazolo[4,3-*d*]pyrimidine-3-carboxylique,
l'acide 7-(4,6-diméthylpyridine-2-ylamino)-1-(2-éthoxy-éthyl)-5-(N-isopropyl-*N*-méthylamino)-1*H*-pyrazolo[4,3-*d*]-pyrimidine-3-carboxylique,
l'acide 5-(*N*-cyclobutyl-*N*-méthylamino)-1-(2-éthoxyéthyl)-7-(4-méthylpyridine-2-ylamino)-1*H*-pyrazolo[4,3-*d*]pyrimidine-3-carboxylique,
l'acide 1-(2-éthoxyéthyl)-5-isopropylamino-7-(4-méthyl-pyridine-2-ylamino)-1*H*-pyrazolo[4,3-*d*]pyrimidine-3-carboxylique,
l'acide 1-(2-éthoxyéthyl)-5-(*N*-éthyl-*N*-méthylamino)-7-(2-méthoxypyrimidine-4-ylamino)-1*H*-pyrazolo[4,3-*d*]pyrimidine-3-carboxylique,
la 3-[1-(2-éthoxyéthyl)-5-(*N*-isopropyl-*N*-méthylamino)-7-(4-méthylpyridine-2-ylamino)-1*H*-pyrazolo[4,3-*d*]pyrimidine-3-yl]-2*H*-1,2,4-oxadiazole-5-one,
la 3-[1-(2-éthoxyéthyl)-5-(*N*-éthyl-*N*-méthylamino)-7-(4-méthylpyridine-2-ylamino)-1*H*-pyrazolo[4,3-*d*]pyrimidine-3-yl]-2*H*-1,2,4-oxadiazole-5-one,
l'acide 1-(2-éthoxyéthyl)-7-(4-fluoro-3-méthylphénylamino)-5-(*N*-isopropyl-*N*-méthylamino)-1*H*-pyrazolo[4,3-*d*]pyrimidine-3-carboxylique,
l'acide 1-(2-éthoxyéthyl)-5-(*N*-éthyl-*N*-méthylamino)-7-(4-fluoro-3-méthylphénylamino)-1*H*-pyrazolo[4,3-*d*]pyrimidine-3-carboxylique,
l'acide 7-(3,4-diméthylphénylamino)-1-(2-éthoxyéthyl)-5-(*N-*éthyl-*N*-méthylamino)-1*H*-pyrazolo[4,3-*d*]pyrimidine-3-carboxylique,
l'acide 1-(2-(cyclopropylméthoxy)éthyl)-5-(*N*-isopropyl-*N-*méthylamino)-7-(4-méthylpyridine-2-ylamino)-1*H*-pyrazolo-[4,3-d]pyrimidine-3-carboxylique,
l'acide 1-(2-(cyclopropylméthoxy)éthyl)-5-(*N*-éthyl-*N-*méthylamino)-7-(4-méthylpyridine-2-ylamino)-1*H*-pyrazolo-[4,3-*d*]pyrimidine-3-carboxylique,
l'acide 1-(2-éthoxyéthyl)-5-(*N*-isopropyl-*N*-méthylamino)-7-(4-méthylpyridine-2-ylamino)-1*H*-pyrazolo[4,3-*d*]pyrimidine-3-carboxylique,
l'acide 1-(2-isopropoxyéthyl)-5-(*N*-isopropyl-*N*-méthyl-amino)-7-(4-méthylpyridine-2-ylamino)-1*H*-pyrazolo[4,3-*d*]-pyrimidine-3-carboxylique,
le *N*-[1-(2-éthoxyéthyl)-5-(*N*-isopropyl-*N*-méthylamino)-7-(4-méthylpyridine-2-ylamino)-1*H*-pyrazolo[4,3-*d*]pyrimidine-3-carbonyl]méthanesulfonamide,
le *N*-[1-(2-éthoxyéthyl)-5-(*N*-éthyl-*N*-méthylamino)-7-(4-méthylpyridine-2-ylamino)-1*H*-pyrazolo[4,3-d]pyrimidine-3-carbonyl]méthanesulfonamide,
le *N*-[5-(éthyl-méthylamino)-1-[2-(3-fluoro-propoxy)-éthyl]-7-(4-méthylpyridine-2-ylamino)-1*H*-pyrazolo[4,3-*d*]-pyrimidine-3-carbonyl]méthanesulfonamide,
le *N*-[1-[2-(3-fluoro-propoxy)-éthyl]-5-(isopropyl-méthyl-amino)-7-(4-méthylpyridine-2-ylamino)-1*H*-pyrazolo[4,3-*d*]-pyrimidine-3-carbonyl]méthanesulfonamide,
le *N*-[5-diéthylamino-1-[2-(3-fluoro-propoxy)-éthyl]-7-(4-méthylpyridine-2-ylamino)-1*H*-pyrazolo[4,3-*d*]pyrimidine-3-carbonyl]méthanesulfonamide,
le *N*-[5-diéthylamino-1-[2-(2,2-difluoréthoxy)-éthyl]-7-(4-méthylpyridine-2-ylamino)-1*H*-pyrazolo[4,3-*d*]pyrimidine-3-carbonyl]méthanesulfonamide,
le *N*-[1-[2-(2,2-difluoréthoxy)-éthyl]-5-(éthyl-méthyl-amino)-7-(4-méthylpyridine-2-ylamino)-1*H*-pyrazolo[4,3-*d*]-pyrimidine-3-carbonyl]méthanesulfonamide, et
le *N*-[1-[2-(2,2-difluoréthoxy)-éthyl]-5-(isopropyl-méthylamino)-7-(4-méthylpyridine-2-ylamino)-1*H*-pyrazolo-[4,3-d]pyrimidine-3-carbonyl]méthanesulfonamide,
et leurs formes tautomères, ou les sels ou produits de solvatation pharmaceutiquement acceptables desdits composés ou desdites formes tautomères.

56. Composé suivant la revendication 1, qui est l'acide 1-(2-éthoxyéthyl)-5-(méthylpropyl)amino)-7-(4-méthylpyridine-2-ylamino)-1H-pyrazolo[4,3-d]pyrimidine-3-carboxylique, une de ses formes tautomères ou un sel ou produit de solvatation pharmaceutiquement acceptables dudit composé ou de ladite forme tautomère.

57. Composé suivant la revendication 1, qui est la 3-1-(2-éthoxyéthyl)-5-(éthyl(méthyl)amino)-7-(4-méthyl-pyridine-2-ylamino)-1H-pyrazolo[4,3-d]pyrimidine-3-yl)-1,2,4-oxadiazole-5-(2H)-one, une de ses formes tautomères ou un sel ou produit de solvatation pharmaceutiquement acceptables dudit composé ou de ladite forme tautomère.

58. Composé suivant la revendication 1, qui est l'acide 1-(2-éthoxyéthyl)-7-(4-fluoro-3-méthylphénylamino)-5-(isopropyl(méthyl)amino)-1H-pyrazolo[4,3-d]pyrimidine-3-carboxylique, une de ses formes tautomères ou un sel ou produit de solvatation pharmaceutiquement acceptables dudit composé ou de ladite forme tautomère.

59. Composé suivant la revendication 1, qui est l'acide 1-(2-éthoxyéthyl)-5-(N-isopropyl-N-méthylamino)-7-(4-méthylpyridine-2-ylamino)-1H-pyrazolo[4,3-d]pyrimidine-3-carboxylique, une de ses formes tautomères ou un sel ou produit de solvatation pharmaceutiquement acceptables dudit composé ou de ladite forme tautomère.

60. Composé suivant la revendication 1, qui est le N-[1-(2-éthoxyéthyl)-5-(N-isopropyl-N-méthylamino)-7-(4-méthylpyridine-2-ylamino)-1H-pyrazolo[4,3-d]pyrimidine-3-carbonyl]méthanesulfonamide, une de ses formes tautomères ou un sel ou produit de solvatation pharmaceutiquement acceptables dudit composé ou de ladite forme tautomère.

61. Composé suivant la revendication 1, qui est 1 e N-[1-(2-éthoxyéthyl)-5-(N-éthyl-N-méthylamino)-7-(4-méthylpyridine-2-ylamino)-1H-pyrazolo[4,3-d]pyrimidine-3-carbonyl]méthanesulfonamide, une de ses formes tautomères ou un sel ou produit de solvatation pharmaceutiquement acceptables dudit composé ou de ladite forme tautomère.

62. Composition pharmaceutique comprenant un composé suivant l'une quelconque des revendications 1 à 61, un diluant ou support pharmaceutiquement acceptables.

63. Composé suivant l'une quelconque des revendications 1 à 61, destiné à être utilisé comme médicament.

64. Composé suivant l'une quelconque des revendications 1 à 61, destiné à être utilisé suivant la revendication 57 comme médicament pour le traitement d'une maladie ou affection dans laquelle on sait, ou il est peut être démontré, que l'inhibition de la PDE5 produit un effet bénéfique.

65. Composé suivant l'une quelconque des revendications 1 à 61, destiné à être utilisé suivant la revendication 57 ou 58 comme médicament pour le traitement d'une maladie ou affection choisie entre l'hypertension, l'insuffisance cardiaque congestive, l'angine de poitrine, un ictus, une maladie des artères coronaires, l'insuffisance cardiaque congestive, des états de patence réduite des vaisseaux sanguins, une maladie vasculaire périphérique, l'athérosclérose, la tolérance induite par les nitrates, la tolérance aux nitrates, le diabète, la tolérance entravée au glucose, le syndrome métabolique, l'obésité, un dysfonctionnement sexuel, l'accouchement prématuré, la prééclampsie, la dysménorrhée, le syndrome d'ovaire polykystique, l'hyperplasie prostatique bénigne, l'obstruction de la sortie de la vessie, l'incontinence, une maladie pulmonaire obstructive chronique, l'insuffisance respiratoire aiguë, la bronchite, l'asthme chronique, l'asthme allergique, la rhinite allergique, des troubles de la motilité intestinale, le syndrome de Kawasaki, la sclérose en plaques, la maladie d'Alzheimer, le psoriasis, la nécrose cutanée, la cicatrisation, la fibrose, la douleur, le cancer, une métastase, la calvitie, l'oesophage en casse-noix, une fissure anale et les hémorroïdes.

66. Composé suivant l'une quelconque des revendications 1 à 61, destiné à être utilisé suivant la revendication 59 comme médicament pour le traitement de l'hypertension pulmonaire.

67. Composé suivant l'une quelconque des revendications 1 à 61, destiné à être utilisé suivant la revendication 59 comme médicament pour le traitement de la douleur neuropathique.

68. Utilisation d'un composé suivant l'une quelconque des revendications 1 à 61 pour la production d'un médicament destiné au traitement d'une maladie ou affection choisie entre l'hypertension, l'insuffisance cardiaque congestive, l'angine de poitrine, un ictus, une maladie des artères coronaires, l'insuffisance cardiaque congestive, des états de patence réduite des vaisseaux sanguins, une maladie vasculaire périphérique, l'athérosclérose, la tolérance induite par les nitrates, la tolérance aux nitrates, le diabète, la tolérance entravée au glucose, le syndrome métabolique, l'obésité, un dysfonctionnement sexuel, l'accouchement prématuré, la prééclampsie, la dysménorrhée, le syndrome d'ovaire polykystique, l'hyperplasie prostatique bénigne, l'obstruction de la sortie de la vessie, l'incontinence, une maladie pulmonaire obstructive chronique, l'insuffisance respiratoire aiguë, la bronchite, l'asthme chronique, l'asthme allergique, la rhinite allergique, des troubles de la motilité intestinale, le syndrome de Kawasaki, la sclérose en plaques, la maladie d'Alzheimer, le psoriasis, la nécrose cutanée, la cicatrisation, la fibrose, la douleur, le cancer, une métastase, la calvitie, l'oesophage en casse-noix, une fissure anale et les hémorroïdes.

69. Composition pharmaceutique comprenant un composé suivant l'une quelconque des revendications 1 à 61 et un second agent pharmaceutiquement acceptable choisi dans le groupe consistant en l'aspirine, des antagonistes du récepteur d'angiotensine II, des agents de blocage du canal calcium, des bêta-bloquants, le C11027, des antagonistes du récepteur CCR5, des imidazolines, des sGCa (activateurs de guanylate-cyclase soluble), des agents antihypertensifs, des diurétiques, des antagonistes alpha-adrénergiques, des inhibiteurs de ACE (enzyme de conversion d'angiotensine), des antagonistes du récepteur d'aldostérone, des inhibiteurs d'endopeptidase neutre, des agents antidiabétiques, des sulfonylurées, des glitazones, des agents abaissant le taux de cholestérol et des ligands d'alpha-2-delta.

70. Composition pharmaceutique comprenant un composé suivant l'une quelconque des revendications 1 à 55 et de la prégabaline.

71. Composé de formule (III) 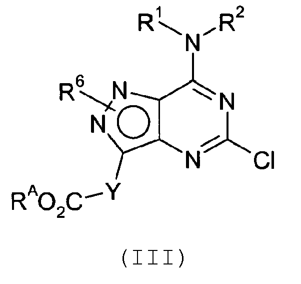 dans laquelle R¹, R², R⁶ et Y sont tels que définis dans la revendication 1 et R^{A} représente un groupe alkyle en C₁ à C₆ ou benzyle,
sous réserve que le composé ne soit pas un composé de formule : 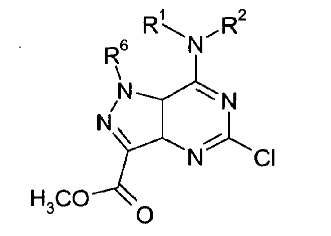 dans laquelle R¹, R² et R⁶ sont tels que définis dans la revendication 1.

72. Composé suivant la revendication 71, de formule (III^{D})

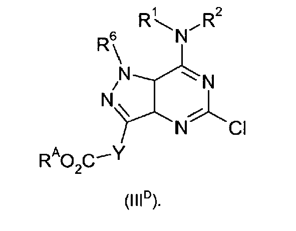

73. Composé de formule (V) 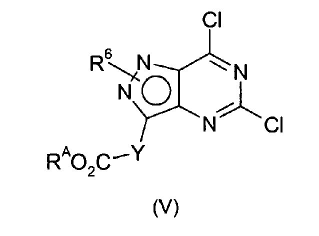 dans laquelle R⁶ et Y sont tels que définis dans la revendication 1 et R^{A} représente un groupe alkyle en C₁ à C₆ ou benzyle,
sous réserve que le composé de formule (V) ne soit pas un composé de formule : 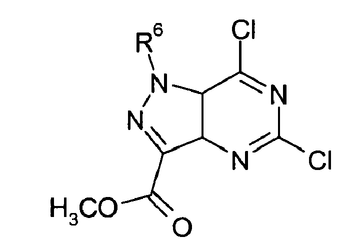 dans laquelle R6 est tel que défini dans la revendication 1.

74. Composé suivant la revendication 73, de formule (VB)
